# EUROPEAN PATENT SPECIFICATION

(11) **EP 2 860 253 B1**
(45) Date of publication and mention of the grant of the patent: **01.08.2018**
(21) Application number: 14193181.6
(22) Date of filing: 01.03.2007
(51) Int. Cl.: C12N 15/74

(54) **Engineered listeria and methods of use thereof**
Manipulierte Listerien und Anwendungsverfahren dafür
Listéria génétiquement modifiée et procédés pour son utilisation

(30) Priority: 01.03.2006 US 778471 P; 21.03.2006 US 784576 P; 30.03.2006 US 396216; 30.03.2006 US 395197
(43) Date of publication of application: 15.04.2015
(62) Divisional of application: 07752174.8
(73) Proprietor: Aduro Biotech, Inc., Berkeley, CA 94710 (US)
(72) Inventor: Dubensky, Thomas W. Jr., Berkeley, CA 94705 (US); Skoble, Justin, Berkeley, CA 94703 (US); Lauer, Peter M., Albany, CA 94706 (US); Cook, David N., Brookline, MA 02445 (US)
(74) Representative: Brasnett, Adrian Hugh

(56) References cited:
- US-A1- 2003 203 472
- CHEREPANOV PETER P ET AL: "Gene disruption in Escherichia coli: Tc-R and Km-R cassettes with the option of Flp-catalyzed excision of the antibiotic-resistance determinant", GENE (AMSTERDAM), vol. 158, no. 1, 1995, pages 9-14, XP002735973, ISSN: 0378-1119
- CHOI KYOUNG- HEE ET AL: "An improved method for rapid generation of unmarked Pseudomonas aeruginosa deletion mutants -", BMC MICROBIOLOGY, vol. 5, May 2005 (2005-05), XP002735974, ISSN: 1471-2180
- WANG YONG ET AL: "Inducible excision of selectable marker gene from transgenic plants by the Cre/lox site-specific recombination system", TRANSGENIC RESEARCH, vol. 14, no. 5, October 2005 (2005-10), pages 605-614, XP019269482, ISSN: 0962-8819
- IWAKI TOMOKO ET AL: "A set of loxP marker cassettes for cre-mediated multiple gene disruption in Schizosaccharomyces pombe", BIOSCIENCE BIOTECHNOLOGY AND BIOCHEMISTRY, vol. 68, no. 3, March 2004 (2004-03), pages 545-550, XP002737156, ISSN: 0916-8451
- JIANG LING-LI ET AL: "Characterization of a mutant Listeria monocytogenes strain expressing green fluorescent protein", ACTA BIOCHIMICA ET BIOPHYSICA SINICA, vol. 37, no. 1, January 2005 (2005-01), pages 19-24, XP55175888, ISSN: 1672-9145

### SEQUENCE LISTING

<110> Aduro Biotech
<120> Engineered Listeria And Methods Of Use Thereof
<130> AHB/FP7075583
<140> EP
   <141> 2007-03-01
<150> EP07752174.8
   <151> 2007-03-01
<150> PCT/US2007/005457
   <151> 2007-03-01
<150> US 11/395,197
   <151> 2006-03-30
<150> US 11/396,216
   <151> 2006-03-30
<150> US 60/778,471
   <151> 2006-03-01
<150> US 60/784,576
   <151> 2006-03-21
<160> 159
<170> FastSEQ for Windows Version 4.0
<210> 1
   <211> 452
   <212> PRT
   <213> Bacteriophage U153
<220>
   <221> VARIANT
   <222> 142
   <223> Xaa = Any Amino Acid
<400> 1
   Met Lys Ala Ala Ile Tyr Ile Arg Val Ser Thr Gln Glu Gln Ile Glu
    1               5                  10                  15
   Asn Tyr Ser Ile Gln Ala Gln Thr Glu Lys Leu Thr Ala Leu Cys Arg
               20                  25                  30
   Ser Lys Asp Trp Asp Val Tyr Asp Ile Phe Ile Asp Gly Gly Tyr Ser
           35                  40                  45
   Gly Ser Asn Met Asn Arg Pro Ala Leu Asn Glu Met Leu Ser Lys Leu
       50                  55                  60
   His Glu Ile Asp Ala Val Val Val Tyr Arg Leu Asp Arg Leu Ser Arg
   65                  70                  75                  80
   Ser Gln Arg Asp Thr Ile Thr Leu Ile Glu Glu Tyr Phe Leu Lys Asn
                   85                  90                  95
   Asn Val Glu Phe Val Ser Leu Ser Glu Thr Leu Asp Thr Ser Ser Pro
               100                 105                 110
   Phe Gly Arg Ala Met Ile Gly Ile Leu Ser Val Phe Ala Gln Leu Glu
           115                 120                 125
   Arg Glu Thr Ile Arg Asp Arg Met Val Met Gly Lys Ile Xaa Arg Ile
       130                 135                 140
   Glu Ala Gly Leu Pro Leu Thr Thr Ala Lys Gly Arg Thr Phe Gly Tyr
   145                 150                 155                 160
   Asp Val Ile Asp Thr Lys Leu Tyr Ile Asn Glu Glu Glu Ala Lys Gln
                   165                 170                 175
   Leu Gln Met Ile Tyr Asp Ile Phe Glu Glu Glu Lys Ser Ile Thr Thr
               180                 185                 190
   Leu Gln Lys Arg Leu Lys Lys Leu Gly Phe Lys Val Lys Ser Tyr Ser
           195                 200                 205
   Ser Tyr Asn Asn Trp Leu Thr Asn Asp Leu Tyr Cys Gly Tyr Val Ser
       210                 215                 220
   Tyr Ala Asp Lys Val His Thr Lys Gly Val His Glu Pro Ile Ile Ser
   225                 230                 235                 240
   Glu Glu Gln Phe Tyr Arg Val Gln Glu Ile Phe Ser Arg Met Gly Lys
                   245                 250                 255
   Asn Pro Asn Met Asn Arg Asp Ser Ala Ser Leu Leu Asn Asn Leu Val
               260                 265                 270
   Val Cys Gly Lys Cys Gly Leu Gly Phe Val His Arg Arg Lys Asp Thr
           275                 280                 285
   Val Ser Arg Gly Lys Lys Tyr His Tyr Arg Tyr Tyr Ser Cys Lys Thr
       290                 295                 300
   Tyr Lys His Thr His Glu Leu Glu Lys Cys Gly Asn Lys Ile Trp Arg
   305                 310                 315                 320
   Ala Asp Lys Leu Glu Glu Leu Ile Ile Asp Arg Val Asn Asn Tyr Ser
                   325                 330                 335
   Phe Ala Ser Arg Asn Val Asp Lys Glu Asp Glu Leu Asp Ser Leu Asn
               340                 345                 350
   Glu Lys Leu Lys Thr Glu His Val Lys Lys Lys Arg Leu Phe Asp Leu
           355                 360                 365
   Tyr Ile Ser Gly Ser Tyr Glu Val Ser Glu Leu Asp Ala Met Met Ala
       370                 375                 380
   Asp Ile Asp Ala Gln Ile Asn Tyr Tyr Glu Ala Gln Ile Glu Ala Asn
   385                 390                 395                 400
   Glu Glu Leu Lys Lys Asn Lys Lys Ile Gln Glu Asn Leu Ala Asp Leu
                   405                 410                 415
   Ala Thr Val Asp Phe Asp Ser Leu Glu Phe Arg Glu Lys Gln Leu Tyr
               420                 425                 430
   Leu Lys Ser Leu Ile Asn Lys Ile Tyr Ile Asp Gly Glu Gln Val Thr
           435                 440                 445
   Ile Glu Trp Leu
       450
<210> 2
   <211> 471
   <212> PRT
   <213> Listeria innocua
<400> 2
   Met Thr Val Gly Ile Tyr Ile Arg Val Ser Thr Glu Glu Gln Val Lys
    1               5                  10                  15
   Glu Gly Phe Ser Ile Ser Ala Gln Lys Glu Lys Leu Lys Ala Tyr Cys
               20                  25                  30
   Thr Ala Gln Gly Trp Glu Asp Phe Lys Phe Tyr Val Asp Glu Gly Lys
           35                  40                  45
   Ser Ala Lys Asp Met His Arg Pro Leu Leu Gln Glu Met Ile Ser His
       50                  55                  60
   Ile Lys Lys Gly Leu Ile Asp Thr Val Leu Val Tyr Lys Leu Asp Arg
   65                  70                  75                  80
   Leu Thr Arg Ser Val Val Asp Leu His Asn Leu Leu Ser Ile Phe Asp
                   85                  90                  95
   Glu Phe Asn Cys Ala Phe Lys Ser Ala Thr Glu Val Tyr Asp Thr Ser
               100                 105                 110
   Ser Ala Met Gly Arg Phe Phe Ile Thr Ile Ile Ser Ser Val Ala Gln
           115                 120                 125
   Phe Glu Arg Glu Asn Thr Ser Glu Arg Val Ser Phe Gly Met Ala Glu
       130                 135                 140
   Lys Val Arg Gln Gly Glu Tyr Ile Pro Leu Ala Pro Phe Gly Tyr Thr
   145                 150                 155                 160
   Lys Gly Thr Asp Gly Lys Leu Ile Val Asn Lys Ile Glu Lys Glu Ile
                   165                 170                 175
   Phe Leu Gln Val Val Glu Met Val Ser Thr Gly Tyr Ser Leu Arg Gln
               180                 185                 190
   Thr Cys Glu Tyr Leu Thr Asn Ile Gly Leu Lys Thr Arg Arg Ser Asn
           195                 200                 205
   Asp Val Trp Lys Val Ser Thr Leu Ile Trp Met Leu Lys Asn Pro Ala
       210                 215                 220
   Val Tyr Gly Ala Ile Lys Trp Asn Asn Glu Ile Tyr Glu Asn Thr His
   225                 230                 235                 240
   Glu Pro Leu Ile Asp Lys Ala Thr Phe Asn Lys Val Ala Lys Ile Leu
                   245                 250                 255
   Ser Ile Arg Ser Lys Ser Thr Thr Ser Arg Arg Gly His Val His His
               260                 265                 270
   Ile Phe Lys Asn Arg Leu Ile Cys Pro Ala Cys Gly Lys Arg Leu Ser
           275                 280                 285
   Gly Leu Arg Thr Lys Tyr Ile Asn Lys Asn Lys Glu Thr Phe Tyr Asn
       290                 295                 300
   Asn Asn Tyr Arg Cys Ala Thr Cys Lys Glu His Arg Arg Pro Ala Val
   305                 310                 315                 320
   Gln Ile Ser Glu Gln Lys Ile Glu Lys Ala Phe Ile Asp Tyr Ile Ser
                   325                 330                 335
   Asn Tyr Thr Leu Asn Lys Ala Asn Ile Ser Ser Lys Lys Leu Asp Asn
               340                 345                 350
   Asn Leu Arg Lys Gln Glu Met Ile Gln Lys Glu Ile Ile Ser Leu Gln
           355                 360                 365
   Arg Lys Arg Glu Lys Phe Gln Lys Ala Trp Ala Ala Asp Leu Met Asn
       370                 375                 380
   Asp Asp Glu Phe Ser Lys Leu Met Ile Asp Thr Lys Met Glu Ile Asp
   385                 390                 395                 400
   Ala Ala Glu Asp Arg Lys Lys Glu Tyr Asp Val Ser Leu Phe Val Ser
                   405                 410                 415
   Pro Glu Asp Ile Ala Lys Arg Asn Asn Ile Leu Arg Glu Leu Lys Ile
               420                 425                 430
   Asn Trp Thr Ser Leu Ser Pro Thr Glu Lys Thr Asp Phe Ile Ser Met
           435                 440                 445
   Phe Ile Glu Gly Ile Glu Tyr Val Lys Asp Asp Glu Asn Lys Ala Val
       450                 455                 460
   Ile Thr Lys Ile Ser Phe Leu
   465                 470
<210> 3
   <211> 434
   <212> PRT
   <213> Bacteriophage PSA
<400> 3
   Met Lys Ile Lys Lys Leu Ala Asn Gly Lys Tyr Cys Val Arg Leu Arg
   1               5                   10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Gln
           35                  40                  45
   His Asp Ser Ser Ser Leu Lys Glu Trp Asn Phe Lys Glu Phe Tyr Thr
       50                  55                  60
   Leu Phe Met Lys Thr Phe Lys Asp Gly Lys Ser Ser Gln Ser Thr Ile
   65                  70                  75                  80
   Asn Leu Tyr Asp Leu Ala Tyr Asn Gln Phe Val Asp Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ser Val Asp Tyr Ala Ile Ser Thr Val Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Thr Ile Gly Ala His Ile Ser Gly Gln Asp Val Ala
   145                 150                 155                 160
   Lys Asn Lys Ala Gln Phe Met Glu Thr Asp Lys Val His Leu Leu Leu
                   165                 170                 175
   Glu Glu Leu Ala Lys Phe His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Thr Val Asn Lys Ala Trp
       210                 215                 220
   Asp Tyr Lys Tyr Thr Asn Thr Phe Ile Asp Thr Lys Thr Lys Lys Ser
   225                 230                 235                 240
   Arg Val Ile Tyr Ile Asp Asn Ser Thr Ala Gln Tyr Leu His Ser Tyr
                   245                 250                 255
   Leu Asn Trp His Thr Asp Tyr Met Lys Glu His Ala Ile Lys Asn Pro
               260                 265                 270
   Leu Met Leu Leu Phe Ile Thr Tyr His Asn Lys Pro Val Asp Asn Ala
           275                 280                 285
   Ser Cys Asn Lys Ala Leu Lys Lys Ile Cys Ser Thr Ile Asn Ser Glu
       290                 295                 300
   Pro Val Thr Leu His Lys Leu Arg His Thr His Thr Gly Leu Cys Val
   305                 310                 315                 320
   Glu Ala Gly Met Asp Ile Ile Tyr Val Ala Asp Arg Leu Gly His Asp
                   325                 330                 335
   Asp Ile Asn Thr Thr Leu Lys Tyr Tyr Ser His Leu Ser Ser Asn Leu
               340                 345                 350
   Arg Gln His Asn Gln Ser Lys Val Asp Arg His Thr His Thr Gly Leu
           355                 360                 365
   Cys Val Glu Ala Gly Met Asp Ile Ile Tyr Val Ala Asp Arg Leu Gly
       370                 375                 380
   His Asp Asp Ile Asn Thr Thr Leu Lys Tyr Tyr Ser His Leu Ser Ser
   385                 390                 395                 400
   Asn Leu Arg Gln His Asn Gln Ser Lys Val Asp Ala Phe Phe Thr Leu
                   405                 410                 415
   Lys Thr Asp Glu Asn Thr Thr Asn Phe Thr Thr Asn Ala Thr Lys Thr
               420                 425                 430
   Thr Glu
<210> 4
   <211> 400
   <212> PRT
   <213> Listeria innocua
<400> 4
   Met Val Lys Lys Val Lys Gly Arg Arg Tyr Glu Gly Ser Ile Glu Gln
    1               5                  10                  15
   Arg Ser Lys Asn Ser Trp Arg Met Arg Val Thr Val Gly Tyr Asp Tyr
               20                  25                  30
   Lys Gly Thr Pro Ile Arg Ala Asp Arg Thr Thr Arg Thr Lys Asn Glu
           35                  40                  45
   Arg Glu Arg Glu Arg Glu Leu Arg Asn Phe Ile Thr Glu Leu Glu Gln
       50                  55                  60
   Asn Gly Tyr Thr Ala Pro Ala Arg Met Thr Phe Lys Ala Phe Val Glu
   65                  70                  75                  80
   Asn Glu Tyr Met Pro Lys His Ala Gln Asn Asn Leu Glu Val Lys Thr
                   85                  90                  95
   Trp Thr Glu Tyr Tyr Lys Ser Ile Val Ala Arg Ala Tyr Pro Ala Phe
               100                 105                 110
   Gly Gly Val Gln Met Asp Lys Ile Thr Thr Leu His Ile Val Asn Leu
           115                 120                 125
   Val Ala Lys Leu Gln Lys Pro Gly Ala Arg Leu Asp Val Lys Pro Thr
       130                 135                 140
   Asp Ser Asp Glu Lys Lys Asn Lys Pro Leu Ser Pro Arg Ser Ile Arg
   145                 150                 155                 160
   Asn Ile Tyr Phe Ala Ile Asn Ser Val Phe Glu Thr Ala Val Glu Trp
                   165                 170                 175
   Lys Val Ile Pro Ile Asn Pro Ala Glu Gly Val Arg Leu Pro Lys Thr
               180                 185                 190
   Thr Lys Arg Pro Pro Thr Ile Tyr Thr Pro Ala Glu Ile Glu Leu Leu
           195                 200                 205
   Asn Ala Ala Leu Val Lys Glu Pro Leu Arg Leu Gln Val Met Ile Tyr
       210                 215                 220
   Ile Ala Leu Ile Ser Gly Cys Arg Glu Ala Glu Leu Ala Ala Leu Glu
   225                 230                 235                 240
   Val Lys His Val Asn Leu Ile Glu Asp Glu Leu Thr Phe Glu Gln Thr
                   245                 250                 255
   Leu Val Ala Lys Ala Gly Glu Gly Leu Leu Leu Lys Glu Ser Thr Lys
               260                 265                 270
   Asn Asp Val Ala Gly Ile Val Ser Ile Pro Ala Trp Leu Thr Asn Leu
           275                 280                 285
   Ile Glu Thr Tyr Ile Ser Asn Glu Val Leu Asp Leu Lys Thr Glu Gly
       290                 295                 300
   Lys Trp Ala Asn His Lys Phe Leu Phe Ala Asp Met Glu Gly Lys Pro
   305                 310                 315                 320
   Ile Arg Pro Asp Ser Ile Tyr Gln Arg Trp Lys Arg Phe Leu Glu Arg
                   325                 330                 335
   His Asn Leu Pro Val Ile Arg Phe His Asp Leu Arg His Thr Ser Ala
               340                 345                 350
   Thr Leu Leu Leu Asn Lys Gly Arg Asp Ile Lys Ile Ile Gln Glu Arg
           355                 360                 365
   Leu Arg His Lys Ser Ser Val Thr Thr Ser Asn Ile Tyr Ala His Val
       370                 375                 380
   Leu Lys Asp Thr His Lys Asp Ala Ala Ser Asp Phe Glu Asn Pro Phe
   385                 390                 395                 400
<210> 5
   <211> 434
   <212> PRT
   <213> Bacteriophage PSA
<400> 5
   Met Lys Ile Lys Lys Leu Ala Asn Gly Lys Tyr Cys Val Arg Leu Arg
    1               5                  10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Gln
           35                  40                  45
   His Asp Ser Ser Ser Leu Lys Glu Trp Asn Phe Lys Glu Phe Tyr Thr
       50                  55                  60
   Leu Phe Met Lys Thr Phe Lys Asp Gly Lys Ser Ser Gln Ser Thr Ile
   65                  70                  75                  80
   Asn Leu Tyr Asp Leu Ala Tyr Asn Gln Phe Val Asp Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ser Val Asp Tyr Ala Ile Ser Thr Val Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Thr Ile Gly Ala His Ile Ser Gly Gln Asp Val Ala
   145                 150                 155                 160
   Lys Asn Lys Ala Gln Phe Met Glu Thr Asp Lys Val His Leu Leu Leu
                   165                 170                 175
   Glu Glu Leu Ala Lys Phe His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Thr Val Asn Lys Ala Trp
       210                 215                 220
   Asp Tyr Lys Tyr Thr Asn Thr Phe Ile Asp Thr Lys Thr Lys Lys Ser
   225                 230                 235                 240
   Arg Val Ile Tyr Ile Asp Asn Ser Thr Ala Gln Tyr Leu His Ser Tyr
                   245                 250                 255
   Leu Asn Trp His Thr Asp Tyr Met Lys Glu His Ala Ile Lys Asn Pro
               260                 265                 270
   Leu Met Leu Leu Phe Ile Thr Tyr His Asn Lys Pro Val Asp Asn Ala
           275                 280                 285
   Ser Cys Asn Lys Ala Leu Lys Lys Ile Cys Ser Thr Ile Asn Ser Glu
       290                 295                 300
   Pro Val Thr Leu His Lys Leu Arg His Thr His Thr Gly Leu Cys Val
   305                 310                 315                 320
   Glu Ala Gly Met Leu Leu Phe Ile Thr Tyr His Asn Lys Pro Val Asp
                   325                 330                 335
   Asn Ala Ser Cys Asn Lys Ala Leu Lys Lys Ile Cys Ser Thr Ile Asn
               340                 345                 350
   Ser Glu Pro Val Thr Leu His Lys Leu Arg His Thr His Thr Gly Leu
           355                 360                 365
   Cys Val Glu Ala Gly Met Asp Ile Ile Tyr Val Ala Asp Arg Leu Gly
       370                 375                 380
   His Asp Asp Ile Asn Thr Thr Leu Lys Tyr Tyr Ser His Leu Ser Ser
   385                 390                 395                 400
   Asn Leu Arg Gln His Asn Gln Ser Lys Val Asp Ala Phe Phe Thr Leu
                   405                 410                 415
   Lys Thr Asp Glu Asn Thr Thr Asn Phe Thr Thr Asn Ala Thr Lys Thr
               420                 425                 430
   Thr Glu
<210> 6
   <211> 441
   <212> PRT
   <213> Listeria innocua
<400> 6
   Met Ala Lys Asn Lys Trp Gln Pro Thr Lys His Leu Gly Ile Tyr Glu
    1               5                  10                  15
   Tyr Met Thr Lys Lys Gly Lys Arg Tyr Gly Ile Arg Val Arg Tyr Lys
               20                  25                  30
   Gln Gly Asn Asp Tyr Pro Glu Ile Asn Lys Ser Gly Phe Glu Thr Ile
           35                  40                  45
   Ala Ala Ala Lys Val Tyr Lys Asn Asn Ile Glu Asn Leu Lys Ala Asn
       50                  55                  60
   Lys Lys Glu Tyr Val Phe Thr Asn Glu Lys Leu Thr Leu Asn Thr Trp
   65                  70                  75                  80
   Phe Ala Ser Tyr Met Glu Met Phe Lys Lys Lys Asn Lys Ser Lys Asp
                   85                  90                  95
   Thr Ile Ala Asn Ala Lys Val Tyr Lys Asn Asn Ile Glu Asn Leu Lys
                   100                 105                 110
   Ala Asn Lys Lys Glu Tyr Val Phe Thr Asn Glu Lys Leu Thr Leu Asn
               115                 120                 125
   Thr Trp Phe Ala Ser Tyr Met Glu Met Phe Lys Lys Lys Asn Lys Ser
           130                 135                 140
   Lys Asp Thr Ile Ala Asn Lys Tyr Ser Ile Tyr Asn Asn His Leu Glu
   145                 150                 155                 160
   Ile Pro Phe Gly Asn Tyr Tyr Leu Thr Asp Ile Ser Leu Asp Ile Tyr
                   165                 170                 175
   Glu Asp Phe Leu Arg Glu Lys Ile Lys Asn Gly Tyr Ala Asn Asn Ser
               180                 185                 190
   Val Lys Ala Met His Lys Leu Met Lys Ser Ile Leu Asn Ala Ala Val
           195                 200                 205
   Arg Tyr Glu Lys Leu Glu Lys Asn Arg Leu Gln Phe Ala Glu Ile Glu
       210                 215                 220
   Gln Leu Glu Glu Asn Glu Val Ile Glu Leu Lys Val Leu Glu Thr Asp
   225                 230                 235                 240
   Glu Phe Asn Val Phe Ile Ser Ala Cys Arg Ala Phe Phe Thr Lys Tyr
                   245                 250                 255
   Asp Phe Thr Met Ile Tyr Leu Ala Val Trp Gly Met Arg Arg Gly Glu
               260                 265                 270
   Val Met Gly Val Lys Leu Lys Asn Leu Thr Phe Asp Asp Ala Lys Gln
           275                 280                 285
   Gln Val Arg Ile Thr Leu Asp Ser Thr Arg Thr Leu Arg Thr Pro Glu
       290                 295                 300
   Gly Lys Gly Thr Lys Thr Pro Ala Gly Arg Arg Ile Leu Leu Ile Asp
   305                 310                 315                 320
   Gly Glu Gly Tyr Arg Leu Leu Lys Tyr Ser Val Glu Lys Ala Val Ser
                   325                 330                 335
   Ile Ala Lys Asp His Gly Ser Val Leu His Gln Asp Asp Phe Ile Phe
               340                 345                 350
   Arg Asn Pro Thr Ser Asn Arg Pro Trp Ala Val Thr Arg Met Asn Asp
           355                 360                 365
   Leu Leu Arg Lys Leu Glu Lys Glu Tyr Asp Ile Lys Val Tyr Pro His
       370                 375                 380
   Leu Leu Arg His Asn Phe Asn Thr Gln Ala Leu Leu Ala Gly Ala Asn
   385                 390                 395                 400
   Ser Asn Asp Leu Arg Lys Phe Ile Gly His Lys Asn Ser Ser Met Thr
                   405                 410                 415
   Asp His Tyr Ser His Ala Thr Asp Glu Gly Arg Glu Lys Leu Met Asn
               420                 425                 430
   Thr Met Lys Asp Arg Leu Ser Gly Ile
           435                 440
<210> 7
   <211> 384
   <212> PRT
   <213> Bacteriophage PSA
<400> 7
   Met Lys Ile Lys Lys Leu Ala Asn Gly Lys Tyr Cys Val Arg Leu Arg
    1               5                  10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Gln
           35                  40                  45
   His Asp Ser Ser Ser Leu Lys Glu Trp Asn Phe Lys Glu Phe Tyr Thr
       50                  55                  60
   Leu Phe Met Lys Thr Phe Lys Asp Gly Lys Ser Ser Gln Ser Thr Ile
   65                  70                  75                  80
   Asn Leu Tyr Asp Leu Ala Tyr Asn Gln Phe Val Asp Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ser Val Asp Tyr Ala Ile Ser Thr Val Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Thr Ile Gly Ala His Ile Ser Gly Gln Asp Val Ala
   145                 150                 155                 160
   Lys Asn Lys Ala Gln Phe Met Glu Thr Asp Lys Val His Leu Leu Leu
                   165                 170                 175
   Glu Glu Leu Ala Lys Phe His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Thr Val Asn Lys Ala Trp
       210                 215                 220
   Asp Tyr Lys Tyr Thr Asn Thr Phe Ile Asp Thr Lys Thr Lys Lys Ser
   225                 230                 235                 240
   Arg Val Ile Tyr Ile Asp Asn Ser Thr Ala Gln Tyr Leu His Ser Tyr
                   245                 250                 255
   Leu Asn Trp His Thr Asp Tyr Met Lys Glu His Ala Ile Lys Asn Pro
               260                 265                 270
   Leu Met Leu Leu Phe Ile Thr Tyr His Asn Lys Pro Val Asp Asn Ala
           275                 280                 285
   Ser Cys Asn Lys Ala Leu Lys Lys Ile Cys Ser Thr Ile Asn Ser Glu
       290                 295                 300
   Pro Val Thr Leu His Lys Leu Arg His Thr His Thr Gly Leu Cys Val
   305                 310                 315                 320
   Glu Ala Gly Met Asp Ile Ile Tyr Val Ala Asp Arg Leu Gly His Asp
                   325                 330                 335
   Asp Ile Asn Thr Thr Leu Lys Tyr Tyr Ser His Leu Ser Ser Asn Leu
               340                 345                 350
   Arg Gln His Asn Gln Ser Lys Val Asp Ala Phe Phe Thr Leu Lys Thr
           355                 360                 365
   Asp Glu Asn Thr Thr Asn Phe Thr Thr Asn Ala Thr Lys Thr Thr Glu
       370                 375                 380
<210> 8
   <211> 384
   <212> PRT
   <213> Listeria innocua
<400> 8
   Met Lys Ile Lys Lys Met Lys Asn Gly Lys Tyr Thr Val Arg Leu Arg
    1               5                  10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Glu
           35                  40                  45
   His Asp Ser Asn Ser Leu Lys Glu Trp Asn Phe Lys Glu Phe Tyr Ser
       50                  55                  60
   Leu Phe Met Lys Thr Phe Lys Glu Asn Lys Ser Ser Gln Ser Thr Ile
   65                  70                  75                  80
   Asn Leu Tyr Asp Leu Ala Tyr Asn Gln Phe Val Asn Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ala Leu Asp Tyr Ala Val Ala Thr Ile Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Ala Leu Gly Ala His Ile Ser Gly His Asp Ile Ala
   145                 150                 155                 160
   Lys Thr Lys Ala Gln Tyr Leu Glu Thr Asp Lys Val His Leu Leu Leu
                   165                 170                 175
   Glu Glu Leu Ala Lys Leu His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Ser Val Asn Lys Ala Trp
       210                 215                 220
   Asp Tyr Lys Tyr Thr Asn Thr Phe Thr Asp Thr Lys Thr Lys Lys Ser
   225                 230                 235                 240
   Arg Val Ile Tyr Ile Asp Asn Ser Thr Val Gln Tyr Leu Gln Ser Tyr
                   245                 250                 255
   Leu Ala Trp His Ala Asp Tyr Met Lys Glu His Ala Ile Glu Asn Pro
               260                 265                 270
   Val Met Leu Leu Phe Ile Thr Tyr His Asn Lys Pro Val Asp Asn Ala
           275                 280                 285
   Ser Cys Asn Lys Ala Leu Lys Lys Ile Cys Thr Thr Ile Asn Ser Glu
       290                 295                 300
   Thr Val Thr Leu His Lys Leu Arg His Thr His Thr Gly Leu Cys Val
   305                 310                 315                 320
   Glu Ala Gly Met Asp Ile Ile Tyr Val Ala Asp Arg Leu Gly His Asp
                   325                 330                 335
   Asp Ile Asn Thr Thr Leu Lys Tyr Tyr Ser His Leu Ser Ser Asn Leu
               340                 345                 350
   Arg Gln Gln Asn Gln Ser Lys Val Asp Ala Phe Phe Thr Leu Lys Thr
           355                 360                 365
   Asp Glu Asn Thr Thr Lys Phe Ala Thr Asn Ala Thr Lys Thr Thr Glu
       370                 375                 380
<210> 9
   <211> 22
   <212> DNA
   <213> Artificial Sequence
<220>
   <223> Primer
<400> 9
   tgaagtaaac ccgcacacga tc 22
<210> 10
   <211> 24
   <212> DNA
   <213> Artificial Sequence
<220>
   <223> Primer
<400> 10
   tgtaacatgg aggttctggc aatc 24
<210> 11
   <211> 613
   <212> PRT
   <213> Bacteriophage phiC31
<400> 11
   Met Thr Gln Gly Val Val Thr Gly Val Asp Thr Tyr Ala Gly Ala Tyr
   1               5                   10                  15
   Asp Arg Gln Ser Arg Glu Arg Glu Asn Ser Ser Ala Ala Ser Pro Ala
               20                  25                  30
   Thr Gln Arg Ser Ala Asn Glu Asp Lys Ala Ala Asp Leu Gln Arg Glu
           35                  40                  45
   Val Glu Arg Asp Gly Gly Arg Phe Arg Phe Val Gly His Phe Ser Glu
       50                  55                  60
   Ala Pro Gly Thr Ser Ala Phe Gly Thr Ala Glu Arg Pro Glu Phe Glu
   65                  70                  75                  80
   Arg Ile Leu Asn Glu Cys Arg Ala Gly Arg Leu Asn Met Ile Ile Val
                   85                  90                  95
   Tyr Asp Val Ser Arg Phe Ser Arg Leu Lys Val Met Asp Ala Ile Pro
               100                 105                 110
   Ile Val Ser Glu Leu Leu Ala Leu Gly Val Thr Ile Val Ser Thr Gln
           115                 120                 125
   Glu Gly Val Phe Arg Gln Gly Asn Val Met Asp Leu Ile His Leu Ile
       130                 135                 140
   Met Arg Leu Asp Ala Ser His Lys Glu Ser Ser Leu Lys Ser Ala Lys
   145                 150                 155                 160
   Ile Leu Asp Thr Lys Asn Leu Gln Arg Glu Leu Gly Gly Tyr Val Gly
                   165                 170                 175
   Gly Lys Ala Pro Tyr Gly Phe Glu Leu Val Ser Glu Thr Lys Glu Ile
               180                 185                 190
   Thr Arg Asn Gly Arg Met Val Asn Val Val Ile Asn Lys Leu Ala His
           195                 200                 205
   Ser Thr Thr Pro Leu Thr Gly Pro Phe Glu Phe Glu Pro Asp Val Ile
       210                 215                 220
   Arg Trp Trp Trp Arg Glu Ile Lys Thr His Lys His Leu Pro Phe Lys
   225                 230                 235                 240
   Pro Gly Ser Gln Ala Ala Ile His Pro Gly Ser Ile Thr Gly Leu Cys
                   245                 250                 255
   Lys Arg Met Asp Ala Asp Ala Val Pro Thr Arg Gly Glu Thr Ile Gly
               260                 265                 270
   Lys Lys Thr Ala Ser Ser Ala Trp Asp Pro Ala Thr Val Met Arg Ile
           275                 280                 285
   Leu Arg Asp Pro Arg Ile Ala Gly Phe Ala Ala Glu Val Ile Tyr Lys
       290                 295                 300
   Lys Lys Pro Asp Gly Thr Pro Thr Thr Lys Ile Glu Gly Tyr Arg Ile
   305                 310                 315                 320
   Gln Arg Asp Pro Ile Thr Leu Arg Pro Val Glu Leu Asp Cys Gly Pro
                   325                 330                 335
   Ile Ile Glu Pro Ala Glu Trp Tyr Glu Leu Gln Ala Trp Leu Asp Gly
               340                 345                 350
   Arg Gly Arg Gly Lys Gly Leu Ser Arg Gly Gln Ala Ile Leu Ser Ala
           355                 360                 365
   Met Asp Lys Leu Tyr Cys Glu Cys Gly Ala Val Met Thr Ser Lys Arg
       370                 375                 380
   Gly Glu Glu Ser Ile Lys Asp Ser Tyr Arg Cys Arg Arg Arg Lys Val
   385                 390                 395                 400
   Val Asp Pro Ser Ala Pro Gly Gln His Glu Gly Thr Cys Asn Val Ser
                   405                 410                 415
   Met Ala Ala Leu Asp Lys Phe Val Ala Glu Arg Ile Phe Asn Lys Ile
               420                 425                 430
   Arg His Ala Glu Gly Asp Glu Glu Thr Leu Ala Leu Leu Trp Glu Ala
           435                 440                 445
   Ala Arg Arg Phe Gly Lys Leu Thr Glu Ala Pro Glu Lys Ser Gly Glu
       450                 455                 460
   Arg Ala Asn Leu Val Ala Glu Arg Ala Asp Ala Leu Asn Ala Leu Glu
   465                 470                 475                 480
   Glu Leu Tyr Glu Asp Arg Ala Ala Gly Ala Tyr Asp Gly Pro Val Gly
                   485                 490                 495
   Arg Lys His Phe Arg Lys Gln Gln Ala Ala Leu Thr Leu Arg Gln Gln
               500                 505                 510
   Gly Ala Glu Glu Arg Leu Ala Glu Leu Glu Ala Ala Glu Ala Pro Lys
                   515                 520                 525
   Leu Pro Leu Asp Gln Trp Phe Pro Glu Asp Ala Asp Ala Asp Pro Thr
       530                 535                 540
   Gly Pro Lys Ser Trp Trp Gly Arg Ala Ser Val Asp Asp Lys Arg Val
   545                 550                 555                 560
   Phe Val Gly Leu Phe Val Asp Lys Ile Val Val Thr Lys Ser Thr Thr
                   565                 570                 575
   Gly Arg Gly Gln Gly Thr Pro Ile Glu Lys Arg Ala Ser Ile Thr Trp
               580                 585                 590
   Ala Lys Pro Pro Thr Asp Asp Asp Glu Asp Asp Ala Gln Asp Gly Thr
           595                 600                 605
   Glu Asp Val Ala Ala
       610
<210> 12
   <211> 84
   <212> DNA
   <213> Artificial Sequence
<220>
   <223> phiC31 target attBB' site
<400> 12
   tgacggtctc gaagccgcgg tgcgggtgcc agggcgtgcc cttgggctcc ccgggcgcgt 60
   actccacctc acccatctgg tcca                                        84
<210> 13
   <211> 285
   <212> DNA
   <213> Artificial Sequence
<220>
   <223> phiC31 target attBB' site
<400> 13
   gtcgacgatg taggtcacgg tctcgaagcc gcggtgcggg tgccagggcg tgcccttggg 60
   ctccccgggc gcgtactcca cctcacccat ctggtccatc atgatgaacg ggtcgaggtg 120
   gcggtagttg atcccggcga acgcgcggcg caccgggaag ccctcgccct cgaaaccgct 180
   gggcgcggtg gtcacggtga gcacgggacg tgcgacggcg tcggcgggtg cggatacgcg 240
   gggcagcgtc agcgggttct cgacggtcac ggcgggcatg tcgac                 285
<210> 14
   <211> 70
   <212> DNA
   <213> Bacteriophage phiC31
<400> 14
   aaggggttgt gaccggggtg gacacgtacg cgggtgctta cgaccgtcag tcgcgcgagc 60
   gcgagaattc                                                        70
<210> 15
   <211> 2791
   <212> DNA
   <213> Artificial Sequence
<220>
   <223> Region of pKSV7
<400> 15
   ccaaattagc gatcttacac cattggctaa tttaacaaga atcacccaac tagggttgaa 60
   tgatcaagca tggacaaatg caccagtaaa ctacaaagca aatgtatcca ttccaaacac 120
   ggtgaaaaat gtgactggcg ctttgattgc acctgctact attagcgatg gcggtagtta 180
   cgcagaaccg gatataacat ggaacttacc tagttataca aatgaagtaa gctatacctt 240
   tagccaacct gtcactattg gaaaaggaac gacaacattt agtggaaccg tgacgcagcc 300
   acttaaggca atttttaatg ctaagtttca tgtggacggc aaagaaacaa ccaaagaagt 360
   ggaagctggg aatttattga ctgaaccagc taagcccgta aaagaaggtc acacatttgt 420
   tggttggttt gatgcccaaa caggcggaac taagtggaat ttcagtacgg ataaaatgcc 480
   gacaaatgac atcaatttat atgcacaatt tagtattaac agctacacag caacctttga 540
   gaatgacggt gtaacaacat ctcaaacagt agattatcaa ggcttgttac aagaacctac 600
   accaccaaca aaagaaggtt atactttcaa aggctggtat gacgcaaaaa ctggtggtga 660
   caagtgggat ttcgcaacta gcaaaatgcc tgctaaaaac atcaccttat atgcccaata 720
   tagcgccaat agctatacag caacgtttga tgttgatgga aaatcaacga ctcaagcagt 780
   agactatcaa ggacttctaa aagaaccaaa ggcaccaacg aaagccggat atactttcaa 840
   aggctggtat gacgaaaaaa cagatgggaa aaaatgggat tttgcgacgg ataaaatgcc 900
   agcaaatgac attacgctgt acgctcaatt tacgaaaaat cctgtggcac caccaacaac 960
   tggagggaac acaccgccta caacaaataa cggcgggaat actacaccac cttccgcaaa 1020
   tatacctgga agcgacacat ctaacacatc aactgggaat tcagccagca caacaagtac 1080
   aatgaacgct tatgaccctt ataattcaaa agaagcttca ctccctacaa ctggcgatag 1140
   cgataatgcg ctctaccttt tgttagggtt attagcagta ggaactgcaa tggctcttac 1200
   taaaaaagca cgtgctagta aatagaagta gtgtaaagag ctagatgtgg ttttcggact 1260
   atatctagct tttttatttt ttaataacta gaatcaagga gaggatagtg gtaccttggt 1320
   gagctcccta cgaaaagcta caactttaaa ttcatgaaaa aagaactgat tcgctgaaaa 1380
   cggatcagtt cttttttctt tagacttatt tttacaaaaa cttttcgata atttccatat 1440
   tctggggtct gtctttgctt tcaagtacag aaatatcacg aacaatgcta tctaatttaa 1500
   ttttttccat tcaaattcta ttttttgttg gagcagatcg tatttactcg taagaacttg 1560
   ttggatattg gctccgacaa cgcagtctgg gttggttttt ggatcaacgt gaattaaatt 1620
   cgtattgcct tctatactct tataaacatc aagcagtgaa atttcttctg gtggtctagc 1680
   aagaatcgga tttgctttgc cagtctgcgt agtaattaaa tcagcttttt ttaaattact 1740
   catgattttt ctaatgttag caggatttgt ttttacgcta ccagcaataa tttcactcga 1800
   taacaaattc gtatttttaa aaatttctat ataagccaaa atgtggatag catcgctaaa 1860
   ttggatagag tatttcattt ttttcaatcc tttcaaattt tctccttgac ttatcttatc 1920
   ataatgttta ttataaaggt gtaaattata aatgtacagc tttagtgtta aaaaatttaa 1980
   aggagtggtt taaatgactt atttagtaac tggtgcaaca ggtggacttg gaggctacgc 2040
   attaaattat ttgaaagagc tggttcccat gtccgatatt tatgctttag ttcgtagcga 2100
   agaaaaaggt acagacttga aagcagcagg atttaatatc cgtattggtg attatagtga 2160
   tgtagaatca atgaagcaag cattcgcagg catcgaccgc gtattatttg tttcaggagc 2220
   acctggtaat cgccaagtag aacacgaaaa tgtggtaaat gcggcaaaag aagcaggcgt 2280
   ttcttacatc gcttacacaa gtttcgcggg cgcagataaa tccacaagcg ctttagcaga 2340
   agatcatttc tttaccgaaa aagtaatcga aaaatccgga atcgcgcaca ctttcttgcg 2400
   taacaactgg tacttcgaaa atgaaatgcc gatgatcggt ggcgcattga gtgctggaaa 2460
   atttgtatac gctgctgaaa atggaaaagt tggctgggca ttaaaacgcg aatacgcaga 2520
   agtagccgca aaagctgttg cggacgctga cttcccagaa atccttgaat tatctggccc 2580
   actcatgcaa ttcgtaatca tgtcatagct gtttcctgtg tgaaattgtt atccgctcac 2640
   aattccacac aacatacgag ccggaagcat aaagtgtaaa gcctggggtg cctaatgagt 2700
   gagctaactc acattaattg cgttgcgctc actgcccgct ttccagtcgg gaaacctgtc 2760
   gtgccagctg gactaaaagg catgcaattc a                                2791
<210> 16
   <211> 1309
   <212> DNA
   <213> Listeria monocytogenes
<400> 16
   ccaaattagc gatcttacac cattggctaa tttaacaaga atcacccaac tagggttgaa 60
   tgatcaagca tggacaaatg caccagtaaa ctacaaagca aatgtatcca ttccaaacac 120
   ggtgaaaaat gtgactggcg ctttgattgc acctgctact attagcgatg gcggtagtta 180
   cgcagaaccg gatataacat ggaacttacc tagttataca aatgaagtaa gctatacctt 240
   tagccaacct gtcactattg gaaaaggaac gacaacattt agtggaaccg tgacgcagcc 300
   acttaaggca atttttaatg ctaagtttca tgtggacggc aaagaaacaa ccaaagaagt 360
   ggaagctggg aatttattga ctgaaccagc taagcccgta aaagaaggtc acacatttgt 420
   tggttggttt gatgcccaaa caggcggaac taagtggaat ttcagtacgg ataaaatgcc 480
   gacaaatgac atcaatttat atgcacaatt tagtattaac agctacacag caacctttga 540
   gaatgacggt gtaacaacat ctcaaacagt agattatcaa ggcttgttac aagaacctac 600
   accaccaaca aaagaaggtt atactttcaa aggctggtat gacgcaaaaa ctggtggtga 660
   caagtgggat ttcgcaacta gcaaaatgcc tgctaaaaac atcaccttat atgcccaata 720
   tagcgccaat agctatacag caacgtttga tgttgatgga aaatcaacga ctcaagcagt 780
   agactatcaa ggacttctaa aagaaccaaa ggcaccaacg aaagccggat atactttcaa 840
   aggctggtat gacgaaaaaa cagatgggaa aaaatgggat tttgcgacgg ataaaatgcc 900
   agcaaatgac attacgctgt acgctcaatt tacgaaaaat cctgtggcac caccaacaac 960
   tggagggaac acaccgccta caacaaataa cggcgggaat actacaccac cttccgcaaa 1020
   tatacctgga agcgacacat ctaacacatc aactgggaat tcagccagca caacaagtac 1080
   aatgaacgct tatgaccctt ataattcaaa agaagcttca ctccctacaa ctggcgatag 1140
   cgataatgcg ctctaccttt tgttagggtt attagcagta ggaactgcaa tggctcttac 1200
   taaaaaagca cgtgctagta aatagaagta gtgtaaagag ctagatgtgg ttttcggact 1260
   atatctagct tttttatttt ttaataacta gaatcaagga gaggatagt            1309
<210> 17
   <211> 1466
   <212> DNA
   <213> Listeria monocytogenes
<400> 17
   cctacgaaaa gctacaactt taaattcatg aaaaaagaac tgattcgctg aaaacggatc 60
   agttcttttt tctttagact tatttttaca aaaacttttc gataatttcc atattctggg 120
   gtctgtcttt gctttcaagt acagaaatat cacgaacaat gctatctaat ttaatttttt 180
   ccatttcaaa ttctattttt tgttggagca gatcgtattt actcgtaaga acttgttgga 240
   tattggctcc gacaacgcag tctgggttgg tttttggatc aacgtgaatt aaattcgtat 300
   tgccttctat actcttataa acatcaagca gtgaaatttc ttctggtggt ctagcaagaa 360
   tcggatttgc tttgccagtc tgcgtagtaa ttaaatcagc tttttttaaa ttactcatga 420
   tttttctaat gttagcagga tttgttttta cgctaccagc aataatttca ctcgataaca 480
   aattcgtatt tttaaaaatt tctatataag ccaaaatgtg gatagcatcg ctaaattgga 540
   tagagtattt catttttttc aatcctttca aattttctcc ttgacttatc ttatcataat 600
   gtttattata aaggtgtaaa ttataaatgt acagctttag tgttaaaaaa tttaaaggag 660
   tggtttaaat gacttattta gtaactggtg caacaggtgg acttggaggc tacgcattaa 720
   attatttgaa agagctggtt cccatgtccg atatttatgc tttagttcgt agcgaagaaa 780
   aaggtacaga cttgaaagca gcaggattta atatccgtat tggtgattat agtgatgtag 840
   aatcaatgaa gcaagcattc gcaggcatcg accgcgtatt atttgtttca ggagcacctg 900
   gtaatcgcca agtagaacac gaaaatgtgg taaatgcggc aaaagaagca ggcgtttctt 960
   acatcgctta cacaagtttc gcgggcgcag ataaatccac aagcgcttta gcagaagatc 1020
   atttctttac cgaaaaagta atcgaaaaat ccggaatcgc gcacactttc ttgcgtaaca 1080
   actggtactt cgaaaatgaa atgccgatga tcggtggcgc attgagtgct ggaaaatttg 1140
   tatacgctgc tgaaaatgga aaagttggct gggcattaaa acgcgaatac gcagaagtag 1200
   ccgcaaaagc tgttgcggac gctgacttcc cagaaatcct tgaattatct ggcccactca 1260
   tgcaattcgt aatcatgtca tagctgtttc ctgtgtgaaa ttgttatccg ctcacaattc 1320
   cacacaacat acgagccgga agcataaagt gtaaagcctg gggtgcctaa tgagtgagct 1380
   aactcacatt aattgcgttg cgctcactgc ccgctttcca gtcgggaaac ctgtcgtgcc 1440
   agctggacta aaaggcatgc aattca                                      1466
<210> 18
   <211> 916
   <212> DNA
   <213> Listeria monocytogenes
<220>
   <221> misc_feature
   <222> 539
   <223> n = A,T,C or G
<400> 18
   agaatttagt tccgcagtgg atgctcattt ttacgcaagt gaagtgtacg aatactataa 60
   aaatgtccac caactagaga gtctagatgg taaaggtgga gaaattgatt cgtttgtcca 120
   ttatggcttg aattgcaata atgccttttg ggatggccaa gaaattcttt atggagatgg 180
   ggacaaaaag aatttcaaac cattttcatg cgccaaaact attgttggtc atgaactaac 240
   gcatgcagtt atccagtatt cggcgggatt ggaatacgaa gggcaatcag gtgcgctaaa 300
   cgagtcgttc gccgatgttt ttggttattt tattgcgcca aatcattggt tgattggtga 360
   ggatgtctgt gtgcgtgggt cgcgagatgg gcgaataaga agcattaaag atcctgacaa 420
   atataatcaa gcggctcata tgaaggatta cgaatcgctt ccaatcacag aggaaggcga 480
   ctggggcgga gttcattata atagtggtat cccgaataaa gcagcctata atactatcnc 540
   taaacttgga aaagaaaaaa cagaacagct ttattttcgc gccttaaagt actatttaac 600
   gaaaaaatcc cagtttaccg atgcgaaaaa agcgcttcaa caagcagcga aagatttata 660
   tggtgaagat gcttctaaaa aagttgctga agcttgggaa gcagttgggg ttaactgatt 720
   aacaaatgtt agagaaaaat taattctcca agtgatattc ttaaaataat tcatgaatat 780
   tttttcttat attagctaat taagaagata attaactgct aatccaattt ttaacggaat 840
   aaattagtga aaatgaaggc cgaattttcc ttgttctaaa aaggttgtat tagcgtatca 900
   cgaggaggga gtataa                                                 916
<210> 19
   <211> 1043
   <212> DNA
   <213> Listeria monocytogenes
<400> 19
   aaacacagaa cgaaagaaaa agtgaggtga atgatatgaa attcaaaaat gtggttctag 60
   gtatgtgctt gaccgcaagt gttctagtct ttccggtaac gataaaagca aatgcctgtt 120
   gtgatgaaat acttacaaac acccgcagct ccgcatatat tgacagcaaa ttaccacata 180
   aacttagttg gtccgcggat aacccgacaa atactgacgt aaatacgcac tattggcttt 240
   ttaaacaagc ggaaaaaata ctagctaaag atgtaaatca tatgcgagct aatttaatga 300
   atgaacttaa aaaattcgat aaacaaatag ctcaaggaat atatgatgcg gatcataaaa 360
   atccatatta tgatactagt acatttttat ctcattttta taatcctgat agagataata 420
   cttatttgcc gggttttgct aatgcgaaaa taacaggagc aaagtatttc aatcaatcgg 480
   tgactgatta ccgagaaggg aaatttgaca cagcgtttta taaattaggc ctagcaatcc 540
   attattatac ggatattagt caacctatgc acgccaataa ttttaccgca atatcatacc 600
   ctccaggcta ccactgtgca tatgaaaatt acgtagatac cattaaacac aattatcaag 660
   caacggaaga catggtagca aaaagatttt gctcagatga cgtgaaagac tggctctatg 720
   aaaatgcgaa aagggcgaaa gcggactacc cgaaaatagt caatgcgaaa actaaaaaat 780
   catatttagt aggaaattcc gaatggaaaa aggatacagt ggaacctact ggagctagac 840
   taagagattc acagcaaact ttggcaggtt ttttagaatt ttggtctaaa aaaacaaatg 900
   aataacaata tttaggaata cattcttatc cactcgttag cgggtggata tattttatgg 960
   ggaggaagta agccaaatgt atataaaagg gaggttaatc tttttctttg taatgttagt 1020
   aatcgcgtta tgttccgaag ggc                                          1043
<210> 20
   <211> 17
   <212> PRT
   <213> Listeria monocytogenes
<400> 20
   Lys Val Phe Lys Lys Ile Lys Asp Ala Gly Lys Trp Val Arg Asp Lys
    1               5                  10                  15
   Ile
<210> 21
   <211> 5
   <212> PRT
   <213> Listeria monocytogenes
<400> 21
   Phe Pro Pro Pro Pro
    1               5
<210> 22
   <211> 5
   <212> PRT
   <213> Listeria monocytogenes
<400> 22
   Phe Pro Pro Ile Pro
    1               5
<210> 23
   <211> 4
   <212> PRT
   <213> Listeria monocytogenes
<400> 23
   Lys Lys Arg Arg
    1
<210> 24
   <211> 27
   <212> PRT
   <213> Listeria monocytogenes
<400> 24
   Met Asn Met Lys Lys Ala Thr Ile Ala Ala Thr Ala Gly Ile Ala Val
    1               5                  10                  15
   Thr Ala Phe Ala Ala Pro Thr Ile Ala Ser Ala
               20                  25
<210> 25
   <211> 52
   <212> PRT
   <213> Listeria monocytogenes
<400> 25
   Met Asp Arg Lys Phe Ile Lys Pro Gly Ile Ile Leu Leu Ile Val Ala
    1               5                  10                  15
   Phe Leu Val Val Ser Ile Asn Val Gly Ala Glu Thr Gly Gly Ser Arg
               20                  25                  30
   Thr Ala Gln Val Asn Leu Thr Thr Ser Gln Gln Ala Phe Ile Asp Glu
           35                  40                  45
   Ile Leu Pro Ala
       50
<210> 26
   <211> 588
   <212> PRT
   <213> Homo sapiens
<400> 26
   Arg Thr Leu Ala Gly Glu Thr Gly Gln Glu Ala Ala Pro Leu Asp Gly
    1               5                  10                  15
   Val Leu Thr Asn Pro Pro Asn Ile Ser Ser Leu Ser Pro Arg Gln Leu
               20                  25                  30
   Leu Gly Phe Pro Cys Ala Glu Val Ser Gly Leu Ser Thr Glu Arg Val
           35                  40                  45
   Arg Glu Leu Ala Val Ala Leu Ala Gln Lys Asn Val Lys Leu Ser Thr
       50                  55                  60
   Glu Gln Leu Arg Cys Leu Ala His Arg Leu Ser Glu Pro Pro Glu Asp
   65                  70                  75                  80
   Leu Asp Ala Leu Pro Leu Asp Leu Leu Leu Phe Leu Asn Pro Asp Ala
                   85                  90                  95
   Phe Ser Gly Pro Gln Ala Cys Thr Arg Phe Phe Ser Arg Ile Thr Lys
               100                 105                 110
   Ala Asn Val Asp Leu Leu Pro Arg Gly Ala Pro Glu Arg Gln Arg Leu
           115                 120                 125
   Leu Pro Ala Ala Leu Ala Cys Trp Gly Val Arg Gly Ser Leu Leu Ser
       130                 135                 140
   Glu Ala Asp Val Arg Ala Leu Gly Gly Leu Ala Cys Asp Leu Pro Gly
   145                 150                 155                 160
   Arg Phe Val Ala Glu Ser Ala Glu Val Leu Leu Pro Arg Leu Val Ser
                   165                 170                 175
   Cys Pro Gly Pro Leu Asp Gln Asp Gln Gln Glu Ala Ala Arg Ala Ala
               180                 185                 190
   Leu Gln Gly Gly Gly Pro Pro Tyr Gly Pro Pro Ser Thr Trp Ser Val
           195                 200                 205
   Ser Thr Met Asp Ala Leu Arg Gly Leu Leu Pro Val Leu Gly Gln Pro
       210                 215                 220
   Ile Ile Arg Ser Ile Pro Gln Gly Ile Val Ala Ala Trp Arg Gln Arg
   225                 230                 235                 240
   Ser Ser Arg Asp Pro Ser Trp Arg Gln Pro Glu Arg Thr Ile Leu Arg
                   245                 250                 255
   Pro Arg Phe Arg Arg Glu Val Glu Lys Thr Ala Cys Pro Ser Gly Lys
               260                 265                 270
   Lys Ala Arg Glu Ile Asp Glu Ser Leu Ile Phe Tyr Lys Lys Trp Glu
           275                 280                 285
   Leu Glu Ala Cys Val Asp Ala Ala Leu Leu Ala Thr Gln Met Asp Arg
       290                 295                 300
   Val Asn Ala Ile Pro Phe Thr Tyr Glu Gln Leu Asp Val Leu Lys His
   305                 310                 315                 320
   Lys Leu Asp Glu Leu Tyr Pro Gln Gly Tyr Pro Glu Ser Val Ile Gln
                   325                 330                 335
   His Leu Gly Tyr Leu Phe Leu Lys Met Ser Pro Glu Asp Ile Arg Lys
               340                 345                 350
   Trp Asn Val Thr Ser Leu Glu Thr Leu Lys Ala Leu Leu Glu Val Asn
           355                 360                 365
   Lys Gly His Glu Met Ser Pro Gln Val Ala Thr Leu Ile Asp Arg Phe
       370                 375                 380
   Val Lys Gly Arg Gly Gln Leu Asp Lys Asp Thr Leu Asp Thr Leu Thr
   385                 390                 395                 400
   Ala Phe Tyr Pro Gly Tyr Leu Cys Ser Leu Ser Pro Glu Glu Leu Ser
                   405                 410                 415
   Ser Val Pro Pro Ser Ser Ile Trp Ala Val Arg Pro Gln Asp Leu Asp
               420                 425                 430
   Thr Cys Asp Pro Arg Gln Leu Asp Val Leu Tyr Pro Lys Ala Arg Leu
           435                 440                 445
   Ala Phe Gln Asn Met Asn Gly Ser Glu Tyr Phe Val Lys Ile Gln Ser
       450                 455                 460
   Phe Leu Gly Gly Ala Pro Thr Glu Asp Leu Lys Ala Leu Ser Gln Gln
   465                 470                 475                 480
   Asn Val Ser Met Asp Leu Ala Thr Phe Met Lys Leu Arg Thr Asp Ala
                   485                 490                 495
   Val Leu Pro Leu Thr Val Ala Glu Val Gln Lys Leu Leu Gly Pro His
               500                 505                 510
   Val Glu Gly Leu Lys Ala Glu Glu Arg His Arg Pro Val Arg Asp Trp
           515                 520                 525
   Ile Leu Arg Gln Arg Gln Asp Asp Leu Asp Thr Leu Gly Leu Gly Leu
       530                 535                 540
   Gln Gly Gly Ile Pro Asn Gly Tyr Leu Val Leu Asp Leu Ser Val Gln
   545                 550                 555                 560
   Glu Ala Leu Ser Gly Thr Pro Cys Leu Leu Gly Pro Gly Pro Val Leu
                   565                 570                 575
   Thr Val Leu Ala Leu Leu Leu Ala Ser Thr Leu Ala
               580                 585
<210> 27
   <211> 546
   <212> PRT
   <213> Homo sapiens
<400> 27
   Arg Thr Leu Ala Gly Glu Thr Gly Gln Glu Ala Ala Pro Leu Asp Gly
    1               5                  10                  15
   Val Leu Thr Asn Pro Pro Asn Ile Ser Ser Leu Ser Pro Arg Gln Leu
               20                  25                  30
   Leu Gly Phe Pro Cys Ala Glu Val Ser Gly Leu Ser Thr Glu Arg Val
           35                  40                  45
   Arg Glu Leu Ala Val Ala Leu Ala Gln Lys Asn Val Lys Leu Ser Thr
       50                  55                  60
   Glu Gln Leu Arg Cys Leu Ala His Arg Leu Ser Glu Pro Pro Glu Asp
   65                  70                  75                  80
   Leu Asp Ala Leu Pro Leu Asp Leu Leu Leu Phe Leu Asn Pro Asp Ala
                   85                  90                  95
   Phe Ser Gly Pro Gln Ala Cys Thr Arg Phe Phe Ser Arg Ile Thr Lys
               100                 105                 110
   Ala Asn Val Asp Leu Leu Pro Arg Gly Ala Pro Glu Arg Gln Arg Leu
           115                 120                 125
   Leu Pro Ala Ala Leu Ala Cys Trp Gly Val Arg Gly Ser Leu Leu Ser
       130                 135                 140
   Glu Ala Asp Val Arg Ala Leu Gly Gly Leu Ala Cys Asp Leu Pro Gly
   145                 150                 155                 160
   Arg Phe Val Ala Glu Ser Ala Glu Val Leu Leu Pro Arg Leu Val Ser
                   165                 170                 175
   Cys Pro Gly Pro Leu Asp Gln Asp Gln Gln Glu Ala Ala Arg Ala Ala
               180                 185                 190
   Leu Gln Gly Gly Gly Pro Pro Tyr Gly Pro Pro Ser Thr Trp Ser Val
           195                 200                 205
   Ser Thr Met Asp Ala Leu Arg Gly Leu Leu Pro Val Leu Gly Gln Pro
       210                 215                 220
   Ile Ile Arg Ser Ile Pro Gln Gly Ile Val Ala Ala Trp Arg Gln Arg
   225                 230                 235                 240
   Ser Ser Arg Asp Pro Ser Trp Arg Gln Pro Glu Arg Thr Ile Leu Arg
                   245                 250                 255
   Pro Arg Phe Arg Arg Glu Val Glu Lys Thr Ala Cys Pro Ser Gly Lys
               260                 265                 270
   Lys Ala Arg Glu Ile Asp Glu Ser Leu Ile Phe Tyr Lys Lys Trp Glu
           275                 280                 285
   Leu Glu Ala Cys Val Asp Ala Ala Leu Leu Ala Thr Gln Met Asp Arg
       290                 295                 300
   Val Asn Ala Ile Pro Phe Thr Tyr Glu Gln Leu Asp Val Leu Lys His
   305                 310                 315                 320
   Lys Leu Asp Glu Leu Tyr Pro Gln Gly Tyr Pro Glu Ser Val Ile Gln
                   325                 330                 335
   His Leu Gly Tyr Leu Phe Leu Lys Met Ser Pro Glu Asp Ile Arg Lys
               340                 345                 350
   Trp Asn Val Thr Ser Leu Glu Thr Leu Lys Ala Leu Leu Glu Val Asn
           355                 360                 365
   Lys Gly His Glu Met Ser Pro Gln Val Ala Thr Leu Ile Asp Arg Phe
       370                 375                 380
   Val Lys Gly Arg Gly Gln Leu Asp Lys Asp Thr Leu Asp Thr Leu Thr
   385                 390                 395                 400
   Ala Phe Tyr Pro Gly Tyr Leu Cys Ser Leu Ser Pro Glu Glu Leu Ser
                   405                 410                 415
   Ser Val Pro Pro Ser Ser Ile Trp Ala Val Arg Pro Gln Asp Leu Asp
               420                 425                 430
   Thr Cys Asp Pro Arg Gln Leu Asp Val Leu Tyr Pro Lys Ala Arg Leu
           435                 440                 445
   Ala Phe Gln Asn Met Asn Gly Ser Glu Tyr Phe Val Lys Ile Gln Ser
       450                 455                 460
   Phe Leu Gly Gly Ala Pro Thr Glu Asp Leu Lys Ala Leu Ser Gln Gln
   465                 470                 475                 480
   Asn Val Ser Met Asp Leu Ala Thr Phe Met Lys Leu Arg Thr Asp Ala
                       485                 490                 495
   Val Leu Pro Leu Thr Val Ala Glu Val Gln Lys Leu Leu Gly Pro His
                   500                 505                 510
   Val Glu Gly Leu Lys Ala Glu Glu Arg His Arg Pro Val Arg Asp Trp
               515                 520                 525
   Ile Leu Arg Gln Arg Gln Asp Asp Leu Asp Thr Leu Gly Leu Gly Leu
       530                 535                 540
   Gln Gly
   545
<210> 28
   <211> 7071
   <212> DNA
   <213> Artificial Sequence
<220>
   <223> pKSV7 vector
<220>
   <221> misc_feature
   <222> 1769, 1859, 1871, 2717, 3473, 3482, 3494, 3560, 5445, 6334
   <223> n = A,T,C or G
<400> 28
   ctcgcggatt gttgatgatt acgaaaatat taagagcaca gactattaca cagaaaatca 60
   agaattaaaa aaacgtagag agagtttgaa agaagtagtg aatacatgga aagaggggta 120
   tcacgaaaaa agtaaagagg ttaataaatt aaagcgagag aatgatagtt tgaatgagca 180
   gttgaatgta tcagagaaat ttcaagatag tacagtgact ttatatcgtg ctgcgagggc 240
   gaatttccct gggtttgaga aagggtttaa taggcttaaa gagaaattct ttaatgattc 300
   caaattcgag cgtgtgggac agtttatgga tgttgtacag gataatgtcc agaaggtcga 360
   tagaaagcgt gagaaacagc gtacagacga tttagagatg tagaggtact tttatgccga 420
   gaaaactttt tgcgtgtgac agtccttaaa atatacttag agcgtaagcg aaagtagtag 480
   cgacagctat taactttcgg ttgcaaagct ctaggatttt taatggacgc agcgcatcac 540
   acgcaaaaag gaaattggaa taaatgcgaa atttgagatg ttaattaaag acctttttga 600
   ggtctttttt tcttagattt ttggggttat ttaggggaga aaacataggg gggtactacg 660
   acctcccccc taggtgtcca ttgtccattg tccaaacaaa taaataaata ttgggttttt 720
   aatgttaaaa ggttgttttt tatgttaaag tgaaaaaaac agatgttggg aggtacagtg 780
   atggttgtag atagaaaaga agagaaaaaa gttgctgtta ctttaagact tacacagaag 840
   aaaatgagat attaaataga atccaagaaa aatataatat tagcaaatca gatgcaccgg 900
   tattctaata aaaaatatgy rmaggaggaa tacsgtgcat tttaacaaaa aaagatagac 960
   agcactggca tgctgcctat ctatgactaa attttgttaa atgtattagc accgttatta 1020
   tatcatgagc gaaaatgtaa taaaagaaac tgaaaacaag aaaaattcaa gaggacgtaa 1080
   ttggacattt gttttatatc cagaatcagc aaaagccgag tggttagagt atttaaaaga 1140
   gttacacatt caatttgtag tgtctccatt acatgatagg gatactgata cagaagatag 1200
   gatgaaaaaa gagcattatc atattctagt gatgtatgag ggtaataaat cttatgaaca 1260
   gataaaaata attacagaag aattgaatgc gactattccg cagattgcag gaagtgtgaa 1320
   aggtcttgtg agatatatgc ttcacatgga cgatcctaat aaatttaaat atcaaaaaga 1380
   agatatgata gtttatggcg gtgtagatgt tgatgaatta ttaaagaaaa caacaacaga 1440
   tagatataaa ttaattaaag aaatgattga gtttattgat gaacaaggaa tcgtagaatt 1500
   taagagttta atggattatg caatgaagtt taaatttgat gattggttcc cgcttttatg 1560
   tgataactcg gcgtatgtta ttcaagaata tataaaatca aatcggtata aatctgaccg 1620
   atagattttg aatttaagag tgtcacaaga cactcttttt tcgcaccaac gaaaactggt 1680
   ttaagccgac tgcgcaaaag acataatcga ttcacaaaaa ataggcacac gaaaaacaag 1740
   ttaagggatg cagtttatgc atcccttanc ttacttatta aataatttat agctattgaa 1800
   aagagataag aattgttcaa gctaatattg tttaaatcgt ccattcctgc atgttttang 1860
   gaawtgttaa nttgattttt tgtaatattt tctkgtatyc tttgttamcc catttcataa 1920
   cgaaataatt atacttttgt ttatctttgt gtgatattct tgattttttt ctacttaatc 1980
   tgataagtga gctattcact ttaggtttag gatgaaaata ttctcttgga accatactta 2040
   atatagaaat atcaacttct gccattaaaa gtaatgccaa tgagcgtttt gtatttaata 2100
   atcttttagc aaacccgtat tccacgatta aataaatctc attagctata ctatcaaaaa 2160
   caattttgcg tattatatcc gtacttatgt tataaggtat attaccatat attttatagg 2220
   attggttttt aggaaattta aactgcaata tatccttgtt taaaacttgg aaattatcgt 2280
   gatcttcctt caggttatga ccatctgtgc cagttcgtaa tgtctggtca actttccgac 2340
   tctgagaaac ttctggaatc gctagagaat ttctggaatg ggattcagga gtggacagaa 2400
   cgacacggat atatagtgga tgtgtcaaaa cgcataccat tttgaacgat gacctctaat 2460
   aattgttaat catgttggtt acgtatttat taacttctcc tagtattagt aattatcatg 2520
   gctgtcatgg cgcattaacg gaataaaggg tgtgcttaaa tcgggccatt ttgcgtaata 2580
   agaaaaagga ttaattatga gcgaattgaa ttaataataa ggtaatagat ttacattaga 2640
   aaatgaaagg ggattttatg cgtgagaatg ttacagtcta tcccggcaat agttaccctt 2700
   attatywsga taagaangaa aggatttttc gctacgctca atcctttaaa aaaacacaaa 2760
   agaccacatt ttttaatgtg gtcttttatt cttcaactaa agcacccatt agttcaacaa 2820
   acgaaaattg gataargtgg gatattttwa awataatwta tktatgttac agtaatattg 2880
   acttttaaaa aaggattgat tctaatgaag aaagcagaca agtaagcctc ctaaattcac 2940
   tttagataaa aatttaggag gcatatcaaa tgaactttaa taaaattgat ttagacaatt 3000
   ggaagagaaa agagatattt aatcattatt tgaaccaaca aacgactttt agtataacca 3060
   cagaaattga tattagtgtt ttataccgaa acataaaaca agaaggatat aaattttacc 3120
   ctgcatttat tttcttagtg acaagggtga taaactcaaa tacagctttt agaactggtt 3180
   acaatagcga cggagagtta ggttattggg ataagttaga gccactttat acaatttttg 3240
   atggtgtatc taaaacattc tctggtattt ggactcctgt aaagaatgac ttcaaagagt 3300
   tttatgattt atacctttct gatgtagaga aatataatgg ttcgggggaa ttgtttccca 3360
   aaacacctat acctgaaaat gctttttctc tttctattat tccatggact tcatttactg 3420
   ggtttaactt aaatatcaat aataatagta attaccttct acccattatt acngcaggaa 3480
   anttcattaa taanggtaat tcaatatatt taccgctatc tttacaggta catcattctg 3540
   tttgtgatgg ttatcatgcn ggattgttta tgaactctat tcaggaattg tcagataggc 3600
   ctaatgactg gcttttatat atgagataat gccgactgta ctttttacrg tcggttttct 3660
   aacgatmcat taataggtmc gaaaaagcma cttttttksc gcttaaaacc agtcatacca 3720
   ataacttaag ggtaactagc ctcgccggaa agagcgaaaa tgcctcacat ttgtgccacc 3780
   taaaaaggag cgatttacat atgagttatg cagtttgtag aatgcaaaaa gtgaaatcag 3840
   ctgcattaat gaatcggcca acgcgcgggg agaggcggtt tgcgtattgg gcgctcttcc 3900
   gcttcctcgc tcactgactc gctgcgctcg gtcgttcggc tgcggcgagc ggtatcagct 3960
   cactcaaagg cggtaatacg gttatccaca gaatcagggg ataacgcagg aaagaacatg 4020
   tgagcaaaag gccagcaaaa ggccaggaac cgtaaaaagg ccgcgttgct ggcgtttttc 4080
   cataggctcc gcccccctga cgagcatcac aaaaatcgac gctcaagtca gaggtggcga 4140
   aacccgacag gactataaag ataccaggcg tttccccctg gaagctccct cgtgcgctct 4200
   cctgttccga ccctgccgct taccggatac ctgtccgcct ttctcccttc gggaagcgtg 4260
   gcgctttctc atagctcacg ctgtaggtat ctcagttcgg tgtaggtcgt tcgctccaag 4320
   ctgggctgtg tgcacgaacc ccccgttcag cccgaccgct gcgccttatc cggtaactat 4380
   cgtcttgagt ccaacccggt aagacacgac ttatcgccac tggcagcagc cactggtaac 4440
   aggattagca gagcgaggta tgtaggcggt gctacagagt tcttgaagtg gtggcctrss 4500
   yacksskmyc ctagaagaac agtatttggt atctgcgctc tgctgaagcc agttaccttc 4560
   ggaaaaagag ttggtagctc ttgatccggc aaamaaacca ccgctggtag cggtggtttt 4620
   tttgtttgca agcagcagat tacgcgcaga aaaaaaggat ctcaagaaga tcctttgatc 4680
   ttttctacgg ggtctgacgc tcagtggaac gaaaactcac gttaagggat tttggtcatg 4740
   agattatcaa aaaggatctt cacctagatc cttttaaatt aaaaatgaag ttttaaatca 4800
   atctaaagta tatatgagta aacttggtct gacagttacc aatgcttaat cagtgaggca 4860
   cctatctcag cgatctgtct atttcgttca tccatagttg cctgactccc cgtcgtgtag 4920
   ataactacga tacgggaggg cttaccatct ggccccagtg ctgcaatgat accgcgagac 4980
   ccacgctcac cggctccaga tttatcagca ataaaccagc cagccggaag ggccgagcgc 5040
   agaagtggtc ctgcaacttt atccgcctcc atccagtcta ttaattgttg ccgggaagct 5100
   agagtaagta gttcgccagt taatagtttg cgcaacgttg ttgccattgc tacaggcatc 5160
   gtggtgtcac gctcgtcgtt tggtatggct tcattcagct ccggttccca acgatcaagg 5220
   cgagttacat gatcccccat gttgtgcaaa aaagcggtta gctccttcgg tcctccgatc 5280
   gttgtcagaa gtaagttggc cgcagtgtta tcactcatgg ttatggcagc actgcataat 5340
   tctcttactg tcatgccatc cgtaagatgc ttttctgtga ctggtgrkka stcwcmcmag 5400
   tcattctgag aatagtgtat gcggcgaccg agttgctctt gcccnggsgt caatacggga 5460
   taataccgcs ccacatagca raactttaaa agtgctcatc attggaaaac gttcttcggg 5520
   gcgaaaactc tcaaggatct taccgctgtt gagatccagt tcgatgtaac ccactcgtgc 5580
   acccaactga tcttcagcat cttttacttt caccagcgtt tctgggtgag caaaaacagg 5640
   aaggcaaaat gccgcaaaaa agggaataag ggcgacacgg aaatgttgaa tactcatact 5700
   cttccttttt caatattatt gaagcattta tcagggttat tgtctcatga gcggatacat 5760
   atttgaatgt atttagaaaa ataaacaaat aggggttccg cgcacatttc cccgaaaagt 5820
   gccacctgac gtctaagaaa ccattattat catgacatta acctataaaa ataggcgtat 5880
   cacgaggccc tttcgtctcg cgcgtttcgg tgatgacggt gaaaacctct gacacatgca 5940
   gctcccggag acggtcacag cttgtctgta agcggatgcc gggagcagac aagcccgtca 6000
   gggcgcgtca gcgggtgttg gcgggtgtcg gggctggctt aactatgcgg catcagagca 6060
   gattgtactg agagtgcacm atatgcggtg tgaaataccg cacagatgcg taaggagaaa 6120
   ataccgcatc aggcgccatt cgccattcag gctgcgcaac tgttgggaag ggcgatcggt 6180
   gcgggcctct tcgctattac gccagctggc gaaaggggga tgtgctgcaa ggcgattaag 6240
   ttgggtaacg ccagggtttt yccagtcacg acgttgtaaa acgacggcca gtgccaagct 6300
   tgcatgcctg caggtcgact ctagaggatc cccngggtac cgagctcgaa ttcgtaatca 6360
   tgtcatagct gtttcctgtg tgaaattgtt atccgctcac aattccacac aacatacgag 6420
   ccggaagcat aaagtgtaaa gcctggggtg cctaatgagt gagctaactc acattaattg 6480
   cgttgcgctc actgcccgct ttccagtcgg gaaacctgtc gtgccagctg gactaaaagg 6540
   catgcaattt cataatcaaa gagagcgaaa aagtagaacg aatgatgata ttgaccatga 6600
   gcgaacacgt gaaaattatg atttgaaaaa tgataaaaat attgattaca acgaacgtgt 6660
   caaagaaatt attgaatcac aaaaaacagg tacaagaaaa acgaggaaag atgctgttct 6720
   tgtaaatgag ttgctagtaa catctgaccg agattttttt gagcaactgg atcagtacaa 6780
   gaaagatact gtatttcata aacaggaact gcaagaagtt aaggatgagt tacagaaggc 6840
   aaataagcag ttacagagtg gaatagagca tatgaggtct acgaaaccct ttgattatga 6900
   aaatgagcgt acaggtttgt tctctggacg tgaagagact ggtagaaaga tattaactgc 6960
   tgatgaattt gaacgcctgc aagaaacaat ctcttcgaac ggattgttga tgattacgaa 7020
   atataagagc ccgactattc ccagaaatca gaattaaaaa cgtagagaga g          7071
<210> 29
   <211> 6055
   <212> DNA
   <213> Artificial Sequence
<220>
   <223> pINT vector
<400> 29
   agatctccaa aaataaacag gtggtggtat taatgaagat aaaaaaatta gcaaacggta 60
   aatattgtgt tcgcctacgt ataaaagtcg atggtgaatg gaaagaaaag cgtttgacag 120
   atacaagtga aacaaactta atgtataaag catctaaatt attaaaacaa gttcagcatg 180
   atagtagttc tctgaaagaa tggaacttca aagaatttta tacgctattc atgaaaacat 240
   ttaaagatgg gaaaagtagt caatctacta ttaatttata cgatcttgct tataatcaat 300
   tcgttgatta tttcgatgaa aaaattaaat ttaattcgat tgatgcggtt caatatcaac 360
   aatttattaa tcatttatct gtagactatg caatatccac tgtagacacc agacaccgca 420
   aaattagagc gatttttaac aaggctgttc atttaggtta catgaagaaa aaccccacta 480
   taggggctca tataagcgga caggacgtag cgaaaaataa agcacaattt atggaaacag 540
   acaaagttca tttactatta gaagaacttg caaaatttca ttctatatca cgagcagtta 600
   tctttctagc tgtccagaca ggcatgaggt tcgaagaaat tattgcacta acaaagaagg 660
   atattaattt cactaaacgt tcaataactg tgaataaagc ttgggattac aagtacacta 720
   atacattcat tgataccaaa acaaaaaaat cacgagtgat ctatattgat aactctaccg 780
   ctcaatattt acattcgtat ttaaattggc atactgaata tatgaaggaa catgctatta 840
   agaatccatt gatgttatta ttcatcactt accacaataa gccagtagac aacgcgtctt 900
   gtaataaagc tttgaagaag atatgtagta caatcaattc tgaaccagtg acattacaca 960
   agctacgaca tacgcataca ggcttatgtg tagaagcggg tatggatatt atttatgtag 1020
   ctgataggct tggtcatgat gacattaata caacattaaa atactatagt catctaagct 1080
   ctaatttaag acaacataat cagtccaaag tagatgcttt tttcacacta aaaacagatg 1140
   aaaataccac aaattttacc acaaatgcca caaaaacaac ggaataacct aggataactt 1200
   cgtataatgt atgctatacg aagttatatg catgggtatt atacgatata aaaaaaactc 1260
   caaaacattc atccgccctt taatatcaag gcttttcaac gttttagaga tttctttaca 1320
   ttactattta acgtcctgag agggattaac acacactgat ataaagccat ttaggatata 1380
   tataccacaa ataataccac aaacatttta tgtaataata aatattattt attattacat 1440
   tgaaataaat attcgttata aatagttttt atatcaagat gttttttctc aaggttttta 1500
   taaaatgact ttaattcttt tgtttcaagt agtccagaga agattttttc aacagcgttc 1560
   ttctttccct ccacgcatgc gacgtcaata cgactcacta tagggcgaat tgggtaccgg 1620
   gccccccctc gaggtcgacg gtatcgataa gcttgatatc gaattcctgc agcccggggg 1680
   atccactagt tctagagcgg ccgccaccgc ggtggagctc cagcttttgt tccctttagt 1740
   gagggttaat taaataactt cgtataatgt atgctatacg aagttatgcg atcgcctctc 1800
   gcctgtcccc tcagttcagt aatttcctgc atttgcctgt ttccagtcgg tagatattcc 1860
   acaaaacagc agggaagcag cgcttttccg ctgcataacc ctgcttcggg gtcattatag 1920
   cgattttttc ggtatatcca tcctttttcg cacgatatac aggattttgc caaagggttc 1980
   gtgtagactt tccttggtgt atccaacggc gtcagccggg caggataggt gaagtaggcc 2040
   cacccgcgag cgggtgttcc ttcttcactg tcccttattc gcacctggcg gtgctcaacg 2100
   ggaatcctgc tctgcgaggc tggccggcta ccgccggcgt aacagatgag ggcaagcggc 2160
   ggagaattac aacttatatc gtatggggct gacttcaggt gctacatttg aagagataaa 2220
   ttgcactgaa atctagaaat attttatctg attaataaga tgatcttctt gagatcgttt 2280
   tggtctgcgc gtaatctctt gctctgaaaa cgaaaaaacc gccttgcagg gcggtttttc 2340
   gaaggttctc tgagctacca actctttgaa ccgaggtaac tggcttggag gagcgcagtc 2400
   accaaaactt gtcctttcag tttagcctta accggcgcat gacttcaaga ctaactcctc 2460
   taaatcaatt accagtggct gctgccagtg gtgcttttgc atgtctttcc gggttggact 2520
   caagacgata gttaccggat aaggcgcagc ggtcggactg aacggggggt tcgtgcatac 2580
   agtccagctt ggagcgaact gcctacccgg aactgagtgt caggcgtgga atgagacaaa 2640
   cgcggccata acagcggaat gacaccggta aaccgaaagg caggaacagg agagcgcacg 2700
   agggagccgc cagggggaaa cgcctggtat ctttatagtc ctgtcgggtt tcgccaccac 2760
   tgatttgagc gtcagatttc gtgatgcttg tcaggggggc ggagcctatg gaaaaacggc 2820
   tttgccgcgg ccctctcact tccctgttaa gtatcttcct ggcatcttcc aggaaatctc 2880
   cgccccgttc gtaagccatt tccgctcgcc gcagtcgaac gaccgagcgt agcgagtcag 2940
   tgagcgagga agcggaatat atcctgtatc acatattctg ctgacgcacc ggtgcagcct 3000
   tttttctcct gccacatgaa gcacttcact gacaccctca tcagtgccaa catagtaagc 3060
   cagtatacac tccgctagcg ctgatgtccg gcggtgcttt tgccgttacg caccaccccg 3120
   tcagtagctg aacaggaggg acagctgata gaaacagaag ccactggagc acctcaaaaa 3180
   caccatcata cactaaatca gtaagttggc agcatcaccc gacgcacttt gcgccgaata 3240
   aatacctgtg acggaagatc acttcgcaga ataaataaat cctggtgtcc ctgttgatac 3300
   cgggaagccc tgggccaact tttggcgaaa atgagacgtt gatcggcacg taagaggttc 3360
   caactttcac cataatgaaa taagatcact accgggcgta ttttttgagt tatcgagatt 3420
   ttcaggagct aaggaagcta aaatggagaa aaaaatcact ggatatacca ccgttgatat 3480
   atcccaatgg catcgtaaag aacattttga ggcatttcag tcagttgctc aatgtaccta 3540
   taaccagacc gttcagctgg atattacggc ctttttaaag accgtaaaga aaaataagca 3600
   caagttttat ccggccttta ttcacattct tgcccgcctg atgaatgctc atccggaatt 3660
   ccgtatggca atgaaagacg gtgagctggt gatatgggat agtgttcacc cttgttacac 3720
   cgttttccat gagcaaactg aaacgttttc atcgctctgg agtgaatacc acgacgattt 3780
   ccggcagttt ctacacatat attcgcaaga tgtggcgtgt tacggtgaaa acctggccta 3840
   tttccctaaa gggtttattg agaatatgtt tttcgtctca gccaatccct gggtgagttt 3900
   caccagtttt gatttaaacg tggccaatat ggacaacttc ttcgcccccg ttttcaccat 3960
   gggcaaatat tatacgcaag gcgacaaggt gctgatgccg ctggcgattc aggttcatca 4020
   tgccgtttgt gatggcttcc atgtcggcag aatgcttaat gaattacaac agtactgcga 4080
   tgagtggcag ggcggggcgt aattttttta aggcagttat tggtgccctt aaacgcctgg 4140
   ttgctacgcc tgaataagtg ataataagcg gatgaatggc agaaattcga aagcaaattc 4200
   gacccggtcg tcggttcagg gcagggtcgt taaatagcga cgtctaagaa accattatta 4260
   tcatgacatt aacctataaa aataggcgta tcacgaggcc ctttcgtctc gcgcgtttcg 4320
   gtgatgacgg tgaaaacctc tgacacatgc agctcccgga gacggtcaca gcttgtctgt 4380
   aagcggatgc cgggagcaga caagcccgtc agggcgcgtc agcgggtgtt ggcgggtgtc 4440
   ggggctggct taactatgcg gcatcagagc agattgtact gagagtgcac aatcgcatcc 4500
   gattgcagta taaatttaac gatcactcat catgttcata tttatcagag ctcgtgctat 4560
   aattatacta attttataag gaggaaaaaa tatgggcatt tttagtattt ttgtaatcag 4620
   cacagttcat tatcaaccaa acaaaaaata agtggttata atgaatcgtt aataagcaaa 4680
   attcatataa ccaaattaaa gagggttata atgaacgaga aaaatataaa acacagtcaa 4740
   aactttatta cttcaaaaca taatatagat aaaataatga caaatataag attaaatgaa 4800
   catgataata tctttgaaat cggctcagga aaaggccatt ttacccttga attagtaaag 4860
   aggtgtaatt tcgtaactgc cattgaaata gaccataaat tatgcaaaac tacagaaaat 4920
   aaacttgttg atcacgataa tttccaagtt ttaaacaagg atatattgca gtttaaattt 4980
   cctaaaaacc aatcctataa aatatatggt aatatacctt ataacataag tacggatata 5040
   atacgcaaaa ttgtttttga tagtatagct aatgagattt atttaatcgt ggaatacggg 5100
   tttgctaaaa gattattaaa tacaaaacgc tcattggcat tacttttaat ggcagaagtt 5160
   gatatttcta tattaagtat ggttccaaga gaatattttc atcctaaacc taaagtgaat 5220
   agctcactta tcagattaag tagaaaaaaa tcaagaatat cacacaaaga taaacaaaag 5280
   tataattatt tcgttatgaa atgggttaac aaagaataca agaaaatatt tacaaaaaat 5340
   caatttaaca attccttaaa acatgcagga attgacgatt taaacaatat tagctttgaa 5400
   caattcttat ctcttttcaa tagctataaa ttatttaata agtaagttaa gggatgcata 5460
   aactgcatcc cttaacttgt ttttcgtgtg cccgatcggt gcgggcctct tcgctattac 5520
   gccagctggc gaaaggggga tgtgctgcaa ggcgattaag ttgggtaacg ccagggtttt 5580
   cccagtcacg acgttgtaaa acgacggcca gtgccaagct agctttcgat catcataatt 5640
   ctgtctcatt atataacatc ctccatacct tctattatag aataccataa actcatctgg 5700
   caattcattt cgagtcacga agaacggaaa aactgccggt ttttatatta caaatgtatt 5760
   aagtttttct attaacaaaa aacaataggt ttcccatagc gaaagttgtt gattaacgtt 5820
   cacatcccac ttacactata aaggtttacc cagcaataca tctcaagccc taagaataca 5880
   cgttcgcttt tcaactgtta cagaattatt acaaatagtt ggtatagtcc tctttagcct 5940
   ttggagctat tatctcatca tttgtttttt aggtgaaaac tgggtaaact tagtattaat 6000
   caatataaaa ttaattctca aatacttaat tacgtactgg gattttctga aaaaa 6055
<210> 30
   <211> 5
   <212> PRT
   <213> Listeria monocytogenes
<400> 30
   Lys Lys Arg Arg Lys
   1 5
<210> 31
   <211> 6
   <212> PRT
   <213> Listeria monocytogenes
<400> 31
   Asp Glu Trp Glu Glu Glu
   1 5
<210> 32
   <211> 12
   <212> PRT
   <213> Listeria monocytogenes
<400> 32
   Asp Arg Leu Ala Asp Leu Arg Asp Arg Gly Thr Gly
   1 5 10
<210> 33
   <211> 13
   <212> PRT
   <213> Listeria monocytogenes
<400> 33
   Ile Lys Lys Lys Arg Arg Lys Ala Ile Ala Ser Ser Asp
   1 5 10
<210> 34
   <211> 5
   <212> PRT
   <213> Listeria monocytogenes
<400> 34
   Thr Asp Ser Glu Asp
   1 5
<210> 35
   <211> 227
   <212> DNA
   <213> Listeria monocytogenes
<400> 35
<210> 36
   <211> 1953
   <212> DNA
   <213> Artificial Sequence
<220>
   <223> Fusion protein coding sequence
<400> 36
   gtgggattaa atagatttat gcgtgcgatg atggtagttt tcattactgc caactgcatt 60
   acgattaacc ccgacataat atttgcagcg acagatagcg aagattccag tctaaacaca 120
   gatgaatggg aagaagaaaa aacagaagag cagccaagcg aggtaaatac gggaccaaga 180
   tacgaaactg cacgtgaagt aagttcacgt gatattgagg aactagaaaa atcgaataaa 240
   gtgaaaaata cgaacaaagc agacctaata gcaatgttga aagcaaaagc agagaaaggt 300
   ggatcccgta cattagcagg tgaaacaggt caagaagcag caccacttga cggtgtatta 360
   acgaatccac caaatatatc aagtttaagt ccacgtcaat tattaggttt tccatgtgca 420
   gaagtttcag gtttaagtac agaacgtgtc cgtgagttag cagttgcatt agcacaaaaa 480
   aacgttaaat tatctacaga acagttacgt tgtttagccc atagattaag cgaaccacca 540
   gaagacttag atgcacttcc tttagacctt cttttattct taaatccaga tgcattttca 600
   ggaccacaag catgtacacg tttttttagt cgaattacaa aagccaatgt tgatttatta 660
   cctcgtgggg ctcctgaaag acaacgttta ttacctgctg cattagcatg ctggggtgtt 720
   cgcggtagct tattaagtga agccgatgtt cgtgctttag ggggtttagc atgtgattta 780
   cctggtcgtt tcgttgcaga atcagcagaa gtgttattac cgagattagt ttcatgccca 840
   ggacctttag atcaagatca acaagaggca gctagagcag ctcttcaagg aggaggccca 900
   ccatatggcc caccaagtac atggagtgtt tctacaatgg atgcgttaag aggtttatta 960
   ccggttttag gacaaccaat tattcgtagt attccacaag gcattgtagc agcatggcgt 1020
   caacgtagtt ctcgtgatcc gtcttggcga caaccagaac gtacaattct acgtccaaga 1080
   tttcgtagag aagtagaaaa aacggcgtgt cctagtggca aaaaagcacg tgaaattgat 1140
   gaaagtttaa ttttttataa aaaatgggaa ttagaagcat gtgtcgatgc agcattacta 1200
   gctacacaaa tggatcgtgt taatgctatt ccattcacat atgaacaatt agatgtttta 1260
   aagcataaat tagacgaatt atatccacaa ggttatccag aatcagttat tcaacattta 1320
   ggttacttat ttttaaaaat gagtccagaa gacatacgca aatggaatgt tacaagttta 1380
   gaaacattaa aagcgctttt agaagttaac aaaggtcatg aaatgagtcc acaagttgct 1440
   acgttaattg atagattcgt taaaggccgt ggtcaattag ataaagatac tttagataca 1500
   ttaacagcat tttatcctgg ctacttatgc agtttatcac cagaagaatt aagttccgtt 1560
   ccaccgagta gtatctgggc agttcgtccg caagatttag atacatgcga cccacgtcaa 1620
   ttagatgttt tatatccaaa agcaagatta gctttccaaa atatgaacgg tagtgaatat 1680
   ttcgtaaaaa ttcaatcctt tttaggtggt gcaccaactg aagatctaaa agcattaagc 1740
   caacaaaatg taagtatgga tttagctacg tttatgaaat tacgtacaga tgcagttcta 1800
   ccattaacag ttgcagaagt tcaaaaatta ttaggtccac acgtagaagg attaaaagca 1860
   gaagaacgtc accgtccagt tcgcgattgg attttacgtc aacgtcaaga tgatttagat 1920
   acattaggtt taggtttaca aggctaagag ctc                              1953
<210> 37
   <211> 1920
   <212> DNA
   <213> Listeria monocytogenes
<400> 37
   gtgggattaa atagatttat gcgtgcgatg atggtagttt tcattactgc caactgcatt 60
   acgattaacc ccgacataat atttgcagcg acagatagcg aagattccag tctaaacaca 120
   gatgaatggg aagaagaaaa aacagaagag cagccaagcg aggtaaatac gggaccaaga 180
   tacgaaactg cacgtgaagt aagttcacgt gatattgagg aactagaaaa atcgaataaa 240
   gtgaaaaata cgaacaaagc agacctaata gcaatgttga aagcaaaagc agagaaaggt 300
   ccgaataaca ataataacaa cggtgagcaa acaggaaatg tggctataaa tgaagaggct 360
   tcaggagtcg accgaccaac tctgcaagtg gagcgtcgtc atccaggtct gtcatcggat 420
   agcgcagcgg aaattaaaaa aagaagaaaa gccatagcgt cgtcggatag tgagcttgaa 480
   agccttactt atccagataa accaacaaaa gcaaataaga gaaaagtggc gaaagagtca 540
   gttgtggatg cttctgaaag tgacttagat tctagcatgc agtcagcaga cgagtctaca 600
   ccacaacctt taaaagcaaa tcaaaaacca tttttcccta aagtatttaa aaaaataaaa 660
   gatgcgggga aatgggtacg tgataaaatc gacgaaaatc ctgaagtaaa gaaagcgatt 720
   gttgataaaa gtgcagggtt aattgaccaa ttattaacca aaaagaaaag tgaagaggta 780
   aatgcttcgg acttcccgcc accacctacg gatgaagagt taagacttgc tttgccagag 840
   acaccgatgc ttctcggttt taatgctcct actccatcgg aaccgagctc attcgaattt 900
   ccgccgccac ctacggatga agagttaaga cttgctttgc cagagacgcc aatgcttctt 960
   ggttttaatg ctcctgctac atcggaaccg agctcattcg aatttccacc gcctccaaca 1020
   gaagatgaac tagaaattat gcgggaaaca gcaccttcgc tagattctag ttttacaagc 1080
   ggggatttag ctagtttgag aagtgctatt aatcgccata gcgaaaattt ctctgatttc 1140
   ccactaatcc caacagaaga agagttgaac gggagaggcg gtagaccaac atctgaagaa 1200
   tttagttcgc tgaatagtgg tgattttaca gatgacgaaa acagcgagac aacagaagaa 1260
   gaaattgatc gcctagctga tttaagagat agaggaacag gaaaacactc aagaaatgcg 1320
   ggttttttac cattaaatcc atttattagt agccctgttc cttcattaac tccaaaggta 1380
   ccgaaaataa gcgcgccggc tctgataagt gacataacta aaaaagcgcc atttaagaat 1440
   ccatcacagc cattaaatgt gtttaataaa aaaactacaa cgaaaacagt gactaaaaaa 1500
   ccaacccctg taaagaccgc accaaagcta gcagaacttc ctgccacaaa accacaagaa 1560
   accgtactta gggaaaataa aacacccttt atagaaaaac aagcagaaac aaacaagcag 1620
   tcaatcaata tgccgagcct accagtaatc caaaaagaag ctacagagag cgataaagag 1680
   gaaatgaaac cacaaaccga ggaaaaaatg gtagaggaaa gcgaatcagc taataacgca 1740
   aacggaaaaa atcgttctgc tggcattgaa gaaggaaaac taattgctaa aagtgcagaa 1800
   gacgaaaaag cgaaggaaga accagggaac catacgacgt taattcttgc aatgttagct 1860
   attggcgtgt tctctttagg ggcgtttatc aaaattattc aattaagaaa aaataattaa 1920
<210> 38
   <211> 639
   <212> PRT
   <213> Listeria monocytogenes
<400> 38
   Val Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Pro Asn Asn Asn Asn Asn Asn Gly Glu Gln Thr Gly
               100                 105                 110
   Asn Val Ala Ile Asn Glu Glu Ala Ser Gly Val Asp Arg Pro Thr Leu
           115                 120                 125
   Gln Val Glu Arg Arg His Pro Gly Leu Ser Ser Asp Ser Ala Ala Glu
       130                 135                 140
   Ile Lys Lys Arg Arg Lys Ala Ile Ala Ser Ser Asp Ser Glu Leu Glu
   145                 150                 155                 160
   Ser Leu Thr Tyr Pro Asp Lys Pro Thr Lys Ala Asn Lys Arg Lys Val
                   165                 170                 175
   Ala Lys Glu Ser Val Val Asp Ala Ser Glu Ser Asp Leu Asp Ser Ser
               180                 185                 190
   Met Gln Ser Ala Asp Glu Ser Thr Pro Gln Pro Leu Lys Ala Asn Gln
           195                 200                 205
   Lys Pro Phe Phe Pro Lys Val Phe Lys Lys Ile Lys Asp Ala Gly Lys
       210                 215                 220
   Trp Val Arg Asp Lys Ile Asp Glu Asn Pro Glu Val Lys Lys Ala Ile
   225                 230                 235                 240
   Val Asp Lys Ser Ala Gly Leu Ile Asp Gln Leu Leu Thr Lys Lys Lys
                   245                 250                 255
   Ser Glu Glu Val Asn Ala Ser Asp Phe Pro Pro Pro Pro Thr Asp Glu
               260                 265                 270
   Glu Leu Arg Leu Ala Leu Pro Glu Thr Pro Met Leu Leu Gly Phe Asn
           275                 280                 285
   Ala Pro Thr Pro Ser Glu Pro Ser Ser Phe Glu Phe Pro Pro Pro Pro
       290                 295                 300
   Thr Asp Glu Glu Leu Arg Leu Ala Leu Pro Glu Thr Pro Met Leu Leu
   305                 310                 315                 320
   Gly Phe Asn Ala Pro Ala Thr Ser Glu Pro Ser Ser Phe Glu Phe Pro
                   325                 330                 335
   Pro Pro Pro Thr Glu Asp Glu Leu Glu Ile Met Arg Glu Thr Ala Pro
               340                 345                 350
   Ser Leu Asp Ser Ser Phe Thr Ser Gly Asp Leu Ala Ser Leu Arg Ser
           355                 360                 365
   Ala Ile Asn Arg His Ser Glu Asn Phe Ser Asp Phe Pro Leu Ile Pro
       370                 375                 380
   Thr Glu Glu Glu Leu Asn Gly Arg Gly Gly Arg Pro Thr Ser Glu Glu
   385                 390                 395                 400
   Phe Ser Ser Leu Asn Ser Gly Asp Phe Thr Asp Asp Glu Asn Ser Glu
                   405                 410                 415
   Thr Thr Glu Glu Glu Ile Asp Arg Leu Ala Asp Leu Arg Asp Arg Gly
               420                 425                 430
   Thr Gly Lys His Ser Arg Asn Ala Gly Phe Leu Pro Leu Asn Pro Phe
           435                 440                 445
   Ile Ser Ser Pro Val Pro Ser Leu Thr Pro Lys Val Pro Lys Ile Ser
       450                 455                 460
   Ala Pro Ala Leu Ile Ser Asp Ile Thr Lys Lys Ala Pro Phe Lys Asn
   465                 470                 475                 480
   Pro Ser Gln Pro Leu Asn Val Phe Asn Lys Lys Thr Thr Thr Lys Thr
                   485                 490                 495
   Val Thr Lys Lys Pro Thr Pro Val Lys Thr Ala Pro Lys Leu Ala Glu
               500                 505                 510
   Leu Pro Ala Thr Lys Pro Gln Glu Thr Val Leu Arg Glu Asn Lys Thr
           515                 520                 525
   Pro Phe Ile Glu Lys Gln Ala Glu Thr Asn Lys Gln Ser Ile Asn Met
       530                 535                 540
   Pro Ser Leu Pro Val Ile Gln Lys Glu Ala Thr Glu Ser Asp Lys Glu
   545                 550                 555                 560
   Glu Met Lys Pro Gln Thr Glu Glu Lys Met Val Glu Glu Ser Glu Ser
                   565                 570                 575
   Ala Asn Asn Ala Asn Gly Lys Asn Arg Ser Ala Gly Ile Glu Glu Gly
               580                 585                 590
   Lys Leu Ile Ala Lys Ser Ala Glu Asp Glu Lys Ala Lys Glu Glu Pro
           595                 600                 605
   Gly Asn His Thr Thr Leu Ile Leu Ala Met Leu Ala Ile Gly Val Phe
       610                 615                 620
   Ser Leu Gly Ala Phe Ile Lys Ile Ile Gln Leu Arg Lys Asn Asn
   625                 630                 635
<210> 39
   <211> 533
   <212> DNA
   <213> Artificial Sequence
<220>
   <223> Expression construct
<400> 39
   ggtaccggga agcagttggg gttaactgat taacaaatgt tagagaaaaa ttaattctcc 60
   aagtgatatt cttaaaataa ttcatgaata ttttttctta tattagctaa ttaagaagat 120
   aattaactgc taatccaatt tttaacggaa taaattagtg aaaatgaagg ccgaattttc 180
   cttgttctaa aaaggttgta ttagcgtatc acgaggaggg agtataagtg ggattaaata 240
   gatttatgcg tgcgatgatg gtagttttca ttactgccaa ctgcattacg attaaccccg 300
   acataatatt tgcagcgaca gatagcgaag attccagtct aaacacagat gaatgggaag 360
   aagaaaaaac agaagagcag ccaagcgagg taaatacggg accaagatac gaaactgcac 420
   gtgaagtaag ttcacgtgat attgaggaac tagaaaaatc gaataaagtg aaaaatacga 480
   acaaagcaga cctaatagca atgttgaaag caaaagcaga gaaaggtgga tcc        533
<210> 40
   <211> 100
   <212> PRT
   <213> Artificial Sequence
<220>
   <223> ActA-N100
<400> 40
   Val Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly
               100
<210> 41
   <211> 648
   <212> PRT
   <213> Artificial Sequence
<220>
   <223> Fusion protein
<400> 41
   Val Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser Arg Thr Leu Ala Gly Glu Thr Gly Gln Glu
               100                 105                 110
   Ala Ala Pro Leu Asp Gly Val Leu Thr Asn Pro Pro Asn Ile Ser Ser
           115                 120                 125
   Leu Ser Pro Arg Gln Leu Leu Gly Phe Pro Cys Ala Glu Val Ser Gly
       130                 135                 140
   Leu Ser Thr Glu Arg Val Arg Glu Leu Ala Val Ala Leu Ala Gln Lys
   145                 150                 155                 160
   Asn Val Lys Leu Ser Thr Glu Gln Leu Arg Cys Leu Ala His Arg Leu
                   165                 170                 175
   Ser Glu Pro Pro Glu Asp Leu Asp Ala Leu Pro Leu Asp Leu Leu Leu
               180                 185                 190
   Phe Leu Asn Pro Asp Ala Phe Ser Gly Pro Gln Ala Cys Thr Arg Phe
           195                 200                 205
   Phe Ser Arg Ile Thr Lys Ala Asn Val Asp Leu Leu Pro Arg Gly Ala
       210                 215                 220
   Pro Glu Arg Gln Arg Leu Leu Pro Ala Ala Leu Ala Cys Trp Gly Val
   225                 230                 235                 240
   Arg Gly Ser Leu Leu Ser Glu Ala Asp Val Arg Ala Leu Gly Gly Leu
                   245                 250                 255
   Ala Cys Asp Leu Pro Gly Arg Phe Val Ala Glu Ser Ala Glu Val Leu
               260                 265                 270
   Leu Pro Arg Leu Val Ser Cys Pro Gly Pro Leu Asp Gln Asp Gln Gln
           275                 280                 285
   Glu Ala Ala Arg Ala Ala Leu Gln Gly Gly Gly Pro Pro Tyr Gly Pro
       290                 295                 300
   Pro Ser Thr Trp Ser Val Ser Thr Met Asp Ala Leu Arg Gly Leu Leu
   305                 310                 315                 320
   Pro Val Leu Gly Gln Pro Ile Ile Arg Ser Ile Pro Gln Gly Ile Val
                   325                 330                 335
   Ala Ala Trp Arg Gln Arg Ser Ser Arg Asp Pro Ser Trp Arg Gln Pro
               340                 345                 350
   Glu Arg Thr Ile Leu Arg Pro Arg Phe Arg Arg Glu Val Glu Lys Thr
           355                 360                 365
   Ala Cys Pro Ser Gly Lys Lys Ala Arg Glu Ile Asp Glu Ser Leu Ile
       370                 375                 380
   Phe Tyr Lys Lys Trp Glu Leu Glu Ala Cys Val Asp Ala Ala Leu Leu
   385                 390                 395                 400
   Ala Thr Gln Met Asp Arg Val Asn Ala Ile Pro Phe Thr Tyr Glu Gln
                   405                 410                 415
   Leu Asp Val Leu Lys His Lys Leu Asp Glu Leu Tyr Pro Gln Gly Tyr
               420                 425                 430
   Pro Glu Ser Val Ile Gln His Leu Gly Tyr Leu Phe Leu Lys Met Ser
           435                 440                 445
   Pro Glu Asp Ile Arg Lys Trp Asn Val Thr Ser Leu Glu Thr Leu Lys
       450                 455                 460
   Ala Leu Leu Glu Val Asn Lys Gly His Glu Met Ser Pro Gln Val Ala
   465                 470                 475                 480
   Thr Leu Ile Asp Arg Phe Val Lys Gly Arg Gly Gln Leu Asp Lys Asp
                   485                 490                 495
   Thr Leu Asp Thr Leu Thr Ala Phe Tyr Pro Gly Tyr Leu Cys Ser Leu
               500                 505                 510
   Ser Pro Glu Glu Leu Ser Ser Val Pro Pro Ser Ser Ile Trp Ala Val
           515                 520                 525
   Arg Pro Gln Asp Leu Asp Thr Cys Asp Pro Arg Gln Leu Asp Val Leu
       530                 535                 540
   Tyr Pro Lys Ala Arg Leu Ala Phe Gln Asn Met Asn Gly Ser Glu Tyr
   545                 550                 555                 560
   Phe Val Lys Ile Gln Ser Phe Leu Gly Gly Ala Pro Thr Glu Asp Leu
                   565                 570                 575
   Lys Ala Leu Ser Gln Gln Asn Val Ser Met Asp Leu Ala Thr Phe Met
               580                 585                 590
   Lys Leu Arg Thr Asp Ala Val Leu Pro Leu Thr Val Ala Glu Val Gln
           595                 600                 605
   Lys Leu Leu Gly Pro His Val Glu Gly Leu Lys Ala Glu Glu Arg His
       610                 615                 620
   Arg Pro Val Arg Asp Trp Ile Leu Arg Gln Arg Gln Asp Asp Leu Asp
   625                 630                 635                 640
   Thr Leu Gly Leu Gly Leu Gln Gly
                   645
<210> 42
   <211> 81
   <212> DNA
   <213> Homo sapiens
<400> 42
   gccatgacag aatataaatt agttgtagtt ggtgcagatg gtgttggtaa aagtgcatta 60
   acaattcaat taattcaata a                                           81
<210> 43
   <211> 26
   <212> PRT
   <213> Homo sapiens
<400> 43
   Ala Met Thr Glu Tyr Lys Leu Val Val Val Gly Ala Asp Gly Val Gly
   1               5                   10                  15
   Lys Ser Ala Leu Thr Ile Gln Leu Ile Gln
               20                  25
<210> 44
   <211> 2039
   <212> DNA
   <213> Artificial Sequence
<220>
   <223> Fusion protein coding sequence
<400> 44
   gtgggattaa atagatttat gcgtgcgatg atggtagttt tcattactgc caactgcatt 60
   acgattaacc ccgacataat atttgcagcg acagatagcg aagattccag tctaaacaca 120
   gatgaatggg aagaagaaaa aacagaagag cagccaagcg aggtaaatac gggaccaaga 180
   tacgaaactg cacgtgaagt aagttcacgt gatattgagg aactagaaaa atcgaataaa 240
   gtgaaaaata cgaacaaagc agacctaata gcaatgttga aagcaaaagc agagaaaggt 300
   ggatcccgta cattagcagg tgaaacaggt caagaagcag caccacttga cggtgtatta 360
   acgaatccac caaatatatc aagtttaagt ccacgtcaat tattaggttt tccatgtgca 420
   gaagtttcag gtttaagtac agaacgtgtc cgtgagttag cagttgcatt agcacaaaaa 480
   aacgttaaat tatctacaga acagttacgt tgtttagccc atagattaag cgaaccacca 540
   gaagacttag atgcacttcc tttagacctt cttttattct taaatccaga tgcattttca 600
   ggaccacaag catgtacacg tttttttagt cgaattacaa aagccaatgt tgatttatta 660
   cctcgtgggg ctcctgaaag acaacgttta ttacctgctg cattagcatg ctggggtgtt 720
   cgcggtagct tattaagtga agccgatgtt cgtgctttag ggggtttagc atgtgattta 780
   cctggtcgtt tcgttgcaga atcagcagaa gtgttattac cgagattagt ttcatgccca 840
   ggacctttag atcaagatca acaagaggca gctagagcag ctcttcaagg aggaggccca 900
   ccatatggcc caccaagtac atggagtgtt tctacaatgg atgcgttaag aggtttatta 960
   ccggttttag gacaaccaat tattcgtagt attccacaag gcattgtagc agcatggcgt 1020
   caacgtagtt ctcgtgatcc gtcttggcga caaccagaac gtacaattct acgtccaaga 1080
   tttcgtagag aagtagaaaa aacggcgtgt cctagtggca aaaaagcacg tgaaattgat 1140
   gaaagtttaa ttttttataa aaaatgggaa ttagaagcat gtgtcgatgc agcattacta 1200
   gctacacaaa tggatcgtgt taatgctatt ccattcacat atgaacaatt agatgtttta 1260
   aagcataaat tagacgaatt atatccacaa ggttatccag aatcagttat tcaacattta 1320
   ggttacttat ttttaaaaat gagtccagaa gacatacgca aatggaatgt tacaagttta 1380
   gaaacattaa aagcgctttt agaagttaac aaaggtcatg aaatgagtcc acaagttgct 1440
   acgttaattg atagattcgt taaaggccgt ggtcaattag ataaagatac tttagataca 1500
   ttaacagcat tttatcctgg ctacttatgc agtttatcac cagaagaatt aagttccgtt 1560
   ccaccgagta gtatctgggc agttcgtccg caagatttag atacatgcga cccacgtcaa 1620
   ttagatgttt tatatccaaa agcaagatta gctttccaaa atatgaacgg tagtgaatat 1680
   ttcgtaaaaa ttcaatcctt tttaggtggt gcaccaactg aagatctaaa agcattaagc 1740
   caacaaaatg taagtatgga tttagctacg tttatgaaat tacgtacaga tgcagttcta 1800
   ccattaacag ttgcagaagt tcaaaaatta ttaggtccac acgtagaagg attaaaagca 1860
   gaagaacgtc accgtccagt tcgcgattgg attttacgtc aacgtcaaga tgatttagat 1920
   acattaggtt taggtttaca aggcgccatg acagaatata aattagttgt agttggtgca 1980
   gatggtgttg gtaaaagtgc attaacaatt caattaattc aataattaat taagagctc  2039
<210> 45
   <211> 674
   <212> PRT
   <213> Artificial Sequence
<220>
   <223> Fusion protein
<400> 45
   Val Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser Arg Thr Leu Ala Gly Glu Thr Gly Gln Glu
               100                 105                 110
   Ala Ala Pro Leu Asp Gly Val Leu Thr Asn Pro Pro Asn Ile Ser Ser
           115                 120                 125
   Leu Ser Pro Arg Gln Leu Leu Gly Phe Pro Cys Ala Glu Val Ser Gly
       130                 135                 140
   Leu Ser Thr Glu Arg Val Arg Glu Leu Ala Val Ala Leu Ala Gln Lys
   145                 150                 155                 160
   Asn Val Lys Leu Ser Thr Glu Gln Leu Arg Cys Leu Ala His Arg Leu
                   165                 170                 175
   Ser Glu Pro Pro Glu Asp Leu Asp Ala Leu Pro Leu Asp Leu Leu Leu
               180                 185                 190
   Phe Leu Asn Pro Asp Ala Phe Ser Gly Pro Gln Ala Cys Thr Arg Phe
           195                 200                 205
   Phe Ser Arg Ile Thr Lys Ala Asn Val Asp Leu Leu Pro Arg Gly Ala
       210                 215                 220
   Pro Glu Arg Gln Arg Leu Leu Pro Ala Ala Leu Ala Cys Trp Gly Val
   225                 230                 235                 240
   Arg Gly Ser Leu Leu Ser Glu Ala Asp Val Arg Ala Leu Gly Gly Leu
                   245                 250                 255
   Ala Cys Asp Leu Pro Gly Arg Phe Val Ala Glu Ser Ala Glu Val Leu
               260                 265                 270
   Leu Pro Arg Leu Val Ser Cys Pro Gly Pro Leu Asp Gln Asp Gln Gln
           275                 280                 285
   Glu Ala Ala Arg Ala Ala Leu Gln Gly Gly Gly Pro Pro Tyr Gly Pro
       290                 295                 300
   Pro Ser Thr Trp Ser Val Ser Thr Met Asp Ala Leu Arg Gly Leu Leu
   305                 310                 315                 320
   Pro Val Leu Gly Gln Pro Ile Ile Arg Ser Ile Pro Gln Gly Ile Val
                   325                 330                 335
   Ala Ala Trp Arg Gln Arg Ser Ser Arg Asp Pro Ser Trp Arg Gln Pro
               340                 345                 350
   Glu Arg Thr Ile Leu Arg Pro Arg Phe Arg Arg Glu Val Glu Lys Thr
           355                 360                 365
   Ala Cys Pro Ser Gly Lys Lys Ala Arg Glu Ile Asp Glu Ser Leu Ile
       370                 375                 380
   Phe Tyr Lys Lys Trp Glu Leu Glu Ala Cys Val Asp Ala Ala Leu Leu
   385                 390                 395                 400
   Ala Thr Gln Met Asp Arg Val Asn Ala Ile Pro Phe Thr Tyr Glu Gln
                   405                 410                 415
   Leu Asp Val Leu Lys His Lys Leu Asp Glu Leu Tyr Pro Gln Gly Tyr
               420                 425                 430
   Pro Glu Ser Val Ile Gln His Leu Gly Tyr Leu Phe Leu Lys Met Ser
           435                 440                 445
   Pro Glu Asp Ile Arg Lys Trp Asn Val Thr Ser Leu Glu Thr Leu Lys
       450                 455                 460
   Ala Leu Leu Glu Val Asn Lys Gly His Glu Met Ser Pro Gln Val Ala
   465                 470                 475                 480
   Thr Leu Ile Asp Arg Phe Val Lys Gly Arg Gly Gln Leu Asp Lys Asp
                   485                 490                 495
   Thr Leu Asp Thr Leu Thr Ala Phe Tyr Pro Gly Tyr Leu Cys Ser Leu
               500                 505                 510
   Ser Pro Glu Glu Leu Ser Ser Val Pro Pro Ser Ser Ile Trp Ala Val
           515                 520                 525
   Arg Pro Gln Asp Leu Asp Thr Cys Asp Pro Arg Gln Leu Asp Val Leu
       530                 535                 540
   Tyr Pro Lys Ala Arg Leu Ala Phe Gln Asn Met Asn Gly Ser Glu Tyr
   545                 550                 555                 560
   Phe Val Lys Ile Gln Ser Phe Leu Gly Gly Ala Pro Thr Glu Asp Leu
                   565                 570                 575
   Lys Ala Leu Ser Gln Gln Asn Val Ser Met Asp Leu Ala Thr Phe Met
               580                 585                 590
   Lys Leu Arg Thr Asp Ala Val Leu Pro Leu Thr Val Ala Glu Val Gln
           595                 600                 605
   Lys Leu Leu Gly Pro His Val Glu Gly Leu Lys Ala Glu Glu Arg His
       610                 615                 620
   Arg Pro Val Arg Asp Trp Ile Leu Arg Gln Arg Gln Asp Asp Leu Asp
   625                 630                 635                 640
   Thr Leu Gly Leu Gly Leu Gln Gly Ala Met Thr Glu Tyr Lys Leu Val
                   645                 650                 655
   Val Val Gly Ala Asp Gly Val Gly Lys Ser Ala Leu Thr Ile Gln Leu
               660                 665                 670
   Ile Gln
<210> 46
   <211> 533
   <212> DNA
   <213> Artificial Sequence
<220>
   <223> Expression construct
<400> 46
   aagcttggga agcagttggg gttaactgat taacaaatgt tagagaaaaa ttaattctcc 60
   aagtgatatt cttaaaataa ttcatgaata tttttcttta tattagctaa ttaagaagat 120
   aattaactgc taatccaatt tttaacggaa taaattagtg aaaatgaagg ccgaattttc 180
   cttgttctaa aaaggttgta ttagcgtatc acgaggaggg agtataagtg ggattaaata 240
   gatttatgcg tgcgatgatg gtagttttca ttactgccaa ctgcattacg attaaccccg 300
   acataatatt tgcagcgaca gatagcgaag attccagtct aaacacagat gaatgggaag 360
   aagaaaaaac agaagagcag ccaagcgagg taaatacggg accaagatac gaaactgcac 420
   gtgaagtaag ttcacgtgat attgaggaac tagaaaaatc gaataaagtg aaaaatacga 480
   acaaagcaga cctaatagca atgttgaaag caaaagcaga gaaaggtgga tcc        533
<210> 47
   <211> 102
   <212> PRT
   <213> Artificial Sequence
<220>
   <223> Fusion protein
<400> 47
   Val Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser
               100
<210> 48
   <211> 1590
   <212> DNA
   <213> Listeria monocytogenes
<400> 48
   atgaaaaaaa taatgctagt ttttattaca cttatattag ttagtctacc aattgcgcaa 60
   caaactgaag caaaggatgc atctgcattc aataaagaaa attcaatttc atccatggca 120
   ccaccagcat ctccgcctgc aagtcctaag acgccaatcg aaaagaaaca cgcggatgaa 180
   atcgataagt atatacaagg attggattac aataaaaaca atgtattagt ataccacgga 240
   gatgcagtga caaatgtgcc gccaagaaaa ggttacaaag atggaaatga atatattgtt 300
   gtggagaaaa agaagaaatc catcaatcaa aataatgcag acattcaagt tgtgaatgca 360
   atttcgagcc taacctatcc aggtgctctc gtaaaagcga attcggaatt agtagaaaat 420
   caaccagatg ttctccctgt aaaacgtgat tcattaacac tcagcattga tttgccaggt 480
   atgactaatc aagacaataa aatcgttgta aaaaatgcca ctaaatcaaa cgttaacaac 540
   gcagtaaata cattagtgga aagatggaat gaaaaatatg ctcaagctta tccaaatgta 600
   agtgcaaaaa ttgattatga tgacgaaatg gcttacagtg aatcacaatt aattgcgaaa 660
   tttggtacag catttaaagc tgtaaataat agcttgaatg taaacttcgg cgcaatcagt 720
   gaagggaaaa tgcaagaaga agtcattagt tttaaacaaa tttactataa cgtgaatgtt 780
   aatgaaccta caagaccttc cagatttttc ggcaaagctg ttactaaaga gcagttgcaa 840
   gcgcttggag tgaatgcaga aaatcctcct gcatatatct caagtgtggc gtatggccgt 900
   caagtttatt tgaaattatc aactaattcc catagtacta aagtaaaagc tgcttttgat 960
   gctgccgtaa gcggaaaatc tgtctcaggt gatgtagaac taacaaatat catcaaaaat 1020
   tcttccttca aagccgtaat ttacggaggt tccgcaaaag atgaagttca aatcatcgac 1080
   ggcaacctcg gagacttacg cgatattttg aaaaaaggcg ctacttttaa tcgagaaaca 1140
   ccaggagttc ccattgctta tacaacaaac ttcctaaaag acaatgaatt agctgttatt 1200
   aaaaacaact cagaatatat tgaaacaact tcaaaagctt atacagatgg aaaaattaac 1260
   atcgatcact ctggaggata cgttgctcaa ttcaacattt cttgggatga agtaaattat 1320
   gatcctgaag gtaacgaaat tgttcaacat aaaaactgga gcgaaaacaa taaaagcaag 1380
   ctagctcatt tcacatcgtc catctatttg cctggtaacg cgagaaatat taatgtttac 1440
   gctaaagaat gcactggttt agcttgggaa tggtggagaa cggtaattga tgaccggaac 1500
   ttaccacttg tgaaaaatag aaatatctcc atctggggca ccacgcttta tccgaaatat 1560
   agtaataaag tagataatcc aatcgaataa                                  1590
<210> 49
   <211> 1593
   <212> DNA
   <213> Artificial Sequence
<220>
   <223> Codon-optimized sequence
<400> 49
   atgaaaaaaa taatgctagt ctttattaca ttaattttag taagtctacc aattgcacaa 60
   caaaccgaag ctaaagatgc atcagcgttc aacaaagaaa attcaattag ttcaatggcc 120
   ccaccagctt ctccaccagc atctccaaaa acaccaattg aaaaaaaaca tgcagacgaa 180
   attgataaat atattcaagg tttagattac aataagaata acgttttagt ataccacggc 240
   gatgcagtaa caaatgtacc tccaagaaaa ggctataaag acggaaatga atatattgtt 300
   gttgaaaaaa aaaagaaatc tattaatcaa aacaatgccg acatccaagt agttaacgcg 360
   attagctcat tgacgtatcc aggcgccctt gtaaaagcta actctgaatt agtggaaaat 420
   caaccagacg tacttccagt caaacgtgat agtctaacct taagtattga tttaccagga 480
   atgacaaatc aagataacaa aattgttgtt aaaaatgcaa ctaaatccaa tgtaaataat 540
   gcagttaaca cattagtaga acgatggaac gaaaaatacg cacaggcata cccaaatgta 600
   tcagctaaaa ttgattacga cgacgaaatg gcctactcag aaagtcaatt aattgctaaa 660
   tttggtacag cattcaaagc agtcaataat agtttaaatg taaattttgg agcgatctct 720
   gaaggaaaga tgcaggaaga agtaatttca ttcaaacaaa tttattataa tgttaacgta 780
   aatgaaccaa cccgtccttc ccgtttcttt ggcaaagcag ttactaaaga acaattacaa 840
   gcactaggtg tgaatgcaga aaacccaccg gcatatattt caagcgtcgc ttacggacga 900
   caagtttact taaaattatc tacaaacagt catagtacaa aagtaaaagc agcattcgat 960
   gcagctgtgt caggaaaatc agttagtgga gatgtagaat taaccaatat tattaaaaat 1020
   tcgagtttta aagctgttat ttatggaggt tctgcaaaag atgaagtaca aattattgac 1080
   ggaaacttag gcgatttacg tgacatttta aaaaaaggcg caacatttaa tagagaaaca 1140
   ccaggggttc caattgctta tacaactaat tttcttaaag ataatgaact tgcagtaatt 1200
   aaaaacaatt cagaatacat tgaaacaact tcgaaagcat atacagacgg aaaaattaat 1260
   attgatcact caggagggta cgttgcacaa tttaatatta gttgggatga agtaaactat 1320
   gatccagaag gcaatgaaat tgtacaacat aaaaattggt ctgaaaataa caaatctaaa 1380
   ctagcacact ttaccagttc tatctattta ccaggaaatg ctcgcaatat taatgtttac 1440
   gcaaaagaat gtaccggatt agcatgggaa tggtggcgca cagttattga cgaccgcaat 1500
   cttcctctag taaaaaacag aaacatcagc atttggggaa caacgcttta tccgaaatac 1560
   agtaataaag ttgataatcc aattgaagga tcc                               1593
<210> 50
   <211> 1593
   <212> DNA
   <213> Artificial Sequence
<220>
   <223> Codon-optimized sequence
<400> 50
   atgaaaaaaa taatgctagt ctttattaca ttaattttag taagtctacc aattgcacaa 60
   caaaccgaag ctaaagatgc atcagcgttc aacaaagaaa attcaattag ttcaatggcc 120
   ccaccagctt ctccaccagc atctccaaaa acaccaattg aaaaaaaaca tgcagacgaa 180
   attgataaat atattcaagg tttagattac aataagaata acgttttagt ataccacggc 240
   gatgcagtaa caaatgtacc tccaagaaaa ggctataaag acggaaatga atatattgtt 300
   gttgaaaaaa aaaagaaatc tattaatcaa aacaatgccg acatccaagt agttaacgcg 360
   attagctcat tgacgtatcc aggcgccctt gtaaaagcta actctgaatt agtggaaaat 420
   caaccagacg tacttccagt caaacgtgat agtctaacct taagtattga tttaccagga 480
   atgacaaatc aagataacaa aattgttgtt aaaaatgcaa ctaaatccaa tgtaaataat 540
   gcagttaaca cattagtaga acgatggaac gaaaaatacg cacaggcata cccaaatgta 600
   tcagctaaaa ttgattacga cgacgaaatg gcctactcag aaagtcaatt aattgctaaa 660
   tttggtacag cattcaaagc agtcaataat agtttaaatg taaattttgg agcgatctct 720
   gaaggaaaga tgcaggaaga agtaatttca ttcaaacaaa tttattataa tgttaacgta 780
   aatgaaccaa cccgtccttc ccgtttcttt ggcaaagcag ttactaaaga acaattacaa 840
   gcactaggtg tgaatgcaga aaacccaccg gcatatattt caagcgtcgc ttacggacga 900
   caagtttact taaaattatc tacaaacagt catagtacaa aagtaaaagc agcattcgat 960
   gcagctgtgt caggaaaatc agttagtgga gatgtagaat taaccaatat tattaaaaat 1020
   tcgagtttta aagctgttat ttatggaggt tctgcaaaag atgaagtaca aattattgac 1080
   ggaaacttag gcgatttacg tgacatttta aaaaaaggcg caacatttaa tagagaaaca 1140
   ccaggggttc caattgctta tacaactaat tttcttaaag ataatgaact tgcagtaatt 1200
   aaaaacaatt cagaatacat tgaaacaact tcgaaagcat atacagacgg aaaaattaat 1260
   attgatcact caggagggta cgttgcacaa tttaatatta gttgggatga agtaaactat 1320
   gatccagaag gcaatgaaat tgtacaacat aaaaattggt ctgaaaataa caaatctaaa 1380
   ctagcacact ttaccagttc tatctattta ccaggaaatg ctcgcaatat taatgtttac 1440
   gcaaaagaat gtaccggatt agcatgggaa ttttttcgca cagttattga cgaccgcaat 1500
   cttcctctag taaaaaacag aaacatcagc atttggggaa caacgcttta tccgaaatac 1560
   agtaataaag ttgataatcc aattgaagga tcc                              1593
<210> 51
   <211> 177
   <212> DNA
   <213> Listeria monocytogenes
<400> 51
   atgaaaaaaa taatgctagt ttttattaca cttatattag ttagtctacc aattgcgcaa 60
   caaactgaag caaaggatgc atctgcattc aataaagaaa attcaatttc atccatggca 120
   ccaccagcat ctccgcctgc aagtcctaag acgccaatcg aaaagaaaca cgcggat    177
<210> 52
   <211> 177
   <212> DNA
   <213> Artificial Sequence
<220>
   <223> Codon-optimized sequence
<400> 52
   atgaaaaaaa ttatgttagt ttttattaca ttaattttag ttagtttacc aattgcacaa 60
   caaacagaag caaaagatgc aagtgcattt aataaagaaa atagtattag tagtatggca 120
   ccaccagcaa gtccaccagc aagtccaaaa acaccaattg aaaaaaaaca tgcagat    177
<210> 53
   <211> 59
   <212> PRT
   <213> Listeria monocytogenes
<400> 53
   Met Lys Lys Ile Met Leu Val Phe Ile Thr Leu Ile Leu Val Ser Leu
    1               5                  10                  15
   Pro Ile Ala Gln Gln Thr Glu Ala Lys Asp Ala Ser Ala Phe Asn Lys
               20                  25                  30
   Glu Asn Ser Ile Ser Ser Met Ala Pro Pro Ala Ser Pro Pro Ala Ser
           35                  40                  45
   Pro Lys Thr Pro Ile Glu Lys Lys His Ala Asp
       50                  55
<210> 54
   <211> 1830
   <212> DNA
   <213> Artificial Sequence
<220>
   <223> Fusion protein coding sequence
<400> 54
   atgaaaaaaa ttatgttagt ttttattaca ttaattttag ttagtttacc aattgcacaa 60
   caaacagaag caaaagatgc aagtgcattt aataaagaaa atagtattag tagtatggca 120
   ccaccagcaa gtccaccagc aagtccaaaa acaccaattg aaaaaaaaca tgcagatgga 180
   tcccgtacat tagcaggtga aacaggtcaa gaagcagcac cacttgacgg tgtattaacg 240
   aatccaccaa atatatcaag tttaagtcca cgtcaattat taggttttcc atgtgcagaa 300
   gtttcaggtt taagtacaga acgtgtccgt gagttagcag ttgcattagc acaaaaaaac 360
   gttaaattat ctacagaaca gttacgttgt ttagcccata gattaagcga accaccagaa 420
   gacttagatg cacttccttt agaccttctt ttattcttaa atccagatgc attttcagga 480
   ccacaagcat gtacacgttt ttttagtcga attacaaaag ccaatgttga tttattacct 540
   cgtggggctc ctgaaagaca acgtttatta cctgctgcat tagcatgctg gggtgttcgc 600
   ggtagcttat taagtgaagc cgatgttcgt gctttagggg gtttagcatg tgatttacct 660
   ggtcgtttcg ttgcagaatc agcagaagtg ttattaccga gattagtttc atgcccagga 720
   cctttagatc aagatcaaca agaggcagct agagcagctc ttcaaggagg aggcccacca 780
   tatggcccac caagtacatg gagtgtttct acaatggatg cgttaagagg tttattaccg 840
   gttttaggac aaccaattat tcgtagtatt ccacaaggca ttgtagcagc atggcgtcaa 900
   cgtagttctc gtgatccgtc ttggcgacaa ccagaacgta caattctacg tccaagattt 960
   cgtagagaag tagaaaaaac ggcgtgtcct agtggcaaaa aagcacgtga aattgatgaa 1020
   agtttaattt tttataaaaa atgggaatta gaagcatgtg tcgatgcagc attactagct 1080
   acacaaatgg atcgtgttaa tgctattcca ttcacatatg aacaattaga tgttttaaag 1140
   cataaattag acgaattata tccacaaggt tatccagaat cagttattca acatttaggt 1200
   tacttatttt taaaaatgag tccagaagac atacgcaaat ggaatgttac aagtttagaa 1260
   acattaaaag cgcttttaga agttaacaaa ggtcatgaaa tgagtccaca agttgctacg 1320
   ttaattgata gattcgttaa aggccgtggt caattagata aagatacttt agatacatta 1380
   acagcatttt atcctggcta cttatgcagt ttatcaccag aagaattaag ttccgttcca 1440
   ccgagtagta tctgggcagt tcgtccgcaa gatttagata catgcgaccc acgtcaatta 1500
   gatgttttat atccaaaagc aagattagct ttccaaaata tgaacggtag tgaatatttc 1560
   gtaaaaattc aatccttttt aggtggtgca ccaactgaag atctaaaagc attaagccaa 1620
   caaaatgtaa gtatggattt agctacgttt atgaaattac gtacagatgc agttctacca 1680
   ttaacagttg cagaagttca aaaattatta ggtccacacg tagaaggatt aaaagcagaa 1740
   gaacgtcacc gtccagttcg cgattggatt ttacgtcaac gtcaagatga tttagataca 1800
   ttaggtttag gtttacaagg ctaagagctc                                  1830
<210> 55
   <211> 607
   <212> PRT
   <213> Artificial Sequence
<220>
   <223> Fusion protein
<400> 55
   Met Lys Lys Ile Met Leu Val Phe Ile Thr Leu Ile Leu Val Ser Leu
    1               5                  10                  15
   Pro Ile Ala Gln Gln Thr Glu Ala Lys Asp Ala Ser Ala Phe Asn Lys
               20                  25                  30
   Glu Asn Ser Ile Ser Ser Met Ala Pro Pro Ala Ser Pro Pro Ala Ser
           35                  40                  45
   Pro Lys Thr Pro Ile Glu Lys Lys His Ala Asp Gly Ser Arg Thr Leu
       50                  55                  60
   Ala Gly Glu Thr Gly Gln Glu Ala Ala Pro Leu Asp Gly Val Leu Thr
   65                  70                  75                  80
   Asn Pro Pro Asn Ile Ser Ser Leu Ser Pro Arg Gln Leu Leu Gly Phe
                   85                  90                  95
   Pro Cys Ala Glu Val Ser Gly Leu Ser Thr Glu Arg Val Arg Glu Leu
               100                 105                 110
   Ala Val Ala Leu Ala Gln Lys Asn Val Lys Leu Ser Thr Glu Gln Leu
           115                 120                 125
   Arg Cys Leu Ala His Arg Leu Ser Glu Pro Pro Glu Asp Leu Asp Ala
       130                 135                 140
   Leu Pro Leu Asp Leu Leu Leu Phe Leu Asn Pro Asp Ala Phe Ser Gly
   145                 150                 155                 160
   Pro Gln Ala Cys Thr Arg Phe Phe Ser Arg Ile Thr Lys Ala Asn Val
                   165                 170                 175
   Asp Leu Leu Pro Arg Gly Ala Pro Glu Arg Gln Arg Leu Leu Pro Ala
               180                 185                 190
   Ala Leu Ala Cys Trp Gly Val Arg Gly Ser Leu Leu Ser Glu Ala Asp
           195                 200                 205
   Val Arg Ala Leu Gly Gly Leu Ala Cys Asp Leu Pro Gly Arg Phe Val
       210                 215                 220
   Ala Glu Ser Ala Glu Val Leu Leu Pro Arg Leu Val Ser Cys Pro Gly
   225                 230                 235                 240
   Pro Leu Asp Gln Asp Gln Gln Glu Ala Ala Arg Ala Ala Leu Gln Gly
                   245                 250                 255
   Gly Gly Pro Pro Tyr Gly Pro Pro Ser Thr Trp Ser Val Ser Thr Met
               260                 265                 270
   Asp Ala Leu Arg Gly Leu Leu Pro Val Leu Gly Gln Pro Ile Ile Arg
           275                 280                 285
   Ser Ile Pro Gln Gly Ile Val Ala Ala Trp Arg Gln Arg Ser Ser Arg
       290                 295                 300
   Asp Pro Ser Trp Arg Gln Pro Glu Arg Thr Ile Leu Arg Pro Arg Phe
   305                 310                 315                 320
   Arg Arg Glu Val Glu Lys Thr Ala Cys Pro Ser Gly Lys Lys Ala Arg
                   325                 330                 335
   Glu Ile Asp Glu Ser Leu Ile Phe Tyr Lys Lys Trp Glu Leu Glu Ala
               340                 345                 350
   Cys Val Asp Ala Ala Leu Leu Ala Thr Gln Met Asp Arg Val Asn Ala
           355                 360                 365
   Ile Pro Phe Thr Tyr Glu Gln Leu Asp Val Leu Lys His Lys Leu Asp
       370                 375                 380
   Glu Leu Tyr Pro Gln Gly Tyr Pro Glu Ser Val Ile Gln His Leu Gly
   385                 390                 395                 400
   Tyr Leu Phe Leu Lys Met Ser Pro Glu Asp Ile Arg Lys Trp Asn Val
                   405                 410                 415
   Thr Ser Leu Glu Thr Leu Lys Ala Leu Leu Glu Val Asn Lys Gly His
               420                 425                 430
   Glu Met Ser Pro Gln Val Ala Thr Leu Ile Asp Arg Phe Val Lys Gly
           435                 440                 445
   Arg Gly Gln Leu Asp Lys Asp Thr Leu Asp Thr Leu Thr Ala Phe Tyr
       450                 455                 460
   Pro Gly Tyr Leu Cys Ser Leu Ser Pro Glu Glu Leu Ser Ser Val Pro
   465                 470                 475                 480
   Pro Ser Ser Ile Trp Ala Val Arg Pro Gln Asp Leu Asp Thr Cys Asp
                   485                 490                 495
   Pro Arg Gln Leu Asp Val Leu Tyr Pro Lys Ala Arg Leu Ala Phe Gln
               500                 505                 510
   Asn Met Asn Gly Ser Glu Tyr Phe Val Lys Ile Gln Ser Phe Leu Gly
           515                 520                 525
   Gly Ala Pro Thr Glu Asp Leu Lys Ala Leu Ser Gln Gln Asn Val Ser
       530                 535                 540
   Met Asp Leu Ala Thr Phe Met Lys Leu Arg Thr Asp Ala Val Leu Pro
   545                 550                 555                 560
   Leu Thr Val Ala Glu Val Gln Lys Leu Leu Gly Pro His Val Glu Gly
                   565                 570                 575
   Leu Lys Ala Glu Glu Arg His Arg Pro Val Arg Asp Trp Ile Leu Arg
               580                 585                 590
   Gln Arg Gln Asp Asp Leu Asp Thr Leu Gly Leu Gly Leu Gln Gly
           595                 600                 605
<210> 56
   <211> 1830
   <212> DNA
   <213> Artificial Sequence
<220>
   <223> Fusion protein coding sequence
<400> 56
   atgaaaaaaa taatgctagt ttttattaca cttatattag ttagtctacc aattgcgcaa 60
   caaactgaag caaaggatgc atctgcattc aataaagaaa attcaatttc atccatggca 120
   ccaccagcat ctccgcctgc aagtcctaag acgccaatcg aaaagaaaca cgcggatgga 180
   tcccgtacat tagcaggtga aacaggtcaa gaagcagcac cacttgacgg tgtattaacg 240
   aatccaccaa atatatcaag tttaagtcca cgtcaattat taggttttcc atgtgcagaa 300
   gtttcaggtt taagtacaga acgtgtccgt gagttagcag ttgcattagc acaaaaaaac 360
   gttaaattat ctacagaaca gttacgttgt ttagcccata gattaagcga accaccagaa 420
   gacttagatg cacttccttt agaccttctt ttattcttaa atccagatgc attttcagga 480
   ccacaagcat gtacacgttt ttttagtcga attacaaaag ccaatgttga tttattacct 540
   cgtggggctc ctgaaagaca acgtttatta cctgctgcat tagcatgctg gggtgttcgc 600
   ggtagcttat taagtgaagc cgatgttcgt gctttagggg gtttagcatg tgatttacct 660
   ggtcgtttcg ttgcagaatc agcagaagtg ttattaccga gattagtttc atgcccagga 720
   cctttagatc aagatcaaca agaggcagct agagcagctc ttcaaggagg aggcccacca 780
   tatggcccac caagtacatg gagtgtttct acaatggatg cgttaagagg tttattaccg 840
   gttttaggac aaccaattat tcgtagtatt ccacaaggca ttgtagcagc atggcgtcaa 900
   cgtagttctc gtgatccgtc ttggcgacaa ccagaacgta caattctacg tccaagattt 960
   cgtagagaag tagaaaaaac ggcgtgtcct agtggcaaaa aagcacgtga aattgatgaa 1020
   agtttaattt tttataaaaa atgggaatta gaagcatgtg tcgatgcagc attactagct 1080
   acacaaatgg atcgtgttaa tgctattcca ttcacatatg aacaattaga tgttttaaag 1140
   cataaattag acgaattata tccacaaggt tatccagaat cagttattca acatttaggt 1200
   tacttatttt taaaaatgag tccagaagac atacgcaaat ggaatgttac aagtttagaa 1260
   acattaaaag cgcttttaga agttaacaaa ggtcatgaaa tgagtccaca agttgctacg 1320
   ttaattgata gattcgttaa aggccgtggt caattagata aagatacttt agatacatta 1380
   acagcatttt atcctggcta cttatgcagt ttatcaccag aagaattaag ttccgttcca 1440
   ccgagtagta tctgggcagt tcgtccgcaa gatttagata catgcgaccc acgtcaatta 1500
   gatgttttat atccaaaagc aagattagct ttccaaaata tgaacggtag tgaatatttc 1560
   gtaaaaattc aatccttttt aggtggtgca ccaactgaag atctaaaagc attaagccaa 1620
   caaaatgtaa gtatggattt agctacgttt atgaaattac gtacagatgc agttctacca 1680
   ttaacagttg cagaagttca aaaattatta ggtccacacg tagaaggatt aaaagcagaa 1740
   gaacgtcacc gtccagttcg cgattggatt ttacgtcaac gtcaagatga tttagataca 1800
   ttaggtttag gtttacaagg ctaagagctc 1830
<210> 57
   <211> 237
   <212> DNA
   <213> Listeria monocytogenes
<400> 57
   ggtacctcct ttgattagta tattcctatc ttaaagttac ttttatgtgg aggcattaac 60
   atttgttaat gacgtcaaaa ggatagcaag actagaataa agctataaag caagcatata 120
   atattgcgtt tcatctttag aagcgaattt cgccaatatt ataattatca aaagagaggg 180
   gtggcaaacg gtatttggca ttattaggtt aaaaaatgta gaaggagagt gaaaccc 237
<210> 58
   <211> 99
   <212> DNA
   <213> Artificial Sequence
<220>
   <223> Codon-optimized sequence
<400> 58
   atgaaaaaac gtaaagtttt aattccatta atggcattaa gtacaatttt agttagtagt 60
   acaggtaatt tagaagttat tcaagcagaa gttggatcc 99
<210> 59
   <211> 33
   <212> PRT
   <213> Bacillus anthracis
<400> 59
   Met Lys Lys Arg Lys Val Leu Ile Pro Leu Met Ala Leu Ser Thr Ile
    1               5                  10                  15
   Leu Val Ser Ser Thr Gly Asn Leu Glu Val Ile Gln Ala Glu Val Gly
               20                  25                  30
   Ser
<210> 60
   <211> 336
   <212> DNA
   <213> Artificial Sequence
<220>
   <223> Expression construct
<400> 60
   ggtacctcct ttgattagta tattcctatc ttaaagttac ttttatgtgg aggcattaac 60
   atttgttaat gacgtcaaaa ggatagcaag actagaataa agctataaag caagcatata 120
   atattgcgtt tcatctttag aagcgaattt cgccaatatt ataattatca aaagagaggg 180
   gtggcaaacg gtatttggca ttattaggtt aaaaaatgta gaaggagagt gaaacccatg 240
   aaaaaacgta aagttttaat tccattaatg gcattaagta caattttagt tagtagtaca 300
   ggtaatttag aagttattca agcagaagtt ggatcc                           336
<210> 61
   <211> 70
   <212> PRT
   <213> Listeria monocytogenes
<400> 61
   Met Asn Met Lys Lys Ala Thr Ile Ala Ala Thr Ala Gly Ile Ala Val
    1               5                  10                  15
   Thr Ala Phe Ala Ala Pro Thr Ile Ala Ser Ala Ser Thr Val Val Val
               20                  25                  30
   Glu Ala Gly Asp Thr Leu Trp Gly Ile Ala Gln Ser Lys Gly Thr Thr
           35                  40                  45
   Val Asp Ala Ile Lys Lys Ala Asn Asn Leu Thr Thr Asp Lys Ile Val
       50                  55                  60
   Pro Gly Gln Lys Leu Gln
   65                  70
<210> 62
   <211> 447
   <212> DNA
   <213> Artificial Sequence
<220>
   <223> Expression construct
<400> 62
   ggtacctcct ttgattagta tattcctatc ttaaagttac ttttatgtgg aggcattaac 60
   atttgttaat gacgtcaaaa ggatagcaag actagaataa agctataaag caagcatata 120
   atattgcgtt tcatctttag aagcgaattt cgccaatatt ataattatca aaagagaggg 180
   gtggcaaacg gtatttggca ttattaggtt aaaaaatgta gaaggagagt gaaacccatg 240
   aatatgaaaa aagctacgat tgcagctaca gccggcattg ccgtaacagc ttttgcagca 300
   ccaactattg cctcagcctc tacagttgtt gtcgaagcag gagacacatt atggggaatc 360
   gcacaatcaa aaggtacaac ggttgatgct attaaaaaag cgaataattt aacaacagat 420
   aaaatcgtgc caggtcaaaa actgcag                                     447
<210> 63
   <211> 28
   <212> DNA
   <213> Artificial Sequence
<220>
   <223> Primer
<400> 63
   cgcctgcagg taaataatga ggttgctg 28
<210> 64
   <211> 29
   <212> DNA
   <213> Artificial Sequence
<220>
   <223> Primer
<400> 64
   cgcggatcct taattatacg cgaccgaag 29
<210> 65
   <211> 1683
   <212> DNA
   <213> Artificial Sequence
<220>
   <223> Expression construct
<400> 65
   ggtacctcct ttgattagta tattcctatc ttaaagttac ttttatgtgg aggcattaac 60
   atttgttaat gacgtcaaaa ggatagcaag actagaataa agctataaag caagcatata 120
   atattgcgtt tcatctttag aagcgaattt cgccaatatt ataattatca aaagagaggg 180
   gtggcaaacg gtatttggca ttattaggtt aaaaaatgta gaaggagagt gaaacccatg 240
   aatatgaaaa aagctacgat tgcagctaca gccggcattg ccgtaacagc ttttgcagca 300
   ccaactattg cctcagcctc tacagttgtt gtcgaagcag gagacacatt atggggaatc 360
   gcacaatcaa aaggtacaac ggttgatgct attaaaaaag cgaataattt aacaacagat 420
   aaaatcgtgc caggtcaaaa actgcaggta aataatgagg ttgctgctgc tgaaaaaaca 480
   gagaaatctg ttagcgcaac ttggttaaac gtccgtactg gcgctggtgt tgataacagt 540
   attattacgt ccatcaaagg tggaacaaaa gtaactgttg aaacaaccga atctaacggc 600
   tggcacaaaa ttacttacaa cgatggaaaa actggtttcg ttaacggtaa atacttaact 660
   gacaaagcag taagcactcc agttgcacca acacaagaag tgaaaaaaga aactactact 720
   caacaagctg cacctgttgc agaaacaaaa actgaagtaa aacaaactac acaagcaact 780
   acacctgcgc ctaaagtagc agaaacgaaa gaaactccag taatagatca aaatgctact 840
   acacacgctg tcaaaagcgg tgacactatt tgggctttat ccgtaaaata cggtgtttct 900
   gttcaagaca ttatgtcatg gaataattta tcttcttctt ctatttatgt aggtcaaaag 960
   cttgctatta aacaaactgc taacacagct actccaaaag cagaagtgaa aacggaagct 1020
   ccagcagctg aaaaacaagc agctccagta gttaaagaaa atactaacac aaatactgct 1080
   actacagaga aaaaagaaac agcaacgcaa caacaaacag cacctaaagc accaacagaa 1140
   gctgcaaaac cagctcctgc accatctaca aacacaaatg ctaataaaac gaatacaaat 1200
   acaaatacaa acaatactaa tacaccatct aaaaatacta atacaaactc aaatactaat 1260
   acgaatacaa actcaaatac gaatgctaat caaggttctt ccaacaataa cagcaattca 1320
   agtgcaagtg ctattattgc tgaagctcaa aaacaccttg gaaaagctta ttcatggggt 1380
   ggtaacggac caactacatt tgattgctct ggttacacta aatatgtatt tgctaaagcg 1440
   ggtatctccc ttccacgtac atctggcgca caatatgcta gcactacaag aatttctgaa 1500
   tctcaagcaa aacctggtga tttagtattc ttcgactatg gtagcggaat ttctcacatt 1560
   ggtatttatg ttggtaatgg tcaaatgatt aacgcgcaag acaatggcgt taaatacgat 1620
   aacatccacg gctctggctg gggtaaatat ctagttggct tcggtcgcgt ataataagga 1680
   tcc                                                               1683
<210> 66
   <211> 29
   <212> DNA
   <213> Artificial Sequence
<220>
   <223> Primer
<400> 66
   aaactgcagg cattgccaac tgcacgtcc 29
<210> 67
   <211> 40
   <212> DNA
   <213> Artificial Sequence
<220>
   <223> Primer
<400> 67
   aaactgcaga gctaatgtac tggctaataa taatgctaac 40
<210> 68
   <211> 32
   <212> DNA
   <213> Artificial Sequence
<220>
   <223> Primer
<400> 68
   cgcctgcagc gtacattagc aggtgaaaca gg 32
<210> 69
   <211> 36
   <212> DNA
   <213> Artificial Sequence
<220>
   <223> Primer
<400> 69
   cgcctgcagg ccttgtaaac ctaaacctaa tgtatc 36
<210> 70
   <211> 99
   <212> DNA
   <213> Homo sapiens
<400> 70
   gcattgccaa ctgcacgtcc attactaggt agttgcggta caccagcact aggttcttta 60
   ttatttttgt tattttctct aggttgggtt caaccaagt 99
<210> 71
   <211> 126
   <212> DNA
   <213> Homo sapiens
<400> 71
   ggtattccga atggatattt agtgttagat ttatctgttc aagaagcatt aagtggtaca 60
   ccgtgtttat taggtccagg tccagtttta acagtgttag cattattatt agccagtaca 120
   ttagct 126
<210> 72
   <211> 1878
   <212> DNA
   <213> Artificial Sequence
<220>
   <223> Codon-optimized sequence
<400> 72
   ggatccgcat tgccaactgc acgtccatta ctaggtagtt gcggtacacc agcactaggt 60
   tctttattat ttttgttatt ttctctaggt tgggttcaac caagtcgtac attagcaggt 120
   gaaacaggtc aagaagcagc accacttgac ggtgtattaa cgaatccacc aaatatatca 180
   agtttaagtc cacgtcaatt attaggtttt ccatgtgcag aagtttcagg tttaagtaca 240
   gaacgtgtcc gtgagttagc agttgcatta gcacaaaaaa acgttaaatt atctacagaa 300
   cagttacgtt gtttagccca tagattaagc gaaccaccag aagacttaga tgcacttcct 360
   ttagaccttc ttttattctt aaatccagat gcattttcag gaccacaagc atgtacacgt 420
   ttttttagtc gaattacaaa agccaatgtt gatttattac ctcgtggggc tcctgaaaga 480
   caacgtttat tacctgctgc attagcatgc tggggtgttc gcggtagctt attaagtgaa 540
   gccgatgttc gtgctttagg gggtttagca tgtgatttac ctggtcgttt cgttgcagaa 600
   tcagcagaag tgttattacc gagattagtt tcatgcccag gacctttaga tcaagatcaa 660
   caagaggcag ctagagcagc tcttcaagga ggaggcccac catatggccc accaagtaca 720
   tggagtgttt ctacaatgga tgcgttaaga ggtttattac cggttttagg acaaccaatt 780
   attcgtagta ttccacaagg cattgtagca gcatggcgtc aacgtagttc tcgtgatccg 840
   tcttggcgac aaccagaacg tacaattcta cgtccaagat ttcgtagaga agtagaaaaa 900
   acggcgtgtc ctagtggcaa aaaagcacgt gaaattgatg aaagtttaat tttttataaa 960
   aaatgggaat tagaagcatg tgtcgatgca gcattactag ctacacaaat ggatcgtgtt 1020
   aatgctattc cattcacata tgaacaatta gatgttttaa agcataaatt agacgaatta 1080
   tatccacaag gttatccaga atcagttatt caacatttag gttacttatt tttaaaaatg 1140
   agtccagaag acatacgcaa atggaatgtt acaagtttag aaacattaaa agcgctttta 1200
   gaagttaaca aaggtcatga aatgagtcca caagttgcta cgttaattga tagattcgtt 1260
   aaaggccgtg gtcaattaga taaagatact ttagatacat taacagcatt ttatcctggc 1320
   tacttatgca gtttatcacc agaagaatta agttccgttc caccgagtag tatctgggca 1380
   gttcgtccgc aagatttaga tacatgcgac ccacgtcaat tagatgtttt atatccaaaa 1440
   gcaagattag ctttccaaaa tatgaacggt agtgaatatt tcgtaaaaat tcaatccttt 1500
   ttaggtggtg caccaactga agatctaaaa gcattaagcc aacaaaatgt aagtatggat 1560
   ttagctacgt ttatgaaatt acgtacagat gcagttctac cattaacagt tgcagaagtt 1620
   caaaaattat taggtccaca cgtagaagga ttaaaagcag aagaacgtca ccgtccagtt 1680
   cgcgattgga ttttacgtca acgtcaagat gatttagata cattaggttt aggtttacaa 1740
   ggcggtattc cgaatggata tttagtgtta gatttatctg ttcaagaagc attaagtggt 1800
   acaccgtgtt tattaggtcc aggtccagtt ttaacagtgt tagcattatt attagccagt 1860
   acattagctt aagagctc                                                1878
<210> 73
   <211> 622
   <212> PRT
   <213> Homo sapiens
<400> 73
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Thr Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Val Ala Thr Leu Ile Asp
                   405                 410                 415
   Arg Phe Val Lys Gly Arg Gly Gln Leu Asp Lys Asp Thr Leu Asp Thr
               420                 425                 430
   Leu Thr Ala Phe Tyr Pro Gly Tyr Leu Cys Ser Leu Ser Pro Glu Glu
           435                 440                 445
   Leu Ser Ser Val Pro Pro Ser Ser Ile Trp Ala Val Arg Pro Gln Asp
       450                 455                 460
   Leu Asp Thr Cys Asp Pro Arg Gln Leu Asp Val Leu Tyr Pro Lys Ala
   465                 470                 475                 480
   Arg Leu Ala Phe Gln Asn Met Asn Gly Ser Glu Tyr Phe Val Lys Ile
                   485                 490                 495
   Gln Ser Phe Leu Gly Gly Ala Pro Thr Glu Asp Leu Lys Ala Leu Ser
               500                 505                 510
   Gln Gln Asn Val Ser Met Asp Leu Ala Thr Phe Met Lys Leu Arg Thr
           515                 520                 525
   Asp Ala Val Leu Pro Leu Thr Val Ala Glu Val Gln Lys Leu Leu Gly
       530                 535                 540
   Pro His Val Glu Gly Leu Lys Ala Glu Glu Arg His Arg Pro Val Arg
   545                 550                 555                 560
   Asp Trp Ile Leu Arg Gln Arg Gln Asp Asp Leu Asp Thr Leu Gly Leu
                   565                 570                 575
   Gly Leu Gln Gly Gly Ile Pro Asn Gly Tyr Leu Val Leu Asp Leu Ser
               580                 585                 590
   Val Gln Glu Ala Leu Ser Gly Thr Pro Cys Leu Leu Gly Pro Gly Pro
           595                 600                 605
   Val Leu Thr Val Leu Ala Leu Leu Leu Ala Ser Thr Leu Ala
       610                 615                 620
<210> 74
   <211> 1653
   <212> DNA
   <213> Artificial Sequence
<220>
   <223> Coding sequence
<400> 74
<210> 75
   <211> 546
   <212> PRT
   <213> Homo sapiens
<400> 75
   Arg Thr Leu Ala Gly Glu Thr Gly Gln Glu Ala Ala Pro Leu Asp Gly
    1               5                  10                  15
   Val Leu Thr Asn Pro Pro Asn Ile Ser Ser Leu Ser Pro Arg Gln Leu
               20                  25                  30
   Leu Gly Phe Pro Cys Ala Glu Val Ser Gly Leu Ser Thr Glu Arg Val
           35                  40                  45
   Arg Glu Leu Ala Val Ala Leu Ala Gln Lys Asn Val Lys Leu Ser Thr
       50                  55                  60
   Glu Gln Leu Arg Cys Leu Ala His Arg Leu Ser Glu Pro Pro Glu Asp
   65                  70                  75                  80
   Leu Asp Ala Leu Pro Leu Asp Leu Leu Leu Phe Leu Asn Pro Asp Ala
                   85                  90                  95
   Phe Ser Gly Pro Gln Ala Cys Thr Arg Phe Phe Ser Arg Ile Thr Lys
               100                 105                 110
   Ala Asn Val Asp Leu Leu Pro Arg Gly Ala Pro Glu Arg Gln Arg Leu
           115                 120                 125
   Leu Pro Ala Ala Leu Ala Cys Trp Gly Val Arg Gly Ser Leu Leu Ser
       130                 135                 140
   Glu Ala Asp Val Arg Ala Leu Gly Gly Leu Ala Cys Asp Leu Pro Gly
   145                 150                 155                 160
   Arg Phe Val Ala Glu Ser Ala Glu Val Leu Leu Pro Arg Leu Val Ser
                   165                 170                 175
   Cys Pro Gly Pro Leu Asp Gln Asp Gln Gln Glu Ala Ala Arg Ala Ala
               180                 185                 190
   Leu Gln Gly Gly Gly Pro Pro Tyr Gly Pro Pro Ser Thr Trp Ser Val
           195                 200                 205
   Ser Thr Met Asp Ala Leu Arg Gly Leu Leu Pro Val Leu Gly Gln Pro
       210                 215                 220
   Ile Ile Arg Ser Ile Pro Gln Gly Ile Val Ala Ala Trp Arg Gln Arg
   225                 230                 235                 240
   Ser Ser Arg Asp Pro Ser Trp Arg Gln Pro Glu Arg Thr Ile Leu Arg
                   245                 250                 255
   Pro Arg Phe Arg Arg Glu Val Glu Lys Thr Ala Cys Pro Ser Gly Lys
               260                 265                 270
   Lys Ala Arg Glu Ile Asp Glu Ser Leu Ile Phe Tyr Lys Lys Trp Glu
           275                 280                 285
   Leu Glu Ala Cys Val Asp Ala Ala Leu Leu Ala Thr Gln Met Asp Arg
       290                 295                 300
   Val Asn Ala Ile Pro Phe Thr Tyr Glu Gln Leu Asp Val Leu Lys His
   305                 310                 315                 320
   Lys Leu Asp Glu Leu Tyr Pro Gln Gly Tyr Pro Glu Ser Val Ile Gln
                   325                 330                 335
   His Leu Gly Tyr Leu Phe Leu Lys Met Ser Pro Glu Asp Ile Arg Lys
               340                 345                 350
   Trp Asn Val Thr Ser Leu Glu Thr Leu Lys Ala Leu Leu Glu Val Asn
           355                 360                 365
   Lys Gly His Glu Met Ser Pro Gln Val Ala Thr Leu Ile Asp Arg Phe
       370                 375                 380
   Val Lys Gly Arg Gly Gln Leu Asp Lys Asp Thr Leu Asp Thr Leu Thr
   385                 390                 395                 400
   Ala Phe Tyr Pro Gly Tyr Leu Cys Ser Leu Ser Pro Glu Glu Leu Ser
                   405                 410                 415
   Ser Val Pro Pro Ser Ser Ile Trp Ala Val Arg Pro Gln Asp Leu Asp
               420                 425                 430
   Thr Cys Asp Pro Arg Gln Leu Asp Val Leu Tyr Pro Lys Ala Arg Leu
           435                 440                 445
   Ala Phe Gln Asn Met Asn Gly Ser Glu Tyr Phe Val Lys Ile Gln Ser
       450                 455                 460
   Phe Leu Gly Gly Ala Pro Thr Glu Asp Leu Lys Ala Leu Ser Gln Gln
   465                 470                 475                 480
   Asn Val Ser Met Asp Leu Ala Thr Phe Met Lys Leu Arg Thr Asp Ala
                   485                 490                 495
   Val Leu Pro Leu Thr Val Ala Glu Val Gln Lys Leu Leu Gly Pro His
               500                 505                 510
   Val Glu Gly Leu Lys Ala Glu Glu Arg His Arg Pro Val Arg Asp Trp
           515                 520                 525
   Ile Leu Arg Gln Arg Gln Asp Asp Leu Asp Thr Leu Gly Leu Gly Leu
       530                 535                 540
   Gln Gly
   545
<210> 76
   <211> 3327
   <212> DNA
   <213> Artificial Sequence
<220>
   <223> Sub-fragment of plasmid
<400> 76
   ggtacctcct ttgattagta tattcctatc ttaaagttac ttttatgtgg aggcattaac 60
   atttgttaat gacgtcaaaa ggatagcaag actagaataa agctataaag caagcatata 120
   atattgcgtt tcatctttag aagcgaattt cgccaatatt ataattatca aaagagaggg 180
   gtggcaaacg gtatttggca ttattaggtt aaaaaatgta gaaggagagt gaaacccatg 240
   aatatgaaaa aagctacgat tgcagctaca gccggcattg ccgtaacagc ttttgcagca 300
   ccaactattg cctcagcctc tacagttgtt gtcgaagcag gagacacatt atggggaatc 360
   gcacaatcaa aaggtacaac ggttgatgct attaaaaaag cgaataattt aacaacagat 420
   aaaatcgtgc caggtcaaaa actgcagcgt acattagcag gtgaaacagg tcaagaagca 480
   gcaccacttg acggtgtatt aacgaatcca ccaaatatat caagtttaag tccacgtcaa 540
   ttattaggtt ttccatgtgc agaagtttca ggtttaagta cagaacgtgt ccgtgagtta 600
   gcagttgcat tagcacaaaa aaacgttaaa ttatctacag aacagttacg ttgtttagcc 660
   catagattaa gcgaaccacc agaagactta gatgcacttc ctttagacct tcttttattc 720
   ttaaatccag atgcattttc aggaccacaa gcatgtacac gtttttttag tcgaattaca 780
   aaagccaatg ttgatttatt acctcgtggg gctcctgaaa gacaacgttt attacctgct 840
   gcattagcat gctggggtgt tcgcggtagc ttattaagtg aagccgatgt tcgtgcttta 900
   ggggctttag catgtgattt acctggtcgt ttcgttgcag aatcagcaga agtgttatta 960
   ccgagattag tttcatgccc aggaccttta gatcaagatc aacaagaggc agctagagca 1020
   gctcttcaag gaggaggccc accatatggc ccaccaagta catggagtgt ttctacaatg 1080
   gatgcgttaa gaggtttatt accggtttta ggacaaccaa ttattcgtag tattccacaa 1140
   ggcattgtag cagcatggcg tcaacgtagt tctcgtgatc cgtcttggcg acaaccagaa 1200
   cgtacaattc tacgtccaag atttcgtaga gaagtagaaa aaacggcgtg tcctagtggc 1260
   aaaaaagcac gtgaaattga tgaaagttta attttttata aaaaatggga attagaagca 1320
   tgtgtcgatg cagcattact agctacacaa atggatcgtg ttaatgctat tccattcaca 1380
   tatgaacaat tagatgtttt aaagcataaa ttagacgaat tatatccaca aggttatcca 1440
   gaatcagtta ttcaacattt aggttactta tttttaaaaa tgagtccaga agacatacgc 1500
   aaatggaatg ttacaagttt agaaacatta aaagcgcttt tagaagttaa caaaggtcat 1560
   gaaatgagtc cacaagttgc tacgttaatt gatagattcg ttaaaggccg tggtcaatta 1620
   gataaagata ctttagatac attaacagca ttttatcctg gctacttatg cagtttatca 1680
   ccagaagaat taagttccgt tccaccgagt agtatctggg cagttcgtcc gcaagattta 1740
   gatacatgcg acccacgtca attagatgtt ttatatccaa aagcaagatt agctttccaa 1800
   aatatgaacg gtagtgaata tttcgtaaaa attcaatcct ttttaggtgg tgcaccaact 1860
   gaagatctaa aagcattaag ccaacaaaat gtaagtatgg atttagctac gtttatgaaa 1920
   ttacgtacag atgcagttct accattaaca gttgcagaag ttcaaaaatt attaggtcca 1980
   cacgtagaag gattaaaagc agaagaacgt caccgtccag ttcgcgattg gattttacgt 2040
   caacgtcaag atgatttaga tacattaggt ttaggtttac aaggcctgca ggtaaataat 2100
   gaggttgctg ctgctgaaaa aacagagaaa tctgttagcg caacttggtt aaacgtccgt 2160
   actggcgctg gtgttgataa cagtattatt acgtccatca aaggtggaac aaaagtaact 2220
   gttgaaacaa ccgaatctaa cggctggcac aaaattactt acaacgatgg aaaaactggt 2280
   ttcgttaacg gtaaatactt aactgacaaa gcagtaagca ctccagttgc accaacacaa 2340
   gaagtgaaaa aagaaactac tactcaacaa gctgcacctg ttgcagaaac aaaaactgaa 2400
   gtaaaacaaa ctacacaagc aactacacct gcgcctaaag tagcagaaac gaaagaaact 2460
   ccagtaatag atcaaaatgc tactacacac gctgtcaaaa gcggtgacac tatttgggct 2520
   ttatccgtaa aatacggtgt ttctgttcaa gacattatgt catggaataa tttatcttct 2580
   tcttctattt atgtaggtca aaagcttgct attaaacaaa ctgctaacac agctactcca 2640
   aaagcagaag tgaaaacgga agctccagca gctgaaaaac aagcagctcc agtagttaaa 2700
   gaaaatacta acacaaatac tgctactaca gagaaaaaag aaacagcaac gcaacaacaa 2760
   acagcaccta aagcaccaac agaagctgca aaaccagctc ctgcaccatc tacaaacaca 2820
   aatgctaata aaacgaatac aaatacaaat acaaacaata ctaatacacc atctaaaaat 2880
   actaatacaa actcaaatac taatacgaat acaaactcaa atacgaatgc taatcaaggt 2940
   tcttccaaca ataacagcaa ttcaagtgca agtgctatta ttgctgaagc tcaaaaacac 3000
   cttggaaaag cttattcatg gggtggtaac ggaccaacta catttgattg ctctggttac 3060
   actaaatatg tatttgctaa agcgggtatc tcccttccac gtacatctgg cgcacaatat 3120
   gctagcacta caagaatttc tgaatctcaa gcaaaacctg gtgatttagt attcttcgac 3180
   tatggtagcg gaatttctca cattggtatt tatgttggta atggtcaaat gattaacgcg 3240
   caagacaatg gcgttaaata cgataacatc cacggctctg gctggggtaa atatctagtt 3300
   ggcttcggtc gcgtataata aggatcc 3327
<210> 77
   <211> 165
   <212> PRT
   <213> Artificial Sequence
<220>
   <223> Fusion protein
<400> 77
<210> 78
   <211> 139
   <212> PRT
   <213> Artificial Sequence
<220>
   <223> Fusion protein
<400> 78
   Gly Ser Ala Lys Val Leu Glu Glu Asp Glu Glu Glu Thr Pro Ala Leu
    1               5                  10                  15
   Gly Ser Leu Leu Phe Leu Leu Phe Ser Leu Gly Trp Val Gln Pro Glu
               20                  25                  30
   Glu Asp Glu Glu Glu Ala Asp Leu Val Leu Ala Lys Val Leu Met Thr
           35                  40                  45
   Glu Tyr Lys Leu Val Val Val Gly Ala Asp Gly Val Gly Lys Ser Ala
       50                  55                  60
   Leu Thr Ile Gln Leu Ile Gln Ala Asp Leu Val Leu Ala Lys Val Leu
   65                  70                  75                  80
   Met Thr Glu Tyr Lys Leu Val Val Val Gly Ala Val Gly Val Gly Lys
                   85                  90                  95
   Ser Ala Leu Thr Ile Gln Leu Ile Gln Ala Asp Leu Val Leu Ala Lys
               100                 105                 110
   Val Leu Glu Ser Ile Ile Asn Phe Glu Lys Leu Ala Asp Leu Val Ala
           115                 120                 125
   Glu Gln Lys Leu Ile Ser Glu Glu Asp Leu Val
       130                 135
<210> 79
   <211> 165
   <212> PRT
   <213> Artificial Sequence
<220>
   <223> Fusion protein
<400> 79
<210> 80
   <211> 139
   <212> PRT
   <213> Artificial Sequence
<220>
   <223> Fusion protein
<400> 80
   Gly Ser Ala Lys Val Leu Met Thr Glu Tyr Lys Leu Val Val Val Gly
    1               5                  10                  15
   Ala Asp Gly Val Gly Lys Ser Ala Leu Thr Ile Gln Leu Ile Gln Ala
               20                  25                  30
   Asp Leu Val Leu Ala Lys Val Leu Met Thr Glu Tyr Lys Leu Val Val
           35                  40                  45
   Val Gly Ala Val Gly Val Gly Lys Ser Ala Leu Thr Ile Gln Leu Ile
       50                  55                  60
   Gln Ala Asp Leu Val Leu Ala Lys Val Leu Glu Glu Asp Glu Glu Glu
   65                  70                  75                  80
   Thr Pro Ala Leu Gly Ser Leu Leu Phe Leu Leu Phe Ser Leu Gly Trp
                   85                  90                  95
   Val Gln Pro Glu Glu Asp Glu Glu Glu Ala Asp Leu Val Leu Ala Lys
               100                 105                 110
   Val Leu Glu Ser Ile Ile Asn Phe Glu Lys Leu Ala Asp Leu Val Ala
           115                 120                 125
   Glu Gln Lys Leu Ile Ser Glu Glu Asp Leu Val
       130                 135
<210> 81
   <211> 25
   <212> PRT
   <213> Homo sapiens
<400> 81
   Met Thr Glu Tyr Lys Leu Val Val Val Gly Ala Asp Gly Val Gly Lys
    1               5                  10                  15
   Ser Ala Leu Thr Ile Gln Leu Ile Gln
               20                  25
<210> 82
   <211> 25
   <212> PRT
   <213> Homo sapiens
<400> 82
   Met Thr Glu Tyr Lys Leu Val Val Val Gly Ala Val Gly Asp Gly Lys
    1               5                  10                  15
   Ser Ala Leu Thr Ile Gln Leu Ile Gln
               20                  25
<210> 83
   <211> 45
   <212> PRT
   <213> Homo sapiens
<400> 83
   Ala Leu Pro Thr Ala Arg Pro Leu Leu Gly Ser Cys Gly Thr Pro Ala
    1              5                   10                  15
   Leu Gly Ser Leu Leu Phe Leu Leu Phe Ser Leu Gly Trp Val Gln Pro
               20                  25                  30
   Ser Arg Thr Leu Ala Gly Glu Thr Gly Gln Glu Ala Ala
           35                  40                  45
<210> 84
   <211> 19
   <212> PRT
   <213> Homo sapiens
<400> 84
   Thr Pro Ala Leu Gly Ser Leu Leu Phe Leu Leu Phe Ser Leu Gly Trp
    1              5                   10                  15
   Val Gln Pro
<210> 85
   <211> 6
   <212> PRT
   <213> Artificial Sequence
<220>
   <223> Spacer
<400> 85
   Glu Glu Asp Glu Glu Glu
    1              5
<210> 86
   <211> 13
   <212> PRT
   <213> Homo sapiens
<400> 86
   Ser Glu Ala Asp Val Arg Ala Leu Gly Gly Leu Ala Cys
    1              5                   10
<210> 87
   <211> 10
   <212> PRT
   <213> Phage lambda
<400> 87
   Leu Thr Ala Asp Glu Tyr Leu Lys Ile Tyr
    1              5                   10
<210> 88
   <211> 10
   <212> PRT
   <213> Artificial Sequence
<220>
   <223> Consensus sequence
<220>
   <221> VARIANT
   <222> 3, 7, 8
   <223> Xaa = Any Amino Acid
<400> 88
   Leu Thr Xaa Glu Glu Val Xaa Xaa Leu Leu
   1               5                   10
<210> 89
   <211> 5
   <212> PRT
   <213> Artificial Sequence
<220>
   <223> Consensus sequence
<220>
   <221> VARIANT
   <222> 1
   <223> Xaa = d or e
<220>
   <221> VARIANT
   <222> 2, 3, 4
   <223> Xaa = f, y, w, v, l, i or a
<220>
   <221> VARIANT
   <222> 5
   <223> Xaa = s or t
<400> 89
   Xaa Xaa Xaa Xaa Xaa
   1               5
<210> 90
   <211> 15
   <212> PRT
   <213> Phage
<220>
   <221> VARIANT
   <222> 2, 7, 10, 12, 13, 14
   <223> Xaa = Any Amino Acid
<400> 90
   His Xaa Leu Arg His Ala Xaa Ala Thr Xaa Leu Xaa Xaa Xaa Gly
   1               5                   10                  15
<210> 91
   <211> 8
   <212> PRT
   <213> Phage
<220>
   <221> VARIANT
   <222> 5
   <223> Xaa = Any Amino Acid
<400> 91
   Thr Gly Leu Arg Xaa Thr Glu Leu
    1               5
<210> 92
   <211> 18
   <212> PRT
   <213> Phage
<220>
   <221> VARIANT
   <222> 2, 3, 4, 8, 9, 10, 11, 12, 14, 15, 17
   <223> Xaa = Any Amino Acid
<400> 92
   Val Xaa Xaa Xaa Leu Gly His Xaa Xaa Xaa Xaa Xaa Thr Xaa Xaa Tyr
    1               5                  10                  15
   Xaa His
<210> 93
   <211> 9
   <212> PRT
   <213> Artificial Sequence
<220>
   <223> Motif
<220>
   <221> VARIANT
   <222> 2, 7, 8
   <223> Xaa = Any Amino Acid
<400> 93
   Tyr Xaa Arg Val Ser Thr Xaa Xaa Gln
    1               5
<210> 94
   <211> 16
   <212> PRT
   <213> Artificial Sequence
<220>
   <223> Motif
<220>
   <221> VARIANT
   <222> 7, 8, 9, 10, 13, 14, 15
   <223> Xaa = Any Amino Acid
<400> 94
   Val Ala Gln Ala Glu Arg Xaa Xaa Xaa Xaa Glu Arg Xaa Xaa Xaa Gly
   1               5                   10                  15
<210> 95
   <211> 1313
   <212> DNA
   <213> Listeria innocua
<400> 95
   aggagggctt atttatggta aaaaaagtaa aaggtaggcg ttatgagggt tctattgaac 60
   aacgtagcaa aaattcatgg cgtatgcgcg tgactgtagg ctatgactac aaaggtacgc 120
   cgattcgagc tgacagaacg acgcgaacaa aaaatgagag ggagcgagaa agagagttaa 180
   gaaatttcat cacagaatta gagcaaaatg gatatacagc tcctgcaaga atgacattta 240
   aagcatttgt tgagaatgag tatatgccga aacatgcaca aaataaccta gaagttaaaa 300
   cctggacaga atactacaaa tctatagtag caagagctta cccagccttt ggcggcgttc 360
   aaatggataa aataactaca cttcatatag ttaacttagt cgcaaaatta caaaagcccg 420
   gcgcaagatt agatgttaaa cctacagatt cagacgaaaa gaaaaataag ccgctttcgc 480
   cgcgatctat cagaaatatt tattttgcga taaattcagt atttgaaact gcggttgagt 540
   ggaaagtaat cccaattaac cccgcagagg gtgtaaggct tccaaaaaca actaaaagac 600
   cgcctactat ttatactcct gctgaaattg aattgttaaa tgcagctcta gtgaaagagc 660
   cacttagatt gcaagtaatg atttatatag cgctgatttc aggttgtaga gaagctgaat 720
   tagcagcatt agaagtaaaa cacgtgaact taatagaaga tgagctaaca ttcgaacaaa 780
   cgctagttgc aaaagcagga gaaggtttac ttcttaaaga atcaactaag aatgatgtag 840
   ctgggatagt ttctataccc gcttggttaa ctaatttaat agaaacatat ataagcaatg 900
   aagttttaga cctaaaaact gaagggaaat gggccaatca caaattttta ttcgccgaca 960
   tggaaggcaa accgattagg cctgattcga tttatcagcg ttggaaacga tttttagaaa 1020
   gacacaactt gccggtgatt cgttttcatg atttgcgtca cacatctgct acacttttat 1080
   tgaacaaagg tagagatata aaaattatcc aagagcggct tagacataaa tctagtgtga 1140
   ccacttcaaa catttatgca catgttttga aagatacgca caaagatgca gctagcgatt 1200
   ttgagaaccc tttttaagct ttctgcccca cctctgcccc acttaataaa aaaaggcaat 1260
   tttaaactaa aatttcacaa acaaaaaacc gcttaaacgc tttgtttagg cgg        1313
<210> 96
   <211> 1203
   <212> DNA
   <213> Listeria innocua
<400> 96
<210> 97
   <211> 400
   <212> **PRT**
   <213> Listeria innocua
<400> 97
   Met Val Lys Lys Val Lys Gly Arg Arg Tyr Glu Gly Ser Ile Glu Gln
   1               5                   10                  15
   Arg Ser Lys Asn Ser Trp Arg Met Arg Val Thr Val Gly Tyr Asp Tyr
               20                  25                  30
   Lys Gly Thr Pro Ile Arg Ala Asp Arg Thr Thr Arg Thr Lys Asn Glu
           35                  40                  45
   Arg Glu Arg Glu Arg Glu Leu Arg Asn Phe Ile Thr Glu Leu Glu Gln
       50                  55                  60
   Asn Gly Tyr Thr Ala Pro Ala Arg Met Thr Phe Lys Ala Phe Val Glu
   65                  70                  75                  80
   Asn Glu Tyr Met Pro Lys His Ala Gln Asn Asn Leu Glu Val Lys Thr
                   85                  90                  95
   Trp Thr Glu Tyr Tyr Lys Ser Ile Val Ala Arg Ala Tyr Pro Ala Phe
               100                 105                 110
   Gly Gly Val Gln Met Asp Lys Ile Thr Thr Leu His Ile Val Asn Leu
           115                 120                 125
   Val Ala Lys Leu Gln Lys Pro Gly Ala Arg Leu Asp Val Lys Pro Thr
       130                 135                 140
   Asp Ser Asp Glu Lys Lys Asn Lys Pro Leu Ser Pro Arg Ser Ile Arg
   145                 150                 155                 160
   Asn Ile Tyr Phe Ala Ile Asn Ser Val Phe Glu Thr Ala Val Glu Trp
                   165                 170                 175
   Lys Val Ile Pro Ile Asn Pro Ala Glu Gly Val Arg Leu Pro Lys Thr
               180                 185                 190
   Thr Lys Arg Pro Pro Thr Ile Tyr Thr Pro Ala Glu Ile Glu Leu Leu
           195                 200                 205
   Asn Ala Ala Leu Val Lys Glu Pro Leu Arg Leu Gln Val Met Ile Tyr
       210                 215                 220
   Ile Ala Leu Ile Ser Gly Cys Arg Glu Ala Glu Leu Ala Ala Leu Glu
   225                 230                 235                 240
   Val Lys His Val Asn Leu Ile Glu Asp Glu Leu Thr Phe Glu Gln Thr
                   245                 250                 255
   Leu Val Ala Lys Ala Gly Glu Gly Leu Leu Leu Lys Glu Ser Thr Lys
               260                 265                 270
   Asn Asp Val Ala Gly Ile Val Ser Ile Pro Ala Trp Leu Thr Asn Leu
           275                 280                 285
   Ile Glu Thr Tyr Ile Ser Asn Glu Val Leu Asp Leu Lys Thr Glu Gly
       290                 295                 300
   Lys Trp Ala Asn His Lys Phe Leu Phe Ala Asp Met Glu Gly Lys Pro
   305                 310                 315                 320
   Ile Arg Pro Asp Ser Ile Tyr Gln Arg Trp Lys Arg Phe Leu Glu Arg
                   325                 330                 335
   His Asn Leu Pro Val Ile Arg Phe His Asp Leu Arg His Thr Ser Ala
               340                 345                 350
   Thr Leu Leu Leu Asn Lys Gly Arg Asp Ile Lys Ile Ile Gln Glu Arg
           355                 360                 365
   Leu Arg His Lys Ser Ser Val Thr Thr Ser Asn Ile Tyr Ala His Val
       370                 375                 380
   Leu Lys Asp Thr His Lys Asp Ala Ala Ser Asp Phe Glu Asn Pro Phe
   385                 390                 395                 400
<210> 98
   <211> 340
   <212> DNA
   <213> Listeria innocua
<400> 98
   taccgaaaaa tatagccgca gcgagtggct gcggctgtgt tttatcgctg aattatggta 60
   taatattttt tgtcggaata cgacaacggg ttgttagctc agttggtaga gcagctgact 120
   cttaatcagc gggtcggggg ttcgaaaccc tcacaaccca taaaaacaaa cgccagtgac 180
   tgttaaagtc gttggtgttt tgtcgttttt acgggcaaaa tgttaataat ttcaataata 240
   agctgatttc tttttgatta tttatcgatt acatagaaaa taagtggaat ttcaaagtat 300
   ctaataattt actacatgat atacaaaagg agttgtttca                       340
<210> 99
   <211> 45
   <212> DNA
   <213> Listeria innocua
<400> 99
   actcttaatc agcgggtcgg gggttcgaaa ccctcacaac ccata 45
<210> 100
   <211> 1433
   <212> DNA
   <213> Listeria innocua
<400> 100
   tggaggtgag aaagttcatg actgtaggga tttatataag ggtttccact gaagaacaag 60
   tgaaggaagg cttttctata tcagcacaga aagagaagtt aaaagcatat tgcacagcgc 120
   aaggatggga agatttcaag ttttacgtcg atgaaggtaa atcagcaaaa gatatgcacc 180
   gccctcttct acaagaaatg atttcacata taaaaaaagg acttatagac acagtcctag 240
   tatataaatt ggatcgtctt actaggtccg ttgtagattt gcataattta ttaagtatat 300
   ttgatgaatt taactgtgca tttaaaagcg ctactgaagt ctacgatact tcttccgcta 360
   tgggcagatt ttttattaca ataataagtt cagttgctca atttgaaaga gagaatacct 420
   ctgaacgagt tagctttggg atggctgaga aagtgcgtca aggagaatat attcctctcg 480
   ctcccttcgg ttatactaag gggactgacg gaaaactaat agtaaataaa atagaaaaag 540
   aaatattttt acaagtagtt gaaatggttt caaccggtta ttctttacga caaacttgtg 600
   aatatttaac aaatattggt ttgaaaacaa ggcgttcaaa tgatgtgtgg aaagtatcta 660
   cattaatttg gatgttaaaa aatcctgctg tctacggagc gataaaatgg aataatgaaa 720
   tatatgaaaa tacacatgag cctctaatcg ataaggcaac atttaataaa gtagccaaaa 780
   tactatcaat aagaagtaaa tcaacaacaa gccgtcgtgg acacgttcat cacattttta 840
   aaaatagatt aatttgtcca gcttgtggaa aaagattatc tggattaaga acaaaatata 900
   taaataaaaa taaggaaact ttttataaca ataactatcg ttgtgctacc tgcaaagaac 960
   atagacgtcc agcagtacag ataagcgagc aaaaaataga gaaagcattt attgattata 1020
   tttcaaacta tacactcaat aaagcaaata tctcttctaa aaaattagat aataatttga 1080
   gaaaacaaga aatgattcaa aaagaaatta tttcacttca aagaaaacgt gaaaagtttc 1140
   agaaagcatg ggctgctgac cttatgaatg atgatgaatt ttctaaatta atgattgata 1200
   caaaaatgga gattgatgct gcagaagata gaaaaaaaga atatgacgta tcattatttg 1260
   tatctcctga agatattgct aaaagaaata acattcttcg tgaactaaaa ataaattgga 1320
   cttcattatc tcctactgaa aaaacagatt ttataagtat gtttattgaa ggaattgaat 1380
   atgtaaaaga tgatgaaaat aaagcggtta taacgaaaat aagtttttta taa        1433
<210> 101
   <211> 471
   <212> PRT
   <213> Listeria innocua
<400> 101
   Met Thr Val Gly Ile Tyr Ile Arg Val Ser Thr Glu Glu Gln Val Lys
    1               5                  10                  15
   Glu Gly Phe Ser Ile Ser Ala Gln Lys Glu Lys Leu Lys Ala Tyr Cys
               20                  25                  30
   Thr Ala Gln Gly Trp Glu Asp Phe Lys Phe Tyr Val Asp Glu Gly Lys
           35                  40                  45
   Ser Ala Lys Asp Met His Arg Pro Leu Leu Gln Glu Met Ile Ser His
       50                  55                  60
   Ile Lys Lys Gly Leu Ile Asp Thr Val Leu Val Tyr Lys Leu Asp Arg
   65                  70                  75                  80
   Leu Thr Arg Ser Val Val Asp Leu His Asn Leu Leu Ser Ile Phe Asp
                   85                  90                  95
   Glu Phe Asn Cys Ala Phe Lys Ser Ala Thr Glu Val Tyr Asp Thr Ser
               100                 105                 110
   Ser Ala Met Gly Arg Phe Phe Ile Thr Ile Ile Ser Ser Val Ala Gln
           115                 120                 125
   Phe Glu Arg Glu Asn Thr Ser Glu Arg Val Ser Phe Gly Met Ala Glu
       130                 135                 140
   Lys Val Arg Gln Gly Glu Tyr Ile Pro Leu Ala Pro Phe Gly Tyr Thr
   145                 150                 155                 160
   Lys Gly Thr Asp Gly Lys Leu Ile Val Asn Lys Ile Glu Lys Glu Ile
                   165                 170                 175
   Phe Leu Gln Val Val Glu Met Val Ser Thr Gly Tyr Ser Leu Arg Gln
               180                 185                 190
   Thr Cys Glu Tyr Leu Thr Asn Ile Gly Leu Lys Thr Arg Arg Ser Asn
           195                 200                 205
   Asp Val Trp Lys Val Ser Thr Leu Ile Trp Met Leu Lys Asn Pro Ala
       210                 215                 220
   Val Tyr Gly Ala Ile Lys Trp Asn Asn Glu Ile Tyr Glu Asn Thr His
   225                 230                 235                 240
   Glu Pro Leu Ile Asp Lys Ala Thr Phe Asn Lys Val Ala Lys Ile Leu
                   245                 250                 255
   Ser Ile Arg Ser Lys Ser Thr Thr Ser Arg Arg Gly His Val His His
               260                 265                 270
   Ile Phe Lys Asn Arg Leu Ile Cys Pro Ala Cys Gly Lys Arg Leu Ser
           275                 280                 285
   Gly Leu Arg Thr Lys Tyr Ile Asn Lys Asn Lys Glu Thr Phe Tyr Asn
       290                 295                 300
   Asn Asn Tyr Arg Cys Ala Thr Cys Lys Glu His Arg Arg Pro Ala Val
   305                 310                 315                 320
   Gln Ile Ser Glu Gln Lys Ile Glu Lys Ala Phe Ile Asp Tyr Ile Ser
                   325                 330                 335
   Asn Tyr Thr Leu Asn Lys Ala Asn Ile Ser Ser Lys Lys Leu Asp Asn
               340                 345                 350
   Asn Leu Arg Lys Gln Glu Met Ile Gln Lys Glu Ile Ile Ser Leu Gln
           355                 360                 365
   Arg Lys Arg Glu Lys Phe Gln Lys Ala Trp Ala Ala Asp Leu Met Asn
       370                 375                 380
   Asp Asp Glu Phe Ser Lys Leu Met Ile Asp Thr Lys Met Glu Ile Asp
   385                 390                 395                 400
   Ala Ala Glu Asp Arg Lys Lys Glu Tyr Asp Val Ser Leu Phe Val Ser
                   405                 410                 415
   Pro Glu Asp Ile Ala Lys Arg Asn Asn Ile Leu Arg Glu Leu Lys Ile
               420                 425                 430
   Asn Trp Thr Ser Leu Ser Pro Thr Glu Lys Thr Asp Phe Ile Ser Met
           435                 440                 445
   Phe Ile Glu Gly Ile Glu Tyr Val Lys Asp Asp Glu Asn Lys Ala Val
       450                 455                 460
   Ile Thr Lys Ile Ser Phe Leu
   465                 470
<210> 102
   <211> 114
   <212> DNA
   <213> Listeria innocua
<400> 102
   taaataattg tcagtcaatc aaaagaatta tttataggtt ttttgtcaaa tatggtgatg 60
   tgtacttata acccattttt cttgcaataa aagcttgtgt tattccccgt tcta       114
<210> 103
   <211> 213
   <212> DNA
   <213> Listeria innocua
<400> 103
   ttcataaaag aatttcaaat cgcacattaa aatttcactt agaataacag catttttgtg 60
   tgatagtcta acagttcctt tttcaatgtt actgtaacct gatgtgtacc tatagcccat 120
   ccgtcgcgca atgaaagctt gggtgattcc tcgctgcaat cgtaattctc gaatttttgt 180
   tgtattaatt cttctggtgt ctactgtttt cat                               213
<210> 104
   <211> 1189
   <212> DNA
   <213> Listeria innocua
<400> 104
   aggatgaaag agaatggcaa agaacaaatg gcaacccact aaacatttag gaatttatga 60
   atacatgact aaaaaaggaa agcgttatgg gatacgagtt cgttataagc aaggtaatga 120
   ttatcctgaa ataaataaat ctggttttga gacaattgca gctgcaaaag tttataaaaa 180
   caacattgaa aatttgaaag ctaataaaaa agaatatgtt tttacaaatg aaaaattaac 240
   attaaatact tggtttgctt cttacatgga aatgtttaaa aagaaaaaca aaagtaaaga 300
   cacaatagcg aataaatata gtatttataa taatcactta gaaatccctt ttggtaatta 360
   ctatttaact gatataagtt tagatattta cgaagacttt ttgcgcgaaa aaattaaaaa 420
   tggatacgca aacaactcag tcaaagcgat gcataaatta atgaaaagca ttttaaacgc 480
   tgctgttaga tatgagaaac tagaaaaaaa cagacttcaa tttgctgaaa tagagcaatt 540
   agaagaaaat gaagttattg agcttaaggt attagaaaca gatgagttta atgtatttat 600
   atcagcttgt agagcatttt ttactaaata tgattttaca atgatttatc ttgcagtttg 660
   ggggatgcgt cgcggtgaag ttatgggggt aaaacttaaa aatcttactt ttgatgatgc 720
   taaacaacaa gtacgtatta cactagattc cactcgaacc cttcgtactc ccgagggaaa 780
   aggtacgaaa acaccagctg gtagaagaat attactaata gacggcgaag gttatcgact 840
   acttaaatat tcggtagaaa aagcggttag cattgctaaa gaccatggat ctgttttgca 900
   ccaggatgat tttattttta gaaacccaac ttctaatcgt ccttgggcgg ttacgcgtat 960
   gaatgattta ctacgaaaat tagaaaaaga atacgacata aaagtttacc ctcatctatt 1020
   acgccataac tttaatactc aggcattatt ggctggagct aatagcaatg atttacgaaa 1080
   atttattggc cacaaaaaca gtagcatgac tgatcattat tcacatgcga cagacgaggg 1140
   acgagaaaaa ttaatgaata cgatgaaaga cagattgtca ggaatctag             1189
<210> 105
   <211> 391
   <212> PRT
   <213> Listeria innocua
<400> 105
   Met Ala Lys Asn Lys Trp Gln Pro Thr Lys His Leu Gly Ile Tyr Glu
   1               5                   10                  15
   Tyr Met Thr Lys Lys Gly Lys Arg Tyr Gly Ile Arg Val Arg Tyr Lys
               20                  25                  30
   Gln Gly Asn Asp Tyr Pro Glu Ile Asn Lys Ser Gly Phe Glu Thr Ile
           35                  40                  45
   Ala Ala Ala Lys Val Tyr Lys Asn Asn Ile Glu Asn Leu Lys Ala Asn
       50                  55                  60
   Lys Lys Glu Tyr Val Phe Thr Asn Glu Lys Leu Thr Leu Asn Thr Trp
   65                  70                  75                  80
   Phe Ala Ser Tyr Met Glu Met Phe Lys Lys Lys Asn Lys Ser Lys Asp
                   85                  90                  95
   Thr Ile Ala Asn Lys Tyr Ser Ile Tyr Asn Asn His Leu Glu Ile Pro
               100                 105                 110
   Phe Gly Asn Tyr Tyr Leu Thr Asp Ile Ser Leu Asp Ile Tyr Glu Asp
           115                 120                 125
   Phe Leu Arg Glu Lys Ile Lys Asn Gly Tyr Ala Asn Asn Ser Val Lys
       130                 135                 140
   Ala Met His Lys Leu Met Lys Ser Ile Leu Asn Ala Ala Val Arg Tyr
   145                 150                 155                 160
   Glu Lys Leu Glu Lys Asn Arg Leu Gln Phe Ala Glu Ile Glu Gln Leu
                   165                 170                 175
   Glu Glu Asn Glu Val Ile Glu Leu Lys Val Leu Glu Thr Asp Glu Phe
               180                 185                 190
   Asn Val Phe Ile Ser Ala Cys Arg Ala Phe Phe Thr Lys Tyr Asp Phe
           195                 200                 205
   Thr Met Ile Tyr Leu Ala Val Trp Gly Met Arg Arg Gly Glu Val Met
       210                 215                 220
   Gly Val Lys Leu Lys Asn Leu Thr Phe Asp Asp Ala Lys Gln Gln Val
   225                 230                 235                 240
   Arg Ile Thr Leu Asp Ser Thr Arg Thr Leu Arg Thr Pro Glu Gly Lys
                   245                 250                 255
   Gly Thr Lys Thr Pro Ala Gly Arg Arg Ile Leu Leu Ile Asp Gly Glu
               260                 265                 270
   Gly Tyr Arg Leu Leu Lys Tyr Ser Val Glu Lys Ala Val Ser Ile Ala
           275                 280                 285
   Lys Asp His Gly Ser Val Leu His Gln Asp Asp Phe Ile Phe Arg Asn
       290                 295                 300
   Pro Thr Ser Asn Arg Pro Trp Ala Val Thr Arg Met Asn Asp Leu Leu
   305                 310                 315                 320
   Arg Lys Leu Glu Lys Glu Tyr Asp Ile Lys Val Tyr Pro His Leu Leu
                   325                 330                 335
   Arg His Asn Phe Asn Thr Gln Ala Leu Leu Ala Gly Ala Asn Ser Asn
               340                 345                 350
   Asp Leu Arg Lys Phe Ile Gly His Lys Asn Ser Ser Met Thr Asp His
           355                 360                 365
   Tyr Ser His Ala Thr Asp Glu Gly Arg Glu Lys Leu Met Asn Thr Met
       370                 375                 380
   Lys Asp Arg Leu Ser Gly Ile
   385                 390
<210> 106
   <211> 160
   <212> DNA
   <213> Listeria innocua
<400> 106
   aaaattgtgg gataaaaatt aaatataaaa atatcccaca aaaaatccca caatagtttg 60
   atattgtatg atattcaaat gaaatcaaaa aaataaaaac cccgtatttc ctaagaaaat 120
   acggggtttt gatatcatat aaaatcaatt aaaaattgac 160
<210> 107
   <211> 574
   <212> DNA
   <213> Listeria innocua
<400> 107
   tcttgttgcc tcctttttgt aatcaatagt tgcaatgcaa gagtatcata aaaaagcgat 60
   gtataaccaa aaatgtaatg aaatgtccga ttcttgtcgt gaacgactag aaaatggagc 120
   ttatttagag atattcttac acaacgtgag tatcattaag ttttttggtc ataagataat 180
   actcattatg agttactatt cacattttaa acattcctgt ttctatttat cacaaaaaat 240
   acatatcaat ccaagatatg cgttatttca cttatgaata ttccttattt atttaattat 300
   ttatcagttt tatttattac taggtgaata atatagtata attattcacc tacgacagac 360
   gagacacgag aaaaattaat gaatacgatg aaagacagat tgtcaggaat ctagaaaatt 420
   gtgggataaa aattaaatat aaaaatatcc cacaaaaaat cccacaataa tttgatattg 480
   tatgatattc aaatgaaatc aaaaaaatca aaaccccgca tttcctaaga aaatacgggg 540
   ttttgatatc atataaaatc gatttaaaat ggac 574
<210> 108
   <211> 1152
   <212> DNA
   <213> Listeria innocua
<400> 108
   atgaaaataa aaaaaatgaa aaatggtaaa tatactgttc gtttgcgtat taaagttgat 60
   ggagagtgga aagaaaaacg tttgacagat acaagtgaaa caaatttgat gtacaaagca 120
   tcaaaattat taaaacaagt tgaacatgat agtaattcac taaaagaatg gaatttcaaa 180
   gaattctatt cgctatttat gaaaactttc aaagaaaata aaagtagtca atcaacaatt 240
   aacttgtatg acttagctta taatcagttc gttaattatt tcgacgaaaa aataaagtta 300
   aattcaattg acgctgttca atatcagcaa tttattaatc atttagcatt agattacgct 360
   gtcgctacta tagataccag acaccgcaaa attagagcga ttttcaataa agccgtccat 420
   ttaggttaca tgaaaaaaaa ccctgctctg ggcgctcaca taagcggtca tgatatagca 480
   aaaacaaaag cgcaatattt agaaacagat aaagtacatc tattattaga agagcttgca 540
   aaacttcatt ctatatcaag agcagttatt tttttagcag ttcaaacagg aatgcgattt 600
   gaagaaatta ttgcactgac aaaaaaagat attaatttta ctaaacgttc tatatcagtg 660
   aataaggcat gggattataa atacactaac acgtttacgg acactaaaac aaaaaagtca 720
   cgagtaatct atattgataa ttcaactgtt caatatttac agtcttacct tgcttggcat 780
   gctgattata tgaaagagca tgcaattgaa aatccggtga tgttgttatt cattacttat 840
   cacaataaac ctgttgacaa cgcttcatgt aacaaagcac tgaagaaaat atgtactaca 900
   attaattctg aaacagtaac attacacaag cttcgacaca cgcacacagg tctatgtgta 960
   gaggctggta tggatattat ttatgtagct gacaggcttg gtcatgatga tattaataca 1020
   acattaaaat attatagtca tctgagttct aatttacgac aacaaaatca atctaaagta 1080
   gatgcttttt tcacactaaa aacagatgaa aataccacaa aatttgccac aaatgccaca 1140
   aaaacaacgg aa 1152
<210> 109
   <211> 384
   <212> PRT
   <213> Listeria innocua
<400> 109
   Met Lys Ile Lys Lys Met Lys Asn Gly Lys Tyr Thr Val Arg Leu Arg
   1               5                   10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Glu
           35                  40                  45
   His Asp Ser Asn Ser Leu Lys Glu Trp Asn Phe Lys Glu Phe Tyr Ser
       50                  55                  60
   Leu Phe Met Lys Thr Phe Lys Glu Asn Lys Ser Ser Gln Ser Thr Ile
   65                  70                  75                  80
   Asn Leu Tyr Asp Leu Ala Tyr Asn Gln Phe Val Asn Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ala Leu Asp Tyr Ala Val Ala Thr Ile Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Ala Leu Gly Ala His Ile Ser Gly His Asp Ile Ala
   145                 150                 155                 160
   Lys Thr Lys Ala Gln Tyr Leu Glu Thr Asp Lys Val His Leu Leu Leu
                   165                 170                 175
   Glu Glu Leu Ala Lys Leu His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Ser Val Asn Lys Ala Trp
       210                 215                 220
   Asp Tyr Lys Tyr Thr Asn Thr Phe Thr Asp Thr Lys Thr Lys Lys Ser
   225                 230                 235                 240
   Arg Val Ile Tyr Ile Asp Asn Ser Thr Val Gln Tyr Leu Gln Ser Tyr
                   245                 250                 255
   Leu Ala Trp His Ala Asp Tyr Met Lys Glu His Ala Ile Glu Asn Pro
               260                 265                 270
   Val Met Leu Leu Phe Ile Thr Tyr His Asn Lys Pro Val Asp Asn Ala
           275                 280                 285
   Ser Cys Asn Lys Ala Leu Lys Lys Ile Cys Thr Thr Ile Asn Ser Glu
       290                 295                 300
   Thr Val Thr Leu His Lys Leu Arg His Thr His Thr Gly Leu Cys Val
   305                 310                 315                 320
   Glu Ala Gly Met Asp Ile Ile Tyr Val Ala Asp Arg Leu Gly His Asp
                   325                 330                 335
   Asp Ile Asn Thr Thr Leu Lys Tyr Tyr Ser His Leu Ser Ser Asn Leu
               340                 345                 350
   Arg Gln Gln Asn Gln Ser Lys Val Asp Ala Phe Phe Thr Leu Lys Thr
           355                 360                 365
   Asp Glu Asn Thr Thr Lys Phe Ala Thr Asn Ala Thr Lys Thr Thr Glu
       370                 375                 380
<210> 110
   <211> 299
   <212> DNA
   <213> Listeria innocua
<400> 110
   taaaacgggt attgcaaggt ataaaaaaat ctctaaaaca ttcgtttatc ctttaatatc 60
   aaggatttcc aacgttttag agatttcttt acatcactac ttaatgccct cggagggaat 120
   cgaaccccca ttttaagaac cggaatctta cgtgctatcc gttgcaccac gagggcttta 180
   tgtacaaaga aaatgtttac cgtacgaata ataattatag cgaaattcgt atgtttttac 240
   aagctttatt ttgaatgaag aagccagcgc atcctgagat ttgctggctt caatagtta  299
<210> 111
   <211> 15
   <212> DNA
   <213> Listeria innocua
<400> 111
   atgccctcgg aggga 15
<210> 112
   <211> 253
   <212> DNA
   <213> Listeria monocytogenes
<400> 112
   taaaatgaaa aaacatctta caacatggct tttgccagat gtgggatgtt tttttagtat 60
   gccctcggag ggaatcgaac ccccatttta agaaccggaa tcttacgtgc tatccgttgc 120
   accacgaggg ctatatgtag gccagaaatg cttaccgtac gaataataat tatagcgaaa 180
   ttcgtagtgt tttacaagtt ttattttaaa tgaagaagcc agcgcctcca aagatttgct 240
   ggctcaagta tta                                                    253
<210> 113
   <211> 1161
   <212> DNA
   <213> Listeria monocytogenes
<400> 113
   atggctagct atgtaaattt aggaaataat aaatatgagc taagagtttc aaagggatat 60
   gatgcacgtg gaaaacaaat acgcaaaaca aaaaacgtca cagttaaaac agtaaaagcg 120
   ttaaaactag aactttctaa ttttgaagct tatgtctatt caagcgatta cacagaaata 180
   aaagatatgc gatttattga ctttgtggaa aaatggcgct taaattacgc aaaaagagaa 240
   ctaaaaggta atactattga taagtataac ctctttctcg aaaactggat tataccttat 300
   tttgagagga agaaaataag taaaattaca actatgcagt tgctcgacta ctttcatgaa 360
   gttcaaaaaa aaggagttgg tccaagcgct ttagagggac atcatcgagt tataagaagt 420
   ttatttaaat atgctacctt gtggggaatt actgaaacag acgtatcttt atcagtgaaa 480
   aaacctacct ataaagtgcc agaaaaaaat atttataata gacgagaaat agaagtgtta 540
   atagatcgca ttaagatatt acaaaaatat caacaagtaa tgattaaatt agcgctatac 600
   tgcggtctta gacgtggcga agttatcggt ttaacaacta aagatatgaa ttacaataaa 660
   aatacaatta acgtttatag agcggttata aagagtgcta gcgaaggtat aaaactagat 720
   gaaactaaaa ataagcgaaa aagaattgtc cccgctcccg ctggactgat gcaagaaatt 780
   aaagaacttg caaaagaaaa gcaaaaaaac aaagataaat taggtttgtt gtggaaagga 840
   acaaaagatt tagatgggaa aactgttgta ttaattttca gtcatgacga cggcaccccc 900
   tttacccccg cttctgtcac tagaatgttt aatcgatttt tagagaaaga agaaaataac 960
   gatcttacta aaatatcatt tcatgatttg cgtcattctg ctgcaagctt ccttctcgaa 1020
   caaggtatta atgtaaaagt cattcaaaac attttaggac attcagacat taaagttaca 1080
   ttaaatacgt atgcacatat cactgaagat ggttactcag aagcagcaaa aacttttgat 1140
   aatttctata aatctagtaa a                                           1161
<210> 114
   <211> 387
   <212> PRT
   <213> Listeria monocytogenes
<400> 114
   Met Ala Ser Tyr Val Asn Leu Gly Asn Asn Lys Tyr Glu Leu Arg Val
    1               5                  10                  15
   Ser Lys Gly Tyr Asp Ala Arg Gly Lys Gln Ile Arg Lys Thr Lys Asn
               20                  25                  30
   Val Thr Val Lys Thr Val Lys Ala Leu Lys Leu Glu Leu Ser Asn Phe
           35                  40                  45
   Glu Ala Tyr Val Tyr Ser Ser Asp Tyr Thr Glu Ile Lys Asp Met Arg
       50                  55                  60
   Phe Ile Asp Phe Val Glu Lys Trp Arg Leu Asn Tyr Ala Lys Arg Glu
   65                  70                  75                  80
   Leu Lys Gly Asn Thr Ile Asp Lys Tyr Asn Leu Phe Leu Glu Asn Trp
                   85                  90                  95
   Ile Ile Pro Tyr Phe Glu Arg Lys Lys Ile Ser Lys Ile Thr Thr Met
               100                 105                 110
   Gln Leu Leu Asp Tyr Phe His Glu Val Gln Lys Lys Gly Val Gly Pro
           115                 120                 125
   Ser Ala Leu Glu Gly His His Arg Val Ile Arg Ser Leu Phe Lys Tyr
       130                 135                 140
   Ala Thr Leu Trp Gly Ile Thr Glu Thr Asp Val Ser Leu Ser Val Lys
   145                 150                 155                 160
   Lys Pro Thr Tyr Lys Val Pro Glu Lys Asn Ile Tyr Asn Arg Arg Glu
                   165                 170                 175
   Ile Glu Val Leu Ile Asp Arg Ile Lys Ile Leu Gln Lys Tyr Gln Gln
               180                 185                 190
   Val Met Ile Lys Leu Ala Leu Tyr Cys Gly Leu Arg Arg Gly Glu Val
           195                 200                 205
   Ile Gly Leu Thr Thr Lys Asp Met Asn Tyr Asn Lys Asn Thr Ile Asn
       210                 215                 220
   Val Tyr Arg Ala Val Ile Lys Ser Ala Ser Glu Gly Ile Lys Leu Asp
   225                 230                 235                 240
   Glu Thr Lys Asn Lys Arg Lys Arg Ile Val Pro Ala Pro Ala Gly Leu
                   245                 250                 255
   Met Gln Glu Ile Lys Glu Leu Ala Lys Glu Lys Gln Lys Asn Lys Asp
               260                 265                 270
   Lys Leu Gly Leu Leu Trp Lys Gly Thr Lys Asp Leu Asp Gly Lys Thr
           275                 280                 285
   Val Val Leu Ile Phe Ser His Asp Asp Gly Thr Pro Phe Thr Pro Ala
       290                 295                 300
   Ser Val Thr Arg Met Phe Asn Arg Phe Leu Glu Lys Glu Glu Asn Asn
   305                 310                 315                 320
   Asp Leu Thr Lys Ile Ser Phe His Asp Leu Arg His Ser Ala Ala Ser
                   325                 330                 335
   Phe Leu Leu Glu Gln Gly Ile Asn Val Lys Val Ile Gln Asn Ile Leu
               340                 345                 350
   Gly His Ser Asp Ile Lys Val Thr Leu Asn Thr Tyr Ala His Ile Thr
           355                 360                 365
   Glu Asp Gly Tyr Ser Glu Ala Ala Lys Thr Phe Asp Asn Phe Tyr Lys
       370                 375                 380
   Ser Ser Lys
   385
<210> 115
   <211> 841
   <212> DNA
   <213> Listeria monocytogenes
<400> 115
   taaggtgtcg aataaggtgt tttgctattt ttaggcaaat aaaaaaagct tcgcatatta 60
   gcgaaacacc tacagcacca acgttttata ttaagccact tgtcggattt gaaccgacga 120
   ccccttcctt accatggaag tgctctacca actgagctaa agcggcagca aagcctttca 180
   aataaaaaaa tggctccaca ggcaggactc gaacctgcga ccgatcggtt aacagccgat 240
   tgctctacca actgagctac tgtggaataa taaattgccc ggcagcgacc tactctcgca 300
   gggggaagcc cccaactacc attggcgcag agaagcttaa ctaccgtgtt cgggatggga 360
   acgggtgtga ccttctcgcc ataactacca gacaatattg agttgttgaa agattgctct 420
   ctcaaaacta gagaagaaag tgttcagtta ggtaacttcg tttcattttt tggttaagtc 480
   ctcgatcgat tagtatttgt ccgctccatg tatcgctaca cttccactcc aaacctatct 540
   acctgatcat ctttcaggga tcttactttc cgaagaaatg ggaaatctca tcttgagggg 600
   ggcttcacgc ttagatgctt tcagcgttta tccctgccac acatagctac ccagcgatgc 660
   tcctggcgga acaactggta caccagcggt gtgtccatcc cggtcctctc gtactaagga 720
   cagctcctct caaatttcct gcgcccgcga cggataggga ccgaactgtc tcacgacgtt 780
   ctgaacccag ctcgcgtgcc gctttaatgg gcgaacagcc caacccttgg gaccgactac 840
   a                                                                 841
<210> 116
   <211> 399
   <212> DNA
   <213> Listeria monocytogenes
<400> 116
   aaaaacaccc cacccgttct gttattatac ccatagtata atcgatttat actacctatt 60
   caagatatcc ataataaata tcattattct tttaaacaat aaaaaaagcc tcgcatacta 120
   gcgaaacata caaattatcc atatattatt taagccactt gtcggatttg aaccgacgac 180
   cccttcctta ccatggaagt gctctaccaa ctgagctaaa gcggcagcaa agcctttcaa 240
   ataaaaaaat ggctccacag gcaggactcg aacctgcgac cgatcggtta acagccgatt 300
   gctctaccaa ctgagctact gtggaataat aaattgcccg gcagcgacct actctcgcag 360
   ggggaagccc ccaactacca ttggcgcaga gaagcttaa                        399
<210> 117
   <211> 399
   <212> DNA
   <213> Listeria monocytogenes
<400> 117
   aaaaacaccc cacccgttct gttattatac ccatagtata atcgatttat actacctatt 60
   caagatatcc ataataaata tcattattct tttaaacaat aaaaaaagcc tcgcatacta 120
   gcgaaacata caaattatcc atatattatt taagccactt gtcggatttg aaccgacgac 180
   cccttcctta ccatggaagt gctctaccaa ctgagctaaa gcggcagcaa agcctttcaa 240
   ataaaaaaat ggctccacag gcaggactcg aacctgcgac cgatcggtta acagccgatt 300
   gctctaccaa ctgagctact gtggaataat aaattgcccg gcagcgacct actctcgcag 360
   ggggaagccc ccaactacca ttggcgcaga gaagcttaa                        399
<210> 118
   <211> 399
   <212> DNA
   <213> Listeria monocytogenes
<400> 118
   aaaaacaccc cacccgttct gttattatac ccatagtata atcgatttat actacctatt 60
   caagatatcc ataataaata tcattattct tttaaacaat aaaaaaagcc tcgcatacta 120
   gcgaaacata caaattatcc atatattatt taagccactt gtcggatttg aaccgacgac 180
   cccttcctta ccatggaagt gctctaccaa ctgagctaaa gcggcagcaa agcctttcaa 240
   ataaaaaaat ggctccacag gcaggactcg aacctgcgac cgatcggtta acagccgatt 300
   gctctaccaa ctgagctact gtggaataat aaattgcccg gcagcgacct actctcgcag 360
   ggggaagccc ccaactacca ttggcgcaga gaagcttaa                        399
<210> 119
   <211> 384
   <212> PRT
   <213> Bacteriophage PSA
<400> 119
   Met Lys Ile Lys Lys Leu Ala Asn Gly Lys Tyr Cys Val Arg Leu Arg
    1               5                  10                  15
   Ile Lys Val Asp Gly Glu Trp Lys Glu Lys Arg Leu Thr Asp Thr Ser
               20                  25                  30
   Glu Thr Asn Leu Met Tyr Lys Ala Ser Lys Leu Leu Lys Gln Val Gln
           35                  40                  45
   His Asp Ser Ser Ser Leu Lys Glu Trp Asn Phe Lys Glu Phe Tyr Thr
       50                  55                  60
   Leu Phe Met Lys Thr Phe Lys Asp Gly Lys Ser Ser Gln Ser Thr Ile
   65                  70                  75                  80
   Asn Leu Tyr Asp Leu Ala Tyr Asn Gln Phe Val Asp Tyr Phe Asp Glu
                   85                  90                  95
   Lys Ile Lys Leu Asn Ser Ile Asp Ala Val Gln Tyr Gln Gln Phe Ile
               100                 105                 110
   Asn His Leu Ser Val Asp Tyr Ala Ile Ser Thr Val Asp Thr Arg His
           115                 120                 125
   Arg Lys Ile Arg Ala Ile Phe Asn Lys Ala Val His Leu Gly Tyr Met
       130                 135                 140
   Lys Lys Asn Pro Thr Ile Gly Ala His Ile Ser Gly Gln Asp Val Ala
   145                 150                 155                 160
   Lys Asn Lys Ala Gln Phe Met Glu Thr Asp Lys Val His Leu Leu Leu
                   165                 170                 175
   Glu Glu Leu Ala Lys Phe His Ser Ile Ser Arg Ala Val Ile Phe Leu
               180                 185                 190
   Ala Val Gln Thr Gly Met Arg Phe Glu Glu Ile Ile Ala Leu Thr Lys
           195                 200                 205
   Lys Asp Ile Asn Phe Thr Lys Arg Ser Ile Thr Val Asn Lys Ala Trp
       210                 215                 220
   Asp Tyr Lys Tyr Thr Asn Thr Phe Ile Asp Thr Lys Thr Lys Lys Ser
   225                 230                 235                 240
   Arg Val Ile Tyr Ile Asp Asn Ser Thr Ala Gln Tyr Leu His Ser Tyr
                   245                 250                 255
   Leu Asn Trp His Thr Asp Tyr Met Lys Glu His Ala Ile Lys Asn Pro
               260                 265                 270
   Leu Met Leu Leu Phe Ile Thr Tyr His Asn Lys Pro Val Asp Asn Ala
           275                 280                 285
   Ser Cys Asn Lys Ala Leu Lys Lys Ile Cys Ser Thr Ile Asn Ser Glu
       290                 295                 300
   Pro Val Thr Leu His Lys Leu Arg His Thr His Thr Gly Leu Cys Val
   305                 310                 315                 320
   Glu Ala Gly Met Asp Ile Ile Tyr Val Ala Asp Arg Leu Gly His Asp
                   325                 330                 335
   Asp Ile Asn Thr Thr Leu Lys Tyr Tyr Ser His Leu Ser Ser Asn Leu
               340                 345                 350
   Arg Gln His Asn Gln Ser Lys Val Asp Ala Phe Phe Thr Leu Lys Thr
           355                 360                 365
   Asp Glu Asn Thr Thr Asn Phe Thr Thr Asn Ala Thr Lys Thr Thr Glu
               370                 375                 380
<210> 120
   <211> 387
   <212> PRT
   <213> Listeria monocytogenes
<400> 120
   Met Ala Ser Tyr Val Asn Leu Gly Asn Asn Lys Tyr Glu Leu Arg Val
   1               5                   10                  15
   Ser Lys Gly Tyr Asp Ala Arg Gly Lys Gln Ile Arg Lys Thr Lys Asn
               20                  25                  30
   Val Thr Val Lys Thr Val Lys Ala Leu Lys Leu Glu Leu Ser Asn Phe
           35                  40                  45
   Glu Ala Tyr Val Tyr Ser Ser Asp Tyr Thr Glu Ile Lys Asp Met Arg
       50                  55                  60
   Phe Ile Asp Phe Val Glu Lys Trp Arg Leu Asn Tyr Ala Lys Arg Glu
   65                  70                  75                  80
   Leu Lys Gly Asn Thr Ile Asp Lys Tyr Asn Leu Phe Leu Glu Asn Trp
                   85                  90                  95
   Ile Ile Pro Tyr Phe Glu Arg Lys Lys Ile Ser Lys Ile Thr Thr Met
               100                 105                 110
   Gln Leu Leu Asp Tyr Phe His Glu Val Gln Lys Lys Gly Val Gly Pro
           115                 120                 125
   Ser Ala Leu Glu Gly His His Arg Val Ile Arg Ser Leu Phe Lys Tyr
       130                 135                 140
   Ala Thr Leu Trp Gly Ile Thr Glu Thr Asp Val Ser Leu Ser Val Lys
   145                 150                 155                 160
   Lys Pro Thr Tyr Lys Val Pro Glu Lys Asn Ile Tyr Asn Arg Arg Glu
                   165                 170                 175
   Ile Glu Val Leu Ile Asp Arg Ile Lys Ile Leu Gln Lys Tyr Gln Gln
               180                 185                 190
   Val Met Ile Lys Leu Ala Leu Tyr Cys Gly Leu Arg Arg Gly Glu Val
           195                 200                 205
   Ile Gly Leu Thr Thr Lys Asp Met Asn Tyr Asn Lys Asn Thr Ile Asn
       210                 215                 220
   Val Tyr Arg Ala Val Ile Lys Ser Ala Ser Glu Gly Ile Lys Leu Asp
   225                 230                 235                 240
   Glu Thr Lys Asn Lys Arg Lys Arg Ile Val Pro Ala Pro Ala Gly Leu
                   245                 250                 255
   Met Gln Glu Ile Lys Glu Leu Ala Lys Glu Lys Gln Lys Asn Lys Asp
               260                 265                 270
   Lys Leu Gly Leu Leu Trp Lys Gly Thr Lys Asp Leu Asp Gly Lys Thr
           275                 280                 285
   Val Val Leu Ile Phe Ser His Asp Asp Gly Thr Pro Phe Thr Pro Ala
       290                 295                 300
   Ser Val Thr Arg Met Phe Asn Arg Phe Leu Glu Lys Glu Glu Asn Asn
   305                 310                 315                 320
   Asp Leu Thr Lys Ile Ser Phe His Asp Leu Arg His Ser Ala Ala Ser
                   325                 330                 335
   Phe Leu Leu Glu Gln Gly Ile Asn Val Lys Val Ile Gln Asn Ile Leu
               340                 345                 350
   Gly His Ser Asp Ile Lys Val Thr Leu Asn Thr Tyr Ala His Ile Thr
           355                 360                 365
   Glu Asp Gly Tyr Ser Glu Ala Ala Lys Thr Phe Asp Asn Phe Tyr Lys
       370                 375                 380
   Ser Ser Lys
   385
<210> 121
   <211> 17
   <212> DNA
   <213> Artificial Sequence
<220>
   <223> Insertion site on pKSV7
<400> 121
   ggtaccttgg tgagctc 17
<210> 122
   <211> 300
   <212> DNA
   <213> Listeria monocytogenes
<400> 122
   gtgggattaa atagatttat gcgtgcgatg atggtagttt tcattactgc caactgcatt 60
   acgattaacc ccgacataat atttgcagcg acagatagcg aagattccag tctaaacaca 120
   gatgaatggg aagaagaaaa aacagaagag cagccaagcg aggtaaatac gggaccaaga 180
   tacgaaactg cacgtgaagt aagttcacgt gatattgagg aactagaaaa atcgaataaa 240
   gtgaaaaata cgaacaaagc agacctaata gcaatgttga aagcaaaagc agagaaaggt 300
<210> 123
   <211> 8
   <212> PRT
   <213> Listeria monocytogenes
<400> 123
   Asp Lys Ser Ala Gly Leu Ile Asp
    1               5
<210> 124
   <211> 6
   <212> PRT
   <213> Listeria monocytogenes
<400> 124
   Leu Lys Glu Lys Ala Glu
    1               5
<210> 125
   <211> 29
   <212> PRT
   <213> Listeria monocytogenes
<400> 125
   Met Gly Leu Asn Arg Phe Met Arg Ala Met Met Val Val Phe Ile Thr
    1               5                   10                  15
   Ala Asn Cys Ile Thr Ile Asn Pro Asp Ile Ile Phe Ala
               20                  25
<210> 126
   <211> 5
   <212> PRT
   <213> Listeria monocytogenes
<400> 126
   Thr Glu Ala Lys Asp
    1               5
<210> 127
   <211> 5
   <212> PRT
   <213> Lactococcus lactis
<400> 127
   Val Tyr Ala Asp Thr
   1 5
<210> 128
   <211> 5
   <212> PRT
   <213> Bacillus anthracis
<400> 128
   Ile Gln Ala Glu Val
   1 5
<210> 129
   <211> 5
   <212> PRT
   <213> Listeria monocytogenes
<400> 129
   Ala Ser Ala Ser Thr
   1 5
<210> 130
   <211> 5
   <212> PRT
   <213> Listeria monocytogenes
<400> 130
   Val Gly Ala Phe Gly
   1 5
<210> 131
   <211> 5
   <212> PRT
   <213> Bacillus anthracis
<400> 131
   Ala Phe Ala Glu Asp
   1 5
<210> 132
   <211> 5
   <212> PRT
   <213> Staphylococcus aureus
<400> 132
   Val Gln Ala Ala Glu
   1 5
<210> 133
   <211> 5
   <212> PRT
   <213> Listeria monocytogenes
<400> 133
   Asp Lys Ala Leu Thr
    1               5
<210> 134
   <211> 5
   <212> PRT
   <213> Bacillus subtillis
<400> 134
   Val Gly Ala Phe Gly
    1               5
<210> 135
   <211> 7
   <212> PRT
   <213> Listeria monocytogenes
<220>
   <221> VARIANT
   <222> 1
   <223> Xaa = e or d
<220>
   <221> VARIANT
   <222> 6
   <223> Xaa = Any Amino Acid
<220>
   <221> VARIANT
   <222> 7
   <223> Xaa = d or e
<400> 135
   Xaa Phe Pro Pro Pro Xaa Xaa
    1               5
<210> 136
   <211> 3552
   <212> DNA
   <213> Artificial Sequence
<220>
   <223> Sub-fragment of plasmid
<400> 136
<210> 137
   <211> 4
   <212> PRT
   <213> Artificial Sequence
<220>
   <223> Consensus sequence
<220>
   <221> VARIANT
   <222> 3
   <223> Xaa = g, a, s, or t
<400> 137
   Leu Leu Xaa His
    1
<210> 138
   <211> 6
   <212> PRT
   <213> Artificial Sequence
<220>
   <223> Consensus sequence
<220>
   <221> VARIANT
   <222> 1
   <223> Xaa = d or e
<220>
   <221> VARIANT
   <222> 2, 3, 4, 5
   <223> Xaa = f, y, w, v, l, i or a
<220>
   <221> VARIANT
   <222> 6
   <223> Xaa = s or t
<400> 138
   Xaa Xaa Xaa Xaa Xaa Xaa
    1               5
<210> 139
   <211> 7
   <212> PRT
   <213> Artificial Sequence
<220>
   <223> Consensus sequence
<220>
   <221> VARIANT
   <222> 1
   <223> Xaa = d or e
<220>
   <221> VARIANT
   <222> 2, 3, 4, 5, 6,
   <223> Xaa = f, y, w, v, l, i or a
<220>
   <221> VARIANT
   <222> 7
   <223> Xaa = s or t
<400> 139
   Xaa Xaa Xaa Xaa Xaa Xaa Xaa
   1               5
<210> 140
   <211> 8
   <212> PRT
   <213> Artificial Sequence
<220>
   <223> Consensus sequence
<220>
   <221> VARIANT
   <222> 1
   <223> Xaa = d or e
<220>
   <221> VARIANT
   <222> 2, 3, 4, 5, 6, 7
   <223> Xaa = f, y, w, v, l, i or a
<220>
   <221> VARIANT
   <222> 8
   <223> Xaa = s or t
<400> 140
   Xaa Xaa Xaa Xaa Xaa Xaa Xaa Xaa
   1               5
<210> 141
   <211> 8
   <212> PRT
   <213> Artificial Sequence
<220>
   <223> Motif
<400> 141
   Val Leu Val Leu Asp Arg Leu Arg
   1               5
<210> 142
   <211> 8
   <212> PRT
   <213> Gallus gallus
<400> 142
   Ser Ile Ile Asn Phe Glu Lys Leu
    1               5
<210> 143
   <211> 18
   <212> PRT
   <213> Listeria monocytogenes
<400> 143
   Ala Thr Asp Ser Glu Asp Ser Ser Leu Asn Thr Asp Glu Trp Glu Glu
    1               5                  10                  15
   Glu Lys
<210> 144
   <211> 9
   <212> PRT
   <213> Listeria monocytogenes
<400> 144
   Lys Tyr Gly Val Ser Val Gln Asp Ile
    1               5
<210> 145
   <211> 121
   <212> PRT
   <213> Homo sapiens
<400> 145
   Met Lys Ala Val Leu Leu Ala Leu Leu Met Ala Gly Leu Ala Leu Gln
    1               5                  10                  15
   Pro Gly Thr Ala Leu Leu Cys Cys Lys Ala Gln Val Ser Asn Glu Asp
               20                  25                  30
   Cys Leu Gln Val Glu Asn Cys Thr Gln Leu Gly Glu Gln Cys Trp Thr
           35                  40                  45
   Ala Arg Ile Arg Ala Val Gly Leu Leu Thr Val Ile Ser Lys Gly Cys
       50                  55                  60
   Ser Leu Asn Cys Val Asp Asp Ser Gln Asp Tyr Tyr Val Gly Lys Lys
   65                  70                  75                  80
   Asn Ile Thr Cys Cys Asp Thr Asp Leu Cys Asn Ala Ser Gly Ala His
                   85                  90                  95
   Ala Leu Gln Pro Ala Ala Ala Ile Leu Ala Leu Leu Pro Ala Leu Gly
               100                 105                 110
   Leu Leu Leu Trp Gly Pro Gly Gln Leu
           115                 120
<210> 146
   <211> 264
   <212> DNA
   <213> Artificial Sequence
<220>
   <223> Synthetic construct
<400> 146
   ggatccggta cagcactttt atgttattcc tgtaaagcac aagtatcaaa tgaagactgt 60
   ttacaagtag aaaattgtac acaattggga gaacaatgtt ggactgcgag aattcgagcc 120
   gtaggtttat taactgtaat tagtaaagga tgttcgttaa actgtgtaga tgactcacaa 180
   gattattacg ttggcaaaaa aaatattaca tgctgtgaca ctgatttatg caatgcaagt 240
   ggcgctcacg ctcttcaaac tagt                                        264
<210> 147
   <211> 86
   <212> PRT
   <213> Artificial Sequence
<220>
   <223> Synthetic construct
<400> 147
   Gly Ser Gly Thr Ala Leu Leu Cys Cys Lys Ala Gln Val Ser Asn Glu
    1               5                  10                  15
   Asp Cys Leu Gln Val Glu Asn Cys Thr Gln Leu Gly Glu Gln Cys Trp
               20                  25                  30
   Thr Ala Arg Ile Arg Ala Val Gly Leu Leu Thr Val Ile Ser Lys Gly
           35                  40                  45
   Cys Ser Leu Asn Cys Val Asp Asp Ser Gln Asp Tyr Tyr Val Gly Lys
       50                  55                  60
   Lys Asn Ile Thr Cys Cys Asp Thr Asp Leu Cys Asn Ala Ser Gly Ala
   65                  70                  75                  80
   His Ala Leu Gln Thr Ser
                   85
<210> 148
   <211> 215
   <212> PRT
   <213> Artificial Sequence
<220>
   <223> Synthetic construct
<400> 148
<210> 149
   <211> 215
   <212> PRT
   <213> Artificial Sequence
<220>
   <223> Synthetic construct
<400> 149
   Met Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
                   20                  25                  30
   Ser Glu Asp Ser Ser Leu Asn Thr Asp Glu Trp Glu Glu Glu Lys Thr
               35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser Gly Thr Ala Leu Leu Cys Tyr Ser Cys Lys
               100                 105                 110
   Ala Gln Val Ser Asn Glu Asp Cys Leu Gln Val Glu Asn Cys Thr Gln
           115                 120                 125
   Leu Gly Glu Gln Cys Trp Thr Ala Arg Ile Arg Ala Val Gly Leu Leu
       130                 135                 140
   Thr Val Ile Ser Lys Gly Cys Ser Leu Asn Cys Val Asp Asp Ser Gln
   145                 150                 155                 160
   Asp Tyr Tyr Val Gly Lys Lys Asn Ile Thr Cys Cys Asp Thr Asp Leu
                   165                 170                 175
   Cys Asn Ala Ser Gly Ala His Ala Leu Gln Thr Ser Gln Leu Ala Asp
               180                 185                 190
   Leu Val Leu Ala Lys Val Leu Gln Leu Glu Ser Ile Ile Asn Phe Glu
           195                 200                 205
   Lys Leu Ala Asp Leu Val Ala
       210                 215
<210> 150
   <211> 120
   <212> DNA
   <213> Artificial Sequence
<220>
   <223> Cloning region of pINT-ActAN100-BamHI-SpeI-MfeI-SIINFEKL
<400> 150
   aaagcagaga aaggtggatc cactagtcaa ttggcagatc ttgtattagc aaaagtttta 60
   caattagaaa gtattattaa ttttgaaaaa ttagcagatc ttgtagcata agcggccgcc 120
<210> 151
   <211> 36
   <212> PRT
   <213> Artificial Sequence
<220>
   <223> Cloning region of pINT-ActAN100-BamHI-SpeI-MfeI-SIINFEKL
<400> 151
   Lys Ala Glu Lys Gly Gly Ser Thr Ser Gln Leu Ala Asp Leu Val Leu
    1               5                  10                  15
   Ala Lys Val Leu Gln Leu Glu Ser Ile Ile Asn Phe Glu Lys Leu Ala
               20                  25                  30
   Asp Leu Val Ala
           35
<210> 152
   <211> 639
   <212> PRT
   <213> Listeria monocytogenes
<400> 152
   Met Gly Leu Asn Arg Phe Met Arg Ala Met Met Val Val Phe Ile Thr
   1               5                   10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Pro Asn Asn Asn Asn Asn Asn Gly Glu Gln Thr Gly
               100                 105                 110
   Asn Val Ala Ile Asn Glu Glu Ala Ser Gly Val Asp Arg Pro Thr Leu
           115                 120                 125
   Gln Val Glu Arg Arg His Pro Gly Leu Ser Ser Asp Ser Ala Ala Glu
       130                 135                 140
   Ile Lys Lys Arg Arg Lys Ala Ile Ala Ser Ser Asp Ser Glu Leu Glu
   145                 150                 155                 160
   Ser Leu Thr Tyr Pro Asp Lys Pro Thr Lys Ala Asn Lys Arg Lys Val
                   165                 170                 175
   Ala Lys Glu Ser Val Val Asp Ala Ser Glu Ser Asp Leu Asp Ser Ser
               180                 185                 190
   Met Gln Ser Ala Asp Glu Ser Thr Pro Gln Pro Leu Lys Ala Asn Gln
           195                 200                 205
   Lys Pro Phe Phe Pro Lys Val Phe Lys Lys Ile Lys Asp Ala Gly Lys
       210                 215                 220
   Trp Val Arg Asp Lys Ile Asp Glu Asn Pro Glu Val Lys Lys Ala Ile
   225                 230                 235                 240
   Val Asp Lys Ser Ala Gly Leu Ile Asp Gln Leu Leu Thr Lys Lys Lys
                   245                 250                 255
   Ser Glu Glu Val Asn Ala Ser Asp Phe Pro Pro Pro Pro Thr Asp Glu
               260                 265                 270
   Glu Leu Arg Leu Ala Leu Pro Glu Thr Pro Met Leu Leu Gly Phe Asn
           275                 280                 285
   Ala Pro Thr Pro Ser Glu Pro Ser Ser Phe Glu Phe Pro Pro Pro Pro
       290                 295                 300
   Thr Asp Glu Glu Leu Arg Leu Ala Leu Pro Glu Thr Pro Met Leu Leu
   305                 310                 315                 320
   Gly Phe Asn Ala Pro Ala Thr Ser Glu Pro Ser Ser Phe Glu Phe Pro
                   325                 330                 335
   Pro Pro Pro Thr Glu Asp Glu Leu Glu Ile Met Arg Glu Thr Ala Pro
               340                 345                 350
   Ser Leu Asp Ser Ser Phe Thr Ser Gly Asp Leu Ala Ser Leu Arg Ser
           355                 360                 365
   Ala Ile Asn Arg His Ser Glu Asn Phe Ser Asp Phe Pro Leu Ile Pro
       370                 375                 380
   Thr Glu Glu Glu Leu Asn Gly Arg Gly Gly Arg Pro Thr Ser Glu Glu
   385                 390                 395                 400
   Phe Ser Ser Leu Asn Ser Gly Asp Phe Thr Asp Asp Glu Asn Ser Glu
                   405                 410                 415
   Thr Thr Glu Glu Glu Ile Asp Arg Leu Ala Asp Leu Arg Asp Arg Gly
               420                 425                 430
   Thr Gly Lys His Ser Arg Asn Ala Gly Phe Leu Pro Leu Asn Pro Phe
           435                 440                 445
   Ile Ser Ser Pro Val Pro Ser Leu Thr Pro Lys Val Pro Lys Ile Ser
       450                 455                 460
   Ala Pro Ala Leu Ile Ser Asp Ile Thr Lys Lys Ala Pro Phe Lys Asn
   465                 470                 475                 480
   Pro Ser Gln Pro Leu Asn Val Phe Asn Lys Lys Thr Thr Thr Lys Thr
                           485                 490                 495
   Val Thr Lys Lys Pro Thr Pro Val Lys Thr Ala Pro Lys Leu Ala Glu
               500                 505                 510
   Leu Pro Ala Thr Lys Pro Gln Glu Thr Val Leu Arg Glu Asn Lys Thr
           515                 520                 525
   Pro Phe Ile Glu Lys Gln Ala Glu Thr Asn Lys Gln Ser Ile Asn Met
       530                 535                 540
   Pro Ser Leu Pro Val Ile Gln Lys Glu Ala Thr Glu Ser Asp Lys Glu
   545                 550                 555                 560
   Glu Met Lys Pro Gln Thr Glu Glu Lys Met Val Glu Glu Ser Glu Ser
                   565                 570                 575
   Ala Asn Asn Ala Asn Gly Lys Asn Arg Ser Ala Gly Ile Glu Glu Gly
               580                 585                 590
   Lys Leu Ile Ala Lys Ser Ala Glu Asp Glu Lys Ala Lys Glu Glu Pro
           595                 600                 605
   Gly Asn His Thr Thr Leu Ile Leu Ala Met Leu Ala Ile Gly Val Phe
       610                 615                 620
   Ser Leu Gly Ala Phe Ile Lys Ile Ile Gln Leu Arg Lys Asn Asn
   625                 630                 635
<210> 153
   <211> 100
   <212> PRT
   <213> Listeria monocytogenes
<400> 153
   Met Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly
               100
<210> 154
   <211> 648
   <212> PRT
   <213> Artificial Sequence
<220>
   <223> Fusion protein
<400> 154
   Met Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
   85 90 95
   Ala Glu Lys Gly Gly Ser Arg Thr Leu Ala Gly Glu Thr Gly Gln Glu
   100 105 110
   Ala Ala Pro Leu Asp Gly Val Leu Thr Asn Pro Pro Asn Ile Ser Ser
   115 120 125
   Leu Ser Pro Arg Gln Leu Leu Gly Phe Pro Cys Ala Glu Val Ser Gly
   130 135 140
   Leu Ser Thr Glu Arg Val Arg Glu Leu Ala Val Ala Leu Ala Gln Lys
   145 150 155 160
   Asn Val Lys Leu Ser Thr Glu Gln Leu Arg Cys Leu Ala His Arg Leu
   165 170 175
   Ser Glu Pro Pro Glu Asp Leu Asp Ala Leu Pro Leu Asp Leu Leu Leu
   180 185 190
   Phe Leu Asn Pro Asp Ala Phe Ser Gly Pro Gln Ala Cys Thr Arg Phe
   195 200 205
   Phe Ser Arg Ile Thr Lys Ala Asn Val Asp Leu Leu Pro Arg Gly Ala
   210 215 220
   Pro Glu Arg Gln Arg Leu Leu Pro Ala Ala Leu Ala Cys Trp Gly Val
   225 230 235 240
   Arg Gly Ser Leu Leu Ser Glu Ala Asp Val Arg Ala Leu Gly Gly Leu
   245 250 255
   Ala Cys Asp Leu Pro Gly Arg Phe Val Ala Glu Ser Ala Glu Val Leu
   260 265 270
   Leu Pro Arg Leu Val Ser Cys Pro Gly Pro Leu Asp Gln Asp Gln Gln
   275 280 285
   Glu Ala Ala Arg Ala Ala Leu Gln Gly Gly Gly Pro Pro Tyr Gly Pro
   290 295 300
   Pro Ser Thr Trp Ser Val Ser Thr Met Asp Ala Leu Arg Gly Leu Leu
   305 310 315 320
   Pro Val Leu Gly Gln Pro Ile Ile Arg Ser Ile Pro Gln Gly Ile Val
   325 330 335
   Ala Ala Trp Arg Gln Arg Ser Ser Arg Asp Pro Ser Trp Arg Gln Pro
   340 345 350
   Glu Arg Thr Ile Leu Arg Pro Arg Phe Arg Arg Glu Val Glu Lys Thr
   355 360 365
   Ala Cys Pro Ser Gly Lys Lys Ala Arg Glu Ile Asp Glu Ser Leu Ile
   370 375 380
   Phe Tyr Lys Lys Trp Glu Leu Glu Ala Cys Val Asp Ala Ala Leu Leu
   385 390 395 400
   Ala Thr Gln Met Asp Arg Val Asn Ala Ile Pro Phe Thr Tyr Glu Gln
   405 410 415
   Leu Asp Val Leu Lys His Lys Leu Asp Glu Leu Tyr Pro Gln Gly Tyr
   420 425 430
   Pro Glu Ser Val Ile Gln His Leu Gly Tyr Leu Phe Leu Lys Met Ser
   435 440 445
   Pro Glu Asp Ile Arg Lys Trp Asn Val Thr Ser Leu Glu Thr Leu Lys
   450 455 460
   Ala Leu Leu Glu Val Asn Lys Gly His Glu Met Ser Pro Gln Val Ala
   465 470 475 480
   Thr Leu Ile Asp Arg Phe Val Lys Gly Arg Gly Gln Leu Asp Lys Asp
   485 490 495
   Thr Leu Asp Thr Leu Thr Ala Phe Tyr Pro Gly Tyr Leu Cys Ser Leu
   500 505 510
   Ser Pro Glu Glu Leu Ser Ser Val Pro Pro Ser Ser Ile Trp Ala Val
   515 520 525
   Arg Pro Gln Asp Leu Asp Thr Cys Asp Pro Arg Gln Leu Asp Val Leu
   530 535 540
   Tyr Pro Lys Ala Arg Leu Ala Phe Gln Asn Met Asn Gly Ser Glu Tyr
   545 550 555 560
   Phe Val Lys Ile Gln Ser Phe Leu Gly Gly Ala Pro Thr Glu Asp Leu
   565 570 575
   Lys Ala Leu Ser Gln Gln Asn Val Ser Met Asp Leu Ala Thr Phe Met
                   580                 585                 590
   Lys Leu Arg Thr Asp Ala Val Leu Pro Leu Thr Val Ala Glu Val Gln
               595                 600                 605
   Lys Leu Leu Gly Pro His Val Glu Gly Leu Lys Ala Glu Glu Arg His
       610                 615                 620
   Arg Pro Val Arg Asp Trp Ile Leu Arg Gln Arg Gln Asp Asp Leu Asp
   625                 630                 635                 640
   Thr Leu Gly Leu Gly Leu Gln Gly
                   645
<210> 155
   <211> 674
   <212> PRT
   <213> Artificial Sequence
<220>
   <223> Fusion protein
<400> 155
   Met Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser Arg Thr Leu Ala Gly Glu Thr Gly Gln Glu
               100                 105                 110
   Ala Ala Pro Leu Asp Gly Val Leu Thr Asn Pro Pro Asn Ile Ser Ser
           115                 120                 125
   Leu Ser Pro Arg Gln Leu Leu Gly Phe Pro Cys Ala Glu Val Ser Gly
       130                 135                 140
   Leu Ser Thr Glu Arg Val Arg Glu Leu Ala Val Ala Leu Ala Gln Lys
   145                 150                 155                 160
   Asn Val Lys Leu Ser Thr Glu Gln Leu Arg Cys Leu Ala His Arg Leu
                   165                 170                 175
   Ser Glu Pro Pro Glu Asp Leu Asp Ala Leu Pro Leu Asp Leu Leu Leu
               180                 185                 190
   Phe Leu Asn Pro Asp Ala Phe Ser Gly Pro Gln Ala Cys Thr Arg Phe
           195                 200                 205
   Phe Ser Arg Ile Thr Lys Ala Asn Val Asp Leu Leu Pro Arg Gly Ala
       210                 215                 220
   Pro Glu Arg Gln Arg Leu Leu Pro Ala Ala Leu Ala Cys Trp Gly Val
   225                 230                 235                 240
   Arg Gly Ser Leu Leu Ser Glu Ala Asp Val Arg Ala Leu Gly Gly Leu
                   245                 250                 255
   Ala Cys Asp Leu Pro Gly Arg Phe Val Ala Glu Ser Ala Glu Val Leu
               260                 265                 270
   Leu Pro Arg Leu Val Ser Cys Pro Gly Pro Leu Asp Gln Asp Gln Gln
           275                 280                 285
   Glu Ala Ala Arg Ala Ala Leu Gln Gly Gly Gly Pro Pro Tyr Gly Pro
       290                 295                 300
   Pro Ser Thr Trp Ser Val Ser Thr Met Asp Ala Leu Arg Gly Leu Leu
   305                 310                 315                 320
   Pro Val Leu Gly Gln Pro Ile Ile Arg Ser Ile Pro Gln Gly Ile Val
                   325                 330                 335
   Ala Ala Trp Arg Gln Arg Ser Ser Arg Asp Pro Ser Trp Arg Gln Pro
   340 345 350
   Glu Arg Thr Ile Leu Arg Pro Arg Phe Arg Arg Glu Val Glu Lys Thr
   355 360 365
   Ala Cys Pro Ser Gly Lys Lys Ala Arg Glu Ile Asp Glu Ser Leu Ile
   370 375 380
   Phe Tyr Lys Lys Trp Glu Leu Glu Ala Cys Val Asp Ala Ala Leu Leu
   385 390 395 400
   Ala Thr Gln Met Asp Arg Val Asn Ala Ile Pro Phe Thr Tyr Glu Gln
   405 410 415
   Leu Asp Val Leu Lys His Lys Leu Asp Glu Leu Tyr Pro Gln Gly Tyr
   420 425 430
   Pro Glu Ser Val Ile Gln His Leu Gly Tyr Leu Phe Leu Lys Met Ser
   435 440 445
   Pro Glu Asp Ile Arg Lys Trp Asn Val Thr Ser Leu Glu Thr Leu Lys
   450 455 460
   Ala Leu Leu Glu Val Asn Lys Gly His Glu Met Ser Pro Gln Val Ala
   465 470 475 480
   Thr Leu Ile Asp Arg Phe Val Lys Gly Arg Gly Gln Leu Asp Lys Asp
   485 490 495
   Thr Leu Asp Thr Leu Thr Ala Phe Tyr Pro Gly Tyr Leu Cys Ser Leu
   500 505 510
   Ser Pro Glu Glu Leu Ser Ser Val Pro Pro Ser Ser Ile Trp Ala Val
   515 520 525
   Arg Pro Gln Asp Leu Asp Thr Cys Asp Pro Arg Gln Leu Asp Val Leu
   530 535 540
   Tyr Pro Lys Ala Arg Leu Ala Phe Gln Asn Met Asn Gly Ser Glu Tyr
   545 550 555 560
   Phe Val Lys Ile Gln Ser Phe Leu Gly Gly Ala Pro Thr Glu Asp Leu
   565 570 575
   Lys Ala Leu Ser Gln Gln Asn Val Ser Met Asp Leu Ala Thr Phe Met
   580 585 590
   Lys Leu Arg Thr Asp Ala Val Leu Pro Leu Thr Val Ala Glu Val Gln
   595 600 605
   Lys Leu Leu Gly Pro His Val Glu Gly Leu Lys Ala Glu Glu Arg His
   610 615 620
   Arg Pro Val Arg Asp Trp Ile Leu Arg Gln Arg Gln Asp Asp Leu Asp
   625 630 635 640
   Thr Leu Gly Leu Gly Leu Gln Gly Ala Met Thr Glu Tyr Lys Leu Val
   645 650 655
   Val Val Gly Ala Asp Gly Val Gly Lys Ser Ala Leu Thr Ile Gln Leu
   660 665 670
   Ile Gln
<210> 156
   <211> 102
   <212> PRT
   <213> Artificial Sequence
<220>
   <223> Fusion protein
<400> 156
   Met Gly Leu Asn Arg Phe Met Arg Ala Met Met Val Val Phe Ile Thr
    1               5                  10                  15
   Ala Asn Cys Ile Thr Ile Asn Pro Asp Ile Ile Phe Ala Ala Thr Asp
               20                  25                  30
   Ser Glu Asp Ser Ser Leu Asn Thr Asp Glu Trp Glu Glu Glu Lys Thr
           35                  40                  45
   Glu Glu Gln Pro Ser Glu Val Asn Thr Gly Pro Arg Tyr Glu Thr Ala
       50                  55                  60
   Arg Glu Val Ser Ser Arg Asp Ile Glu Glu Leu Glu Lys Ser Asn Lys
   65                  70                  75                  80
   Val Lys Asn Thr Asn Lys Ala Asp Leu Ile Ala Met Leu Lys Ala Lys
                   85                  90                  95
   Ala Glu Lys Gly Gly Ser
               100
<210> 157
   <211> 230
   <212> DNA
   <213> Listeria monocytogenes
<400> 157
   ggtaccggga agcagttggg gttaactgat taacaaatgt tagagaaaaa ttaattctcc 60
   aagtgatatt cttaaaataa ttcatgaata ttttttctta tattagctaa ttaagaagat 120
   aattaactgc taatccaatt tttaacggaa taaattagtg aaaatgaagg ccgaattttc 180
   cttgttctaa aaaggttgta ttagcgtatc acgaggaggg agtataagtg            230
<210> 158
   <211> 2070
   <212> DNA
   <213> Artificial Sequence
<220>
   <223> Coding sequence of fusion protein
<400> 158
<210> 159
   <211> 690
   <212> PRT
   <213> Artificial Sequence
<220>
   <223> Fusion protein
<400> 159

## Description

### STATEMENT REGARDING FEDERALLY SPONSORED

### RESEARCH OR DEVELOPMENT

This invention was made, in part, with U.S. government support under National Cancer Institute NHI 1 K23CA104160-01. The government may have certain rights in the invention.

### FIELD OF THE INVENTION

The invention provides engineered *Listeria* bacteria, useful for stimulating the immune system and treating cancers and infections. Also provided are polynucleotides, fusion protein partners, and integration vectors useful for modifying *Listeria* and other bacterial species.

### BACKGROUND OF THE INVENTION

Cancers and infections can be treated by administering reagents that modulate the immune system. These reagents include vaccines, cytokines, antibodies, and small molecules, such as CpG oligodeoxynucleotides and imidazoquinolines (see, e.g., Becker (2005) Virus Genes 30:251-266; Schetter and Vollmer (2004) Curr. Opin. Drug Devel. 7:204-210; Majewski, et al. (2005) Int. J. Dermatol. 44:14-19), Hofmann, et al. (2005) J. Clin. Virol. 32:86-91; Huber, et al. (2005) Infection 33:25-29; Carter (2001) Nature Revs. Cancer 1: 118-129; Dechant and Valaerius (2001) Crit. Revs. Oncol. 39:69-77; O'Connor, et al. (2004) Neurology 62:2038-2043). Vaccines, including classical vaccines (inactivated whole organisms, extracts, or antigens), dendritic cell (DC) vaccines, and nucleic acid-based vaccines, are all useful for treating cancers and infections (see, e.g., Robinson and Amara (2005) Nat. Med. Suppl. 11:S25-S32; Plotkin (2005) Nat. Med. Suppl. 11:85-811; Pashine, et al. (2005) Nat. Med. Suppl. 11:S63-S68; Larche and Wraith (2005) Nat. Med. Suppl. 11:S69-S76). Another reagent useful for modulating the immune system is *Listeria monocytogenes* (*L. monocytogenes*), and this reagent has proven to be successful in treating cancers and tumors (see, e.g., Brockstedt, et al. (2004) Proc. Natl. Acad. Sci. USA 101:13832-13837; Brockstedt, et al (2005) Nat. Med. 11:853-860); Starks, et al. (2004) J. Immunol. 173:420-427; Shen, et al. (1995) Proc. Natl. Acad. Sci. USA 92:3987-3991).

Recombinant *Listeria* strains have been developed as vaccines against viruses and tumors (see, e.g., Starks, et al. (2004) J. Immunol. 173:420-427; Gunn, et al. (2001) J. Immunol. 167:6471-6479; Ikonomidis, et al. (1994) J. Exp. Med. 180:2209-2218; Mata, et al. (2001) Vaccine 19:1435-1445; Mata and Paterson (1999) J. Immunol. 163:1449-1456; Mata, et al. (1998) J. Immunol. 161:2985-2993; Friedman, et al. (2000) J. Virol. 74:9987-9993; Soussi, et al. (2002) Vaccine 20:2702-2712; Saklani-Jusforgues, et al. (2003) Infect. Immun. 71:1083-1090; Soussi, et al. (2000) Infect. Immunity 68:1498-1506; Tvinnereim, et al. (2002) Infect. Immunity 70:153-162; Rayevskaya, et al. (2002) J. Virol. 76:918-922; Frankel, et al. (1995) J. Immunol. 55:4775-4782; Jensen, et al. (1997) J. Virol. 71:8467-8474; Jensen, el al. (1997) Immunol. Rev. 158:147-157; Lin, et al. (2002) Int. J. Cancer 102:629-637; Peters, et al. (2003) FEMS Immunol. Med. Microbiol. 35:243-253; Peters, et al. (2003) J. Immunol. 170:5176-5187; Paterson (2003) Immunol. Res. 27:451-462; Paterson and Johnson (2004) Expert Rev. Vaccines 3:S119-S134; Ochsenbein, et al. (1999) Proc. Natl. Acad. Sci USA 96:9293-9298; Hess, et al. (2000) Adv. Immunol. 75:1-88).

*L. monocytogenes* has a natural tropism for the liver and spleen and, to some extent, other tissues such as the small intestines (see, e.g., Dussurget, et al. (2004) Ann. Rev. Microbiol. 58:587-610; Gouin, et al. (2005) Curr. Opin. Microbiol. 8:35-45; Cossart (2002) Int. J. Med. Microbiol. 291:401-409; Vazquez-Boland, et al. (2001) Clin. Microbiol. Rev. 14:584-640; Schluter, et al. (1999) Immunobiol, 201:188-195). Where the bacterium resides in the intestines, passage to the bloodstream is mediated by listerial proteins, such as ActA and internalin A (see, e.g., Manohar, et al. (2001) Infection Immunity 69:3542-3549; Lecuit, et al. (2004) Proc. Natl. Acad. Sci. USA 101:6152-6157; Lecuit and Cossart (2002) Trends Mol. Med. 8:537-542). Once the bacterium enters a host cell, the life cycle of *L. monocytogenes* involves escape from the phagolysosome and to the cytosol. This life cycle contrasts with that of *Mycobacterium,* which remains inside the phagolysosome (see, e.g., Clemens, et al. (2002) Infection Immunity 70:5800-5807; Schluter, et al. (1998) Infect. Immunity 66:5930-5938; Gutierrez, et al. (2004) Cell 119:753-766). *L. monocytogenes'* escape from the phagolysosome is mediated by listerial proteins, such as listeriolysin (LLO), PI-PLC, and PC-PLC (see Portnoy, et al. (2002) J. Cell Biol. 158:409-414). US 2003/203472 describes site-specific Listeria integration vectors and methods for their use. Vaccines for treating cancers or infections are often ineffective because of a lack of appropriate reagents.

The present invention relates to method of integrating a plasmid at a genomic attBB' site in a *Listeria* genome. This method may be used to provide bacterial vaccines, useful for enhancing the expression or immune processing of antigens, and for increasing survival to cancers and infections.

### SUMMARY OF THE INVENTION

The present invention provides a method of integrating a plasmid at a genomic attBB' site in a *Listeria* genome, wherein said attBB' site has been integrated into the genome into a gene that mediates growth or spread, wherein said plasmid comprises a phage attachment attPP' site and comprises: a first nucleic acid encoding a phage integrase; a second nucleic acid encoding said phage attPP' attachment site; a third nucleic acid encoding a heterologous antigen; fourth and fifth nucleic acids encoding first and second lox sites, or first and second Frt sites, and; a sixth nucleic acid encoding a selection marker, wherein the first lox or Frt sites and second lox or Frt sites are operably linked with the sixth nucleic acid, and wherein the operably linked lox or Frt sites are useful for mediating Cre or FLP recombinase, respectively, catalyzed excision of the sixth nucleic acid; said method comprising transfecting the *Listeria* with the plasmid and allowing integrase-catalysed integration of the heterologous antigen into the *Listeria* genome under conditions suitable for integration.

The present disclosure also sets out ("provides") a number of other products and methods as discussed herein below which may facilitate understanding of the present invention, which is defined by the claims.

The present disclosure is based, in part, on the recognition that administering an attenuated *Listeria* to a mammal bearing a tumor results in enhanced survival, where the *Listeria* was engineered to contain a nucleic acid encoding an ActA-based fusion protein linked to a tumor antigen.

In one aspect, the disclosure provides a polynucleotide comprising a promoter operably linked to a nucleic acid sequence encoding a fusion protein, wherein the fusion protein comprises (a) modified ActA and (b) a heterologous antigen. In some embodiments, the promoter is a bacterial promoter (e.g., a *Listerial* promoter). In some embodiments, the promoter is an ActA promoter. In some embodiments, the modified ActA comprises at least the first 59 amino acids of ActA. In some embodiments, the modified ActA comprises more than the first 59 amino acids of ActA. In some embodiments, the modified ActA comprises less than the first 380 amino acids or less than the first 265 amino acids. In some embodiments, the modified ActA comprises more than the first 59 amino acids of ActA, and less than the first 380 amino acids of ActA. For example, in some embodiments, the modified ActA comprises at least about the first 59 amino acids of ActA, but less than about the first 265 amino acids of ActA. In some embodiments, the modified ActA comprises more than the first 59 amino acids of ActA, but less than about the first 265 amino acids of ActA. In other embodiments, the modified ActA comprises more than the first 59 amino acids of ActA, and less than the first 380 amino acids of ActA. In still further embodiments, the modified ActA comprises at least the first 85 amino acids of ActA and less than the first 125 amino acids of ActA. In some embodiments, the modified ActA comprises amino acids 1-100 of ActA. In some embodiments, the modified ActA consists of amino acids 1-100 of ActA. The heterologous antigen may be non*-Listerial.* In some embodiments, the heterologous antigen is from, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is a tumor antigen or is derived from a tumor antigen. In some embodiments, the heterologous antigen is, or is derived from, mesothelin. For example, in some embodiments, the heterologous antigen is, or is derived from, human mesothelin. In some embodiments, the *Listeria* is hMeso26 or hMeso38 (see Table 11 of Example VII, below). In some embodiments, the heterologous antigen does not comprise an EphA2 antigenic peptide. In some embodiments, the nucleic acid sequence encoding the fusion protein is codon-optimized for expression in *Listeria.* The disclosure provides plasmids and cells comprising the polynucleotide. The disclosure further provides a *Listeria* bacterium (e.g., *Listeria monocytogenes)* comprising the polynucleotide, as well as vaccines comprising the *Listeria.* The *Listeria* bacterium may be attenuated (e.g., an actA deletion mutant or an actA insertion mutant). In certain embodiments, the bacterium may comprise an attenuating mutation in *actA* and/or in *inlB.* In some embodiments, the *Listeria* comprises the polynucleotide in its genome. In some embodiments, the polynucleotide has been integrated into a virulence gene in the Listerial genome. In some embodiments, a polynucleotide (or nucleic acid) has been integrated into a virulence gene in the genome of the *Listeria,* wherein the integration of the polynucleotide (a) disrupts expression of the virulence gene and/or (b) disrupts a coding sequence of the virulence gene. In some embodiments, the virulence gene is prfA-dependent. In other embodiments, the virulence gene is prfA-independent. In some embodiments, the nucleic acid or the polynucleotide has been integrated into the genome of the *Listeria* at the *actA* locus and/or *inlB* locus. In some embodiments, the *Listeria* comprises a plasmid comprising the polynucleotide. The disclosure further provides immunogenic and pharmaceutical compositions comprising the *Listeria.* The disclosure also provides methods for stimulating immune responses to the heterologous antigen in a mammal (e.g., a human), comprising administering an effective amount of the *Listeria* (or an effective amount of a composition comprising the *Listeria*) to the mammal. For instance, the disclosure also provides methods for stimulating immune responses to an antigen from, or derived from, a cancer or infectious agent, comprising administering an effective amount of the *Listeria* (or a composition comprising the *Listeria*) to a mammal having the cancer or infectious agent, wherein the heterologous antigen shares at least one epitope with or is immunologically cross-reactive with the antigen from, or derived from, the cancer or infectious agent. In some embodiments, inclusion of the modified Act A sequence in the fusion protein enhances the immunogenicity of the *Listeria* comprising the polynucleotide (e.g., relative to the immunogenicity of *Listeria* comprising a polynucleotide encoding a fusion protein comprising the heterologous antigen and a non-ActA signal sequence and/or leader sequence, instead of the modified ActA). In some embodiments, inclusion of the modified Act A sequence in the fusion protein enhances expression and/or secretion of the heterologous antigen in *Listeria* (e.g., relative to the expression and/or secretion in *Listeria* of the heterologous antigen fused to a non-ActA signal sequence and/or leader sequence instead of the modified ActA).

In another aspect, the disclosure provides a polynucleotide comprising a first nucleic acid encoding a modified ActA (e.g., actA-N-100), operably linked and in frame with, a second nucleic acid encoding a heterologous antigen. In some embodiments, the modified ActA comprises at least the first 59 amino acids of ActA, but less than about the first 265 amino acids of ActA. In some embodiments, the modified ActA comprises more than the first 59 amino acids of ActA, but less than about the first 265 amino acids of ActA. In some embodiments, the first nucleic acid encodes amino acids 1-100 of ActA. In some embodiments, the polynucleotide is genomic. For instance, the polynucleotide may be integrated into the *actA* or *inlB* gene. In some alternative embodiments, the polynucleotide is plasmid-based. In some embodiments, the polynucleotide is operably linked with one or more of the following: (a) actA promoter; or (b) a bacterial promoter that is not actA promoter. In some embodiments, the heterologous antigen is, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is, or is derived from, mesothelin (e.g., human mesothelin). The disclosure further provides a *Listeria* bacterium e.g., *Listeria monocytogenes*) comprising the polynucleotide, as well as vaccines comprising the *Listeria.* In some embodiments, the *Listeria* is hMeso26 or hMeso38 (see Table 11 of Example VII, below). The disclosure also provides methods for stimulating immune responses to an antigen from, or derived from, a cancer (e.g., a tumor or pre-cancerous cell) or infectious agent (e.g., a virus, pathogenic bacterium, or parasitic organism), comprising administering the *Listeria* to a mammal having the cancer or infectious agent, wherein the heterologous antigen shares at least one epitope with or is immunologically cross-reactive with the antigen from, or derived from, the cancer or infectious agent. In some embodiments of the methods, the stimulating is relative to immune response without administering the *Listeria.* In some embodiments of the methods, the heterologous antigen is from, or is derived from, the cancer cell, tumor, or infectious agent.

In another aspect, the disclosure provides a polynucleotide comprising a first nucleic acid encoding a modified actA, wherein the modified actA comprises (a) amino acids 1-59 of actA, (b) an inactivating mutation in, deletion of, or truncation prior to, at least one domain for actA-mediated regulation of the host cell cytoskeleton, wherein the first nucleic acid is operably linked and in frame with a second nucleic acid encoding a heterologous antigen. In some embodiments the modified ActA comprises more than the first 59 amino acids of ActA. In some embodiments, the domain is the cofilin homology region (KKRR (SEQ ID NO:23)). In some embodiments, the domain is the phospholipid core binding domain (KVFKKIKDAGKWVRDKI (SEQ ID NO:20)). In some embodiments, the at least one domain comprises all four proline-rich domains (FPPPP (SEQ ID NO:21), FPPPP (SEQ ID NO:21), FPPPP (SEQ ID NO:21), FPPIP (SEQ ID NO:22)) of ActA. In some embodiments, the modified actA is actA-N100. In some embodiments, the polynucleotide is genomic. In some embodiments, the polynucleotide is not genomic. In some embodiments, the polynucleotide is operably linked with one or more of the following: (a) actA promoter; or (b) a bacterial (e.g., listerial) promoter that is not actA promoter. The disclosure further provides a *Listeria* bacterium (e.g., *Listeria monocytogenes)* comprising the polynucleotide, as well as vaccines comprising the *Listeria.* In some embodiments, the *Listeria* comprises an attenuating mutation in *actA* and/or *inlB.* In some embodiments, the *Listeria* is is hMeso26 or hMeso38 (see Table 11 of Example VII, below). The disclosure also provides methods for stimulating immune responses to an antigen from, or derived from, a cancer or infectious agent, comprising administering the *Listeria* to a mammal having the cancer or infectious agent, wherein the heterologous antigen shares at least one epitope with or is immunologically cross-reactive with the antigen from, or derived from, the cancer or infectious agent. In some embodiments, the stimulating is relative to immune response without administering the *Listeria.* In some embodiments, the cancer comprises a tumor or pre-cancerous cell. In some embodiments, the infectious agent comprises a virus, pathogenic bacterium, or parasitic organism. In some embodiments, the heterologous antigen is, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is, or is derived from, mesothelin. For instance, in some embodiments, the heterologous antigen is, or is derived from, human mesothelin. In some embodiments, inclusion of the modified Act A sequence in the polynucleotide enhances expression and/or secretion of the heterologous antigen in *Listeria.* In some embodiments, inclusion of the modified Act A sequence in the polynucleotide enhances the immunogenicity of vaccine compositions comprising the *Listeria.*

In still another aspect, this disclosure provides a plasmid comprising a first nucleic acid encoding a phage integrase, a second nucleic acid encoding a phage attachment site (attPP' site), and a third nucleic acid encoding a heterologous antigen or regulatory nucleic acid, wherein the plasmid is useful for mediating site-specific integration of the nucleic acid encoding the heterologous antigen at a bacterial attachment site (attBB' site) in a bacterial genome that is compatible with the attPP' site of the plasmid. In some embodiments, each of the nucleic acids is derivable from *L. innocua* 0071, each of the nucleic acids is derivable from *L. innocua* 1765, each of the nucleic acids is derivable from *L. innocua* 2601, or each of the nucleic acids is derivable from *L. monocytogenes* f6854_2703. In some embodiments, the first nucleic acid encodes a phiC31 integrase. In some embodiments, the plasmid is the polynucleotide sequence of pINT; or a polynucleotide hybridizable under stringent conditions to a polynucleotide encoding pINT, wherein the polynucleotide that is hybridizable is capable of mediating site specific integration at the same bacterial attachment site (attBB') in a bacterial genome as that used by pINT. In some embodiments, the bacterial genome is of a *Listeria, Bacillus anthracis,* or *Francisella tularensis.* In some embodiments, the heterologous antigen is, or is derived from, a cancer cell, tumor, or infectious agent. In some embodiments, the regulatory nucleic acid is a bacterial attachment site (attBB'). In some embodiments, the plasmid further comprises a fourth nucleic acid encoding a first lox site, a fifth nucleic acid encoding a second lox site, and a sixth nucleic acid encoding a selection marker, wherein the first lox site and second lox site are operably linked with the sixth nucleic acid, and wherein the operably linked lox sites are useful for mediating Cre recombinase catalyzed excision of the sixth nucleic acid. In some embodiments, the first lox site is a loxP site and the second lox site is a loxP site. In some embodiments, the plasmid further comprises a non compatible bacterial attachment site (attBB'), wherein the non compatible attBB' site is not compatible with the phage attachment site (attPP'). In some embodiments, the plasmid further comprises a first promoter operably linked with the first nucleic acid, and a second promoter operably linked with the third nucleic acid. This disclosure further provides a method of modifying a bacterial genome, comprising transfecting the bacterium with the plasmid, and allowing integrase-catalyzed integration of the third nucleic acid into the bacterial genome under conditions suitable for integration. In some embodiments of the method, the bacterium is *Listeria, Bacillus anthracis,* or *Francisella tularensis.*

This disclosure further provides a plasmid comprising: (a) a first nucleic acid encoding a first region of homology to a bacterial genome, (b) a second nucleic acid encoding a second region of homology to the bacterial genome, and (c) a third nucleic acid comprising a bacterial attachment site (attBB'), wherein the third nucleic acid is flanked by the first and second nucleic acids, wherein the first nucleic acid and second nucleic acid are operably linked with each other and able to mediate homologous integration of the third nucleic acid into the bacterial genome. In some embodiments, the bacterial attachment site (attBB') comprises the attBB' of: listerial tRNAArg-attBB'; listerial comK attBB'; *Listeria innocua* 0071; *Listeria innocua* 1231; *Listeria innocua* 1765; *Listeria innocua* 2610; or *Listeria monocytogenes* f6854_2703; or phiC31. In some embodiments, the genome is of a *Listeria, Bacillus anthracis,* or *Francisella tularensis.* In some embodiments, the third nucleic acid encodes a selection marker flanked by a first lox site and a second lox site, wherein the lox sites are recognized as substrates by Cre recombinase and allow Cre recombinase catalyzed excision of the third nucleic acid, and wherein the selection marker is useful for detecting integration of the third nucleic acid into the bacterial genome. In some embodiments, the first lox site is a loxP site, and the second lox site is a loxP site. In some embodiments, the third nucleic acid comprises an antibiotic resistance gene. In some embodiments, the first nucleic acid is homologous to a first region of a virulence factor gene and the second nucleic acid is homologous to a second region of the virulence factor gene, wherein the first and second regions of the virulence factor gene are distinct from each other and do not overlap each other. In some embodiments, the first region of the virulene factor gene covalently contacts or abuts the second region of the virulence factor gene. In other embodiments, the first region of the virulence factor gene is not in covalent contact with, and does not covalently about, the second region of the virulence factor gene. This disclosure further provides bacteria modified by integration of the plasmid. In some embodiments, the integration is in a region of the genome that is necessary for mediating growth or spread. In other embodiments, the integration is in a region of the genome that is not necessary for mediating growth or spread.

In yet another aspect, this disclosure provides a bacterium wherein the genome comprises a polynucleotide containing two operably linked heterologous recombinase binding sites flanking a first nucleic acid, wherein the two sites are: (a) two lox sites; or (b) two Frt sites, and wherein the nucleic acid flanked by the two lox sites is excisable by Cre recombinase, and wherein the nucleic acid flanked by the two Frt sites is excisable by FLP recombinase. In some embodiments, the two lox sites are both loxP sites. In some embodiments, the first nucleic acid encodes a selection marker or a heterologous antigen. In some embodiments, the first nucleic acid encodes an antibiotic resistance gene. In some embodiments, the bacterium is *Listeria, Bacillus anthracis,* or *Francisella tularensis.* In some embodiments, the polynucleotide further comprises a second nucleic acid, wherein the second nucleic acid is not flanked by, and is not operably linked with, the first and second heterologous recombinase binding site. In some embodiments, the second nucleic acid encodes one or both of: heterologous antigen; or a bacterial attachment site (attBB'). In some embodiments, the heterologous antigen is, or is derived from, a cancer cell, tumor, or infectious agent. This disclosure further provides a method of excising the first nucleic acid from the bacterial genome, comprising contacting the genome with Cre recombinase or FLP recombinase, and allowing the recombinase to catalyze excision of the first nucleic acid, under conditions allowing or facilitating excision: (a) wherein the first nucleic acid is flanked by lox sites and the recombinase is Cre recombinase; or (b) wherein the first nucleic acid is flanked by Frt sites and the recombinase is FLP recombinase. In some embodiments, the recombinase is transiently expressed in the bacterium.

In another aspect, this disclosure provides *Listeria* (e.g., *Listeria monocytogenes)* in which the genome comprises a polynucleotide comprising a nucleic acid encoding a heterologous antigen. In some embodiments, the nucleic acid encoding the heterologous antigen has been integrated into the genome by site-specific recombination or homologous recombination. In some embodiments, the site of integration into the genome is the tRNA^{Arg} locus. In some embodiments, the presence of the nucleic acid in the genome attenuates the *Listeria.* In some embodiments, the nucleic acid encoding the heterologous antigen has been integrated into the locus of a virulence gene. In some embodiments, the nucleic acid encoding the heterologous antigen has been integrated into the *actA* locus. In some embodiments, the nucleic acid encoding the heterologous antigen has been integrated into the *inlB* locus. In some embodiments, the genome of the *Listeria* comprises a first nucleic acid encoding a heterologous antigen that has been integrated into a first locus (e.g., the *actA* locus) and a second nucleic acid encoding a second heterologous antigen that has been integrated into a second locus (e.g., the inlB locus). The first and second heterologous antigens may be identical to each other or different. In some embodiments, the first and second heterologous antigens differ from each other, but are derived from the same tumor antigen or infectious agent antigen. In some embodiments, the first and second heterologous antigens are each a different fragment of an antigen derived from a cancer cell, tumor, or infectious agent. In some embodiments, the integrated nucleic acid encodes a fusion protein comprising a modified ActA and the heterologous antigen. In some embodiments, at least two, at least three, at least four, at least five, at least six, or at least seven nucleic acid sequences encoding heterologous antigens have been integrated into the Listerial genome.

In another aspect, this disclosure provides a *Listeria* bacterium comprising a genome, wherein the genome comprises a polynucleotide comprising a nucleic acid encoding a heterologous antigen, wherein the nucleic acid has been integrated into a virulence gene in the genome. In some embodiments, the *Listeria* is attenuated by disruption of expression of the virulence gene or disruption of a coding sequence of the virulence gene. In some embodiments, integration of the polynucleotide disrupts expression of the virulence gene or disrupts a coding sequence of the virulence gene. In some embodiments, all or part of the virulence gene has been deleted. In some embodiments, none of the virulence gene has been deleted. In some embodiments, the integration attenuates the *Listeria.* In some embodiments, the virulence gene is prfA-dependent. In other embodiments, the virulence gene is prfA-independent. In some embodiments, the virulence gene is necessary for mediated growth or spread of the bacterium. In some embodiments, the virulence gene is not necessary for growth and spread of the bacterium. In some embodiments, the virulence gene is *actA* or *inlB.* In some embodiments, the *Listeria* bacterium is *Listeria monocytogenes.* In some embodiments, the heterologous antigen is from, or is derived from, a cancer cell, tumor, or infectious agent. In some embodiments, the heterologous antigen is mesothelin (e.g., human mesothelin), or derived from mesothelin. In some embodiments, the nucleic acid encodes a fusion protein comprising a modified ActA and the heterologous antigen. In some embodiments, the bacterium comprises a second nucleic acid encoding a second heterologous antigen that has been integrated into a second virulence gene. This disclosure provides vaccines comprising the *Listeria* bacterium. This disclosure further provides a method for stimulating an immune response to the heterologous antigen in a mammal, comprising administering an effective amount of the *Listeria* bacterium, or an effective amount of a composition comprising the *Listeria* bacterium, to the mammal.

In still another aspect, this disclosure provides a method of producing a *Listeria* bacterium (e.g., an attenuated bacterium), comprising integrating a polynucleotide into a virulence gene in the genome of the *Listeria* bacterium, wherein the polynucleotide comprises a nucleic acid encoding a heterologous antigen. In some embodiments, the *Listeria* is attenuated by disruption of expression of the virulence gene or disruption of a coding sequence of the virulence gene. In some embodiments, the integration of the polynucleotide disrupts expression of the virulence gene or disrupts a coding sequence of the virulence gene. In some embodiments, the integration of the polynucleotide results in both (a) and (b). In some embodiments the method produces a *Listeria* bacterium for use in a vaccine. In some embodiments, the polynucleotide is integrated into the virulence gene by homologous recombination. In some embodiments, the polynucleotide is integrated via site-specific recombination. In some embodiments, all or part of the virulence gene is deleted during integration of the polynucleotide. In other embodiments, none of the virulence gene is deleted during the integration. In some embodiments, the virulence gene is *actA* or *inlB.* In some embodiments, the heterologous antigen is from, or is derived from, a cancer cell, tumor, or infectious agent. In some embodiments, the heterologous antigen is mesothelin (e.g., human mesothelin), or derived from mesothelin. In some embodiments, the nucleic acid encodes a fusion protein comprising a modified ActA and the heterologous antigen. This disclosure further provides a *Listeria* bacterium produced by the method, and vaccine compositions comprising the bacterium. This disclosure also provides a *Listeria* bacterium having the properties of a *Listeria* bacterium produced by the method, as well as vaccines comprising the bacterium. Methods for stimulating an immune response to the heterologous antigen in a mammal, comprising administering an effective amount of the *Listeria* bacterium, or an effective amount of a composition comprising the *Listeria* bacterium, are also provided.

In an additional aspect, this disclosure provides a *Listeria* bacterium comprising a genome, wherein the genome comprises a polynucleotide comprising a nucleic acid encoding a heterologous antigen, wherein the nucleic acid has been integrated into a gene necessary for mediating growth or spread. In some embodiments, integration of the polynucleotide attenuates the *Listeria* for growth or spread. In some embodiments, part or all of the gene has been deleted. In some embodiments, none of the gene has been deleted. In some embodiments, the gene is *actA.* In some embodiments, the *Listeria* bacterium is *Listeria monocytogenes.* In some embodiments, the heterologous antigen is from, or is derived from, a cancer cell, tumor, or infectious agent. In some embodiments, the heterologous antigen is mesothelin (e.g., human mesothelin), or derived from mesothelin. In some embodiments, the nucleic acid encodes a fusion protein comprising a modified ActA and the heterologous antigen. This disclosure provides vaccines comprising the *Listeria* bacterium. This disclosure further provides a method for stimulating an immune response to the heterologous antigen in a mammal, comprising administering an effective amount of the *Listeria* bacterium, or an effective amount of a composition comprising the *Listeria* bacterium, to the mammal.

In still another aspect, this disclosure provides a method of producing a *Listeria* bacterium (e.g., an attenuated bacterium), comprising integrating a polynucleotide into a gene in the genome of the *Listeria* bacterium that is necessary for mediating growth or spread, wherein the polynucleotide comprises a nucleic acid encoding a heterologous antigen. In some embodiments, the integration of the polynucleotide attenuates the *Listeria* for growth or spread. In some embodiments the method produces a *Listeria* bacterium for use in a vaccine. In some embodiments, the polynucleotide is integrated into the gene by homologous recombination. In some embodiments, the polynucleotide is integrated via site-specific recombination. In some embodiments, all or part of the gene necessary for mediating growth or spread is deleted during integration of the polynucleotide. In other embodiments, none of the gene is deleted during the integration. In some embodiments, the gene necessary for mediating growth or spread is *actA.* In some embodiments, the heterologous antigen is from, or is derived from, a cancer cell, tumor, or infectious agent. In some embodiments, the heterologous antigen is mesothelin (e.g., human mesothelin), or derived from mesothelin. In some embodiments, the nucleic acid encodes a fusion protein comprising a modified ActA and the heterologous antigen. This disclosure further provides a *Listeria* bacterium produced by the method, and vaccine compositions comprising the bacterium. This disclosure also provides a *Listeria* bacterium having the properties of a *Listeria* bacterium produced by the method, as well as vaccines comprising the bacterium. Methods for stimulating an immune response to the heterologous antigen in a mammal, comprising administering an effective amount of the *Listeria* bacterium, or an effective amount of a composition comprising the *Listeria* bacterium, are also provided.

In an additional aspect, this disclosure provides a *Listeria* bacterium comprising a genome, wherein the genome comprises a polynucleotide comprising a nucleic acid encoding a heterologous antigen, wherein the nucleic acid has been integrated into a virulence gene in the genome and the bacterium is attenuated by disruption of expression of the virulence gene or disruption of a coding sequence of the virulence gene. In some embodiments, all or part of the virulence gene has been deleted. In some embodiments, none of the virulence gene has been deleted. In some embodiments, the virulence gene is *actA* or *inlB.* In some embodiments, the *Listeria* is *Listeria* monocytogenes. In some embodiments, the heterologous antigen is from, or is derived from, a cancer cell, tumor, or infectious agent. In some embodiments, bacterium further comprises a second nucleic acid encoding a second heterologous antigen that has been integrated into a second virulence gene. In some embodiments, the nucleic acid encodes a fusion protein comprising a modified ActA and the heterologous antigen. Vaccines comprising the *Listeria* bacterium are further provided, as are methods for for stimulating an immune response to the heterologous antigen in a mammal, comprising administering an effective amount of the *Listeria* bacterium or an effective amount of a composition comprising the *Listeria* bacterium, to the mammal.

In still another aspect, this disclosure provides a method of producing a *Listeria* bacterium for use in a vaccine, comprising: integrating a polynucleotide into a virulence gene in the genome of the *Listeria* bacterium, wherein the polynucleotide comprises a nucleic acid encoding a heterologous antigen and wherein the bacterium is attenuated by disruption of the expression of the virulence gene or disruption of a coding sequence of the virulence gene. In some embodiments, the polynucleotide is integrated into the virulence gene by homologous recombination. In some embodiments, all or part of the virulence gene is deleted during integration of the polynucleotide into the virulence gene. In some embodiments, the virulence gene is *actA* or *inlB.* This disclosure further provides a *Listeria* bacterium produced by the method.

In another aspect, this disclosure provides a method of producing a *Listeria* bacterium for use in a vaccine, comprising integrating a polynucleotide into a virulence gene in the genome of the *Listeria* bacterium, wherein the polynucleotide comprises a nucleic acid encoding a heterologous antigen and wherein the *Listeria* bacterium is or has been attenuated by mutation of the virulence gene. This disclosure further provides a *Listeria* bacterium produced by this method.

In some embodiments of each of the aforementioned aspects, as well as other aspects described herein, the *Listeria* is attenuated with respect to cell-to-cell spread and/or entry into nonphagocytic cells. For instance, in some embodiments, the *Listeria* comprises an attenuating mutation in *actA* and/or *inlB.*

In some embodiments of each of the aforementioned aspects, as well as other aspects described herein, the polynucleotide and/or the nucleic acid encoding the fusion protein comprising the heterologous antigen has been integrated into a virulence gene in the genome.

In some embodiments of each of the aforementioned aspects, as well as other aspects described herein, the heterologous antigen is prostate stem cell antigen (PSCA) (e.g., human PSCA), or an antigen derived from PSCA.

In some embodiments of each of the aforementioned aspects, as well as other aspects described herein, the heterologous antigen is mesothelin, or an antigen derived from mesothelin.

In some embodiments of each of the aforementioned aspects, as well as other aspects described herein, the polynucleotide comprises two nucleic acid sequences, each of which encodes a fusion protein comprising a heterologous antigen. In further embodiments, the polynucleotide comprises three nucleic acids, each of which encodes a fusion protein comprising a heterologous antigen. In some embodiments, the polynucleotide comprises four or more nucleic acids, each of which encodes a fusion protein comprising a heterologous antigen.

In another aspect, this disclosure provides a *Listeria* bacterium comprising a genome, wherein the genome comprises: (a) a first polynucleotide comprising a first nucleic acid encoding a first heterologous antigen, wherein the first polynucleotide has been integrated into a virulence gene in the genome and wherein the polynucleotide comprises a nucleic acid encoding a heterologous antigen; and (b) a second polynucleotide comprising a second nucleic acid encoding a second heterologous antigen, wherein the second polynucleotide has also been integrated into the genome. In some embodiments, the bacterium is attenuated by disruption of the expression of the virulence gene or by disruption of a coding sequence of the virulence gene. In some embodiments, the integration of the first polynucleotide disrupts expression of the virulence gene or disrupts a coding sequence of the virulence gene. In some embodiments, all or part of the virulence gene has been deleted. In some other embodiments, none of the virulence gene has been deleted. In some embodiments, the virulence gene is *actA* or *inlB.* In some embodiments, the *Listeria* is *Listeria monocytogenes.* In some embodiments, the first and second heterologous antigens are from, or are derived from, a cancer cell, tumor, or infectious agent. In some embodiments, the first and/or second heterologous antigen is mesothelin, an antigen derived from mesothelin, PSCA, or an antigen derived from PSCA (e.g., the first heterologous antigen may be mesothelin, and the second heterologous antigen may be PSCA). In some embodiments, the second polynucleotide has been integrated into a virulence gene in the genome and the *Listeria* may be attenuated by disruption of the expression of the virulence gene or disruption of a coding sequence of the virulence gene. In some embodiments, the second polynucleotide has been integrated into *actA* or *inlB.* In some embodiments, the second polynucleotide has been integrated into a region of the genome that does not comprise a virulence gene. In some embodiments, the second polynucleotide has been integrated into a tRNA^{Arg} locus. In some embodiments, the first and/or second nucleic acid encodes a fusion protein comprising a modified ActA (e.g., ActA-N100) and the first or second heterologous antigen. In some embodiments, the first and/or second nucleic acid encodes a fusion protein comprising a signal sequence and the first or second heterologous antigen, wherein the signal sequence is an ActA signal sequence, p60 signal sequence, an LLO signal sequence, a BaPa signal sequence, a Llusp45 signal sequence, or a PhoD signal sequence. Vaccines comprising the *Listeria* bacterium are further provided. Methods of stimulating an immune response to the heterologous antigen in a mammal, comprising administering an effective amount of the *Listeria* bacterium, or an effective amount of a composition comprising the *Listeria* bacterium, to the mammal are also provided. In some embodiments, the genome of the *Listeria* further comprises a third polynucleotide comprising a third nucleic acid encoding a third heterologous antigen, wherein the third polynucleotide has also been integrated into the genome.

In some embodiments of each of the aforementioned aspects, as well as other aspects described herein, the polynucleotides comprising the nucleic acid(s) encoding a heterologous antigen(s) or the fusion protein(s) comprising a heterologous antigen that are integrated into a region of the genome further comprise a promoter and/or other regulatory sequences necessary for expression of the nucleic acids once integrated. In some alternative embodiments, the polynucleotides comprising the nucleic acids encoding the heterologous antigen or the fusion protein comprising the heterologous antigen are integrated into a region of the genome such that the nucleic acid is operably linked to a promoter and/or secretory signal already present in the *Listeria* genome. In some embodiments, the polynucleotides that are inserted in virulence genes are inserted in coding regions. In some other embodiments, the polynucleotides are inserted into non-coding regions of the virulence genes.

In some embodiments of each of the aforementioned aspects, as well as other aspects described herein, polynucleotides integrated into the *Listeria* genome are integrated via homologous recombination. In some alternative embodiments, the polynucleotides are integrated into the *Listeria* genome via site-specific integration.

In some embodiments, this disclosure provides a *Listeria* bacterium containing a polynucleotide comprising a first nucleic acid encoding a fusion protein partner, operably linked and in frame with and a second nucleic acid encoding human mesothelin, or a derivative thereof. The first nucleic acid can encode, e.g., LLO62 (non-codon optimized); LLO26 (codon *optimized);* LLO441 (non-codon optimized); LLO441 (codon optimized); full length LLO (non-codon optimized); full length LLO (codon optimized); BaPA secretory sequence; *B. subtilis* phoD secretory sequence (Bs phoD SS); p60 (non-codon optimized); p60 (codon optimized); actA (non-codon optimized); actA (codon optimized); actA-N100 (non-codon optimized); actA-N100 (codon optimized); actA (A30R). The second nucleic acid can encode full length human mesothelin; human mesothelin deleted in its signal sequence; human mesothelin deleted in its GPI anchor; or human mesothelin deleted in both the signal sequence and the GPI anchor, where codon-optimized and non-codon optimized versions of mesothelin are provided. In another aspect, the present invention provides the above polynucleotide integrated at the position of the *inlB* gene, *actA* gene, *hly* gene, where integration can be mediated by homologous recombination, and where integration can optionally be with operable linking with the promoter of the *inlB, actA,* or *hly* gene. In yet another aspect, this disclosure provides listerial embodiments where the above polynucleotide is integrated into the listerial genome by way of site-specific integration, e.g., at the tRNA^{Arg} site. Each of the individual embodiments disclosed herein, optionally, encompasses a *Listeria* comprising a constitutively active pfrA gene (prfA*). The listerial constructs are not limited to polynucleotides operably linked with an actA promoter or hly promoter. What is also encompassed is operable linkages with other bacterial promoters, synthetic promoters, bacteriovirus promoters, and combinations of two or more promoters.

In some embodiments, the heterologous antigen encoded by a nucleic acid in the polynucleotides, *Listeria* bacteria, and/or vaccines described above, or elsewhere herein, does not comprise an EphA2 antigenic peptide. In some embodiments, the heterologous antigen encoded by a nucleic acid in the polynucleotides, *Listeria* bacteria, and/or vaccines, does not comprise full-length EphA2 or an antigenic fragment, analog or derivative thereof.

### BRIEF DESCRIPTION OF THE DRAWINGS

Figure 1 discloses pINT, a 6055 bp plasmid. Once pINT is integrated in a listerial genome, the *Listeria* can be isolated by erythromycin resistance (ErmC), followed by treatment with Cre recombinase to remove a region of the plasmid encoding the antibiotic resistance genes (CAT and ErmC).
Figure 2 shows pKSV7, a 7096 plasmid that mediates homologous recombination.
Figure 3 shows steps, or intermediates, occurring with pKSV7-mediated homologous recombination into a bacterial genome.
Figure 4 discloses a method for preparing an insert bearing homologous arms, where the insert bearing the homologous arms is placed into pKSV7. The loxP-flanked region is bracketed by the homologous arms. After integration into a bacterial genome, transient exposure to Cre recombinase catalyzes removal of the antibiotic resistance gene. Integration occurs with deletion of part of the genome, corresponding to the region between areas matching the homologous arms.
Figure 5 shows an alternate method for preparing an insert bearing homologous arms, where the insert bearing homologous arms is placed into pKSV7. The loxP-flanked region resides outside the homologous arms. After integration into a bacterial genome, transient exposure to Cre recombinase catalyzes removal of the antibiotic resistance gene (or other selection marker). Integration occurs with deletion of part of the genome, corresponding to the region between areas matching the homologous arms.
Figure 6 discloses the preparation of an insert bearing homologous arms, where the insert bearing homologous arms is placed into pKSV7. The loxP-flanked region resides in between the homologous arms. In vectors prepared according to this figure, integration is not followed by deletion of any corresponding region of the genome.
Figure 7 is a schematic disclosing some of the mesothelin constructs of the present invention, including, e.g., any promoters, secretory sequences, fusion protein partners, and so on.
Figure 8 is a gel showing expression of mesothelin from various listerial constructs.
Figure 9 is a gel showing expression of mesothelin from a number of listerial constructs.
Figures 10-12 show expression of interferon-gamma (IFNgamma) from spot forming cell (SFC) assays, and compare immune responses where mice had been vaccinated with various numbers (colony forming units; c.f.u.) of engineered *L. monocytogenes.*
Figures 13 disclose numbers of tumor metastases on the surfaces of livers, after treating tumor-bearing mice with various preparations of recombinant *L. monocytogenes.* Figure 13 reveals the raw data (photographs of fixed livers).
Figure 14 also disclose numbers of tumor metastases on the surfaces of livers, after treatment of tumor-bearing mice with various preparations of recombinant *L. monocytogenes.*
Figure 15A-G further disclose numbers of tumor metastases on the surfaces of livers, after treating tumor-bearing mice with recombinant *L. monocytogenes.*
Figure 16 demonstrates increased survival to tumors by tumor-bearing mice with treatment with various preparations of recombinant *L. monocytogenes.*
Figure 17 illustrates mesothelin constructs and secretion of mesothelin by various preparations of recombinant *L. monocytogenes.*
Figure 18 discloses secretion of mesothelin and immune responses stimulated by various preparations of recombinant *L*. *monocytogenes.*
Figure 19 shows secretion of mesothelin and immune responses stimulated by various preparations of recombinant *L. monocytogenes.*
Figure 20 further reveals mesothelin expression and immune responses stimulated by various preparations of recombinant *L. monocytogenes.*
Figure 21 additionally illustrates secretion of mesothelin and immune responses stimulated by various preparations of recombinant *L. monocytogenes.*
Figure 22 demonstrates mesothelin expression and immune responses stimulated by various preparations of recombinant *L. monocytogenes.*
Figure 23 discloses immune responses stimulated by vaccination with various preparations of recombinant *Listeria.*
Figure 24 further discloses secretion of mesothelin and immune responses stimulated by various preparations of recombinant *L. monocytogenes.*
Figure 25 reveals immune responses stimulated after vaccination with a number of preparations of recombinant *Listeria.*
Figure 26 additionally discloses secretion of mesothelin and immune responses stimulated by various preparations of recombinant *L. monocytogenes.* hMeso6: *L. monocytogenes* ΔactAΔinIB encoding actA promoter; actA-N100-hMeso ΔSSAGPI; integrated at *actA* locus. hMeso25: *L. monocytogenes* ΔactAΔinlB encoding actA promoter; actA-N100-hMeso ΔSSAGPI; integrated at *inlB* locus.
Figure 27 further demonstrates secretion of mesothelin and immune responses stimulated by various preparations of recombinant *L. monocytogenes.*
Figure 28 shows photographs of fixed lungs.

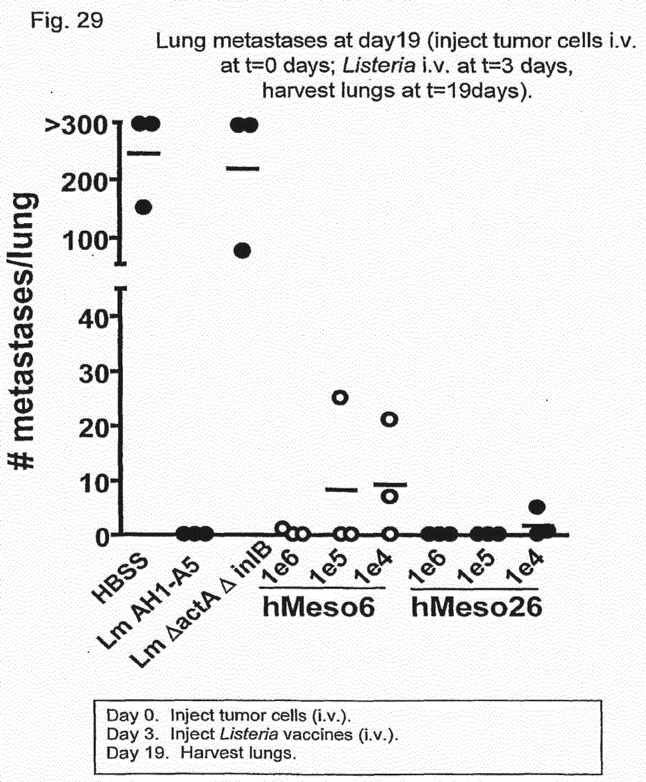

Figure 29 shows a histogram of data from the photographs of fixed lung.
Figure 30 reveals the effectiveness of various preparations of recombinant *Listeria* in improving survival of tumor-bearing mice.
Figure 31 discloses secretion of mesothelin and immune responses stimulated by various preparations of recombinant *L. monocytogenes.*
Figure 32 compares mesothelin expression from various preparations of recombinant *Listeria.*
Figure 33 depicts mesothelin secretion and immune responses stimulated after vaccination with recombinant *L. monocytogenes.*
Figure 34 demonstrates immune response stimulated after vaccination with the preparations and doses of recombinant *Listeria.*
Figures 35A and 35B disclose numbers of tumor metastases on livers, after treatment of tumor-bearing mice with various preparations of recombinant *L. monocytogenes.* Figure 35A illustrates raw data (photographs of fixed livers).
Figure 36 demonstrates the effectiveness of various preparations of recombinant *Listeria* in improving survival of tumor-bearing mice.
Figure 37 discloses immune response after vaccination with various preparations of recombinant *Listeria,* and compares CD4⁺ T cell and CD8⁺ T cell responses.
Figure 38 reveals survival of tumor-bearing mice to the tumors after vaccination with various preparations of recombinant *Listeria.*
Figure 39 further illustrates survival of tumor-bearing mice to the tumors after vaccination with various preparations of recombinant *Listeria.*
Figure 40 discloses alignment of a phage integrase of the present invention with a another phage integrase (U153 int: SEQ ID NO:1; lin 1231: SEQ ID NO:2).
Figure 41 discloses alignment of yet another phage integrase of the present invention another phage integrase (PSA int: SEQ ID NO:3; lin 0071: SEQ ID NO:4).
Figure 42 shows alignment of still another phage integrase of the present invention with a different phage integrase (PSA int: SEQ ID NO:5; lin 1765: SEQ ID NO:6).
Figure 43 discloses alignment of a further phage integrase of the present invention with another phage integrase (PSA int: SEQ ID NO:7; lin 2601: SEQ ID NO:8).
Figure 44 provides an alignment of an additional phage integrase of the present invention with a nucleic acid encoding another phage integrase (PSA int: SEQ ID NO:119; lmof6854_2703: SEQ ID NO:120).
Figure 45A and 45B discloses pINT-ActAN100-BamHI-SpeI-MfeI-SIINFEKL, a 6594 bp plasmid based on pINT (Fig. 1) and containing an ActA promoter, actA-N100 and a SIINFEKL sequence. Figure 45B shows the cloning region of pINT-ActAN100-BamHI-SpeI-MfeI-SIINFEKL. (Nucleic acid sequence is SEQ ID NO:150, Peptide sequence is SEQ ID NO:151)
Figure 46 discloses the schematic configuration of PSCA molecular constructs with Kyte-Doolittle overlay.
Figure 47A shows the results of dendritic cell presentation of peptides to the reporter cell line B3Z. Figure 47B is a Western blot analysis of expression and secretion of ActA-N100-PSCA fusion proteins in J774 cells.
Figure 48 demonstrates immune response stimulated after vaccination with recombinant *Listeria monocytogenes* expressing ActA-N100-PSCA fusion proteins.
Figure 49A and 49B disclose bivalent vaccine strains expressing two different human tumor antigens. Figure 49A discloses fusion molecules encoded by integrated constructs. Figure 49B discloses the expression and secretion of ActA fusion proteins following infection of J774 cells with monovalent or bivalent *Listeria monocytogenes* vaccines.
Figure 50 demonstrates immune response stimulated after vaccination with monovalent or divalent recombinant *Listeria monocytogenes* vaccines.

### DETAILED DESCRIPTION

As used herein, including the appended claims, the singular forms of words such as "a," "an," and "the," include their corresponding plural references unless the context clearly dictates otherwise.

### I. DEFINITIONS.

Abbreviations used to indicate a mutation in a gene, or a mutation in a bacterium comprising the gene, are as follows. By way of example, the abbreviation "*L. monocytogenes* ΔActA" means that part, or all, of the ActA gene was deleted. The delta symbol (Δ) means deletion. An abbreviation including a superscripted minus sign (*Listeria* ActA⁻) means that the ActA gene was mutated, e.g., by way of a deletion, point mutation, or frameshift mutation, but not limited to these types of mutations. Exponentials are abbreviated, where, for example, "3e7" means 3 x 10⁷.

"Administration" as it applies to a human, mammal, mammalian subject, animal, veterinary subject, placebo subject, research subject, experimental subject, cell, tissue, organ, or biological fluid, refers without limitation to contact of an exogenous ligand, reagent, placebo, small molecule, pharmaceutical agent, therapeutic agent, diagnostic agent, or composition to the subject, cell, tissue, organ, or biological fluid, and the like. "Administration" can refer, e.g., to therapeutic, pharmacokinetic, diagnostic, research, placebo, and experimental methods. Treatment of a cell encompasses contact of a reagent to the cell, as well as contact of a reagent to a fluid, where the fluid is in contact with the cell. "Administration" also encompasses *in vitro* and ex *vivo* treatments, e.g., of a cell, by a reagent, diagnostic, binding composition, or by another cell.

An "agonist," as it relates to a ligand and receptor, comprises a molecule, combination of molecules, a complex, or a combination of reagents, that stimulates the receptor. For example, an agonist of granulocyte-macrophage colony stimulating factor (GM-CSF) can encompass GM-CSF, a mutein or derivative of GM-CSF, a peptide mimetic of GM-CSF, a small molecule that mimics the biological function of GM-CSF, or an antibody that stimulates GM-CSF receptor. An antagonist, as it relates to a ligand and receptor, comprises a molecule, combination of molecules, or a complex, that inhibits, counteracts, downregulates, and/or desensitizes the receptor. "Antagonist" encompasses any reagent that inhibits a constitutive activity of the receptor. A constitutive activity is one that is manifest in the absence of a ligand/receptor interaction. "Antagonist" also encompasses any reagent that inhibits or prevents a stimulated (or regulated) activity of a receptor. By way of example, an antagonist of GM-CSF receptor includes, without implying any limitation, an antibody that binds to the ligand (GM-CSF) and prevents it from binding to the receptor, or an antibody that binds to the receptor and prevents the ligand from binding to the receptor, or where the antibody locks the receptor in an inactive conformation.

As used herein, an "analog" in the context of an EphA2 polypeptide (or a fragment of an EphA2 polypeptide) refers to a proteinaceous agent (e.g., a peptide, polypeptide or protein) that possesses a similar or identical function as the EphA2 polypeptide (or fragment of an EphA2 polypeptide), but does not necessarily comprise a similar or identical amino acid sequence or structure of the EphA2 polypeptide (or fragment). An analog of an EphA2 polypeptide that has a similar amino acid sequence to an EphA2 polypeptide refers to a proteinaceous agent that satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the amino acid sequence of an EphA2 polypeptide; (b) a proteinaceous agent encoded by a nucleotide sequence that hybridizes under stringent conditions to a nucleotide sequence encoding an EphA2 polypeptide of at least 20 amino acid residues, at least 30 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, or at least 150 amino acid residues; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding an EphA2 polypeptide. A proteinaceous agent with similar structure to an EphA2 polypeptide refers to a proteinaceous agent that has a similar secondary, tertiary or quaternary structure of the EphA2 polypeptide.

"Antigen presenting cells" (APCs) are cells of the immune system used for presenting antigen to T cells. APCs include dendritic cells, monocytes, macrophages, marginal zone Kupffer cells, microglia, Langerhans cells, T cells, and B cells (see, e.g., Rodriguez-Pinto and Moreno (2005) Eur. J. Immunol. 35:1097-1105). Dendritic cells occur in at least two lineages. The first lineage encompasses pre-DC1, myeloid DC1, and mature DC1. The second lineage encompasses CD34⁺⁺CD45RA⁻ early progenitor multipotent cells, CD34⁺⁺CD45RA ⁺ cells, CD34⁺⁺CD45RA⁺⁺CD4⁺ IL-3Ralpha⁺⁺ pro-DC2 cells, CD4⁺CD11c⁻plasmacytoid pre-DC2 cells, lymphoid human DC2 plasmacytoid-derived DC2s, and mature DC2s (see, e.g., Gilliet and Liu (2002) J. Exp. Med. 195:695-704; Bauer, et al. (2001) J. Immunol. 166:5000-5007; Arpinati, et al. (2000) Blood 95:2484-2490; Kadowaki, et al. (2001) J. Exp. Med. 194:863-869; Liu (2002) Human Immunology 63:1067-1071; McKenna, et al. (2005) J. Virol. 79:17-27; O'Neill, et al. (2004) Blood 104:2235-2246; Rossi and Young (2005) J. Immunol. 175:1373-1381; Banchereau and Palucka (2005) Nat. Rev. Immunol. 5:296-306).

"Attenuation" and "attenuated" encompasses a bacterium, virus, parasite, infectious organism, prion, tumor cell, gene in the infectious organism, and the like, that is modified to reduce toxicity to a host. The host can be a human or animal host, or an organ, tissue, or cell. The bacterium, to give a non-limiting example, can be attenuated to reduce binding to a host cell, to reduce spread from one host cell to another host cell, to reduce extracellular growth, or to reduce intracellular growth in a host cell. Attenuation can be assessed by measuring, e.g., an indicum or indicia of toxicity, the LD₅₀, the rate of clearance from an organ, or the competitive index (see, e.g., Auerbuch, et al. (2001) Infect. Immunity 69:5953-5957). Generally, an attenuation results an increase in the LD₅₀ and/or an increase in the rate of clearance by at least 25%; more generally by at least 50%; most generally by at least 100% (2-fold); normally by at least 5-fold; more normally by at least 10-fold; most normally by at least 50-fold; often by at least 100-fold; more often by at least 500-fold; and most often by at least 1000-fold; usually by at least 5000-fold; more usually by at least 10,000-fold; and most usually by at least 50,000-fold; and most often by at least 100,000-fold.

"Attenuated gene" encompasses a gene that mediates toxicity, pathology, or virulence, to a host, growth within the host, or survival within the host, where the gene is mutated in a way that mitigates, reduces, or eliminates the toxicity, pathology, or virulence. The reduction or elimination can be assessed by comparing the virulence or toxicity mediated by the mutated gene with that mediated by the non-mutated (or parent) gene. "Mutated gene" encompasses deletions, point mutations, and frameshift mutations in regulatory regions of the gene, coding regions of the gene, non-coding regions of the gene, or any combination thereof.

"Cancerous condition" and "cancerous disorder" encompass, without implying any limitation, a cancer, a tumor, metastasis, angiogenesis of a tumor, and precancerous disorders such as dysplasias.

"Conservatively modified variants" applies to both amino acid and nucleic acid sequences. With respect to particular nucleic acid sequences, a conservatively modified variant refers to nucleic acids encoding identical amino acid sequences, or amino acid sequences that have one or more conservative substitutions. An example of a conservative substitution is the exchange of an amino acid in one of the following groups for another amino acid of the same group (U.S. Pat. No. 5,767,063 issued to Lee, et al.; Kyte and Doolittle (1982) J. Mol. Biol. 157:105-132).
(1) Hydrophobic: Norleucine, Ile, Val, Leu, Phe, Cys, Met;
(2) Neutral hydrophilic: Cys, Ser, Thr;
(3) Acidic: Asp, Glu;
(4) Basic: Asn, Gln, His, Lys, Arg;
(5) Residues that influence chain orientation: Gly, Pro;
(6) Aromatic: Trp, Tyr, Phe; and
(7) Small amino acids: Gly, Ala, Ser.

A "derivative" in the context of an EphA2 polypeptide or a fragment of an EphA2 polypeptide refers to a proteinaceous agent that comprises an amino acid sequence of an EphA2 polypeptide or a fragment of an EphA2 polypeptide that has been altered by the introduction of amino acid residue substitutions, deletions or additions (i.e., mutations). The term "derivative" in the context of EphA2 proteinaceous agents also refers to an EphA2 polypeptide or a fragment of an EphA2 polypeptide which has been modified, i.e, by the covalent attachment of any type of molecule to the polypeptide. For example, but not by way of limitation, an EphA2 polypeptide or a fragment of an EphA2 polypeptide may be modified, e.g., by glycosylation, acetylation, pegylation, phosphorylation, amidation, derivatization by known protecting/blocking groups, proteolytic cleavage, linkage to a cellular ligand or other protein, etc. A derivative of an EphA2 polypeptide or a fragment of an EphA2 polypeptide may be modified by chemical modifications using techniques known to those of skill in the art, including, but not limited to, specific chemical cleavage, acetylation, formylation, metabolic synthesis of tunicamycin, etc. Further, a derivative of an EphA2 polypeptide or a fragment of an EphA2 polypeptide may contain one or more non-classical amino acids. In one embodiment, a polypeptide derivative possesses a similar or identical function as an EphA2 polypeptide or a fragment of an EphA2 polypeptide described herein. In another embodiment, a derivative of EphA2 polypeptide or a fragment of an EphA2 polypeptide has an altered activity when compared to an unaltered polypeptide. For example, a derivative of an EphA2 polypeptide or fragment thereof can differ in phosphorylation relative to an EphA2 polypeptide or fragment thereof.

"Effective amount" encompasses, without limitation, an amount that can ameliorate, reverse, mitigate, prevent, or diagnose a symptom or sign of a medical condition or disorder. Unless dictated otherwise, explicitly or by context, an "effective amount" is not limited to a minimal amount sufficient to ameliorate a condition.

"EphA2 antigenic peptides" (sometimes referred to as "EphA2 antigenic polypeptides"), are defined and described in U.S. Patent Publication No. 2005/0281783 A1. EphA2 is a 130 kDa receptor tyrosine kinase expressed in adult epithelia (Zantek et al. (1999) Cell Growth & Differentiation 10:629; Lindberg et al. (1990) Molecular & Cellular Biology 10:6316). An "EphA2 antigenic peptide" or an "EphA2 antigenic polypeptide" refers to an EphA2 polypeptide, or a fragment, analog or derivative thereof comprising one or more B cell epitopes or T cell epitopes of EphA2. The EphA2 polypeptide may be from any species. For example the EphA2 polypeptide may be a human EphA2 polypeptide. The term "EphA2 polypeptide" includes the mature, processed form of EphA2, as well as immature forms of EphA2. In some embodiments, the EphA2 polypeptide is the sequence shown as SEQ ID NO:2 of U.S. Patent Publication No. 2005/0281783 A1. Examples of the nucleotide sequence of human EphA2 can be found in the GenBank database (see, e.g., Accession Nos. BC037166, M59371 and M36395). Examples of the amino acid sequence of human EphA2 can also be found in the GenBank database (see, e.g., Accession Nos. NP-004422, AAH37166, and AAA53375). Additional examples of amino acid sequences of EphA2 include those listed as GenBank Accession Nos. NP_034269 (mouse), AAH06954 (mouse), XP_345597 (rat), and BAB63910 (chicken).

An "extracellular fluid" encompasses, e.g., serum, plasma, blood, interstitial fluid, cerebrospinal fluid, secreted fluids, lymph, bile, sweat, fecal matter, and urine. An "extracelluar fluid" can comprise a colloid or a suspension, e.g., whole blood or coagulated blood.

The term "fragments" in the context of EphA2 polypeptides include an EphA2 antigenic peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, at least 200 contiguous amino acid residues, or at least 250 contiguous amino acid residues of the amino acid sequence of an EphA2 polypeptide.

"Gene" refers to a nucleic acid sequence encoding an oligopeptide or polypeptide. The oligopeptide or polypeptide can be biologically active, antigenically active, biologically inactive, or antigenically inactive, and the like. The term gene encompasses, e.g., the sum of the open reading frames (ORFs) encoding a specific oligopeptide or polypeptide; the sum of the ORFs plus the nucleic acids encoding introns; the sum of the ORFs and the operably linked promoter(s); the sum of the ORFS and the operably linked promoter(s) and any introns; the sum of the ORFS and the operably linked promoter(s), intron(s), and promoter(s), and other regulatory elements, such as enhancer(s). In certain embodiments, "gene" encompasses any sequences required in cis for regulating expression of the gene. The term gene can also refer to a nucleic acid that encodes a peptide encompassing an antigen or an antigenically active fragment of a peptide, oligopeptide, polypeptide, or protein. The term gene does not necessarily imply that the encoded peptide or protein has any biological activity, or even that the peptide or protein is antigenically active. A nucleic acid sequence encoding a non-expressable sequence is generally considered a pseudogene. The term gene also encompasses nucleic acid sequences encoding a ribonucleic acid such as rRNA, tRNA, or a ribozyme.

"Growth" of a *Listeria* bacterium encompasses, without limitation, functions of bacterial physiology and genes relating to colonization, replication, increase in listerial protein content, increase in listerial lipid content. Unless specified otherwise explicitly or by context, growth of a *Listeria* encompasses growth of the bacterium outside a host cell, and also growth inside a host cell. Growth related genes include, without implying any limitation, those that mediate energy production (e.g., glycolysis, Krebs cycle, cytochromes), anabolism and/or catabolism of amino acids, sugars, lipids, minerals, purines, and pyrimidines, nutrient transport, transcription, translation, and/or replication. In some embodiments, "growth" of a *Listeria* bacterium refers to intracellular growth of the *Listeria* bacterium, that is, growth inside a host cell such as a mammalian cell. While intracellular growth of a *Listeria* bacterium can be measured by light microscopy or colony forming unit (CFU) assays, growth is not to be limited by any technique of measurement. Biochemical parameters such as the quantity of a listerial antigen, listerial nucleic acid sequence, or lipid specific to the *Listeria* bacterium, can be used to assess growth. In some embodiments, a gene that mediates growth is one that specifically mediates intracellular growth. In some embodiments, a gene that specifically mediates intracellular growth encompasses, but is not limited to, a gene where inactivation of the gene reduces the rate of intracellular growth but does not detectably, substantially, or appreciably, reduce the rate of extracellular growth (e.g., growth in broth), or a gene where inactivation of the gene reduces the rate of intracellular growth to a greater extent than it reduces the rate of extracellular growth. To provide a non-limiting example, in some embodiments, a gene where inactivation reduces the rate of intracellular growth to a greater extent than extracellular growth encompasses the situation where inactivation reduces intracellular growth to less than 50% the normal or maximal value, but reduces extracellular growth to only 1-5%, 5-10%, or 10-15% the maximal value. This disclosure, in certain aspects, encompasses a *Listeria* attenuated in intracellular growth but not attenuated in extracellular growth, a *Listeria* not attenuated in intracellular growth and not attenuated in extracellular growth, as well as a *Listeria* not attenuated in intracellular growth but attenuated in extracellular growth.

"Immune condition" or "immune disorder" encompasses a disorder, condition, syndrome, or disease resulting from ineffective, inappropriate, or pathological response of the immune system, e.g., to a persistent infection or to a persistent cancer (see, e.g., Jacobson, et al. (1997) Clin. Immunol. Immunopathol. 84:223-243). "Immune condition" or "immune disorder" encompasses, e.g., pathological inflammation, an inflammatory disorder, and an autoimmune disorder or disease. "Immune condition" or "immune disorder" also can refer to infections, persistent infections, cancer, tumors, precancerous disorders, cancers that resist irradication by the immune system, and angiogenesis of tumors. "Immune condition" or "immune disorder" also encompasses cancers induced by an infective agent, including the non-limiting examples of cancers induced by hepatitis B virus, hepatitis C virus, simian virus 40 (SV40), Epstein-Barr virus, papillomaviruses, polyomaviruses, Kaposi's sarcoma herpesvirus, human T-cell leukemia virus, and *Helicobacter pylori* (see, e.g., Young and Rickinson (2004) Nat. Rev. Cancer 4:757-768; Pagano, et αl. (2004) Semin. Cancer Biol. 14:453-471; Li, et al. (2005) Cell Res. 15:262-271).

A composition that is "labeled" is detectable, either directly or indirectly, by spectroscopic, photochemical, biochemical, immunochemical, isotopic, or chemical methods. For example, useful labels include ³²P, ³³P, ³⁵S, ¹⁴C, ³H, ¹²⁵I, stable isotopes, epitope tags, fluorescent dyes, electron-dense reagents, substrates, or enzymes, e.g., as used in enzyme-linked immunoassays, or fluorettes (see, e.g., Rozinov and Nolan (1998) Chem. Biol. 5:713-728).

"Ligand" refers to a small molecule, peptide, polypeptide, or membrane associated or membrane-bound molecule, that is an agonist or antagonist of a receptor. "Ligand" also encompasses a binding agent that is not an agonist or antagonist, and has no agonist or antagonist properties. By convention, where a ligand is membrane-bound on a first cell, the receptor usually occurs on a second cell. The second cell may have the same identity (the same name), or it may have a different identity (a different name), as the first cell. A ligand or receptor may be entirely intracellular, that is, it may reside in the cytosol, nucleus, or in some other intracellular compartment. The ligand or receptor may change its location, e.g., from an intracellular compartment to the outer face of the plasma membrane. The complex of a ligand and receptor is termed a "ligand receptor complex." Where a ligand and receptor are involved in a signaling pathway, the ligand occurs at an upstream position and the receptor occurs at a downstream position of the signaling pathway.

"Nucleic acid" refers to deoxyribonucleotides or ribonucleotides and polymers thereof in either single stranded, double-stranded form, or multi-stranded form. Non-limiting examples of a nucleic acid are a, e.g., cDNA, mRNA, oligonucleotide, and polynucleotide. A particular nucleic acid sequence can also implicitly encompasses "allelic variants" and "splice variants."

"Operably linked" in the context of a promoter and a nucleic acid encoding a mRNA means that the promoter can be used to initiate transcription of that nucleic acid.

The terms "percent sequence identity" and "% sequence identity" refer to the percentage of sequence similarity found by a comparison or alignment of two or more amino acid or nucleic acid sequences. Percent identity can be determined by a direct comparison of the sequence information between two molecules by aligning the sequences, counting the exact number of matches between the two aligned sequences, dividing by the length of the shorter sequence, and multiplying the result by 100. An algorithm for calculating percent identity is the Smith-Waterman homology search algorithm (see, e.g., Kann and Goldstein (2002) Proteins 48:367-376; Arslan, et al. (2001) Bioinformatics 17:327-337).

"Precancerous condition" encompasses, without limitation, dysplasias, preneoplastic nodules; macroregenerative nodules (MRN); low-grade dysplastic nodules (LG-DN); high-grade dysplastic nodules (HG-DN); biliary epithelial dysplasia; foci of altered hepatocytes (FAH); nodules of altered hepatocytes (NAH); chromosomal imbalances; aberrant activation of telomerase; re-expression of the catalytic subunit of telomerase; expression of endothelial cell markers such as CD31, CD34, and BNH9 (see, e.g., Terracciano and Tornillo (2003) Pathologica 95:71-82; Su and Bannasch (2003) Toxicol. Pathol. 31:126-133; Rocken and Carl-McGrath (2001) Dig. Dis. 19:269-278; Kotoula, et al. (2002) Liver 22:57-69; Frachon, et al. (2001) J. Hepatol. 34:850-857; Shimonishi, et al. (2000) J. Hepatobiliary Pancreat. Surg. 7:542-550; Nakanuma, et al. (2003) J. Hepatobiliary Pancreat. Surg. 10:265-281). Methods for diagnosing cancer and dysplasia are disclosed (see, e.g., Riegler (1996) Semin. Gastrointest. Dis. 7:74-87; Benvegnu, et al. (1992) Liver 12:80-83; Giannini, et al. (1987) Hepatogastroenterol. 34:95-97; Anthony (1976) Cancer Res. 36:2579-2583).

By "purified" and "isolated" is meant, when referring to a polypeptide, that the polypeptide is present in the substantial absence of the other biological macromolecules with which it is associated in nature. The term "purified" as used herein means that an identified polypeptide often accounts for at least 50%, more often accounts for at least 60%, typically accounts for at least 70%, more typically accounts for at least 75%, most typically accounts for at least 80%, usually accounts for at least 85%, more usually accounts for at least 90%, most usually accounts for at least 95%, and conventionally accounts for at least 98% by weight, or greater, of the polypeptides present. The weights of water, buffers, salts, detergents, reductants, protease inhibitors, stabilizers (including an added protein such as albumin), and excipients, and molecules having a molecular weight of less than 1000, are generally not used in the determination of polypeptide purity. See, e.g., discussion of purity in U.S. Pat. No. 6,090,611 issued to Covacci, et al.

"Peptide" refers to a short sequence of amino acids, where the amino acids are connected to each other by peptide bonds. A peptide may occur free or bound to another moiety, such as a macromolecule, lipid, oligo- or polysaccharide, and/or a polypeptide. Where a peptide is incorporated into a polypeptide chain, the term "peptide" may still be used to refer specifically to the short sequence of amino acids. A "peptide" may be connected to another moiety by way of a peptide bond or some other type of linkage. A peptide is at least two amino acids in length and generally less than about 25 amino acids in length, where the maximal length is a function of custom or context. The terms "peptide" and "oligopeptide" may be used interchangeably.

"Protein" generally refers to the sequence of amino acids comprising a polypeptide chain. Protein may also refer to a three dimensional structure of the polypeptide. "Denatured protein" refers to a partially denatured polypeptide, having some residual three dimensional structure or, alternatively, to an essentially random three dimensional structure, i.e., totally denatured. This disclosure encompasses reagents of, and methods using, polypeptide variants, e.g., involving glycosylation, phosphorylation, sulfation, disulfide bond formation, deamidation, isomerization, cleavage points in signal or leader sequence processing, covalent and non-covalently bound cofactors, oxidized variants, and the like. The formation of disulfide linked proteins is described (see, e.g., Woycechowsky and Raines (2000) Curr. Opin. Chem. Biol. 4:533-539; Creighton, et al. (1995) Trends Biotechnol. 13:18-23).

"Recombinant" when used with reference, e.g., to a nucleic acid, cell, animal, virus, plasmid, vector, or the like, indicates modification by the introduction of an exogenous, non-native nucleic acid, alteration of a native nucleic acid, or by derivation in whole or in part from a recombinant nucleic acid, cell, virus, plasmid, or vector. Recombinant protein refers to a protein derived, e.g., from a recombinant nucleic acid, virus, plasmid, vector, or the like. "Recombinant bacterium" encompasses a bacterium where the genome is engineered by recombinant methods, e.g., by way of a mutation, deletion, insertion, and/or a rearrangement. "Recombinant bacterium" also encompasses a bacterium modified to include a recombinant extra-genomic nucleic acid, e.g., a plasmid or a second chromosome, or a bacterium where an existing extra-genomic nucleic acid is altered.

"Sample" refers to a sample from a human, animal, placebo, or research sample, e.g., a cell, tissue, organ, fluid, gas, aerosol, slurry, colloid, or coagulated material. The "sample" may be tested *in vivo,* e.g., without removal from the human or animal, or it may be tested *in vitro.* The sample may be tested after processing, e.g., by histological methods. "Sample" also refers, e.g., to a cell comprising a fluid or tissue sample or a cell separated from a fluid or tissue sample. "Sample" may also refer to a cell, tissue, organ, or fluid that is freshly taken from a human or animal, or to a cell, tissue, organ, or fluid that is processed or stored.

A "selectable marker" encompasses a nucleic acid that allows one to select for or against a cell that contains the selectable marker. Examples of selectable markers include, without limitation, e.g.: (1) A nucleic acid encoding a product providing resistance to an otherwise toxic compound (e.g., an antibiotic), or encoding susceptibility to an otherwise harmless compound (e.g., sucrose); (2) A nucleic acid encoding a product that is otherwise lacking in the recipient cell (e.g., tRNA genes, auxotrophic markers); (3) A nucleic acid encoding a product that suppresses an activity of a gene product; (4) A nuclei c acid that encodes a product that can be readily identified (e.g., phenotypic markers such as beta-galactosidase, green fluorescent protein (GFP), cell surface proteins, an epitope tag, a FLAG tag); (5) A nucleic acid that can be identified by hybridization techniques, for example, PCR or molecular beacons.

"Specifically" or "selectively" binds, when referring to a ligand/receptor, nucleic acid/complementary nucleic acid, antibody/antigen, or other binding pair (e.g., a cytokine to a cytokine receptor) indicates a binding reaction which is determinative of the presence of the protein in a heterogeneous population of proteins and other biologics. Thus, under designated conditions, a specified ligand binds to a particular receptor and does not bind in a significant amount to other proteins present in the sample. Specific binding can also mean, e.g., that the binding compound, nucleic acid ligand, antibody, or binding composition derived from the antigen-binding site of an antibody, of the contemplated method binds to its target with an affinity that is often at least 25% greater, more often at least 50% greater, most often at least 100% (2-fold) greater, normally at least ten times greater, more normally at least 20-times greater, and most normally at least 100-times greater than the affinity with any other binding compound.

In a typical embodiment an antibody will have an affinity that is greater than about 10⁹ liters/mol, as determined, e.g., by Scatchard analysis (Munsen, et al. (1980) Analyt. Biochem. 107:220-239). It is recognized by the skilled artisan that some binding compounds can specifically bind to more than one target, e.g., an antibody specifically binds to its antigen, to lectins by way of the antibody's oligosaccharide, and/or to an Fc receptor by way of the antibody's Fc region.

"Spread" of a bacterium encompasses "cell to cell spread," that is, transmission of the bacterium from a first host cell to a second host cell, as mediated, for example, by a vesicle. Functions relating to spread include, but are not limited to, e.g., formation of an actin tail, formation of a pseudopod-like extension, and formation of a double-membraned vacuole.

The "target site" of a recombinase is the nucleic acid sequence or region that is recognized, bound, and/or acted upon by the recombinase (see, e.g., U.S. Pat. No. 6,379,943 issued to Graham, et al.*;* Smith and Thorpe (2002) Mol. Microbiol. 44:299-307; Groth and Calos (2004) J. Mol. Biol. 335:667-678; Nunes-Duby, et al. (1998) Nucleic Acids Res. 26:391-406).

"Therapeutically effective amount" is defined as an amount of a reagent or pharmaceutical composition that is sufficient to show a patient benefit, i.e., to cause a decrease, prevention, or amelioration of the symptoms of the condition being treated. When the agent or pharmaceutical composition comprises a diagnostic agent, a "diagnostically effective amount" is defined as an amount that is sufficient to produce a signal, image, or other diagnostic parameter. Effective amounts of the pharmaceutical formulation will vary according to factors such as the degree of susceptibility of the individual, the age, gender, and weight of the individual, and idiosyncratic responses of the individual (see, e.g., U.S. Pat. No. 5,888,530 issued to Netti, et al.).

"Treatment" or "treating" (with respect to a condition or a disease) is an approach for obtaining beneficial or desired results including and preferably clinical results. For purposes of this invention, beneficial or desired results with respect to a disease include, but are not limited to, one or more of the following: improving a condition associated with a disease, curing a disease, lessening severity of a disease, delaying progression of a disease, alleviating one or more symptoms associated with a disease, increasing the quality of life of one suffering from a disease, and/or prolonging survival. Likewise, for purposes of this invention, beneficial or desired results with respect to a condition include, but are not limited to, one or more of the following: improving a condition, curing a condition, lessening severity of a condition, delaying progression of a condition, alleviating one or more symptoms associated with a condition, increasing the quality of life of one suffering from a condition, and/or prolonging survival. For instance, in some embodiments where the compositions described herein are used for treatment of cancer, the beneficial or desired results include, but are not limited to, one or more of the following: reducing the proliferation of (or destroying) neoplastic or cancerous cells, reducing metastasis of neoplastic cells found in cancers, shrinking the size of a tumor, decreasing symptoms resulting from the cancer, increasing the quality of life of those suffering from the cancer, decreasing the dose of other medications required to treat the disease, delaying the progression of the cancer, and/or prolonging survival of patients having cancer. Depending on the context, "treatment" of a subject can imply that the subject is in need of treatment, e.g., in the situation where the subject comprises a disorder expected to be ameliorated by administration of a reagent.

"Vaccine" encompasses preventative vaccines. Vaccine also encompasses therapeutic vaccines, e.g., a vaccine administered to a mammal that comprises a condition or disorder associated with the antigen or epitope provided by the vaccine.

### II. GENERAL.

The present invention provides reagents and methods useful for the treatment and diagnosis of cancer, tumors, precancerous disorders, and infections. Provided are nucleic acids, *Listeria* bacteria, and vaccines comprising a *Listeria* bacterium. This disclosure encompasses listerial cells that have been modified *in vitro,* including during storage, or *in vivo,* including products of bacterial cell division and products of bacterial deterioration.

Provided are nucleic acids encoding at least one heterologous antigen (heterologous to the *Listeria* bacterium). The heterologous antigen can be derived from a tumor, cancer cell, or and/or infective agent, e.g., a virus, bacterium, or protozoan. The heterologous antigen can also be a listerial antigen, for example, where the antigen is expressed in greater amounts than that which naturally occurs within the *Listeria* bacterium, where the listerial antigen is operably linked with a non-native regulatory sequence, or where the listerial antigen is modified to be attenuated or to increase its antigenicity.

Where a *Listeria* contains a nucleic acid encoding a heterologous antigen, the term "heterologous" encompasses, but is not necessarily limited to, an antigen from, or derived from: (1) A non-listerial organism; (2) An antigen of synthetic origin; (3) An antigen of listerial origin where the nucleic acid is integrated at a position in the listerial genome that is different from that found in the wild type; and (4) An antigen of listerial origin, but where the nucleic acid is operably linked with a regulatory sequence not normally used in a wild type *Listeria.* The preceding commentary also applies to the term "heterologous antigen," when used, for example, in the context of a viral vector. Here, heterologous antigen encompasses antigens that are not from, and not derived from, that viral vector, as well as, for example, antigens from the viral vector that are controlled by a non-native nucleic acid regulatory sequence.

Provided are reagents and methods for stimulating the mammalian immune system, for reducing the number and/or size of tumors, for reducing metastasis, and for reducing titer of an infectious organism. The present invention also provides reagents and methods for improving survival of a cell, tissue, organ, or mammal, to a cancer or infection. The present invention also provides reagents and methods for improving survival of a cell (*in vivo* or *in vitro*), a tissue (*in vivo* or *in vitro*), an organ (*in vivo* or *in vitro*), an organism, a mammal, a veterinary subject, a research subject, or a human subject, to a cancer, tumor, or infection. What is encompassed is administration that is *in vivo* or *in vitro,* survival of the cell, tissue, or organ *in vitro* or *in vivo,* or any combination thereof. Any combination includes, e.g., administration that is *in vivo* where subsequent survival is *in vitro,* or administration that is *in vitro* and where subsequent survival is *in vivo.*

Provided is a *Listeria* comprising a polynucleotide encoding at least one heterologous antigen wherein the one polynucleotide is genomic. Also encompassed is a *Listeria* comprising a polynucleotide encoding at least one heterologous antigen, wherein the polynucleotide is genomic and not residing on a plasmid within the *Listeria.* Moreover, encompassed is a *Listeria* comprising a polynucleotide encoding at least one heterologous antigen, wherein the polynucleotide resides on a plasmid within the *Listeria.* Furthermore, what is provided is a *Listeria* comprising a polynucleotide encoding at least one heterologous antigen, where the polynucleotide resides on a plasmid and does not occur integrated in the genome. In another aspect, the present disclosure provides a *Listeria* comprising a polynucleotide encoding at least one heterologous antigen, where the polynucleotide is integrated in the genome and also separately resides in a plasmid.

The mouse is an accepted model for human immune response. In detail, mouse T cells are a model for human T cells, mouse dendritic cells (DCs) are a model for human DCs, mouse NK cells are a model for human NK cells, mouse NKT cells are a model for human NKT cells, mouse innate response is an accepted model for human innate response, and so on. Model studies are disclosed, for example, for CD8⁺ T cells, central memory T cells, and effector memory T cells (see, e.g., Walzer, et al. (2002) J. Immunol. 168:2704-2711); the two subsets of NK cells (see, e.g., Chakir, et al. (2000) J. Immunol. 165:4985-4993; Smith, et al. (2000) J. Exp. Med. 191:1341-1354; Ehrlich, et al. (2005) J. Immunol. 174:1922-1931; Peritt, et al. (1998) J. Immunol. 161:5821-5824); NKT cells (see, e.g., Couedel, et al. (1998) Eur. J. Immunol. 28:4391-4397; Sakamoto, et al. (1999) J. Allergy Clin. Immunol. 103:S445-S451; Saikh, et al. (2003) J. Infect. Dis. 188:1562-1570; Emoto, et al. (1997) Infection Immunity 65:5003-5009; Taniguchi, et al. (2003) Annu. Rev. Immunol. 21:483-513; Sidobre, et al. (2004) Proc. Natl. Acad. Sci. 101:12254-12259); monocytes/macrophages (Sunderkotter, et al. (2004) J. Immunol. 172:4410-4417); the two lineages of DCs (Boonstra, et al. (2003) J. Exp. Med. 197:101-109; Donnenberg, et al. (2001) Transplantation 72:1946-1951; Becker (2003) Virus Genes 26:119-130; Carine, et al. (2003) J. Immunol. 171:6466-6477; Penna, et al, (2002) J. Immunol. 169:6673-6676; Alferink, et al. (2003) J. Exp. Med. 197:585-599).

Mouse innate response, including the Toll-Like Receptors (TLRs), is a model for human innate immune response, as disclosed (see, e.g., Janssens and Beyaert (2003) Clinical Microb. Revs. 16:637-646). Mouse neutrophils are an accepted model for human neutrophils (see, e.g., Kobayashi, et al. (2003) Proc. Natl. Acad. Sci. USA 100:10948-10953; Torres, *et al.* (2004) 72:2131-2139; Sibelius, et al. (1999) Infection Immunity 67:1125-1130; Tvinnereim, et al. (2004) J. Immunol. 173:1994-2002). Murine immune response to *Listeria* is an accepted model for human response to *Listeria* (see, e.g., Kolb-Maurer, et al. (2000) Infection Immunity 68:3680-3688; Brzoza, et al. (2004) J. Immunol. 173:2641-2651; Esplugues, et al. (2005) Blood Feb. 3 (epub ahead of print); Paschen, et al. (2000) Eur. J. Immunol. 30:3447-3456; Way and Wilson (2004) J. Immunol. 173:5918-5922; Ouadrhiri, et al. (1999) J. Infectious Diseases 180:1195-1204; Neighbors, et al. (2001) J. Exp. Med. 194:343-354; Calorini, et al. (2002) Clin. Exp. Metastasis 19:259-264; Andersson, et al. (1998) J. Immunol. 161:5600-5606; Flo, et al. (2000) J. Immunol. 164:2064-2069; Calorini, et al. (2002) Clin. Exp. Metastasis 19:259-264; Brzoza, et al. (2004) J. Immunol. 173:2641-2651; Brzoza, et al. (2004) J. Immunol. 173:2641-2651; Cleveland, et al. (1996) Infection Immunity 64:1906-1912; Andersson, et al. (1998) J. Immunol. 161:5600-5606).

U.S. Patent Publication Nos. 2004/0228877 and 2004/0197343, each of which is incorporated by reference herein in its entirety, describe *Listeria* useful in some embodiments of the present invention. U.S. Patent Publication No. 2005/0249748, incorporated by reference herein in its entirety, further describes *Listeria* and polynucleotides useful in some embodiments of the present invention.

### (a). Secretory or signal sequences.

The present invention embraces a nucleic acid encoding a secretory sequence, or encoding a listerial protein, or a fragment thereof, suitable for use as a fusion protein partner. What is encompassed is a nucleic acid encoding:
i. a secretory sequence,
ii. a signal sequence,
iii. a listerial polypeptide containing its native secretory sequence,
iv. a listerial protein with its native secretory sequence replaced with that of another listerial protein,
v. a listerial protein with its native secretory sequence replaced with the secretory sequence of a non-listerial bacterial protein,
vi. a non-secreted listerial protein, or fragment thereof, not containing any secretory sequence; and
vii. a non-listerial bacterial secretory sequence fused with, and in frame with,
a non-secreted listerial protein, or fragment thereof.

These embodiments can encompass the following listerial proteins, and fragments or domains thereof:
i. Listeriolysin (LLO). The secretory signal sequence of listeriolysin O (hly gene) has been identified (see, e.g., Lety, et al. (2003) Microbiol. 149:1249-1255).
ii. ActA. The ribosomal binding site, promoter, and signal sequence have been identified for listerial ActA. The ribosomal binding site occurs 6 bp upstream of the start codon of the ActA gene (Vazquez-Boland, et al. (1992) Infect. Immunity 60:219-230).
iii. Internalins. All of the internalin (Inl) proteins contain an N-terminal sequence of 30-35 amino acids with characteristics of bacterial signal peptides (see, e.g., Dramsi, et al. (1997) Infect. Immunity 65:1615-1625).
iv. p60 (iap gene). A 27-amino acid region between the start codon and nucleotide 524 functions as a signal sequence, and directs transport of p60 across the *Listeria* cell membrane (Kohler, et al. (1990) Infect. Immunity 58:1943-1950). Kohler, *et al., supra,* also disclose a purine-rich ribosome (16S RNA) binding site of the p60 mRNA of *L. monocytogenes.*

Table 1 discloses a number of non-limiting examples of signal peptides for use in fusing with a fusion protein partner sequence such as a heterologous antigen. The SignalP algorithm can be used to determine signal sequences in Gram positive bacteria. This program is available on the world wide web at: cbs.dtu.dk/services/SignalP/. Signal peptides tend to contain three domains: a positively charged N-terminus (1-5 residues long); a central hydrophobic comain (7-15 residues long); and a neutral but polar C-terminal domain (see, e.g., Lety, et al. (2003) Microbiology 149:1249-1255; Paetzel, et al. (2000) Pharmacol. Ther. 87:27-49). As signal peptides and secretory seqeuences encoded by a *Listeria* genome, or by a genome or plasmid of another bacterium, are not necessarily codon optimized for optimal expression in *Listeria*, the present disclosure also provides nucleic acids originating from the *Listeria* genome, or from a genome or plasmid of another bacterium, that are altered by codon optimized for expressing by a *L. monocytogenes.* The present disclosure is not to be limited to polypeptide and peptide antigens that are secreted, but also embraces polypeptides and peptides that are not secreted or cannot be secreted from a *Listeria* or other bacterium.

**Table 1. Bacterial signal pathway. Signal peptides are identified by the signal peptidase site.**

| Signal peptidase site (cleavage site represented by') | Gene | Genus/species |
|---|---|---|
| **secA1 pathway** | | |
| TEA'KD (SEQ ID NO: 126) | *hly* (LLO) | *Listeria monocytogenes* |
| VYA'DT (SEQ ID NO:127) | Usp45 | *Lactococcus lactis* (see, e.g., Steidler, et al. (2003) Nat. Biotech. 21:785-789; Schotte, et al. (2000) Enzyme Microb. Technol. 27:761-765). |
| IQA'EV (SEQ ID NO:128) | *pag* (protective antigen) | *Bacillus anthracis* |

| **secA2 pathway** | | |
|---|---|---|
| ASA'ST (SEQ ID NO:129) | *iap* (invasion-associated protein) p60 | *Listeria monocytogenes* |
| VGA'FG (SEQ ID NO:130) | NamA lmo2691 (autolysin) | *Listeria monocytogenes* |
| AFA'ED (SEQ ID NO:131) | * BA_0281 (NLP/P60 Family) | *Bacillus anthracis* |
| VQA'AE (SEQ ID NO:132) | * *atl* (autolysin) | *Staphylococcus aureus* |

| **Tat pathway** | | |
|---|---|---|
| DKA'LT (SEQ ID NO:133) | lmo0367 | *Listeria monocytogenes* |
| VGA'FG (SEQ ID NO:134) | PhoD (alkaline phosphatase) | *Bacillus subtillis* |
| * Bacterial autolysins secreted by sec pathway (not determined whether secA1 or secA2). | | |
| Secretory sequences are encompassed by the indicated nucleic acids encoded by the *Listeria* EGD genome (GenBank Acc. No. NC_ 003210) at, e.g., nucleotides 45434-456936 (inlA); nucleotides 457021-457125 (inlB); nucleotides 1860200-1860295 (inlC); nucleotides 286219-287718 (inlE); nucleotides 205819-205893 (hly gene; LLO) (see also GenBank Acc. No. P13128); nucleotides 209470-209556 (ActA) (see also GenBank Acc. No. S20887). | | |

In some embodiments, the polypeptide comprising the heterologous antigen that is expressed by the *Listeria* comprises a signal sequence which is a non-listerial signal sequence. In some embodiments, the signal sequence is a secA1 signal peptide. In some other embodiments, the signal sequence is a secA2 or Tat signal peptide. In some embodiments, the signal peptide is an actA signal peptide. In some embodiments, the signal peptide used to effect secretion of the heterologous antigen is selected from p60 signal sequence, a *Listeria monocytogenes* LLO signal sequence, a *Bacillus anthracis* Protective Antigen (BaPa) signal sequence, a *Lactococcus lactis* usp45 signal sequence, and a *Bacillus subtilis* PhoD signal sequence. Signal peptides that can be used for the expression and secretion of heterologous antigens are described in, e.g., U.S. Patent Publication No. 2005/0249748.

### (b). Codon optimization.

The present disclosure, in certain embodiments, provides codon optimization of a nucleic acid heterologous to *Listeria,* or of a nucleic acid endogenous to *Listeria.* The optimal codons utilized by *L. monocytogenes* for each amino acid are shown (Table 2). A nucleic acid is codon-optimized if at least one codon in the nucleic acid is replaced with a codon that is more frequently used by *L. monocytogenes* for that amino acid than the codon in the original sequence.

Normally, at least one percent of any non-optimal codons are changed to provide optimal codons, more normally at least five percent are changed, most normally at least ten percent are changed, often at least 20% are changed, more often at least 30% are changed, most often at least 40%, usually at least 50% are changed, more usually at least 60% are changed, most usually at least 70% are changed, optimally at least 80% are changed, more optimally at least 90% are changed, most optimally at least 95% are changed, and conventionally 100% of any non-optimal codons are codon-optimized for *Listeria* expression (Table 2).

**Table 2. Optimal codons for expression in Listeria.**

| | | | | | | | | | | |
|---|---|---|---|---|---|---|---|---|---|---|
| Amino Acid | A | R | N | D | C | Q | E | G | H | I |
| Optimal *Listeria* codon | GCA | CGU | AAU | GAU | UGU | CAA | GAA | GGU | CAU | AUU |
| Amino Acid | L | K | M | F | P | S | T | W | Y | V |
| Optimal *Listeria* codon | UUA | AAA | AUG | UUU | CCA | AGU | ACA | UGG | UAU | GUU |

In some embodiments, the nucleic acid encoding the heterologous antigen is codon-optimized for expression in *Listeria monocytogenes*. In some embodiments, the polynucleotide encoding the fusion protein comprising the modified ActA or an alternative signal peptide sequence and the heterologous antigen is codon-optimized.

Additional information regarding the codon-optimization of nucleotide sequences for expression in *Listeria monocytogenes* can be found in U.S. Patent Publication No. 2005/0249748.

### (c). Virulence factors and attenuation.

*L. monocytogenes* expresses various genes and gene products that contribute to invasion, growth, or colonization of the host (Table 3). Some of these are classed as "virulence factors." These virulence factors include ActA, listeriolysin (LLO), protein 60 (p60), internalin A (inlA), internalin B (inlB), phosphatidylcholine phospholipase C (PC-PLC), phosphatidylinositol-specific phospholipase C (PI-PLC; plcA gene). A number of other internalins have been characterized, e.g., InlC2, InlD, InlE, and InlF (Dramsi, et al. (1997) Infect. Immunity 65:1615-1625). Mpl, a metalloprotease that processes proPL-PLC to active PL-PLC, is also a virulence factor (Chakraborty, et al. (2000) Int. J. Med. Microbiol. 290:167-174; Williams, et al. (2000) J. Bact. 182:837-841). In some embodiments, a virulence gene is a gene that encodes a virulence factor. Without limiting the present disclosure to the attenuated genes disclosed herein, the present disclosure supplies a *Listeria* that is altered, mutated, or attenuated in one or more of the sequences of Table 3.

In some embodiments, the virulence gene is a prf-A dependent gene. In other embodiments, the virulence gene is a prf-A independent gene.

In some embodiments, the *Listeria* comprises an attenuating mutation in *actA* and/or *inlB.* In some embodiments, a polynucleotide encoding a heterologous antigen has been integrated into the *actA* and/or *inlB* gene.

DNA repair genes can also be the target of an attenuating mutation. Mutating or deleting a DNA repair gene can result in an attenuated bacterium (see, e.g., Darwin and Nathan (2005) Infection Immunity 73:4581-4587).

**Table 3. Sequences of L. monocytogenes nucleic acids and proteins.**

| Protein/Gene | Nucleotides | GenBank Acc. No. |
|---|---|---|
| Actin assembly inducing protein precursor (ActA gene) | 209470-211389 (coding sequence) 209456-211389 (gene) | NC_003210 |
| ActA in various *L. monocytogenes* subtypes. | -- | AF497169; AF497170; AF497171; AF497172; AF497173; AF497174; AF497175; AF497176; AF497177; AF497178; AF497179; AF497180; AF497181; AF497182; AF497183 (Lasa, *et al.* (1995) Mol. Microbiol. 18:425-436). |
| Listeriolysin O precursor (LLO) (hly gene) | 205819-207408 | NC_003210 |
| Internalin A (InlA) | 454534-456936 | NC_003210 |
| Internalin B (inlB) | 457021-458913 | NC_003210 |
| SvpA | --- | Bierne, et al. (2004) J. Bacteriol. 186:1972-1982; Borezee, et al. (2000) Microbiology 147:2913-2923. |
| pl104 (a.k.a. LAP) | | Pandiripally, et al. (1999) J. Med. Microbiol. 48:117-124; Jaradat, et al. (2003) Med. Microbiol. Immunol. 192:85-91. |
| Phosphatidylinositol-specific phospholipase C(PI-PLC) (plcA gene) | 204624-205577 | NC_003210 |
| Phosphatidylcholine-specific phospholipase C(PC-PLC) (plcB gene) | 1-3031 | X59723 |
| Zinc metalloprotease precursor (Mpl) | 20773 9-209271 | NC_003210 |
| p60 (protein 60; invasion associated protein (iap)). | Complement of 618932-620380 | NC_003210 (Lenz, et al. (2003) Proc. Natl. Acad. Sci. USA 100:12432-12437). |
| Sortase | 966245-966913 | NC_003210 |
| Listeriolysin positive regulatory protein (PrfA gene) | 203607-203642 | NC_003210 |
| Listeriolysin positive regulatory protein (PrfA gene) | 1-801 | AY318750 |
| PrfB gene | 2586114-2587097 | NC_003210 |
| FbpA gene | 570 amino acids | Dramsi, et al. (2004) Mol. Microbiol. 53:639-649. |
| Auto gene | -- | Cabanes, et al. (2004) Mol. Microbiol. 51:1601-1614. |
| Ami (amidase that mediates adhesion) | -- | Dussurget, et al. (2004) Annu. Rev. Microbiol. 58:587-610. |
| dlt operon (dltA; dltB; dltC; dltD). | 487-2034 (dltA) | GenBank Acc. No: AJ012255 (Abachin, et al. (2002) Mol. Microbiol. 43:1-14.) |
| prfA boxes | -- | Dussurget, et al. (2002) Mol. Microbiol. 45:1095-1106. |
| Htp (sugar-P transporter) | 1-1386 | GenBank Acc. No. AJ315765 (see, e.g., Milohanic, et al. (2003) Mol. Microbiol. 47:1613-1625). |

Listeriolysin (LLO) biology is described (see, e.g., Glomski, et al. (2003) Infect. Immun. 71:6754-6765; Gedde, et al. (2000) Infect. Immun. 68:999-1003; Glomski, et al. (2002) J. Cell Biol. 156:1029-1038; Dubail, et al. (2001) Microbiol. 147:2679-2688; Dramsi and Cosssart (2002) J. Cell Biol. 156:943-946). ActA biochemistry and physiology is disclosed (see, e.g., Machner, et al. (2001) J. Biol. Chem. 276:40096-40103; Lauer, et al. (2001) Mol. Microbiol. 42:1163-1177; Portnoy, et al. (2002) J. Cell Biol. 158:409-414). Internalin biochemistry and physiology is available (see, e.g., Bierne and Cossart (2000) J. Cell Sci. 115:3357-3367; Schluter, et al. (1998) Infect. Immun. 66:5930-5938; Dormann, et al. (1997) Infect. Immun. 65:101-109). Sortase proteins are described (see, e.g., Bierne, et al. (2002) Mol. Microbiol. 43:869-881). Two phospholipases, PI-PLC (encoded by plcA gene) and PC-PLC (encoded by plcB gene) are disclosed (see, e.g., Camilli, et al. (1993) Mol. Microbiol. 8:143-157; Schulter, et al. (1998) Infect. Immun. 66:5930-5938). Protein p60 is described (Pilgrim, et al. (2003) Infect. Immun. 71:3473-3484).

This disclosure also contemplates a *Listeria* attenuated in at least one regulatory factor, e.g., a promoter or a transcription factor. The following concerns promoters. ActA expression is regulated by two different promoters (Lauer, et al. (2002) J. Bacteriol. 184:4177-4186). Together, inlA and inlB are regulated by five promoters (Lingnau, et al. (1995) Infect. Immun. 63:3896-3903). The transcription factor prfA is required for transcription of a number of *L. monocytogenes* genes, e.g., hly, plcA, ActA, mpl, prfA, and iap. PrfA's regulatory properties are mediated by, e.g., the PrfA-dependent promoter (PinlC) and the PrfA-box. The present disclosure, in certain embodiments, provides a nucleic acid encoding inactivated, mutated, or deleted in at least one of ActA promoter, inlB promoter, PrfA, PinlC, PrfA-box, and the like (see, e.g., Lalic-Mullthaler, et al. (2001) Mol. Microbiol. 42:111-120; Shetron-Rama, et al. (2003) Mol. Microbiol. 48:1537-1551; Luo, et al. (2004) Mol. Microbiol. 52:39-52). PrfA can be made constitutively active by a Glyl45Ser mutation, Glyl55Ser mutation, or Glu77Lys mutation (see, e.g., Mueller and Freitag (2005) Infect. Immun. 73:1917-1926; Wong and Freitag (2004) J. Bacteriol. 186:6265-6276; Ripio, et al. (1997) J. Bacteriol. 179:1533-1540).

Attenuation can be effected by, e.g., heat-treatment or chemical modification. Attenuation can also be effected by genetic modification of a nucleic acid that modulates, e.g., metabolism, extracellular growth, or intracellular growth, genetic modification of a nucleic acid encoding a virulence factor, such as *listeria*l prfA, ActA, listeriolysin (LLO), an adhesion mediating factor (e.g., an internalin such as inlA or inlB), mpl, phosphatidylcholine phospholipase C (PC-PLC), phosphatidylinositol-specific phospholipase C (PI-PLC; plcA gene), any combination of the above, and the like. Attenuation can be assessed by comparing a biological function of an attenuated *Listeria* with the corresponding biological function shown by an appropriate parent *Listeria.*

The present disclosure, in other embodiments, provides a *Listeria* that is attenuated by treating with a nucleic acid targeting agent, such as a cross-linking agent, a psoralen, a nitrogen mustard, cis-platin, a bulky adduct, ultraviolet light, gamma irradiation, any combination thereof, and the like. Typically, the lesion produced by one molecule of cross-linking agent involves cross-linking of both strands of the double helix. The *Listeria* of this disclosure can also be attenuated by mutating at least one nucleic acid repair gene, e.g., uvrA, uvrB, uvrAB, uvrC, uvrD, uvrAB, phrA, and/or a gene mediating recombinational repair, e.g., recA. Moreover, this disclosure provides a *Listeria* attenuated by both a nucleic acid targeting agent and by mutating a nucleic acid repair gene. Additionally, this disclosure encompasses treating with a light sensitive nucleic acid targeting agent, such as a psoralen, and/or a light sensitive nucleic acid cross-linking agent, such as psoralen, followed by exposure to ultraviolet light.

In some embodiments, the *Listeria* of this disclosure are attenuated. Attenuated *Listeria* useful in the present invention are described in, e.g., in U.S. Pat. Publ. Nos. 2004/0228877 and 2004/0197343. Various assays for assessing whether a particular strain of *Listeria* has the desired attenuation are provided, e.g., in U.S. Pat. Publ. Nos. 2004/0228877, 2004/0197343, and 2005/0249748.

### (d). Listeria strains.

This disclosure supplies a number of listerial species and strains for making or engineering an attenuated *Listeria* of the present invention (Table 4). The *Listeria* of the present invention is not to be limited by the species and strains disclosed in this table.

**Table 4. Strains of Listeria suitable for use in the present invention, e.g., as a vaccine or as a source of nucleic acids.**

| | |
|---|---|
| *L. monocytogenes* 10403S wild type. | Bishop and Hinrichs (1987) J. Immunol. 139:2005-2009; Lauer, et al. (2002) J. Bact. 184:4177-4186. |
| *L. monocytogenes* DP-L4056 (phage cured). The prophage-cured 10403S strain is designated DP-L4056. | Lauer, et al. (2002) J. Bact. 184:4177-4186. |
| *L. monocytogenes* DP-L4027, which is DP-L2161, phage cured, deleted in hly gene. | Lauer, et al. (2002) J. Bact. 184:4177-4186; Jones and Portnoy (1994) Infect. Immunity 65:5608-5613. |
| *L. monocytogenes* DP-L4029, which is DP-L3078, phage cured, deleted in ActA. | Lauer, et al. (2002) J. Bact. 184:4177-4186; Skoble, et al. (2000) J. Cell Biol. 150:527-538. |
| *L. monocytogenes* DP-L4042 (delta PEST) | Brockstedt, et al. (2004) Proc. Natl. Acad. Sci. USA 101:13832-13837; supporting information. |
| *L. monocytogenes* DP-L4097 (LLO-S44A). | Brockstedt, et al. (2004) Proc. Natl. Acad. Sci. USA 101:13832-13837; supporting information. |
| *L. monocytogenes* DP-L4364 (delta lplA; lipoate protein ligase). | Brockstedt, et al. (2004) Proc. Natl. Acad. Sci. USA 101:13832-13837; supporting information. |
| *L. monocytogenes* DP-L4405 (delta inlA). | Brockstedt, et al. (2004) Proc. Natl. Acad. Sci. USA 101:13832-13837; supporting information. |
| *L. monocytogenes* DP-L4406 (delta inlB). | Brockstedt, et al. (2004) Proc. Natl. Acad. Sci. USA 101:13832-13837; supporting information. |
| *L. monocytogenes* CS-L0001 (delta ActA-delta inlB). | Brockstedt, et al. (2004) Proc. Natl. Acad. Sci. USA 101:13832-13837; supporting information. |
| *L. monocytogenes* CS-L0002 (delta ActA-delta lplA). | Brockstedt, et al. (2004) Proc. Natl. Acad. Sci. USA 101:13832-13837; supporting information. |
| *L. monocytogenes* CS-L0003 (L461T-delta lplA). | Brockstedt, et al. (2004) Proc. Natl. Acad. Sci. USA 101:13832-13837; supporting information. |
| *L. monocytogenes* DP-L4038 (delta ActA-LLO L461T). | Brockstedt, et al. (2004) Proc. Natl. Acad. Sci. USA 101:13832-13837; supporting information. |
| *L. monocytogenes* DP-L4384 (S44A-LLO L461T). | Brockstedt, et al. (2004) Proc. Natl. Acad. Sci. USA 101:13832-13837; supporting information. |
| *L. monocytogenes.* Mutation in lipoate protein lipase (LplA1). | O'Riordan, et al. (2003) Science 302:462-464. |
| *L. monocytogenes* DP-L4017 (10403S hly (L461T) point mutation in hemolysin gene. | U.S. Provisional Pat. Appl. Ser. No. 60/490,089 filed July 24, 2003. |
| *L. monocytogenes* EGD. | GenBank Acc. NO. AL591824. |
| *L. monocytogenes* EGD-e. | GenBank Acc. No. NC_003210. ATCC Acc. No. BAA-679. |
| *L. monocytogenes* strain EGD, complete genome, segment 3/12 | GenBank Acc. No. AL591975 |
| *L. monocytogenes.* | ATCC Nos. 13932; 15313; 19111-19120; 43248-43251; 51772-51782. |
| *L. monocytogenes* DP-L4029 deleted in *uvr*AB. | U.S. Provisional Pat. Appl. Ser. No. 60/541,515 filed February 2, 2004; U.S. Provisional Pat. Ser. No. 60/490,080 filed 24, |
| *L. monocytogenes* DP-L4029 deleted in *uvr*AB treated with a psoralen. | U.S. Provisional Pat. Appl. Ser. No. 60/541,515 filed February 2, 2004. |
| *L. monocytogenes* ActA-/inlB-double mutant. | Deposited with ATCC on October 3, 2003. Acc. No. PTA-5562. |
| *L. monocytogenes* lplA mutant or hly mutant. | U.S. Pat. Applic. No. 20040013690 of Portnoy, et al. |
| *L. monocytogenes* DAL/DAT double mutant. | U.S. Pat. Applic. No. 20050048081 of Frankel and Portnoy. |
| *L. monocytogenes* str. 4b F2365. | GenBank Acc. No. NC_ 002973. |
| *Listeria ivanovii* | ATCC No. 49954 |
| *Listeria innocua* Clip11262. | GenBank Acc. No. NC_003212; ALS92022. |
| *Listeria innocua*, a naturally occurring hemolytic strain containing the PrfA-regulated virulence gene cluster. | Johnson, et al. (2004) Appl. Environ. Microbiol. 70:4256-4266. |
| *Listeria seeligeri.* | Howard, et al. (1992) Appl. Eviron. Microbiol. 58:709-712. |
| *Listeria innocua* with *L. monocytogenes* pathogenicity island genes. | Johnson, et al. (2004) Appl. Environ. Microbiol. 70:4256-4266. |
| *Listeria innocua* with *L. monocytogenes* internalin A gene, e.g., as a plasmid or as a genomic nucleic acid. | See, e.g., Lingnau, et al. (1995) Infection Immunity 63:3896-3903; Gaillard, et al. (1991) Cell 65:1127-1141). |
| The present invention encompasses reagents and methods that comprise the above listerial strains, as well as these strains that are modified, e.g., by a plasmid and/or by genomic integration, to contain a nucleic acid encoding one of, or any combination of, the following genes: hly (LLO; listeriolysin); iap (p60); inlA; inlB; inlC; dal (alanine racemase); daaA (dat; D-amino acid aminotransferase); plcA; plcB; ActA; or any nucleic acid that mediates growth, spread, breakdown of a single walled vesicle, breakdown of a double walled vesicle, binding to a host cell, uptake by a host cell. The present invention is not to be limited by the particular strains disclosed above. | |

### (e). Antigens.

The present disclosure, in certain embodiments, provides a nucleic acid encoding at least one antigen, an antigen with one or more conservative changes, one or more epitopes from a specified antigen, or a peptide or polypeptide that is immunologically cross-reactive with an antigen (Table 5). The nucleic acids and antigens of this disclosure are not to be limited to those disclosed in the table.

**Table 5. Antigens.**

| Antigen | Reference |
|---|---|
| **Tumor antigens** | |
| Mesothelin | GenBank Acc. No. NM_005823; U40434; NM_013404; BC003512 (see also, e.g., Hassan, et al. (2004) Clin. Cancer Res. 10:3937-3942; Muminova, et al. (2004) BMC Cancer 4:19; Iacobuzio-Donahue, et al. (2003) Cancer Res. 63:8614-8622). |
| Wilms' tumor-1 associated protein (Wt-1), including isoform A; isoform B; isoform C; isoform D. | WT-1 isoform A (GenBank Acc. Nos. NM_000378; NP_000369). WT-1 isoform B (GenBank Acc. Nos. NM_024424; NP_077742). WT-1 isoform C (GenBank Acc. Nos. NM_024425; NP_077743). WT-1 isoform D (GenBank Acc. Nos. NM_024426; NP_077744). |
| Stratum corneum chymotryptic enzyme (SCCE), and variants thereof. | GenBank Acc. No. NM_005046; NM_139277; AF332583. See also, e.g., Bondurant, et al. (2005) Clin. Cancer Res. 11:3446-3454; Santin, et al. (2004) Gynecol. Oncol. 94:283-288; Shigemasa, et al. (2001) Int. J. Gynecol. Cancer 11:454-461; Sepehr, et al. (2001) Oncogene 20:7368-7374. |
| MHC class I chain-related protein A (MICA); MHC class I chain-related protein A (MICB). | See, e.g., Groh, et al. (2005) Proc. Natl. Acad. Sci. USA 102:6461-6466; GenBank Acc. Nos. NM_000247; BC_016929; AY750850; NM_005931. |
| Gastrin and peptides derived from gastrin; gastrin/CCK-2 receptor (also known as CCK-B). | Harris, et al. (2004) Cancer Res. 64:5624-5631; Gilliam, et al. (2004) Eur. J. Surg. Oncol. 30:536-543; Laheru and Jaffee (2005) Nature Reviews Cancer 5:459-467. |
| Glypican-3 (an antigen of, e.g., hepatocellular carcinoma and melanoma). | GenBank Acc. No. NM_004484. Nakatsura, et al. (2003) Biochem. Biophys. Res. Commun. 306:16-25; Capurro, et al. (2003) Gasteroenterol. 125:89-97; Nakatsura, et al. (2004) Clin. Cancer Res. 10:6612-6621). |
| Coactosin-like protein. | Nakatsura, et al. (2002) Eur. J. Immunol. 32:826-836; Laheru and Jaffee (2005) Nature Reviews Cancer 5:459-467. |
| Prostate stem cell antigen (PSCA). | GenBank Acc. No. AF043498; AR026974; AR302232 (see also, e.g., Argani, et al. (2001) Cancer Res. 61:4320-4324; Christiansen, et al. (2003) Prostate 55:9-19; Fuessel, *et al.* (2003) 23:221-228). |
| Prostate acid phosphatase (PAP); prostate-specific antigen (PSA); PSM; PSMA. | Small, et al. (2000) J. Clin. Oncol. 18:3894-3903; Altwein and Luboldt (1999) Urol. Int. 63:62-71; Chan, et al. (1999) Prostate 41:99-109; Ito, et al. (2005) Cancer 103:242-250; Schmittgen, et al. (2003) Int. J. Cancer 107:323-329; Millon, et al. (1999) Eur. Urol. 36:278-285. |
| Six-transmembrane epithelial antigen of prostate (STEAP). | See, e.g., Machlenkin, et al. (2005) Cancer Res. 65:6435-6442; GenBank Acc. No. NM_018234; NM_001008410; NM_182915; NM_024636; NM_012449; BC011802. |
| Prostate carcinoma tumor antigen-1 (PCTA-1). | See, e.g., Machlenkin, et al. (2005) Cancer Res. 65:6435-6442; GenBank Acc. No. L78132. |
| Prostate tumor-inducing gene-1 (PTI-1). | See, e.g., Machlenkin, et al. (2005) Cancer Res. 65:6435-6442). |
| Prostate-specific gene with homology to G protein-coupled | receptor. See, e.g., Machlenkin, et al. (2005) Cancer Res. 65:6435-6442). |
| Prostase (an antrogen regulated serine protease). | See, e.g., Machlenkin, et al. (2005) Cancer Res. 65:6435-6442; GenBank Acc. No. BC096178; BC096176; BC096175. |
| Proteinase 3. | GenBank Acc. No. X55668. |
| Cancer-testis antigens, e.g., NY-ESO-1; SCP-1; SSX-1; SSX-2; SSX-4; GAGE, CT7; CT8; CT10; MAGE-1; MAGE-2; MAGE-3; MAGE-4; MAGE-6; LAGE-1. | GenBank Acc. No. NM_001327 (NY-ESO-1) (see also, e.g., Li, et al. (2005) Clin. Cancer Res. 11:1809-1814; Chen, et al. (2004) Proc. Natl. Acad. Sci. USA. 101(25):9363-9368; Kubuschok, et al. (2004) Int. J. Cancer. 109:568-575; Scanlan, et al. (2004) Cancer Immun. 4:1; Scanlan, et al. (2002) Cancer Res. 62:4041-4047; Scanlan, et al. (2000) Cancer Lett. 150:155-164; Dalerba, et al. (2001) Int. J. Cancer 93:85-90; Ries, et al. (2005) Int. J. Oncol. 26:817-824. |
| MAGE-A1, MAGE-A2; MAGE-A3; MAGE-A4; MAGE-A6; MAGE-A9; MAGE-A 10; MAGE-A12; GAGE-3/6; NT-SAR-35; BAGE; CA125. | Otte, et al. (2001) Cancer Res. 61:6682-6687; Lee, et al. (2003) Proc. Natl. Acad. Sci. USA 100:2651-2656; Sarcevic, et al. (2003) Oncology 64:443-449; Lin, et al. (2004) Clin. Cancer Res. 10:5708-5716. |
| GAGE-1; GAGE-2; GAGE-3; GAGE-4; GAGE-5; GAGE-6; GAGE-7; GAGE-8; GAGE-65; GAGE-11; GAGE-13; GAGE-7B. | De Backer, et al. (1999) Cancer Res. 59:3157-3165; Scarcella, et al. (1999) Clin. Cancer Res. 5:335-341. |
| HIP1R; LMNA; KIAA1416; Seb4D; KNSL6; TRIP4; MBD2; HCAC5; MAGEA3. | Scanlan, et al. (2002) Cancer Res. 62:4041-4047. |
| DAM family of genes, e.g., DAM-1; DAM-6. | Fleishhauer, et al. (1998) Cancer Res. 58:2969-2972. |
| RCAS1. | Enjoji, et al. (2004) Dig. Dis. Sci. 49:1654-1656. |
| RU2. | Van Den Eynde, et al. (1999) J. Exp. Med. 190:1793-1800. |
| CAMEL. | Slager, et al. (2004) J. Immunol. 172:5095-5102; Slager, et al. (2004) Cancer Gene Ther. 11:227-236. |
| Colon cancer associated antigens, e.g., NY-CO-8; NY-CO-9; NY-CO-13; NY-CO-16; NY-CO-20; NY-CO-38; NY-CO-45; NY-CO-9/HDAC5; NY-CO-41/MBD2; NY-CO-42/TRIP4; NY-CO-95/KIAA1416; KNSL6; seb4D. | Scanlan, et al. (2002) Cancer Res. 62:4041-4047. |
| N-Acetylglucosaminyl-tranferase V (GnT-V). | Dosaka-Akita, et al. (2004) Clin. Cancer Res. 10:1773-1779. |
| Elongation factor 2 mutated (ELF2M). | Renkvist, et al. (2001) Cancer Immunol Immunother. 50:3-15. |
| HOM-MEL-40/SSX2 | Neumann, et al. (2004) Int. J. Cancer 112:661-668; Scanlan, et al. (2000) Cancer Lett. 150:155-164. |
| BRDT. | Scanlan, et al. (2000) Cancer Lett. 150:155-164. |
| SAGE; HAGE. | Sasaki, et al. (2003) Eur. J. Surg. Oncol. 29:900-903. |
| RAGE. | See, e.g., Li, et al. (2004) Am. J. Pathol. 164:1389-1397; Shirasawa, et al. (2004) Genes to Cells 9:165-174. |
| MUM-1 (melanoma ubiquitous mutated); MUM-2; MUM-2 Arg-Gly mutation; MUM-3. | Gueguen, et al. (1998) J. Immunol. 160:6188-6194; Hirose, et al. (2005) Int. J. Hematol. 81:48-57; Baurain, et al. (2000) J. Immunol. 164:6057-6066; Chiari, et al. (1999) Cancer Res. 59:5785-5792. |
| LDLR/FUT fusion protein antigen of melanoma. | Wang, et al. (1999) J. Exp. Med. 189:1659-1667. |
| NY-REN series of renal cancer antigens. | Scanlan, et al. (2002) Cancer Res. 62:4041-4047; Scanlan, et al. (1999) Cancer Res. 83:456-464. |
| NY-BR series of breast cancer antigens, e.g., NY-BR-62; NY-BR-75; NY-BR-85; NY-BR-62; NY-BR-85. | Scanlan, et al. (2002) Cancer Res. 62:4041-4047; Scanlan, et al. (2001) Cancer Immunity 1:4. |
| BRCA-1; BRCA-2. | Stolier, et al. (2004) Breast J. 10:475-480; Nicoletto, et al. (2001) Cancer Treat Rev. 27:295-304. |
| DEK/CAN fusion protein. | von Lindem, et al. (1992) Mol. Cell. Biol. 12:1687-1697. |
| Ras, e.g., wild type ras, ras with mutations at codon 12, 13, 59, or 61, e.g., mutations G12C; G12D; G12R; G12S; G12V; G13D; A59T; Q61H. K-RAS; H-RAS; N-RAS. | GenBank Acc. Nos. P01112; P01116; M54969; M54968; P01111; P01112; K00654. See also, e.g., GenBank Acc. Nos. M26261; M34904; K01519; K01520; BC006499; NM_006270; NM_002890; NM_004985; TSM_033360; NM_176795; NM_005343. |
| BRAF (an isoform of RAF). | Tannapfel, et al. (2005) Am. J. Clin. Pathol. 123:256-2601; Tsao and Sober (2005) Dermatol. Clin. 23:323-333. |
| Melanoma antigens, including HST-2 melanoma cell antigens. | GenBank Acc. No. NM_206956; NM_206955; NM_206954; NM_206953; NM_006115; NM_005367; NM_004988; AY 148486; U10340; U10339; M77481. See, e.g., Suzuki, et al. (1999) J. Immunol. 163:2783-2791. |
| Survivin | GenBank Acc. No. AB028869; U75285 (see also, e.g., Tsuruma, et al. (2004) J. Translational Med. 2:19 (11 pages); Pisarev, et al. (2003) Clin. Cancer Res. 9:6523-6533; Siegel, et al. (2003) Br. J. Haematol. 122:911-914; Andersen, et al. (2002) Histol. Histopathol. 17:669-675). |
| MDM-2 | NM_002392; NM_006878 (see also, e.g., Mayo, et al. (1997) Cancer Res. 57:5013-5016; Demidenko and Blagosklonny (2004) Cancer Res. 64:3653-3660). |
| Methyl-CpG-binding proteins (MeCP2; MBD2). | Muller, et al. (2003) Br. J. Cancer 89:1 934-1939; Fang, et al. (2004) World J. Gastreenterol. 10:3394-3398. |
| NA88-A. | Moreau-Aubry, et al. (2000) J. Exp. Med. 191:1617-1624. |
| Histone deacetylases (HDAC), e.g., HDAC5. | Waltregny, et al. (2004) Eur. J. Histochem. 48:273-290; Scanlan, et al. (2002) Cancer Res. 62:4041-4047. |
| Cyclophilin B (Cyp-B). | Tamura, et al. (2001) Jpn. J. Cancer Res. 92:762-767. |
| CA 15-3; CA 27.29. | Clinton, et al. (2003) Biomed. Sci. Instrum. 39:408-414. |
| Heat shock protein Hsp70. | Faure, et al. (2004) Int. J. Cancer 108:863-870. |
| GAGE/PAGE family, e.g., PAGE-1; PAGE-2; PAGE-3; PAGE-4; XAGE-1; XAGE-2; XAGE-3. | Brinkmann, et al. (1999) Cancer Res. 59:1445-1448. |
| MAGE-A, B, C, and D families. MAGE-B5; MAGE-B6; MAGE-C2; MAGE-C3; MAGE-3; MAGE-6. | Lucas, et al. (2000) Int. J. Cancer 87:55-60; Scanlan, et al. (2001) Cancer Immun. 1:4. |
| Kinesin 2; TATA element modulatory factor 1; tumor protein D53; NY | Scanlan, et al. (2001) Cancer Immun. 30:1-4. |
| Alpha-fetoprotein (AFP) | Grimm, et al. (2000) Gastroenterol. 119:1104-1112. |
| SART1; SART2; SART3; ART4. | Kumamuru, et al. (2004) Int. J. Cancer 108:686-695; Sasatomi, et al. (2002) Cancer 94:1636-1641; Matsumoto, et al. (1998) Jpn. J. Cancer Res. 89:1292-1295; Tanaka, et al. (2000) Jpn. J. Cancer Res. 91:1177-1184. |
| Preferentially expressed antigen of melanoma (PRAME). | Matsushita, et al. (2003) Leuk. Lymphoma 44:439-444; Oberthuer, et al. (2004) Clin. Cancer Res. 10:4307-4313. |
| Carcinoembryonic antigen (CEA), CAP1-6D enhancer agonist peptide. | GenBank Acc. No. M29540; E03352; X9831 1; M17303 (see also, e.g., Zaremba (1997) Cancer Res. 57:4570-4577; Sarobe, et al. (2004) Curr. Cancer Drug Targets 4:443-454; Tsang, et al. (1997) Clin. Cancer Res. 3:2439-2449; Fong, et al. (2001) Proc. Natl. Acad. Sci. USA 98:8809-8814). |
| HER-2/neu. | Disis, et al. (2004) J. Clin. Immunol. 24:571-578; Disis and Cheever (1997) Adv. Cancer Res. 71:343-371. |
| cdk4; cdk6; p16 (INK4); Rb protein. | Ghazizadeh, et al. (2005) Respiration 72:68-73; Ericson, et al. (2003) Mol. Cancer Res. 1:654-664. |
| TEL; AML1; TEL/AML1. | Stams, et al. (2005) Clin. Cancer Res. 11:2974-2980. |
| Telomerase (TERT). | Nair, et al. (2000) Nat. Med. 6:101 1-1017. |
| 707-AP. | Takahashi, et al. (1997) Clin. Cancer Res. 3:1363-1370. |
| Annexin, e.g., Annexin II. | Zimmerman, et al. (2004) Virchows Arch. 445:368-374. |
| BCR/ABL; BCR/ABL p210; BCR/ABL p190; CML-66; CML-28. | Cobaldda, et al. (2000) Blood 95:1007-1013; Hakansson, et al. (2004) Leukemia 18:538-547; Schwartz, et al. (2003) Semin. Hematol. 40:87-96; Lim, et al. (1999) Int. J. Mol. Med. 4:665-667. |
| BCL2; BLC6; CD10 protein. | Iqbal, et al. (2004) Am. J. Pathol. 165:159-166. |
| CDC27 (this is a melanoma antigen). | Wang, et al. (1999) Science 284:1351-1354. |
| Sperm protein 17 (SP17); 14-3-3-zeta; MEMD; KIAA0471; TC21. | Arora, et al. (2005) Mol. Carcinog. 42:97-108. |
| Tyrosinase-related proteins 1 and 2 (TRP-1 and TRP-2). | GenBank Acc. No. NM_001922. (see also, e.g., Bronte, et al. (2000) Cancer Res. 60:253-258). |
| gp100/pmel-17. | GenBank Acc. Nos. AH003567; U31798; U31799;U31 807; U31799 (see also, e.g., Bronte, et al. (2000) Cancer Res. 60:253-258). |
| TARP. | See, e.g., Clifton, et al. (2004) Proc. Natl. Acad. Sci. USA 101:10166-10171; Virok, et al. (2005) Infection Immunity 73:1939-1946. |
| Tyrosinase-related proteins 1 and 2 (TRP-1 and TRP-2). | GenBank Acc. No. NM_001922. (see also, e.g., Bronte, et al. (2000) Cancer Res. 60:253-258). |
| Melanocortin 1 receptor (MC1R); MAGE-3; gp100; tyrosinase; dopachrome tautomerase (TRP-2); MART-1. | Salazar-Onfray, et al. (1997) Cancer Res. 57:4348-4355; Reynolds, et al. (1998) J. Immunol. 161:6970-6976; Chang, et al. (2002) Clin. Cancer Res. 8:1021-1032. |
| MUC-1; MUC-2. | See, e.g., Davies, et al. (1994) Cancer Lett. 82:179-184; Gambus, et al. (1995) Int. J. Cancer 60:146-148; McCool, et al. (1999) Biochem. J. 341:593-600. |
| Spas-1. | U.S. Published Pat. Appl. No. 20020150588 of Allison, et al. |
| CASP-8; FLICE; MACH. | Mandruzzato, et al. (1997) J. Exp. Med. 186:785-793. |
| CEACAM6; CAP-1. | Duxbury, et al. (2004) Biochem. Biophys. Res. Commun. 317:837-843; Morse, et al. (1999) Clin. Cancer Res. 5:1331-1338. |
| HMGB1 (a DNA binding protein and cytokine). | Brezniceanu, et al. (2003) FASEB J. 17:1295-1297. |
| ETV6/AML1. | Codrington, et al. (2000) Br. J. Haematol. 111:1071-1079. |
| Mutant and wild type forms of adenomatous polyposis coli (APC); beta-catenin; c-met; p53; E-cadherin; cyclooxygenase-2 (COX-2). | Clements, et al. (2003) Clin. Colorectal Cancer 3:113-120; Gulmann, et al. (2003) Appl. Immunohistochem. Mol. Morphol. 11:230-237; Jungck, et al. (2004) Int. J. Colorectal. Dis. 19:438-445; Wang, et al. (2004) J. Surg. Res. 120:242-248; Abutaily, et al. (2003) J. Pathol. 201:355-362; Liang, et al. (2004) Br. J. Surg. 91:355-361; Shirakawa, et al. (2004) Clin. Cancer Res. 10:4342-4348. |
| Renal cell carcinoma antigen bound by mAB G250. | Mulders, et al. (2003) Urol. Clin. North Am. 30:455-465; Steffens, et al. (1999) Anticancer Res. 19:1197-1200. |

| ***Francisella tularensis* antigens** | |
|---|---|
| *Francisella tularensis* A and B. | Complete genome of subspecies Schu S4 (GenBank Acc. No. AJ749949); of subspecies Schu 4 (GenBank Acc. No. NC_006570). Outer membrane protein (43 kDa) Bevanger, et al. (1988) J. Clin. Microbiol. 27:922-926; Porsch-Ozcurumez, et al. (2004) Clin. Diagnostic. Lab. Immunol. 11:1008-1015). Antigenic components of *F. tularensis* include, e.g., 80 antigens, including 10 kDa and 60 kDa chaperonins (Havlasova, et al. (2002) Proteomics 2:857-86), nucleoside diphosphate kinase, isocitrate dehydrogenase, RNA-binding protein Hfq, the chaperone ClpB (Havlasova, et al. (2005) Proteomics 5:2090-2103). See also, e.g., Oyston and Quarry (2005) Antonie Van Leeuwenhoek 87:277-281; Isherwood, et al. (2005) Adv. Drug Deliv. Rev. 57:1403-1414; Biagini, et al. (2005) Anal. Bioanal. Chem. 382:1027-1034. |

| **Malarial antigens** | |
|---|---|
| Circumsporozoite protein (CSP); SSP2; HEP17; Exp-1 orthologs found in P. falciparum; and LSA-1. | See, e.g., Haddad, et al. (2004) Infection Immunity 72:1594-1602; Hoffman, et al. (1997) Vaccine 15:842-845; Oliveira-Ferreira and Daniel-Ribeiro (2001) Mem. Inst. Oswaldo Cruz, Rio de Janeiro 96:221-227. CSP (see, e.g., GenBank Acc. No. AB121024). SSP2 (see, e.g., GenBank Acc. No. AF249739). LSA-1 (see, e.g., GenBank Acc. No. Z30319). |
| Ring-infected erythrocyte survace protein (RESA); merozoite surface protein 2 (MSP2); Spf66; merozoite surface protein 1(MSP1); 195A; BVp42. | See, e.g., Stirnadel, et al. (2000) Int. J. Epidemiol. 29:579-586; Krzych, et al. (1995) J. Immunol. 155:4072-4077. See also, Good, et al. (2004) Immunol. Rev. 201:254-267; Good, et al. (2004) Ann. Rev. Immunol. 23:69-99. MSP2 (see, e.g., GenBank Acc. No. X96399; X96397). MSP1 (see, e.g., GenBank Acc. No. X03371). RESA (see, e.g., GenBank Acc. No. X05181; X05182). |
| Apical membrane antigen 1 (AMA1). | See, e.g., Gupta, et al. (2005) Protein Expr. Purif. 41:186-198. AMA1 (see, e.g., GenBank Acc. No. A'13; AJ494905; AJ490565). |

| **Viruses and viral antigens** | |
|---|---|
| Hepatitis A | GenBank Acc. Nos., e.g., NC_001489; AY644670; X83302; K02990; M14707. |
| Hepatitis B | Complete genome (see, e.g., GenBank Acc. Nos. AB214516; NC_003977; AB205192; AB205191; AB205190; AJ748098; AB198079; AB198078; AB198076; AB074756). |
| Hepatitis C | Complete genome (see, e.g., GenBank Acc. Nos. NC_004102; AJ238800; AJ238799; AJ132997; AJ132996; AJ000009; D84263). |
| Hepatitis D | GenBank Acc. Nos, e.g. NC_001653; AB118847; AY261457. |
| Human papillomavirus, including all 200+subtypes (classed in 16 groups), such as the high risk subtypes 16, 18, 30, 31, 33,45. | See, e.g., Trimble, et al. (2003) Vaccine 21:4036-4042; Kim, et al. (2004) Gene Ther. 11:1011-1018; Simon, et al. (2003) Eur. J. Obstet. Gynecol. Reprod. Biol. 109:219-223; Jung, et al. (2004) J. Microbiol. 42:255-266; Damasus-Awatai and Freeman-Wang (2003) Curr. Opin. Obstet. Gynecol. 15:473-477; Jansen and Shaw (2004) Annu. Rev. Med. 55:319-331; Roden and Wu (2003) Expert Rev. Vaccines 2:495-516; de Villiers, et al. (2004) Virology 324:17-24; Hussain and Paterson (2005) Cancer Immunol. Immunother. 54:577-586; Molijn, et al. (2005) J. Clin. Virol. 32 (Suppl. 1) S43-S51. GenBank Acc. Nos. AY686584; AY686583; AY686582; NC_006169; NC_006168; NC_006164; NC_001355; NC_001349; NC_005351; NC_001596). |
| Human T-cell lymphotropic virus (HTLV) types I and II, including the HTLV type I subtypes Cosmopolitan, Central African, and Austro-Melanesian, and the HTLV type II subtypes IIa, IIb, IIc, and IId. | See, e.g., Capdepont, et al. (2005) AIDS Res. Hum. Retrovirus 21:28-42; Bhigjee, et al. (1999) AIDS Res. Hum. Restrovirus 15:1229-1233; Vandamme, et al. (1998) J. Virol. 72:4327-4340; Vallejo, et al. (1996) J. Acquir. Immune Defic. Syndr. Hum. Retrovirol. 13:384-391. HTLV type I (see, e.g., GenBank Acc. Nos. AY563954; AY563953. HTLV type II (see, e.g., GenBank Acc. Nos. L03561; Y13051; AF139382). |
| Coronaviridae, including Coronaviruses, such as SARS-coronavirus (SARS-CoV), and Toroviruses. | See, e.g., Brian and Baric (2005) Curr. Top. Microbiol. Immunol. 287:1-30; Gonzalez, et al. (2003) Arch. Virol. 148:2207-2235; Smits, et al. (2003) J. Virol. 77:9567-9577; Jamieson, et al. (1998) J. Infect. Dis. 178:1263-1269 (GenBank Acc. Nos. AY348314; NC_004718; AY394850). |
| Rubella virus. | GenBank Acc. Nos. NC_001545; AF435866. |
| Mumps virus, including the genotypes A, C, D, G, H, and I. | See, e.g., Orvell, et al. (2002) J. Gen. Virol. 83:2489-2496. See, e.g., GenBank Acc. Nos. AY681495; NC_002200; AY685921; AF201473. |
| Coxsackie virus A including the serotypes 1, 11, 13, 15, 17, 18, 19, 20, 21, 22, and 24 (also known as Human enterovirus C; HEV-C). | See, e.g., Brown, et al. (2003) J. Virol. 77:8973-8984. GenBank Acc. Nos. AY421768; AY790926: X67706. |
| Coxsackie virus B, including subtypes 1-6. | See, e.g., Ahn, et al. (2005) J. Med. Virol. 75:290-294; Patel, et al. (2004) J. Virol. Methods 120:167-172; Rezig, et al. (2004) J. Med. Virol. 72:268-274. GenBank Acc. No. X05690. |
| Human enteroviruses including, e.g., human enterovirus A (HEV-A, CAV2 to CAV8, CAV10, CAV12, CAV14, CAV16, and EV71) and also including HEV-B (CAV9, CBV1 to CBV6, E1 to E7, E9, E11 to E21, E24 to E27, E29 to E33, and EV69 and E73), as well as HEV. | See, e.g., Oberste, et al. (2004) J. Virol. 78:855-867. Human enterovirus A (GenBank Acc. Nos. NC_001612); human enterovirus B (NC_001472); human enterovirus C (NC_001428); human enterovirus D (NC_001430). Simian enterovirus A (GenBank Acc. No. NC_003988). |
| Polioviruses including PV1, PV2, and PV3. | See, e.g., He, et al. (2003) J. Virol. 77:4827-4835; Hahsido, et al. (1999) Microbiol. Immunol. 43:73-77. GenBank Acc. No. AJ132961 (type 1); AY278550 (type 2); X04468 (type 3). |
| Viral encephalitides viruses, including equine encephalitis, Venezuelan equine encephalitis (VEE) (including subtypes IA, IB, IC, ID, IIIC, IIID), Eastern equine encephalitis (EEE), Western equine encephalitis (WEE), St. Louis encephalitis, Murray Valley (Australian) encephalitis, Japanese encephalitis, and tick-born encephalitis. | See, e.g., Hoke (2005) Mil. Med. 170:92-105; Estrada-Franco, et al. (2004) Emerg. Infect. Dis. 10:2113-2121; Das, et al. (2004) Antiviral Res. 64:85-92; Aguilar, et al. (2004) Emerg. Infect. Dis. 10:880-888; Weaver, et al. (2004) Arch. Virol. Suppl. 18:43-64; Weaver, et al. (2004) Annu. Rev. Entomol. 49:141-174. Eastern equine encephalitis (GenBank Acc. No. NC_003899; AY722102); Western equine encephalitis (NC_003908). |
| Human herpesviruses, including cytomegalovirus (CMV), Epstein-Barr virus (EBV), human herpesvirus-1 (HHV-1), HHV-2, HHV-3, HHV-4, HHV-5, HHV-6, HHV-7, HHV-8, herpes B virus, herpes simplex virus types 1 and 2 (HSV-1, HSV-2), and varicella zoster virus (VZV). | See, e.g., Studahl, et al. (2000) Scand. J. Infect. Dis. 32:237-248; Padilla, et al. (2003) J. Med. Virol. 70 (Suppl. 1) S103-S110; Jainkittivong and Langlais (1998) Oral Surg. Oral Med. 85:399-403. GenBank Nos. NC_001806 (herpesvirus 1); NC_001798 (herpesvirus 2); X04370 and NC_001348 (herpesvirus 3); NC_001345 (herpesvirus 4); NC_001347 (herpesvirus 5); X83413 and NC_000898 (herpesvirus 6); NC_001716 (herpesvirus 7). Human herpesviruses types 6 and 7 (HHV-6; HHV-7) are disclosed by, e.g., Padilla, et al. (2003) J. Med. Virol. 70 (Suppl. 1)S103-S110. Human herpesvirus 8 (HHV-8), including subtypes A-E, are disclosed in, e.g., Treurnicht, et al. (2002) J. Med. Virul. 66:235-240. (VZV). |
| HIV-1 including group M (including subtypes A to J) and group O (including any distinguishable subtypes) (HIV-2, including subtypes A-E. | See, e.g., Smith, et al. (1998) J. Med. Virol. 56:264-268. See also, e.g., GenBank Acc. Nos. DQ054367; NC_001802; AY968312; DQ011180; DQ011179, DQ011178; DQ01 1177; AY588971; AY588970; AY781127; AY781126; AY970950; AY970949; AY970948; X61240; AJ006287; AJ508597; and AJ508596. |
| Epstein-Barr virus (EBV), including subtypes A and B. | See, e.g., Peh, et al. (2002) Pathology 34:446-450. Epstein-Barr virus strain B95-8 (GenBank Acc. No. V01555). |
| Reovirus, including serotypes and strains 1, 2, and 3, type 1 Lang, type 2 Jones, and type 3 Dearing. | See, e.g., Barthold, et al. (1993) Lab. Anim. Sci. 43:425-430; Roner, et al. (1995) Proc. Natl. Acad. Sci. USA 92:12362-12366; Kedl, et al. (1995) J. Virol. 69:552-559. GenBank Acc. No. K02739 (sigma-3 gene surface protein). |
| Cytomegalovirus (CMV) subtypes include CMV subtypes I-VII. | See, e.g., Chern, et al. (1998) J. Infect. Dis. 178:1149-1153; Vilas Boas, et al. (2003) J. Med. Virol. 71:404-407; Trincado, et al. (2000) J. Med. Virol. 61:481-487. GenBank Acc. No. X17403. |
| Rhinovirus, including all serotypes. | Human rhinovirus 2 (GenBank Acc. No. X02316); Human rhinovirus B (GenBank Acc. No. NC_001490); Human rhinovirus 89 (GenBank Acc. No. NC_001617); Human rhinovirus 39 (GenBank Acc. No. AY751783). |
| Adenovirus, including all serotypes. | AY803294; NC_004001; AC_000019; AC_000018; AC_000017; AC_000015; AC_000008; AC_000007; AC_000006; AC_000005; AY737798; AY737797;NC_003266; NC_002067; AY594256; AY594254; AY875648; AJ854486; AY163756; AY594255; AY594253; NC_001460; NC_001405; AY598970; AY458656; AY487947; NC_001454; AF534906; AY45969; AY128640; L19443; AY339865; AF532578. |
| Varicella-zoster virus, including strains and genotypes Oka, Dumas, European, Japanese, and Mosaic. | See, e.g., Loparev, et al. (2004) J. Virol. 78:8349-8358; Carr, et al. (2004) J. Med. Virol. 73:131-136; Takayama and Takayama (2004) J. Clin. Virol. 29:113-119. |
| Filoviruses, including Marburg virus and Ebola virus, and strains such as Ebola-Sudan (EBO-S), Ebola-Zaire (EBO-Z), and Ebola-Reston (EBO-R). | See, e.g., Geisbert and Jahrling (1995) Virus Res. 39:129-150; Hutchinson, et al. (2001) J. Med. Virol. 65:561-566. Marburg virus (see, e.g., GenBank Acc. No. NC_001608). Ebola virus (see, e.g., GenBank Acc. Nos. NC_006432; AY769362; NC_002549; AF272001; AF086833). |
| Arenaviruses, including lymphocytic choriomeningitis (LCM) virus, Lassa virus, Junin virus, and Machupo virus. | Junin virus, segment S (GenBank Acc. No. NC_005081); Junin virus, segment L (GenBank Acc. No. NC_005080). |
| Rabies virus. | See, e.g., GenBank Acc. Nos. NC_ 001542; AY956319; AY705373; AF499686; AB 128149; AB085828; AB009663. |
| Arboviruses, including West Nile virus, Dengue viruses 1 to 4, Colorado tick fever virus, Sindbis virus, Togaviraidae, Flaviviridae, Bunyaviridae, Reoviridae, Rhabdoviridae, Orthomyxoviridae, and the like. | Dengue virus type 1 (see, e.g., GenBank Acc. Nos. AB 195673; AY762084). Dengue virus type 2 (see, e.g., GenBank Acc. Nos. NC_001474; AY702040; AY702039; AY702037). Dengue virus type 3 (see, e.g., GenBank Acc. Nos. AY923865; AT858043). Dengue virus type 4 (see, e.g., GenBank Acc. Nos. AY947539; AY947539; AF326573). Sindbis virus (see, e.g., GenBank Acc. Nos. NC_001547; AF429428; J02363; AF103728). West Nile virus (see, e.g., GenBank Acc. Nos. NC_001563; AY603654). |
| Poxvirus including orthopoxvirus (variola virus, monkeypox virus, vaccinia virus, cowpox virus), yatapoxvirus (tanapox virus, Yaba monkey tumor virus), parapoxvirus, and molluscipoxvirus. | Viriola virus (see, e.g., GenBank Acc. Nos. NC_001611; Y16780; X72086; X69198). |
| Yellow fever. | See, e.g., GenBank Acc. No. NC_002031; AY640589; X03700. |
| Hantaviruses, including serotypes Hantaan (HTN), Seoul (SEO), Dobrava (DOB), Sin Nombre Puumala (PUU), and Dobrava-like Saaremaa (SAAV). | See, e.g., Elgh, et al. (1997) J. Clin. Microbiol. 35:1122-1130; Sjolander, et al. (2002) Epidemiol. Infect. 128:99-103; Zeier, et al. (2005) Virus Genes 30:157-180. GenBankAcc. No. NC_005222 and NC_005219 (Hantavirus). See also, e.g., GenBank Acc. Nos. NC_005218; NC_005222; NC_005219. |
| Flaviviruses, including Dengue virus, Japanese encephalitis virus, West Nile virus, and yellow fever virus. | See, e.g., Mukhopadhyay, et al. (2005) Nature Rev. Microbiol. 3:13-22. GenBank Acc. Nos NC_001474 and AY702040 (Dengue). GenBank Acc. Nos. NC_001563 and AY603654. |
| Measles virus. | See, e.g., GenBank Acc. Nos. AB040874 and AY486084. |
| Human parainfluenzaviruses (HPV), including HPV types 1-56. | Human parainfluenza virus 2 (see, e.g., GenBank Acc. Nos. AB176531; NC003443). Human parainfluenza virus 3 (see, e.g., GenBank Acc. No. NC_00 1 796). |
| Influenza virus, including influenza virus types A, B, and C. | Influenza nucleocapsid (see, e.g., GenBank Acc. No. AY626145). Influenza hemagglutinin (see, e.g., GenBank Acc. Nos. AY627885; AY555153). Influenza neuraminidase (see, e.g., GenBank Acc. Nos. AY555151; AY577316). Influenza matrix protein 2 (see, e.g., GenBank Acc. Nos. AY626144(. Influenza basic protein 1 (see, e.g., GenBank Acc. No. AY627897). Influenza polymerase acid protein (see, e.g., GenBank Acc. No. AY627896). Influenza nucleoprotein (see, e.g., GenBank Acc. Nno. AY627895). |
| Influenza A virus subtypes, e.g., swine viruses (SIV): H1N1 influenzaA and swine influenza virus. | Hemagglutinin of H1N1 (GenBank Acc. No. S67220). Influenza A virus matrix protein (GenBank Acc. No. AY700216). Influenza virus A H5H1 nucleoprotein (GenBank Acc. No. AY646426). H1N1 haemagglutinin (GenBank Acc. No. D00837). See also, GenBank Acc. Nos. BD006058; BD006055; BD006052. See also, e.g., Wentworth, et al. (1994) J. Virol. 68:2051-2058; Wells, et al. (1991) J.A.M.A. 265:478-481. |
| Respiratory syncytial virus (RSV), including subgroup A subgroup B. | Respiratory syncytial virus (RSV) (see, e.g., GenBank Acc. Nos. AY353550; NC_001803; NC001781). |
| Rotaviruses, including human rotaviruses A to E, bovine rotavirus, rhesus monkey rotavirus, and human-RVV reassortments. | Human rotavirus C segment 8 (GenBank Acc. No. AJ549087); Human rotavirus G9 strain outer capsid protein (see, e.g., GenBank Acc. No. DQ056300); Human rotavirus B strain non-structural protein 4 (see, e.g., GenBank Acc. No. AY548957); human rotavirus A strain major inner capsid protein (see, e.g., GenBank Acc. No. AY601554). |
| Polyomavirus, including simian virus 40 (SV40), JC virus (JCV) and BK virus (BKV). | See, e.g., Engels, et al. (2004) J. Infect. Dis. 190:2065-2069; Vilchez and Butel (2004) Clin. Microbiol. Rev. 17:495-508; Shivapurkar, et al. (2004) Cancer Res. 64:3757-3760; Carbone, et al. (2003) Oncogene 2:5173-5180; Barbanti-Brodano, et al. (2004) Virology 318:1-9) (SV40 complete genome in, e.g., GenBank Acc. Nos. NC_ 001669; AF168994; AY271817; AY271816; AY120890; AF345344; AF332562). |
| Coltiviruses, including Colorado tick fever virus, Eyach virus. | Attoui, et al. (1998) J. Gen. Virol. 79:2481-2489. Segments of Eyach virus (see, e.g., GenBank Acc. Nos. AF282475; AF282472; AF282473; AF282478; AF282476; NC_003707; NC_003702; NC_003703; NC_003704; NC_003705; NC_003696; NC_003697; NC_003698; NC_003699; NC_003701; NC_003706; NC_003700; AF282471; AF282477). |
| Calciviruses, including the genogroups Norwalk, Snow Mountain group (SMA), and Saaporo. | Snow Mountain virus (see, e.g., GenBank Acc. No. AY134748). |
| Parvoviridae, including dependovirus, parvovirus (including parvovirus B19), and erythrovirus. | See, e.g., Brown (2004) Dev. Biol. (Basel) 118:71-77; Alvarez-Lafuente, et al. (2005) Ann. Rheum. Dis. 64:780-782; Ziyaeyan, et al. (2005) Jpn. J. Infect. Dis. 58:95-97; Kaufman, et al. (2005) Virology 332:189-198. |
| The present disclosure provides, but is not limited by, an attenuated *Listeria* comprising a nucleic acid that encodes at least one of the above-disclosed antigens, or at least one antigen encoded by one of the above-disclosed complete genomes. The present disclosure encompasses nucleic acids encoding mutants, muteins, splice variants, fragments, truncated variants, soluble variants, extracellular domains, intracellular domains, mature sequences, and the like, of the disclosed antigens. Provided are nucleic acids encoding epitopes, oligo- and polypeptides of these antigens. Also provided are codon optimized embodiments, that is, optimized for expression in *Listeria.* | |

In some embodiments, the antigen is non-Listerial. In some embodiments, the antigen is from a cancer cell, tumor, or infectious agent. In some embodiments, the antigen is derived from an antigen from a cancer cell, tumor, or infectious agent. In some embodiments, an antigen that is "derived from" another antigen is a fragment or other derivative of the antigen. In some embodiments, the derived antigen comprises a fragment of at least 8 amino acids, at least 12 amino acids, at least 20 amino acids, at least 50 amino acids, at least 75 amino acids, at least 100 amino acids, or at least 200 amino acids. In some embodiments, the derivative of the antigen has at least about 80 % sequence identity, at least about 85% sequence identity, at least about 90% sequence identity, at least about 95% sequence identity, or at least about 98% sequence identity to the antigen from which it is derived, or a fragment thereof. In some embodiments, a derived antigen comprises an antigen deleted of its signal sequence and/or membrane anchor. In some embodiments, an antigen derived from another antigen comprises at least one MHC class I epitope and/or at least one MHC class II epitope from the original (full-length) antigen. In some embodiments, the antigen is a tumor antigen. Assays for testing the immunogenicity of antigens are described herein and are well known in the art.

In some embodiments, the heterologous antigen is a human tumor antigen or an antigen derived from a human tumor antigen. In some embodiments, the antigen derived from a tumor antigen consists of an amino acid sequence that exhibits at least about 65% sequence identity to the tumor antigen, at least 70% sequence identity to the tumor antigen, or at least about 75% sequence identity to the tumor antigen. In some embodiments, the derived antigen consists of an amino acid sequence that exhibits at least 85% sequence identity to the tumor antigen, at least 90% sequence identity to the tumor antigen, or at least about 95% sequence identity to the tumor antigen. In some embodiments, the antigen derived from the tumor antigen comprises at least 10, 20, 30, 40, 50, 75, 100, or 200 amino acids of the tumor antigen. In some embodiments, the antigen derived from the tumor antigen consists of at least 10, 20, 30, 40, 50, 75, 100, or 200 continguous amino acids of the tumor antigen.

In some embodiments, the antigen is mesothelin, or derived from mesothelin. In some embodiments, the mesothelin is human. In some embodiments, the mesothelin is full-length (e.g., full length human mesothelin). In some embodiments, the antigen derived from mesothelin comprises mesothelin (e.g., human mesothelin) deleted in its signal sequence, deleted in its GPI anchor, or deleted in both the signal sequence and the GPI anchor. The polynucleotide encoding the mesothelin may be codon-optimized or non-codon optimized for expression in *Listeria.*

In some embodiments, the antigen derived from mesothelin consists of an amino acid sequence that exhibits at least about 65% sequence identity to human mesothelin , at least 70% sequence identity to human mesothelin, or at least about 75% sequence identity to human mesothelin. In some embodiments, the mesothelin polypeptide sequence consists of an amino acid sequence that exhibits at least 85% sequence identity to human mesothelin , at least 90% sequence identity to human mesothelin, or at least about 95% sequence identity to human mesothelin. In some embodiments, the antigen derived from mesothelin comprises at least 10, 20, 30, 40, 50, 75, 100, or 200 amino acids of a mesothelin polypeptide, such as human mesothelin or human mesothelin deleted of its signal peptide sequence and/or GPI anchor. In some embodiments, the antigen derived from mesothelin consists of at least 10, 20, 30, 40, 50, 75, 100, or 200 continguous amino acids of a mesothelin polypeptide, such as human mesothelin or human mesothelin deleted of its signal peptide sequence and/or GPI anchor.

In certain embodiments, the antigen is prostate stem cell antigen (PSCA), or is an antigen derived from PSCA. In some embodiments, the PSCA is human PSCA. In some embodiments, the PSCA is full-length (e.g., full length human PSCA). In some embodiments, the antigen derived from PSCA comprises PSCA (e.g., human PSCA) deleted of (a) part or all its signal peptide region, (b) its GPI anchor region, or (c) its GPI anchor region and part or all of its signal peptide region. The polynucleotide encoding the PSCA may be codon-optimized or non-codon optimized for expression in *Listeria.*

Various assays for assessing whether a particular antigen derivative can stimulate the desired immunogenicity when expressed in *Listeria* are known to those of ordinary skill in the art. Specific examples are provided in the Examples below. Various assays are also provided, e.g., in U.S. Pat. Publ. Nos. 2004/0228877, 2004/0197343, and 2005/0249748.

In some embodiments, the antigen derived from PSCA consists of an amino acid sequence that exhibits at least about 65% sequence similarity to human PSCA, at least 70% sequence similarity to human PSCA, or at least about 75% sequence similarity to human PSCA. In some embodiments, the PSCA polypeptide sequence consists of an amino acid sequence that exhibits at least 85% sequence similarity to human PSCA, at least 90% sequence similarity to human PSCA, or at least about 95% sequence similarity to human PSCA. In some embodiments, the antigen derived from PSCA comprises at least 10, 20, 30, 40, 50, 75, 100, or 200 amino acids of a PSCA polypeptide, such as human PSCA or human PSCA deleted of its signal peptide sequence and/or GPI anchor. In some embodiments, the antigen derived from PSCA consists of at least 10, 20, 30, 40, 50, 75, 100, or 200 continguous amino acids of a PSCA polypeptide, such as human PSCA or human PSCA deleted of its signal peptide sequence and/or GPI anchor.

In some embodiments, the antigen (e.g., heterologous antigen) does not comprise an EphA2 antigenic peptide (sometimes referred to as an "EphA2 antigenic polypeptide"), as defined and described in U.S. Patent Publication No. 2005/0281783 A1. In some embodiments, the EphA2 antigenic peptide excluded from use in the methods and compositions described herein can be any EphA2 antigenic peptide that is capable of eliciting an immune response against EphA2-expressing cells involved in a hyperproliferative disorder. Thus, in some embodiments, the excluded EphA2 antigenic peptide can be an EphA2 polypeptide (e.g., the EphA2 polypeptide of SEQ ID NO:2 in U.S. Patent Publication No. 2005/0281783 A1), or a fragment or derivative of an EphA2 polypeptide that (1) displays ability to bind or compete with EphA2 for binding to an anti-EphA2 antibody, (2) displays ability to generate antibody which binds to EphA2, and/or (3) contains one or more T cell epitopes of EphA2. In some embodiments, the EphA2 antigenic peptide is a sequence encoded by one of the following nucleotide sequences, or a fragment or derivative thereof: Genbank Accession No. NM_004431 (Human); Genbank Accession No. NM_010139 (Mouse); or Genbank Accession No. AB038986 (Chicken, partial sequence). In some embodiments, the EphA2 antigenic peptide is full-length human EphA2 (e.g., SEQ ID NO:2 of U.S. Patent Publication No. 2005/0281783 A1). In some embodiments, the EphA2 antigenic peptide comprises the extracellular domain of EphA2 or the intracellular domain of EphA2. In some embodiments, the EphA2 antigenic peptide consists of full-length EphA2 or a fragment thereof with a substitution of lysine to methionine at amino acid residue 646 of EphA2. In some embodiments, the EphA2 antigenic peptide sequence consists of an amino acid sequence that exhibits at least about 65% sequence similarity to human EphA2, at least 70% sequence similarity to human EphA2, or at least about 75% sequence similarity to human EphA2. In some embodiments, the EphA2 polypeptide sequence consists of an amino acid sequence that exhibits at least 85% sequence similarity to human EphA2, at least 90% sequence similarity to human EphA2, or at least about 95% sequence similarity to human EphA2. In some embodiments, the excluded EphA2 antigenic peptide consists of at least 10, 20, 30, 40, 50, 75, 100, or 200 amino acids of an EphA2 polypeptide. In some embodiments, the EphA2 antigenic peptide consists of at least 10, 20, 30, 40, 50, 75, 100, or 200 continguous amino acids of an EphA2 polypeptide.

This disclosure supplies methods and reagents for stimulating immune response to infections, e.g., infections of the liver. These include infections from hepatotropic viruses and viruses that mediate hepatitis, e.g., hepatitis B virus, hepatitis C virus, and cytomegalovirus. This disclosure contemplates methods to treat other hepatotropic viruses, such as herpes simplex virus, Epstein-Barr virus, and dengue virus (see, e.g., Ahlenstiel and Rehermann (2005) Hepatology 41:675-677; Chen, et al. (2005) J. Viral Hepat. 12:38-45; Sun and Gao (2004) Gasteroenterol. 127:1525-1539; Li, et al. (2004) J. Leukoc. Biol. 76:1171-1179; Ahmad and Alvarez (2004) J. Leukoc. Biol. 76:743-759; Cook (1997) Eur. J. Gasteroenterol. Hepatol. 9:1239-1247; Williams and Riordan (2000) J. Gasteroenterol. Hepatol. 15 (Suppl.)G17-G25; Varani and Landini (2002) Clin. Lab. 48:39-44; Rubin (1997) Clin. Liver Dis. 1:439-452; Loh, et al. (2005) J. Virol. 79:661-667; Shresta, et al. (2004) Virology 319:262-273; Fjaer, et al. (2005) Pediatr. Transplant 9:68-73; Li, et al. (2004) World J. Gasteroenterol. 10:3409-3413; Collin, et al. (2004) J. Hepatol. 41:174-175; Ohga, et al. (2002) Crit. Rev. Oncol. Hematol. 44:203-215).

In another aspect, the present invention provides methods and reagents for the treatment and/or prevention of parasitic infections, e.g., parasitic infections of the liver. These include, without limitation, liver flukes (e.g., *Clonorchis*, *Fasciola hepatica, Opisthorchis*), *Leishmania*, *Ascaris lumbricoides*, *Schistosoma,* and helminths. Helminths include, e.g., nematodes (roundworms), cestodes (tapeworms), and trematodes (flatworms or flukes) (see, e.g., Tliba, et al. (2002) Vet. Res. 33:327-332; Keiser and Utzinger (2004) Expert Opin. Pharmacother. 5:1711-1726; Kaewkes (2003) ActA Trop. 88:177-186; Srivatanakul, et al. (2004) Asian Pac. J. Cancer Prev. 5:118-125; Stuaffer, et al. (2004) J. Travel Med. 11:157-159; Nylen, et al. (2003) Clin. Exp. Immunol. 131:457-467; Bukte, et al. (2004) Abdom. Imaging 29:82-84; Singh and Sivakumar (2003) 49:55-60; Wyler (1992) Parisitol. Today 8:277-279; Wynn, et al. (2004) Immunol. Rev. 201:156-167; Asseman, et al. (1996) Immunol. Lett. 54:11-20; Becker, et al. (2003) Mol. Biochem. Parasitol. 130:65-74; Pockros and Capozza (2005) Curr. Infect. Dis. Rep. 7:61-70; Hsieh, et al. (2004) J. Immunol. 173:2699-2704; Korten, et al. (2002) J. Immunol. 168:5199-5206; Pockros and Capozza (2004) Curr. Gastroenterol. Rep. 6:287-296).

Yet another aspect of the present invention provides methods and reagents for the treatment and/or prevention of bacterial infections, e.g., by hepatotropic bacteria. Provided are methods and reagents for treating, e.g., *Mycobacterium tuberculosis, Treponema pallidum*, and *Salmonella spp* (see, e.g., Cook (1997) Eur. J. Gasteroenterol. Hepatol. 9:1239-1247; Vankayalapati, et al. (2004) J. Immunol. 172:130-137; Sellati, et al. (2001) J. Immunol. 166:4131-4140; Jason, et al. (2000) J. Infectious Dis. 182:474-481; Kirby, et al. (2002) J. Immunol. 169:4450-4459; Johansson and Wick (2004) J. Immunol. 172:2496-2503; Hayashi, et al. (2004) Intern. Med. 43:521-523; Akcay, et al. (2004) Int. J. Clin. Pract. 58:625-627; de la Barrera, et al. (2004) Clin. Exp. Immunol. 135:105-113).

In a further embodiment, the heterologous of the present invention is derived from Human Immunodeficiency Virus (HIV), e.g., gp120; gp160; gp41; gag antigens such as p24gag or p55 gag, as well as protein derived from the pol, env, tat, vir, rev, nef, vpr, vpu, and LTR regions of HIV. The heterologous antigens contemplated include those from herpes simplex virus (HSV) types 1 and 2, from cytomegalovirus, from Epstein-Barr virus, or Varicella Zoster Virus. Also encompassed are antigens derived from a heptatis virus, e.g., hepatitis A, B, C, delta, E, or G. Moreover, the antigens also encompass antigens from *Picornaviridae* (poliovirus; rhinovirus); *Caliciviridae*; *Togaviridae* (rubella; dengue); *Flaviviridiae*; *Coronaviridae*; *Reoviridae*; *Birnaviridae*; *Rhabdoviridae*; *Orthomyxoviridae; Filoviridae*; *Paramyxoviridae* (mumps; measle); *Bunyviridae*; *Arenaviridae*; *Retroviradae* (HTLV-I; HIV-1); Papillovirus, tick-borne encephalitis viruses, and the like.

In yet another aspect, the present invention provides reagents and methods for the prevention and treatment of bacterial and parasitic infections, e.g., *Salmonella, Neisseria*, *Borrelia*, *Chlamydia*, *Bordetella*, plasmodium, *Toxoplasma*, *Mycobacterium tuberculosis*, *Bacillus anthracis, Yersiniapestis*, Diphtheria, Pertussis, Tetanus, bacterial or fungal pneumonia, Otitis Media, Gonorrhea, Cholera, Typhoid, Meningitis, Mononucleosis, Plague, Shigellosis, Salmonellosis, Legionaire's Disease, Lyme disease, Leprosy, Malaria, Hookworm, *Onchocerciasis*, *Schistosomiasis,* Trypanasomes, Leshmania, Giardia, Amoebiasis, Filariasis, Borelia, and Trichinosis (see, e.g., Despommier, et al. (2000) Parasitic Dieases, 4th ed., Apple Trees Productions, New York, NY; U.S. Government (2002) 21st Century Collection Centers for Disease Control (CDC) Emerging Infectious Diseases (EID) - Comprehensive Collection from 1995 to 2002 with Accurate and Detailed Information on Dozens of Serious Virus and Bacteria Illnesses - Hantavirus, Influenza, AIDS, Malaria, TB, Pox, Bioterrorism, Smallpox, Anthrax, Vaccines, Lyme Disease, Rabies, West Nile Virus, Hemorrhagic Fevers, Ebola, Encephalitis (Core Federal Information Series).

The present invention, at least in some embodiments, provides reagents and methods for treating a disorder or condition, or stimulating an immune response to a disorder or condition, that comprises both a cancer and infection. In some viral infections, for example, an antigen can be both a tumor antigen and a viral antigen (see, e.g., Montesano, et al. (1990) Cell 62:435-445; Ichaso and Dilworth (2001) Oncogene 20:7908-7916; Wilson, et al. (1999) J. Immunol. 162:3933-3941; Daemen, et al. (2004) Antivir. Ther. 9:733-742; Boudewijn, et al. (2004) J. Natl. Cancer Inst. 96:998-1006; Liu, et al. (2004) Proc. Natl. Acad. Sci. USA 101:14567-14571).

In some embodiments, the heterologous antigen shares at least one epitope with, or is immunologically cross-reactive with, an antigen from, or derived from, a cancer or infectious agent.

### (f). DNA repair mutants and nucleic acid targeting agents.

The present disclosure, in other embodiments, provides *Listeria* mutants, where the mutant is defective in repair of DNA damage, including, e.g., the repair of UV-light induced DNA damage, radiation induced damage, interstrand cross-links, intrastrand cross-links, covalent adducts, bulky adduct-modified DNA, deamidated bases, depurinated bases, depyrimidinated bases, oxidative damage, psoralen adducts, cis-platin adducts, combinations of the above, and the like (Mu and Sancar (1997) Prog. Nucl. Acid Res. Mol. Biol. 56:63-81; Sancar (1994) Science 266:1954-1956; Lin and Sancar (1992) Mol. Microbiol. 6:2219-2224; Selby and Sancar (1990) 236:203-211; Grossman (1994) Ann. N.Y. Acad. Sci. 726:252-265). Provided is a *Listeria* mutated in, e.g., uvrA, uvrB, uvrAB, uvrC, any combination of the above, and the like.

Moreover, what is provided is a *Listeria* that comprises at least one interstrand cross-link in its genomic DNA, or at least two, at least three, at least four, at least five, at least ten, at least 20, at least 30, at least 40, at least 50, at least 100, or more, cross-links in its genomic DNA.

One embodiment of the present disclosure comprises *Listeria* uvrAB engineered to express a heterologous antigen, where the engineered bacterium is treated with a nucleic acid cross-linking agent, a psoralen compound, a nitrogen mustard compound, 4'-(4-amino-2-oxa)butyl-4,5',8-trimethylpsoralen, or beta-alanine,N-(acridine-9-yl),2-[bis(2-chloroethyl)amino]ethyl ester (see, e.g., U.S. Publ. Pat. Appl. No. US 2004/0197343 of Dubensky; Brockstedt, et al (2005) Nat. Med. 11:853-860).

### (g) Hybridization under stringent conditions.

Hybridization of a polynucleotide such as a plasmid to a variant of that polynucleotide bearing at least one mutation, can be accomplished under the following stringent conditions. The plasmid can be between 2-3 kb, 3-4 kb, 4-5 kb, 5-6 kb, 6-7 kb, and so on. The mutation can consist of 1-10 nucleotides (nt), 10-20 nt, 20-30 nt, 30-40 nt, 40-50 nt, 50-60 nt, 60-70 kb, 70-80 kb, 80-90 kb, 90-100 kb, and the like.

Stringent conditions for hybridization in formamide can use the following hybridization solution: 48 ml formamide; 24 ml 20 times SSC; 1.0 ml 2 M Tris Cl, pH 7.6; 1.0 ml 100 times Denhardt's solution; 5.0 ml water; 20 ml 50% dextran sulfate, 1.0 ml 10% sodium dodecylsulfate (total volume 100 ml). Hybridization can be for overnight at 42° C (see, e.g., (1993) Current Protocols in Molecular Biology, Suppl. 23, pages 6.3.3-6.3.4). More stringent hybridization conditions comprise use of the above buffer but at the temperature of 43°, 44°, 45°, 46°, 47°, 48°, 49°, 50°, 51°, 52°, 53°, 54°, and the like.

Stringent hybridization under aqueous conditions are 1% bovine serum albumin; 1 mM EDTA; 0.5 M NaHPO₄, pH 7.2, 7% sodium dodecyl sulfate, with overnight incubation at 65° C. More stringent aqueous hybridization conditions comprise the use of the above buffer, but at a temperature of 66°, 67°, 68°, 69°, 70°, 71°, 72°, 73°, 74°, 75°, and so on (see, e.g., (1993) Current Protocols in Molecular Biology, Suppl. 23, pages 6.3.3-6.3.4).

Increasing formamide concentration increases the stringency of hybridization. Mismatches between probe DNA and target DNA slows down the rate of hybridization by about 2-fold, for every 10% mismatching. Similarly, the melting temperature of mismatched DNA duplex decreases by about one degree centigrade for every 1.7% mismatching (Anderson (1999) Nucleic Acid Hybridization, Springer-Verlag, New York, N.Y., pp. 70-72; Tijssen (1993) Hybridization with Nucleic Acid Probes, Elsevier Publ. Co., Burlington, MA; Ross (ed.) (1998) Nucleic Acid Hybridization:Essential Techniques, John Wiley and Sons, Hoboken, NJ; U.S. Pat. No. 6,551,784 issued to Fodor, et al.).

This disclosure encompasses a variant first plasmid that hybridizes under stringent conditions to a second plasmid of the present disclosure, where both plasmids are functionally equivalent, and where hybridization is determinable by hybridizing the first plasmid directly to the second plasmid, or by hybridizing oligonucleotide probes spanning the entire length (individually or as a collection of probes) of the first variant plasmid to the second plasmid, and so on.

The skilled artisan will be able to adjust, or elevate, the hybridization temperature to allow distinction between a probe nucleic acid and a target nucleic acid where the sequences of the probe and target differ by 5-10 nucleotides, 10-15 nucleotides, 15-20 nucleotides, 20-25 nucleotides, 25-30 nucleotides, 30-35 nucleotides, 35-40 nucleotides, 40-45 nucleotides, 45-50 nucleotides, 50-55 nucleotides, 55-60 nucleotides, 60-65 nucleotides, 65-70 nucleotides, 70-80 nucleotides, and the like.

This disclosure provides polynucleotides that hybridize under stringent conditions to each of the polynucleotides described herein or their complements.

### III. SOME DETAILED EMBODIMENTS OF THIS DISCLOSURE.

### (a). Integration by site-specific recombination and by homologous recombination.

In some embodiments, nucleic acids, polynucleotides, bacterial genomes including listerial genomes, and bacteria including *Listeria* and *Bacillus anthracis*, of the present disclosure are modified by site-specific recombination and/or by homologous recombination. Site specific recombinases are described (see, e.g., Landy (1993) Curr. Op. Biotechnol. 3:699-707; Smith and Thorpe (2002) Mol. Microbiol. 44:299-307; Groth and Calos (2004) J. Mol. Biol. 335:667-678; Nunes-Duby, et al. (1998) Nucleic Acids Res. 26:391-406; Sauer (1993) Methods Enzymol. 225:890-900). Transposition is distinguished from site-specific recombination (see, e.g., Hallett and Sherratt (1997) FEMS Microbiol. Rev. 21:157-178; Grindley (1997) Curr. Biol. 7:R608-R612).

### A. Site-specific recombination.

The present disclosure provides systems for mediating site-specific integration into a nucleic acid, vector, or genome. By "system" is meant, a first nucleic acid encoding an integrase, as well as the expressed integrase polypeptide, a second nucleic acid encoding a phage attachment site (attPP'), and a third nucleic acid encoding a corresponding bacterial attachment site (attBB'). Generally, any given attPP' site corresponds to, or is compatible with, a particular attBB' site. The availability of the integration systems of the present disclosure allow for the integration of one or more nucleic acids into any given polynucleotide or genome.

The integration site of the present disclosure can be implanted at a pre-determined position in a listerial genome by way of site-specific integration at an existing site (e.g., at the tRNA^{Arg} integration site or the comK integration site). In addition, or in the alternative, the integration system site can be implanted at a pre-determined location by way of homologous integration.

Homologous recombination can result in deletion of material from the integration site, or no deletion of material, depending on the design of the regions of homology (the "homologous arms"). Any deletion that occurs, during homologous recombination corresponds to the region of the target DNA that resides in between regions of the target DNA that can hybridize with the "homologous arms." Homologous recombination can be used to implant an integration site (attBB') within a bacterial genome, for future use in site-specific recombination.

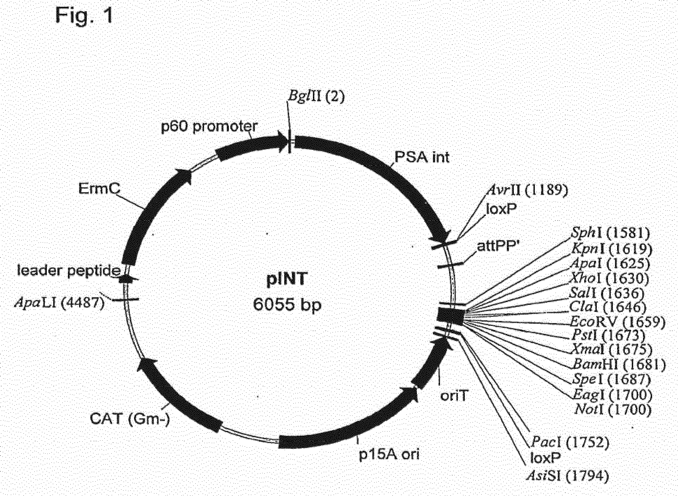

Figure 1 discloses a strategy for preparing the plasmid, pINT, for use in site-directed integration into a bacterial genome. pINT contains a chloramphenicol resistance gene and an erythromycin resistance gene (see, e.g., Roberts, et al. (1996) Appl. Environ. Microbiol. 62:269-270). When pINT mediates site-specific integration of a nucleic acid into the listerial genome, the antibiotic resistance genes can be subsequently eliminated by transient exposure to Cre recombinase. As shown in Figure 1, the antibiotic resistance genes reside in between a first loxP site and a second loxP site. Cre recombinase can catalyze removal of material residing in between the two loxP sites. Transient expression of Cre recombinase can be effected by electroporation by a plasmid encoding Cre recombinase, or by any number of other techniques.

The *Listeria* genome or chromosome of the present disclosure is modified using the plasmids pPL1, pPL2, and/or pINT1 (Lauer, et al. (2002) J. Bact. 184:4177-4186). The plasmid pPL1 (GenBank Acc. No. AJ417488) comprises a nucleic acid encoding U153 integrase, where this integrase catalyzes integration at the comK-attBB' location of the listerial genome (Lauer, et al. (2002) J. Bact. 184:4177-4186). The structure of comK is available (nucleotides 542-1114 of GenBank Acc. No.AF174588). pPL1 contains a number of restriction sites suitable for inserting a cassette. For example, in some embodiments, a cassette of the present disclosure encodes at least one heterologous antigen and a loxP-flanked region, where the loxP-flanked region comprises: a first nucleic acid encoding an integrase and a second nucleic acid encoding an antibiotic resistance factor. Some of the restriction sites are disclosed in Table 6. Restriction sites can also be introduced *de novo* by standard methods.

**Table 6. Restriction sites in pPL1 and pPL2.**

| pPL1 | | pPL2 | |
|---|---|---|---|
| Site | Cut position | Site | Cut position |
| *HindII* | 56 | *HindII* | 56 |
| *SmaI* | 95 | *SmaI* | 95 |
| *BamHI* | 99 | *BamHI* | 99 |
| *HindIII* | 69 | *ClaI* | 64 |
| *NotI* | 118 | *NotI* | 118 |
| *SalI* | 54 | *SalI* | 54 |
| *KpnI* | 37 | *SpeI* | 105 |
| *PstI* | 91 | *KpnI* | 37 |
| *SacI* | 139 | *PstI* | 91 |
| *AatII* | 5 and 175 | *SacI* | 139 |
| *BalI* | 490 (in chloramphenicol resistance gene) | *AatII* | 5 and 175 |
| *ScaI* | 340 (in chloramphenicol resistance gene) | *AvaI* | 48 and 93 |
| *BaeI* | 3942 and 3975 (in U153 integrase gene) | *BalI* | 490 (in chloramphenicol resistance gene) |
| *BsePI* | 3753 (in U153 integrase gene) | *ScaI* | 340 (in chloramphenicol resistance gene) |
| *MluI* | 4074 (in U153 integrase gene) | *AflIII* | 3259 and 4328 (in PSA integrase gene) |
| -- | -- | *SnaBI* | 4077 and 4177 (in PSA integrase gene) |
| -- | -- | *Eam1105I* | 3263 (in PSA integrase gene) |
| -- | -- | *BseYI* | 4357 (in PSA integrase gene) |
| -- | -- | *SwaI* | 3353 (in PSA integrase gene) |
| -- | -- | *BglII* | 4150 (in PSA integrase gene) |

The skilled artisan will appreciate that the techniques used for preparing pPL1 and pPL2, and for using pPL1 and pPL2 to mediate site-specific integration, can be applied to the integrases, phage attachment sites (attPP'), and bacterial attachment sites (attBB'), of the present disclosure.

pPL2 (GenBank Acc. No. AJ417499) comprises a nucleic acid encoding PSA integrase, where this integrase catalyzes integration at the tRNA^{Arg} gene of the *L. monocytogenes* genome (Lauer, et al. (2002) J. Bact. 184:4177-4186). The 74 nucleotide tRNA^{Arg} gene is found at nucleotide 1,266,675 to 1,266,748 of *L. monocytogenes* strain EGD genome (see, e.g., GenBank Acc. No. NC_003210), and at nucleotides 1,243,907 to 1,243,980 of *L. monocytogenes* strain 4bF265 (see, e.g., GenBank Acc. No. NC_002973). pPL2 contains a number of restriction sites suitable for inserting a cassette. The present disclosure provides a cassette encoding, e.g., a heterologous antigen and loxP-flanked region, where the loxP-flanked region comprises: a first nucleic acid encoding an integrase and a second nucleic acid encoding an antibiotic-resistance factor. Some of the restriction sites are disclosed in Table 6. Standard methods can be used to introduce other restriction sites *de novo.*

A first embodiment of site-specific recombination involves integrase-catalyzed site-specific integration of a nucleic acid at an integration site located at a specific tRNA^{Arg} region of the *Listeria* genome.

A second embodiment uses integration of a nucleic acid at the ComK region of the *Listeria* genome.

Additional embodiments comprise prophage attachment sites where the target is found at, e.g., tRNA-Thr4 of *L. monocytogenes* F6854 ϕ F6854.3 (nucleotides 277,661-277710 of *L. monocytogenes* EGD GenBank Acc. No. AL591983.1), tRNA-Lys4 of *L. innocua* 11262 ϕ 11262.1 (nucleotides 115,501-115,548 of GenBank Acc. No. AL596163.1); similar to *L. monocytogenes* 1262 of *L. innocua* 11262 phi11262.3; intergenic *of L. innocua* 11262 ϕ 11262.4 (nucleotides 162,123-162,143 of GenBank Acc. No. AL596169.1); and tRNA-Arg4 *of L. innocua* 11262 ϕ 11262.6 (nucleotides 15908-15922 of GenBank Acc. No. AL596173.1 of *L. innocua* or nucleotides 145,229-145,243 of GenBank Acc. No. AL591983.1 of *L. monocytogenes* EGD) (see, e.g., Nelson, et al. (2004) Nucleic Acids Res. 32:2386-2395)

A further embodiment of site-specific recombination comprises insertion of a loxP sites (or Frt site) by site-specific intregration at the tRNA^{Arg} region or ComK region, where insertion of the loxP sites is followed by Cre recombinase-mediated insertion of a nucleic acid into the *Listeria* genome.

pPL1 integrates at the comK-attBB' chromosomal location (6,101 bp; GenBank Acc. No. AJ417488). This integration is catalyzed by U153 integrase. The *L. monocytogenes* comK gene is disclosed (nucleotides 542-1114 of GenBank Acc. No. AF174588). The pPL1 integration site comprises nucleotides 2694-2696 of the plasmid sequence AJ417488. The following two PCR primers bracket the attachment site comK-attBB' of the *Listeria* genome: Primer PL60 is 5'-TGA AGT AAA CCC GCA CAC GATC-3' (SEQ ID NO:9); Primer PL61 is 5'-TGT AAC ATG GAG GTT CTG GCA ATC-3' (SEQ ID NO:10). The primer pair PL60 and PL61 amplifies comK-attBB' resulting in a 417 bp product in non-lysogenic strains, e.g., DP-L4056.

pPL2 integrates at the tRNA^{Arg}-attBB' chromosomal location (6,123 bp; GenBank Acc. No. AJ417449). This integration is catalyzed by PSA integrase. pPL2 is similar to pPL1, except that the PSA phage attachment site and U153 integrase of pPL1 were deleted and replaced with PSA integrase and the PSA phage attachment site. The pPL2 integration site comprises a 17 bp region that resides at at nucleotides 2852-2868 of the plasmid pPL2 (AJ417449), with the corresponding bacterial region residing at nucleotides 1,266,733-1,266,749 of *L. monocytogenes* strain EGD genome (GenBank Acc. No. NC_003210).

For listeriophage A118, a phage closely related to U153 listeriophage, the attB position resides at nucleotides 187-189 of the 573 bp comK ORF (Loessner, et al. (2000) Mol. Microbiol. 35:324-340). This 573 bp ORG (nucleotide 542-1114 of GenBank Acc. No. AF174588) and the attB site (nucleotide 701-757 of GenBank Acc. No. AF174588) are both disclosed in GenBank Acc. No. AF174588. The attP site resides in the listeriophage A118 genome at nucleotides 23500-23444 (GenBank Acc. No. AJ242593).

The present disclosure provides reagents and methods for catalyzing the integration of a nucleic acid, e.g., a plasmid, at an integration site in a *Listeria* genome. The *L. monocytogenes* genome is disclosed (see, e.g., GenBank Acc. No. NC_003210; GenBank Acc. No. NC_003198, He and Luchansky (1997) Appl. Environ. Microbiol. 63:3480-3487, Nelson, et al. (2004) Nucl. Acids Res. 32:2386-2395; Buchrieser, et al. (2003) FEMS Immunol. Med. Microbiol. 35:207-213; Doumith, et al. (2004) Infect. Immun. 72:1072-1083; Glaser, et al. (2001) Science 294:849-852).

Suitable enzymes for catalyzing integration of a nucleic acid into a *Listeria* genome include, e.g., U153 integrase (see, e.g., complement of nucleotides 2741-4099 of GenBank Acc. No. AJ417488; Lauer, et al. (2002) J. Bact. 184:4177-4186)) and PSA integrase (see, e.g., complement of nucleotides 19,413-20,567 of PSA phage genome (37,618 bp genome) (GenBank Acc. No. NC_003291)).

A similar or identical nucleotide sequence for tRNA^{Arg} gene, and for the core integration site that is found within this gene, has been disclosed for a number of strains of *L. monocytogenes.* The *L. monocytogenes* strain EGD complete genome (2,944,528 bp total) (GenBank Acc. No. NC_003210) contains an integration site in the tRNA^{Arg} gene. The 74 nucleotide tRNA^{Arg} gene is found at nucleotide 1,266,675 to 1,266,748 of GenBank Acc. No. NC_003210. Similarly, the tRNA^{Arg} gene occurs in *L. monocytogenes* strain 4bF265 (GenBank Acc. No. NC_002973) at nucleotides 1,243,907 to 1,243,980. The sequence of tRNA^{Arg} gene for *L. monocytogenes* strain WSLC 1042 is disclosed in Lauer, et al. (2002) J. Bact. 184:4177-4186. Lauer, *et al., supra,* disclose the bacterial core integration site and the corresponding phage core integration site.

Residence in a functional cluster establishes function of nucleic acids residing in that cluster. The function of a bacterial gene, or bacteriophage gene, can be identified according to its grouping in a functional cluster with other genes of known function, its transcriptional direction as relative to other genes of similar function, and occurrence on one operon with other genes of similar function (see, e.g., Bowers, et al. (2004) Genome Biology 5:R35.1-R35.13). For example, the gene encoding phage integrase has been identified in the genomes of a number of phages (or phages integrated into bacterial genomes), where the phage integrase gene resides in a lysogeny control cluster, where this cluster contains a very limited number of genes (three genes to nine genes) (see, e.g., Loessner, et al. (2000) Mol. Microbiol. 35:324-340; Zimmer, et al. (2003) Mol. Microbiol. 50:303-317; Zimmer, et al. (2002) J. Bacteriol. 184:4359-4368).

The phage attachment site (attPP') resides essentially immediately adjacent to the phage integrase gene. According to Zhao and Williams, the integrase gene (int) and attP are typically adjacent, facilitating their co-evolution (Zhao and Williams (2002) J. Bacteriol. 184:859-860). For example, in phiC31 phage, phage integrase is encoded by nucleotide (nt): 38,447 to 40,264, while the attP site resides nearby at nt 38,346 to 38,429. PhiC31 phage integrase does not require cofactors for catalyzing the integration reaction, and can function in foreign cellular environments, such as mammalian cells (see, e.g., Thorpe and Smith (1998) Proc. Natl. Acad. Sci. USA 95:5505-5510; Groth, et al. (2000) Proc. Natl. Acad. Sci. USA 97:5995-6000; GenBank Acc. No. AJ006589). Furthermore, for phage SM1, phage HP1, phage phi3626, for various actinomycete bacteriophages (intM gene), phage lambda, and for phage Aa phi23, the integrase gene and attP site are located immediately next to each other. The integrase gene and attP site can occur together in small group of genes known as a "lysogeny control cluster." Methods for determining the genomic location, approximate size, maximally active size, and/or minimal size of an attPP' site (or attP site) are available (see, e.g., Zimmer, et al. (2002) J. Bacteriol. 184:4359-4368; Siboo, et al. (2003) J. Bacteriol. 185:6968-6975; Mayer, et al. (1999) Infection Immunity 67:1227-1237; Alexander, et al. (2003) Microbiology 149:2443-2453; Hoess and Landy (1978) Proc. Natl. Acad. Sci. USA 75:5437-5441; Resch (2005) Sequence and analysis of the DNA genome of the temperate bacteriophage Aaphi23, Inauguraldissertation, Univ. Basel; Campbell (1994) Ann. Rev. Microbiol. 48:193-222).

The present disclosure provides a vector for use in modifying a listerial genome, where the vector encodes phiC31 phage integrase, phiC31 attPP' site, and where the listerial genome was modified to include the phiC31 attBB' site. A bacterial genome, e.g., of *Listeria* or *B. anthracis,* can be modified to include an attBB' site by homologous recombination. The phiC31 attBB' site is disclosed by Thorpe and Smith (1998) Proc. Natl. Acad. Sci. USA 95:5505-5510. The amino acid sequence of phiC31 integrase is disclosed below (GenBank Acc. No. AJ414670):
MTQGVVTGVDTYAGAYDRQSRERENSSAASPATQRSANEDKAADLQREVERDGGRFRFVGHFSEAPGT SAFGTAERPEFERILNECRAGRLNMIIVYDVSRFSRLKVMDAIPIVSELLALGVTIVSTQEGVFRQGN VMDLIHLIMRLDASHKESSLKSAKILDTKNLQRELGGYVGGKAPYGFELVSETKEITRNGRMVNVVIN KLAHSTTPLTGPFEFEPDVIRWWWREIKTHKHLPFKPGSQAAIHPGSITGLCKRMDADAVPTRGETIG KKTASSAWDPATVMRILRDPRIAGFAAEVIYKKKPDGTPTTKIEGYRIQRDPITLRPVELDCGPIIEP AEWYELQAWLDGRGRGKGLSRGQAILSAMDKLYCECGAVMTSKRGEESIKDSYRCRRRKVVDPSAPGQ HEGTCNVSMAALDKFVAERIFNKIRHAEGDEETLALLWEAARRFGKLTEAPEKSGERANLVAERADAL NALEELYEDRAAGAYDGPVGRKHFRKQQAALTLRQQGAEERLAELEAAEAPKLPLDQWFPEDADADPT GPKSWWGRASVDDKRVFVGLFVDKIVVTKSTTGRGQGTPIEKRASITWAKPPTDDDEDDAQDGTEDVA A (GenBank Acc. No. AJ414670) (SEQ ID NO:11)

The present disclosure provides the following relevant phiC31 target attBB' sites, and functional variants therof:
TGACGGTCTCGAAGCCGCGGTGCGGGTGCCAGGGCGTGCCCTTGGGCTCCCCGGGCGCGTAC TCCACCTCACCCATCTGGTCCA (SEQ ID NO:12) (see, e.g., Thorpe and Smith (1998) Proc. Natl. Acad. Sci. USA 95:5505-5510).
gtcgacgatgtaggtcacggtctcgaagccgcggtgcgggtgccagggcgtgcccttgggct ccccgggcgcgtactccacctcacccatctggtccatcatgatgaacgggtcgaggtggcgg tagttgatcccggcgaacgcgcggcgcaccgggaagccctcgccctcgaaaccgctgggcgc ggtggtcacggtgagcacgggacgtgcgacggcgtcggcgggtgcggatacgcggggcagcg tcagcgggttctcgacggtcacggcgggcatgtcgac (SEQ ID NO:13) (GenBank Acc. No. X60952)

Furthermore, this disclosure provides the following relevant phiC31 attPP' sites, and functional variants therof:
AAGGGGTTGTGACCGGGGTGGACACGTACGCGGGTGCTTACGACCGTCAGTCGC GCGAGCGCGAGAATTC (SEQ ID NO:14) (see, e.g., GenBank Acc. Nos. X57036 and AJ006589; Thorpe and Smith (1998) Proc. Natl. Acad. Sci. USA 95:5505-5510).

The present disclosure encompasses a vector that encodes a phage integrase and a functionally active attPP' site, but does not encode the phage integrase and attPP' site of pPL1. Also encompassed is a vector that encodes a phage integrase and a functionally active attPP' site, but does not encode the phage integrase and attPP' site of pPL2. Moreover, the present disclosure encompasses a vector that encodes a phage integrase and a functionally active attPP' site, but does not encode the phage integrase and attPP' site of pPL1 or of pPL2.

The present disclosure encompasses a vector useful for integrating a heterologous nucleic acid into a bacterial genome that encodes a phage integrase and a functionally active attPP' site, but does not encode the phage integrase and attPP' site of U153 phage. Also encompassed is a vector, useful for integrating a heterologous nucleic acid into a bacterial genome, that encodes a phage integrase and a functionally active attPP' site, but does not encode the phage integrase and attPP' site of PSA phage. Moreover, the present disclosure encompasses a vector, useful for integrating a heterologous nucleic acid into a bacterial genome, that encodes a phage integrase and a functionally active attPP' site, but does not encode the phage integrase and attPP' site from any of U153 phage and PSA phage. In another aspect, the present disclosure encompasses a vector, useful for integrating a heterologous nucleic acid into a bacterial genome, that encodes a phage integrase and a functionally active attPP' site, but does not encode the phage integrase and attPP' site of A118 phage. Further encompassed by this disclosure is a vector, useful for integrating a heterologous nucleic acid into a bacterial genome, that encodes a phage integrase and a functionally active attPP' site, but does not encode the phage integrase and attPP' site from any of A118 phage, U153 phage, or PSA phage.

### B. Homologous recombination.

The target site for homologous recombination can be an open reading frame, a virulence gene, a gene of unknown function, a pseudogene, a region of DNA shown to have no function, a gene that mediates growth, a gene that mediates spread, a regulatory region, a region of the genome that mediates listerial growth or survival, a gene where disruption leads to attenuation, an intergenic region, and the like.

To give a first example, once a nucleic acid encoding an antigen (operably linked with a promoter) is implanted into a virulence gene, the result is two fold, namely the inactivation of the virulence gene, plus the creation of an expressable antigen.

This disclosure provides a *Listeria* bacterium comprising an expression cassette, integrated via homologous recombination (or by allelic exchange, and the like), in a *listerial* virulence gene. Integration can be with or without deletion of a corresponding nucleic acid from the *listerial* genome.

The expression cassette can be operably linked with one or more promoters of the virulence gene (promoters already present in the parental or wild type *Listeria*)*.* Alternatively, the expression cassette can be operably linked with both: (1) One or more promoters supplied by the expression cassette; and (2) One or more promoters supplied by the parent or wild type *Listeria.*

In some embodiments, the expression cassette can be operably linked with one or more promoters supplied by the expression cassette, and not at all operably linked with any promoter of the *Listeria.*

Without implying any limitation, the virulence factor gene can be one or more of *actA, inlB,* both *actA* and *inlB,* as well as one or more of the genes disclosed in Table 3. In another aspect, homologous recombination can be at the locus of one or more genes that mediate growth, spread, or both growth and spread.

In another aspect, this disclosure provides a *Listeria* bacterium having a polynucleotide, where the polynucleotide comprises a nucleic acid (encoding a heterologous antigen) integrated at the locus of a virulence factor. In some embodiments, integration is by homologous recombination. In some embodiments, this disclosure provides integration in a regulatory region of the virulence factor gene, in an open reading frame (ORF) of the virulence factor gene, or in both a regulatory region and the ORF of the virulence factor. Integration can be with deletion or without deletion of all or part of the virulence factor gene.

Expression of the nucleic acid encoding the heterologous antigen can be mediated by the virulence factor's promoter, where this promoter is operably linked and with the nucleic acid. For example, a nucleic acid integrated in the *actA* gene can be operably linked with the *actA* promoter. Also, a nucleic acid integrated at the locus of the *inlB* gene can be operably linked and in frame with the *inlB* promoter. In addition, or as an alternative, the regulation of expression of the open reading frame can be mediated entirely by a promoter supplied by the nucleic acid.

The expression cassette and the above-identified nucleic acid can provide one or more listerial promoters, one or more bacterial promoters that are non-listerial, an actA promoter, an inlB promoter, and any combination thereof. The promoter mediates expression of the expression cassette. Also, the promoter mediates expression of the above-identified nucleic acid. Moreover, the promoter is operably linked with the ORF.

In some embodiments, integration into the virulence gene, or integration at the locus of the virulence gene, results in deletion of all or part of the virulence gene, and/or disruption of regulation of the virulence gene. In some embodiments, integration results in an attenuation of the virulence gene, or in inactivation of the virulence gene. Moreover, this disclosure provides a promoter that is prfA-dependent, a promoter that is prfA-independent, a promoter of synthetic origin, a promoter of partially synthetic origin, and so on.

Provided is a method for manufacturing the above-disclosed *Listeria.* Also provided are methods of using the above-disclosed *Listeria* for expressing the expression cassette or for expressing the above-identified nucleic acid. Moreover, in some embodiments, what is provided are methods for stimulating a mammalian immune system, comprising administering the above-disclosed *Listeria* to a mammal.

To give another example, once a bacterial attachment site (attBB') is implanted in a virulence gene, the result is two fold, namely the inactivation of that gene, plus the creation of a tool that enables efficient integration of a nucleic acid at that attBB' site.

In directing homologous integration of the pKSV7 plasmid, or another suitable plasmid, into the listerial genome, the present disclosure provides a region of homology that is normally at least 0.01 kb, more normally at least 0.02 kb, most normally at least 0.04 kb, often at least 0.08 kb, more often at least 0.1 kb, most often at least 0.2 kb, usually at least 0.4 kb, most usually at least 0.8 kb, generally at least 1.0 kb, more generally at least 1.5 kb, and most generally at least 2.0 kb.

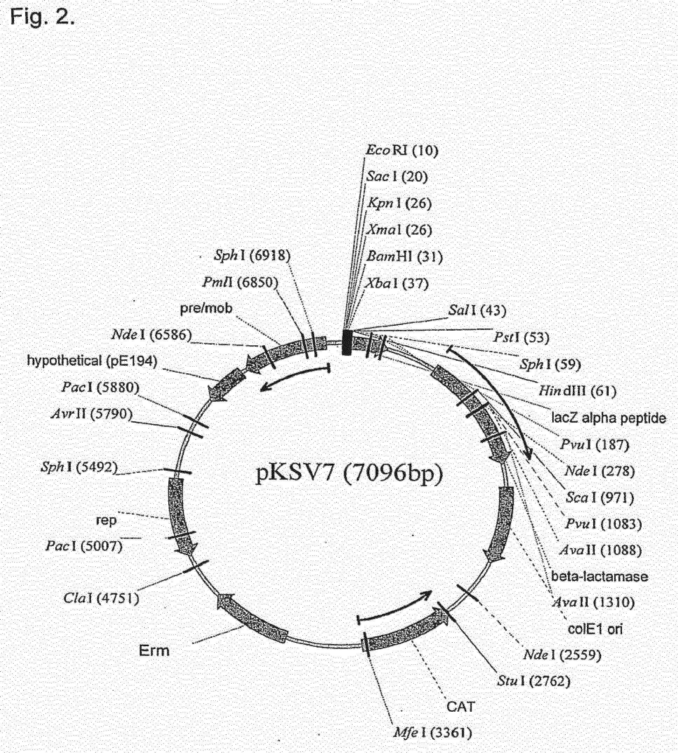

Figure 2 demonstrates a strategy using pKSV7 in homologous recombination into a bacterial genome. In Step 1, the plasmid crosses over with a region of homology in the genome. In Step 2, the plasmid integrates into the genome, producing a merodiploid intermediate. WXYZ represents any sequence in the pKSV7, such as an antibiotic-resistance encoding gene. Step 3 shows a second crossover, while Step 4 shows elimination of the "body" of the pKSV7 plasmid and elimination of WXYZ. Subsequent treatment with Cre recombinase, e.g., by transient expression of Cre recombination, catalyzes removal of material between the loxP sites.

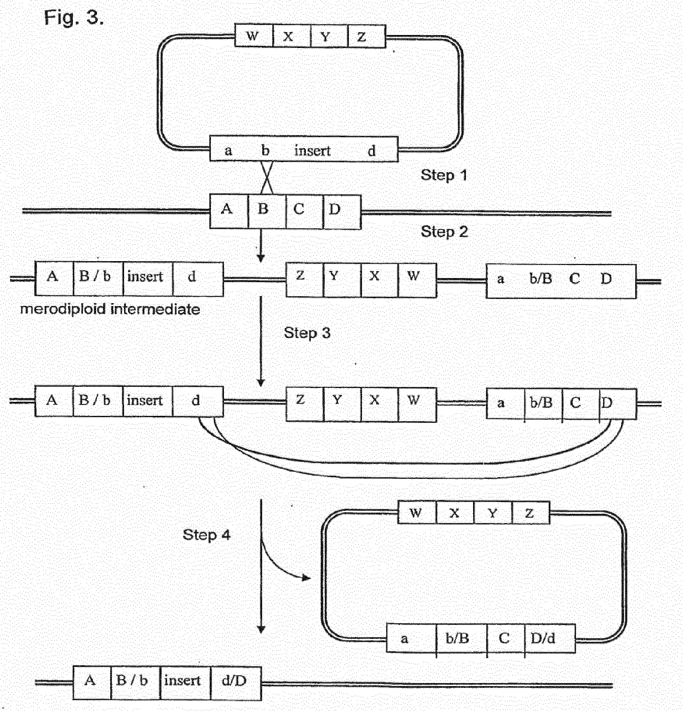

Figure 3 shows a method for preparing an insert, where the insert is placed into pKSV7. The insert mediates homologous recombination into a listerial genome, resulting in integration of various elements into the listerial geneome (nucleic acids encoding an antigen, loxP sites, and an antibiotic resistance gene). Subsequent treatment with Cre recombinase catalyzes removal of material between the loxP sites.

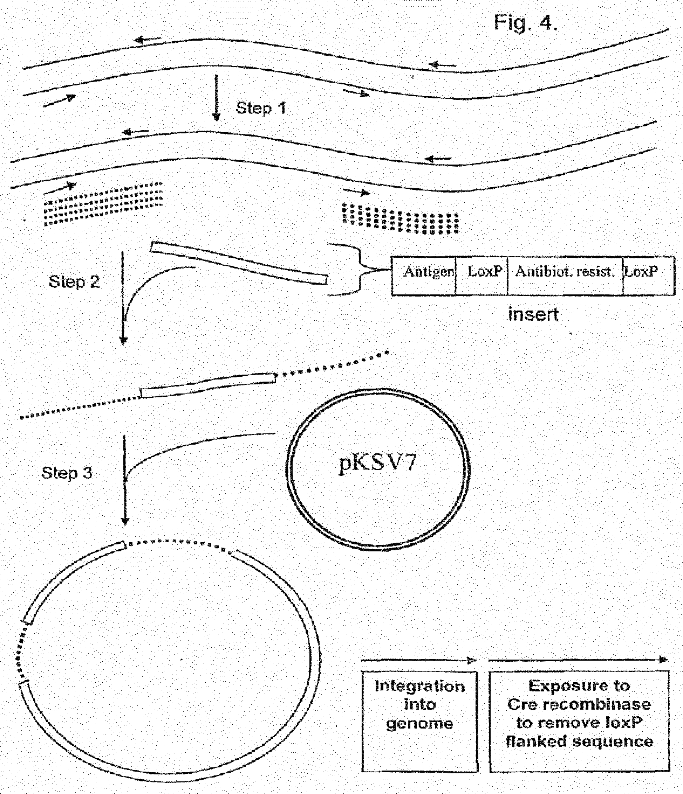

Figure 4 shows a method for preparing an insert, where the insert is placed into pKSV7. The insert mediates homologous recombination into a listerial genome, resulting in integration of various elements into the listerial genome (nucleic acid encoding an antigen). Nucleic acids encoding loxP sites and an antibiotic resistance gene are encoded by a modified pKSV7. Subsequent treatment with Cre recombinase, e.g., by transient expression of Cre recombination, catalyzes removal of material between the loxP sites.

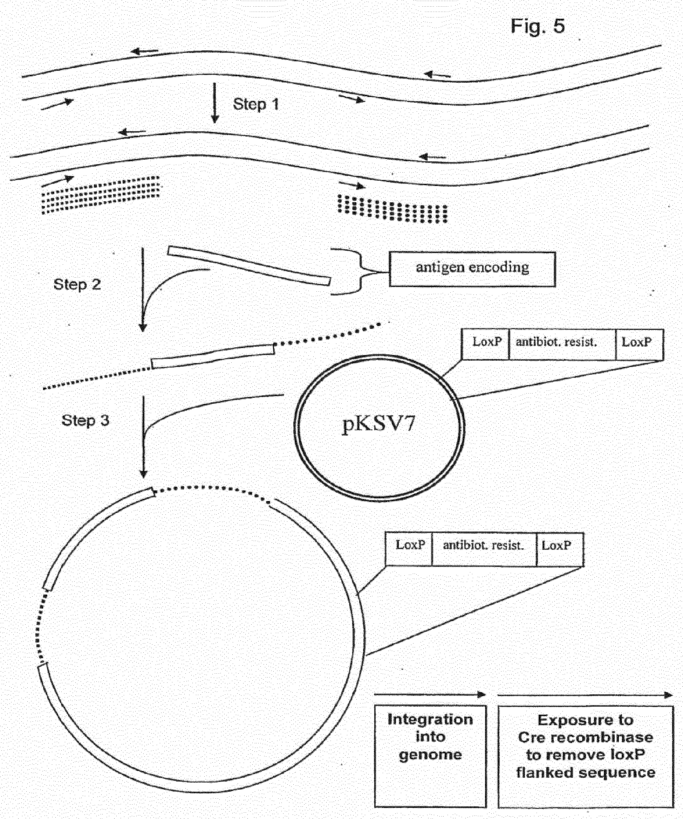

Figure 5 discloses an embodiment that results in only integration with no deletion. Subsequent treatment with Cre recombinase, e.g., by transient expression of Cre recombination, catalyzes removal of material between the loxP sites.

The reagents and methods of the present disclosure, prepared by homologous recombination, are not limited to use of pKSV7, or to derivatives thereof. Other vectors suitable for homologous recombination are available (see, e.g., Merlin, et al. (2002) J. Bacteriol. 184:4573-4581; Yu, et al. (2000) Proc. Natl. Acad. Sci. USA 97:5978-5983; Smith (1988) Microbiol. Revs. 52:1-28; Biswas, et al. (1993) J. Bact. 175:3628-3635; Yu, et al. (2000) Proc. Natl. Acad. Sci. USA 97:5978-5983; Datsenko and Wannter (2000) Proc. Natl. Acad. Sci. USA 97:6640-6645; Zhang, et al. (1998) Nature Genetics 20:123-128).

For integrating a nucleic acid by way of homologous recombination, bacteria are electroporated with a pKSV7, where the pKSV7 encodes a heterologous protein or where the pKSV7 contains an expression cassette. Bacteria are selected by plating on BHI agar media (or media not based on animal proteins) containing a suitable antibiotic, e.g., chloramphenicol (0.01 mg/ml), and incubated at the permissive temperature of 30°C. Single cross-over integration into the bacterial chromosome is selected by passaging several individual colonies for multiple generations at the non-permissive temperature of 41°C in medium containing the antibiotic. Finally, plasmid excision and curing (double cross-over) is achieved by passaging several individual colonies for multiple generations at the permissive temperature of 30°C in BHI media not containing the antibiotic.

Homologous recombination can be used to insert a nucleic acid into a target DNA, with or without deletion of material from the target DNA. A vector that mediates homologous recombination includes a first homologous arm (first nucleic acid), a second homologous arm (second nucleic acid), and a third nucleic acid encoding a heterologous antigen that resides in between the two homologous arms. Regarding the correspondence of the homologous arms and the target genomic DNA, the target regions can abut each other or the target regions can be spaced apart from each other. Where the target regions abut each other, the event of homologous recombination merely results in insertion of the third nucleic acid. But where the target regions are spaced apart from each other, the event of homologous recombination results in insertion of the third nucleic acid and also deletion of the DNA residing in between the two target regions.

Homologous recombination at the inlB gene can be mediated by pKSV7, where the pKSV7 contains the following central structure. The following central structure consists essentially of a first homologous arm (upstream of inlB gene in a *L*. *monocytogenes* genome), a region containing *KpnI* and *BamHI* sites (underlined), and a second homologous arm (downstream of inlB gene in L. *monocytogenes*). The region containing *KpnI* and *BamHI* sites is suitable for receiving an insert, where the insert also contains *KpnI* and *BamHI* sites at the 5'-prime and 3'-prime end (or 3'-end and 5'-end):

The following is the region of the "central region" that contains *KpnI* and *BamHI* sites for inserting an expression cassette: GGTACCTTGGTGAGCTC (SEQ ID NO:121).

The upstream homologous arm is shown below (upstream of inlB gene). The present sequences are from *L. monocytogenes* 10403S. The following provides comparison with another listerial strain, *L. monocytogenes* 4bF2365. In this strain, the inlB gene resides at nt 196,241-198,133 (GenBank AE017323; segment 2 of 10 segments). The upstream homologous arm, disclosed here for *L. monocytogenes* 10403 S, has a corresponding sequence in *L. monocytogenes* 4bF2365 at nt 194,932 to 196,240 (GenBank AE017323; segment 2 of 10 segments). The downstream homologous arm, disclosed here for *L*. *monocytogenes* 10403S, has a corresponding (but not totally identical) sequence in *L. monocytogenes* 4bF2365 at nt 198,134 to 199,629 (GenBank AE017323; segment 2 of 1 0 segments).

### (upstream homologous arm)

The downstream homologous arm is shown below (downstream of inlB gene):

### (downstream homologous arm)

Regarding insertion at the ActA gene in a listerial genome, the following discloses a suitable upstream and downstream homologous arms for mediating homologous-recombination integration at the ActA locus of *L*. *monocytogenes* 10403S:

### (upstream homologous arm)

The following discloses a suitable downstream homologous arm, for mediating insertion at the listerial ActA gene:

### (homologous downstream arm)

### (b). LoxP-flanked antibiotic resistance genes.

The present disclosure, in some embodiments, provides reagents and methods for mediating the rapid or efficient excision of a first nucleic acid from a bacterial genome. The method depends on recombinase-mediated excision, where the recombinase recognizes heterologous recombinase binding sites that flank the first nucleic acid. The heterologous recombinase binding sites can be, for example, a pair of loxP sites or a pair of frt sites. To provide a non-limiting example, the first nucleic acid can encode a selection marker such as an antibiotic resistance gene.

The reagents of this embodiment include plasmids comprising two heterologous recombinase binding sites that flank the first nucleic acid; a bacterial genome comprising two heterologous recombinase bindings sites that flank the first nucleic acid; and a bacterium containing a genome comprising two heterologous recombinase bindings sites that flank the first nucleic acid.

The method of this embodiment is set forth in the following steps:
i. Transfect a bacterium with a plasmid, where the plasmid can mediate integration of a first nucleic acid (flanked by a pair of heterologous recombinase binding sites) into the bacterial genome;
ii. Allow integration of the first nucleic acid (flanked by two heterologous recombinase binding sites) into the bacterial genome. Without implying any limitation as to the mechanism, integration can be by way of site-specific recombination or homologous recombination;
iii. Select for the bacterium containing the integrated first nucleic acid. Where the first nucleic acid encodes an antibiotic resistance gene, selection can involve culturing the bacterium in a medium containing the antibiotic. The selection step can result in a genotypically pure bacterium;
iv. Treat the genotypically pure bacterium with conditions that facilitate recombinase-catalyzed excision of the first nucleic acid from the bacterial genome. Where the pair of heterologous recombinase binding sites are loxP sites, the recombinase can be Cre recombinase. Cre recombinase can be introduced into the bacterium by transfecting with a plasmid encoding this enzyme. In one embodiment, expression of Cre recombinase is transient. Cre recombinase and FLP recombinase use the same enzymatic reaction mechanism, and mediate precise site-specific excision between a pair of their specific target sequences;
v. After allowing for Cre recombinase-catalyzed excision of the first nucleic, the bacterium can be cultured until the plasmid is lost by dilution or nuclease action;
vi. The resulting bacterium can be identified by the presence of the first nucleic acid in the genome. Also, the resulting bacterium can be identified by the loss of one of the two heterologous recombinase binding sites from the genome, that is, only one of the two sites will be left.

The above disclosure is not intended to limit the method to the recited steps, is not intended to limit the method to the disclosed order of steps, and is not intended to mean that all of these steps must occur. This disclosure is not necessarily limited to two heterologous recombinase binding sites. Polynucleotides containing two loxP sites and two Frt sites can be used, for example, where the two loxP sites flank a first nucleic acid, and the two Frt sites flank a second nucleic acid, and where transient expression of Cre recombinase allows excision of the first nucleic acid, and where transient expression of FLP recombinase (perhaps at a different time) results in excision of the second nucleic acid.

The canonical DNA target site for site-specific recombinases consists of two recombinase binding sites, where the two recombinase binding sites flank a core region (spacer region). The present disclosure provides two canonical DNA target sites (a pair of canonical DNA target sites), where the sites flank a first nucleic acid. LoxP is one type of canonical DNA target site. LoxP has two 13bp recombinase binding sites (13bp inverted repeats) that flank an 8bp core region or spacer. Thus, each loxP site is a sequence of 34 continuous nucleotides (34bp).

Cre recombinase and FLP recombinase are members of the integrase family of site-specific recombinases. Cre and FLP recombinase utilize a tyrosine residue to catalyze DNA cleavage. Cre recombinase recognizes lox sites, while FLP recombinase recognizes Frt sites.

Guidance for designing alternate and variant Lox sites and Frt sites is available. Where an alternate spacer region is desired, the skilled artisan will recognize that Cre recombinase-mediated excision is likely to require identical spacer regions in the first lox site and the second lox site (see, e.g., Araki, et al. (2000) Nucleic Acids Res. 30:e103; Nagy (2000) Genetics 26:99-109; Guo, et al. (1997) Nature 389:40-46; Sauer (1993) Methods Enzymol. 225:890-900; Langer, et al. (2002) Nucleic Acids Res. 30:3067-3077; Lath, et al. (2002) Nucleic Acids Res. 30:e115; Baer and Bode (2001) Curr. Opinion Biotechnol. 12:473-480; Nakano, et al. (2001) Microbiol. Immunol. 45:657-665).

The present disclosure contemplates a polynucleotide comprising a first lox site and a second lox site, where the pair of lox sites flanks a first nucleic acid, and where the first nucleic acid can encode, e.g., a selection marker, antibiotic resistance gene, regulatory region, or antigen. Also contemplated is a polynucleotide comprising a first lox site and a second lox site, where the pair of lox sites flanks a first nucleic acid, and where the first nucleic acid can encode, e.g., a selection marker, antibiotic resistance gene, regulatory region, or antigen.

The skilled artisan will readily appreciate that variant Lox sites where the recombinase binding site is under 13bp are available, in light of reports that Cre recombinase can function with a recombinase binding site as short as 8-10 bp.

An alternate lox site, loxY is available, to provide a non-limiting example. The present disclosure contemplates a polynucleotide comprising a first loxY site and a second loxY site, where the pair of loxY sites flanks a first nucleic acid, and where the first nucleic acid can encode, e.g., a selection marker, an antibiotic resistance gene, a regulatory region, or an antigen, and so on. Note also, that the core region of loxP has alternating purine and pyrimidine bases. However, this alternating pattern is necessary for recognition by Cre recombinase, and the present disclosure encompasses LoxP site variants with mutated core regions (see, e.g., Sauer (1996) Nucleic Acids Res. 24:4608-4613; Hoess, et al. (1986) Nucleic Acids Res. 14:2287-2300).

The Frt site contains three 13bp symmetry elements and one 8bp core region (48bp altogether). FLP recombinase recognizes Frt as a substrate, as well as variant Frt sites, including Frt sites as short as 34bp, and Frt site with variant core regions (see, e.g., Schweizer (2003) J. Mol. Microbiol. Biotechnol. 5:67-77; Bode, et al. (2000) Biol. Chem. 381:801-813).

The present disclosure provides a polynucleotide containing a first loxP site and an operably linked second loxP site, wherein the first and second loxP sites flank a first nucleic acid, to provide a non-limiting example. It will be appreciated that this disclosure encompasses other heterologous recombinase binding sites, such as variants of loxP, as well as frt sites and frt site variants.

The term "operably linked," as it applies to a first loxP site and a second loxP site, where the two loxP sites flank a first nucleic acid, encompasses the following. Here, "operably linked" means that Cre recombinase is able to recognize the first loxP site and the second loxP site as substrates, and is able to catalyze the excision of the first nucleic acid from the bacterial genome. The term "operably linked" is not to be limited to loxP sites, as it encompasses any "heterologous recombinase binding sites" such as other lox sites, or frt sites. Also, the term "operably linked" is not to be limited to recombinase-catalyzed excision, the term also embraces recombinase-catalyzed integration. Moreover, the term "operably linked" is not to be limited to nucleic acids residing in a genome -- also encompassed are nucleic acids residing in plasmids, intermediates used in genetic engineering, and the like.

Nucleic acids encoding recombinases are disclosed in Table 7A, and nucleic acid target sites recognized by these recombinases appear in Table 7B.

**Table 7A. Recombinases.**

| Recombinase | Location and GenBank Accession No. |
|---|---|
| Cre recombinase | Nucleotides 5347-6195 (exon 1) and 6262-6465 (exon 1) of GenBank Acc. No. AJ627603. |
| FLP recombinase | Complement of nucleotides 4426-5697 of GenBank Acc. No. AF048702. |
| FLP recombinase | Complement of nucleotides 6054-7325 of GenBank Acc. No. AY597273. |
| FLP recombinase | Nucleotides 5570-6318, 1-523 of GenBank Acc. No. J01347. The upstream region of the coding sequence begins at nucleotide 5570, while the downstream region of the coding sequence ends at nucleotide 523. |

| Table 7B. Binding sites for recombinases. | |
|---|---|
| Target site | Location and GenBank Accession No. |
| Target sites of FLP recombinase | |
| Frt | Nucleotides 260-307 of GenBank Acc. No. AY562545. |
| Frt | Nucleotides 464-511 of GenBank Acc.No. AY597272. |
| Frt | Nucleotides 3599-3646 of GenBank Acc. No. AY423864. |

| Target sites of Cre recombinase | |
|---|---|
| LoxP | Nucleotides 415-448 of GenBank Acc. No. AF143506. |
| LoxP | Nucleotides 118-151 of GenBank Acc. No. U51223. |
| LoxP | Nucleotides 1050-1083 of GenBank Acc. No. AY093430. |
| LoxP | Nucleotides 759-792 of GenBank Acc. No. AJ401047. |

Nucleic acid sequences encoding various antibiotic resistance factors are disclosed (Table 8). Typical sequences are those encoding resistance to an antibiotic that is toxic to *Listeria* e.g., chloramphenicol acetyltransferase (CAT) (Table 8).

A first nucleic acid encoding the antibiotic resistance factor is operably linked to a ribosome binding site, a promoter, and contains a translation start site, and/or a translation stop site, and is flanked by two heterologous recombinase binding sites.

This disclosure provides a polynucleotide containing a pair of operably linked loxP sites flanking a first nucleic acid, and a second nucleic acid (not flanked by the loxP sites), where the polynucleotide consists of a first strand and a second strand, and where the first nucleic acid has a first open reading frame (ORF) and the second nucleic acid has a second open reading frame (ORF). In one aspect, the first ORF is on the first strand, and the second ORF is also on the first strand. In another aspect, the first ORF is on the first strand and the second ORF is on the second strand. Yet another aspect provides a first ORF on the second strand and the second ORF on the first strand. Moreover, both ORFs can reside on the second strand. The present disclosure, in one aspect, provides a plasmid comprising the above-disclosed polynucleotide. Also provided is a *Listeria* containing the above-disclosed polynucleotide, where the polynucleotide can be on a plasmid and/or integrated in the genome. Each of the above-disclosed embodiments can comprise heterologous recombinase binding sites other than loxP. For example, lox variants, Frt sites, Frt variants, and recombinas binding sites unrelated to lox or Frt are available.

**Table 8. Antibiotic resistance genes.**

| Antibiotic resistance gene. | GenBank Accession No. |
|---|---|
| Chloramphenicol (chloramphenicol acetyltransferase; CAT). | Complement of nucleotides 312-971 of GenBank Acc. No. AJ417488 (pPL1 of Lauer, *et al*.). |
| Chloramphenicol (CAT). | Complement of nucleotides 4898-5548 of GenBank Acc. No. AJ417488 (pPL1 of Lauer, *et al*.). |
| Chloramphenicol (CAT). | Complement of nucleotides 312-971 of GenBank Acc. No. AJ417449 (pPL2 of Lauer, *et al*.). |
| Chloramphenicol (CAT). | Complement of nucleotides 4920-5570 of GenBank Acc. No. AJ417449 (pPL2 of Lauer, *et al*.). |
| Chloramphenicol (CAT). | Nucleotides 3021-3680 of GenBank Acc. No. AJ007660. |
| Penicillin (penicillin-binding protein 2). | Nucleotides 25-1770 of GenBank Acc. No. X59629. |
| Erythromycin (erythromycin resistance determinant). | Nucleotides 864-1601 of GenBank Acc. No. AY680862. |
| Ampicillin (penicillin beta-lActAmase). | Complement of nucleotides 3381-4311 of GenBank Acc. No.AJ401049. |
| Tetracycline (tetracycline resistance protein). | Complement of nucleotides of 4180-5454 of GenBank Acc. No. AY608912. |
| Gentamycin (aminoglycoside acetyltransferase). | Complement of nucleotides 1326-1859 of GenBank Acc. No. EVE414668. |

### (c). ActA fusion protein partners, and derivatives thereof.

### A. Modified ActA fusion protein partners, polynucleotides encoding the fusion proteins comprising modified ActA, and Listeria comprising the polynucleotides

### i. GENERAL.

The present disclosure, in certain aspects, provides a polynucleotide comprising a first nucleic acid encoding a modified ActA, operably linked and in frame with a second nucleic acid encoding a heterologous antigen. The disclosure also provides a *Listeria* containing the polynucleotide, where expression of the polynucleotide generates a fusion protein comprising the modified ActA and the heterologous antigen. The modified ActA can include the natural secretory sequence of ActA, a secretory sequence derived from another listerial protein, a secretory sequence derived from a non-listerial bacterial protein, or the modified ActA can be devoid of any secretory sequence.

The ActA-derived fusion protein partner finds use in increasing expression, increasing stability, increasing secretion, enhancing immune presentation, stimulating immune response, improving survival to a tumor, improving survival to a cancer, increasing survival to an infectious agent, and the like.

In one aspect, the disclosure provides a polynucleotide comprising a promoter operably linked to a nucleic acid sequence encoding a fusion protein, wherein the fusion protein comprises (a) modified ActA and (b) a heterologous antigen. In some embodiments, the promoter is ActA promoter. In some embodiments, the modified ActA comprises at least the first 59 amino acids of ActA. In some embodiments, the modified ActA comprises more than the first 59 amino acids of ActA. In some embodiments, the modified ActA is a fragment of ActA comprising the signal sequence of ActA (or is derived from a fragment of ActA comprising the signal sequence of ActA). In some embodiments, the modified ActA comprises at least the first 59 amino acids of ActA, but less than about the first 265 amino acids of ActA. In some embodiments, the modified ActA comprises more than the first 59 amino acids of ActA, but less than about the first 265 amino acids of ActA. In other words, in some embodiments, the modified ActA sequence corresponds to an N-terminal fragment of ActA (including the ActA signal sequence) that is truncated somewhere between amino acid 59 and about amino acid 265 of the Act A sequence. In some embodiments, the modified ActA comprises the first 59 to 200 amino acids of ActA, the first 59 to 150 amino acids of ActA, the first 59 to 125 amino acids of ActA, or the first 59 to 110 amino acids of ActA. In some embodiments, the modified ActA consists of the first 59 to 200 amino acids of ActA, the first 59 to 150 amino acids of ActA, the first 59 to 125 amino acids of ActA, or the first 59 to 110 amino acids of ActA. In some embodiments, the modified ActA comprises about the first 65 to 200 amino acids of ActA, about the first 65 to 150 amino acids of ActA, about the first 65 to 125 amino acids of ActA, or about the first 65 to 110 amino acids of ActA. In some embodiments, the modified ActA consists of about the first 65 to 200 amino acids of ActA, about the first 65 to 150 amino acids of ActA, about the first 65 to 125 amino acids of ActA, or about the first 65 to 110 amino acids of ActA. In some embodiments, the modified ActA comprises the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the modified ActA consists of the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the modified ActA comprises amino acids 1-100 of ActA. In some embodiments, the modified ActA consists of amino acids 1-100 of ActA. In some embodiments, the heterologous antigen is, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is a tumor antigen or is derived from a tumor antigen. In some embodiments, the heterologous antigen is, or is derived from, human mesothelin. In some embodiments, the nucleic acid sequence encoding the fusion protein is codon-optimized for expression in *Listeria.* The disclosure provides plasmids and cells comprising the polynucleotide. The disclosure further provides a *Listeria* bacterium e.g., *Listeria monocytogenes*) comprising the polynucleotide, as well as vaccines comprising the *Listeria.* In some embodiments, the genomic DNA of the *Listeria* comprises the polynucleotide. In some embodiments, the polynucleotide is positioned in the genomic DNA at the site of the *actA* gene or the site of the *inlB* gene. In some embodiments, the *Listeria* comprises a plasmid comprising the polynucleotide. The disclosure further provides immunogenic and pharmaceutical compositions comprising the *Listeria.* The disclosure also provides methods for stimulating immune responses to the heterologous antigen in a mammal (e.g., a human), comprising administering an effective amount of the *Listeria* (or an effective amount of a composition comprising the *Listeria*) to the mammal. For instance, the disclosure also provides methods for stimulating immune responses to an antigen from, or derived from, a cancer or infectious agent, comprising administering an effective amount of the *Listeria* (or a composition comprising the *Listeria*) to a mammal having the cancer or infectious agent, wherein the heterologous antigen shares at least one epitope with or is immunologically cross-reactive with the antigen from, or derived from, the cancer or infectious agent.

The preferred embodiments of antigen expression cassettes utilizing ActA-N-100 heterologous antigen fusion partner configurations is to functionally link the said antigen fusion construct with the native actA promoter and 5' untranslated region (UTR) RNA. PrfA-dependent transcription from the actA promoter results in synthesis of a 150 nucleotide 5' UTR RNA prior to the ActA protein GUG translation initiation site. *L. monocytogenes* mutants deleted of the actA promoter 5' UTR express low levels of ActA, resulting in a phenotype characterized by absence of intracellular actin recruitment, inability to spread from cell-to-cell, and attenuated, as compared to the wild-type parent bacterium (Wong et. al. defective Cellular Microbiology 6:155-166).

In some embodiments, the promoter used to express the fusion protein comprising the modified ActA and a heterologous antigen is an hly promoter. In another embodiments, the promoter is an actA promoter. These listerial promoters may, of instance, be derived from any strain of *Listeria monocytogenes.* For instance, the hly or actA promoter may be derived from the *L. monocytogenes* strain EGD. The EGD strain genome sequence is publicly available at GenBank Acc. No. AL591974 (complete genome segment 2/12) (hly promoter: nts 5581-5818; actA gene: nts 9456-11389), incorporated by reference herein in its entirety. Alternatively, the sequences used may be from another strain such as the *L. monocytogenes* strain 10403S.

In another aspect, the disclosure provides a polynucleotide comprising a first nucleic acid encoding a modified ActA, operably linked and in frame with, a second nucleic acid encoding a heterologous antigen. In some embodiments, the modified ActA comprises at least the first 59 amino acids of ActA, but less than about the first 265 amino acids of ActA. In some embodiments, the modified ActA comprises the first 59 to 200 amino acids of ActA, the first 59 to 150 amino acids of ActA, the first 59 to 125 amino acids of ActA, or the first 59 to 110 amino acids of ActA. In some embodiments, the modified ActA comprises the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the first nucleic acid encodes amino acids 1-100 of ActA. In some embodiments, the polynucleotide is genomic. In some alternative embodiments, the polynucleotide is plasmid-based. In some embodiments, the polynucleotide is operably linked with a promoter. For instance, the polynucleotide may be operably linked with one or more of the following: (a) actA promoter; or (b) a bacterial promoter that is not actA promoter. In some embodiments, the heterologous antigen is, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is, or is derived from human mesothelin. The disclosure further provides a *Listeria* bacterium e.g., *Listeria monocytogenes*) comprising the polynucleotide, as well as vaccines comprising the *Listeria.* In some embodiments, the *Listeria* is hMeso26 or hMeso38 (see Table 11 of Example VII, below). The disclosure also provides methods for stimulating immune responses to an antigen from, or derived from, a cancer or infectious agent, comprising administering the *Listeria* to a mammal having the cancer or infectious agent, wherein the heterologous antigen shares at least one epitope with or is immunologically cross-reactive with the antigen from, or derived from, the cancer or infectious agent.

In some embodiments, the *L. monocytogenes* native sequence encoding the first 100 amino acids of ActA is functionally linked in frame with a desired heterologous antigen sequence. In the some embodiments, the heterologous antigen sequence is synthesized according to the optimal codon usage of *L. monocytogenes,* a low GC percentage organism. In some embodiments, compositions utilizing the actA promoter together with the 5' untranslated sequences are desired.

In another aspect, the disclosure provides a polynucleotide comprising a first nucleic acid encoding a modified actA, where the modified actA comprises (a) amino acids 1-59 of actA, (b) an inactivating mutation in, deletion of, or truncation prior to, at least one domain for actA-mediated regulation of the host cell cytoskeleton, wherein the first nucleic acid is operably linked and in frame with a second nucleic acid encoding a heterologous antigen. In some embodiments, the domain is the cofilin homology region (KKRR (SEQ ID NO:23)). In some embodiments, the domain is the phospholipid core binding domain (KVFKKIKDAGKWVRDKI (SEQ ID NO:20)). In some embodiments, at least one domain comprises all four proline-rich domains (FPPPP (SEQ ID NO:21), FPPPP (SEQ ID NO:21), FPPPP (SEQ ID NO:21), FPPIP (SEQ ID NO:22)) of ActA. In some embodiments, the modified actA is actA-N100. In some embodiments, the polynucleotide is genomic. In some embodiments, the polynucleotide is not genomic. In some embodiments, the polynucleotide is operably linked with one or more of the following: (a) actA promoter; or (b) a bacterial (e.g., listerial) promoter that is not actA promoter. The disclosure further provides a *Listeria* bacterium (e.g., *Listeria monocytogenes*) comprising the polynucleotide, as well as vaccines comprising the *Listeria.* In some embodiments, the *Listeria* is is hMeso26 or hMeso38 (see Table 11 of Example VII, below). The disclosure also provides methods for stimulating immune responses to an antigen from, or derived from, a cancer or infectious agent, comprising administering the *Listeria* to a mammal having the cancer or infectious agent, wherein the heterologous antigen shares at least one epitope with or is immunologically cross-reactive with the antigen from, or derived from, the cancer or infectious agent. Insome embodiments, the stimulating is relative to immune response without administering the *Listeria.* In some embodiments, the cancer comprises a tumor or pre-cancerous cell. In some embodiments, the infectious agent comprises a virus, pathogenic bacterium, or parasitic organism. In some embodiments, the heterologous antigen is, or is derived from, a cancer cell, tumor, or infectious agent. In some embodiments, the heterologous antigen is immunologically cross-reactive with, or shares at least one epitope with, the cancer, tumor, or infectious agent. In some embodiments, the heterologous antigen is, or is derived from, human mesothelin.

In some embodiments, what is provided is a polynucleotide comprising a first nucleic acid encoding a modified ActA comprising at least amino acids 1-59 of ActA, further comprising at least one modification in a wild type ActA sequence, wherein the at least one modification is an inactivating mutation in, deletion of, or truncation at or prior to, a domain specifically used for ActA-mediated regulation of the host cell cytoskeleton, wherein the first nucleic acid is operably linked and in frame with a second nucleic acid encoding a heterologous antigen.

Also encompassed is the above polynucleotide, where the at least one modification is an inactivating mutation in, deletion of, or termination at, comprising the cofilin homology region KKRR (SEQ ID NO:23). Moreover, what is encompassed is the above polynucleotide where the at least one modification is an inactivating mutation in, deletion of, or termination at, comprising the phospholipid core binding domain (KVFKKIKDAGKWVRDKI (SEQ ID NO:20)).

In yet another aspect, what is contemplated is the above polynucleotide, wherein the at least one modification comprises an inactivating mutation in, or deletion of, in each of the first proline-rich domain (FPPPP (SEQ ID NO:21)), the second proline-rich domain (FPPPP (SEQ ID NO:21)), the third proline-rich domain (FPPPP (SEQ ID NO:21)), and the fourth proline-rich domain (FPPIP (SEQ ID NO:22)), or a termination at the first proline-rich domain. In another aspect, what is provided is the above polynucleotide where the modified ActA is ActA-N100.

Yet another embodiment provides a *Listeria* bacterium comprising one or more of the above polynucleotide. The polynucleotide can be genomic, it can be plasmid-based, or it can reside on both a plasmid and the listerial genome. Also provided is the above *Listeria* where the polynucleotide is not genomic, as well as the above *Listeria* where the polynucleotide is not plasmidic. The *Listeria* can be *Listeria monocytogenes, L. innocua,* or some other listerial species.

Moreover, what is supplied by yet another embodiment, is a method of stimulating immune response to an antigen from, or derived from, a tumor, cancer cell, or infectious agent, comprising administering to a mammal the above-disclosed *Listeria* and where the heterologous antigen is shares at least one epitope with the antigen derived from the tumor, cancer cell, or infectious agent. What is also supplied is the above method, where the stimulating is relative to antigen-specific immune response in absence of the administering the *Listeria* (specific to the antigen encoded by the second nucleic acid).

Optionally, the heterologous antigen can be identical to the antigen from (or derived from) the tumor, cancer cell, or infectious agent.

ActA-N100 encompasses a nucleic acid encoding amino acids 1-100 of ActA, as well as the polypeptide expressed from this nucleic acid. (This numbering includes all of the secretory sequence of ActA.) What is provided is a polynucleotide comprising a first nucleic acid encoding ActA-N100 operably linked and in frame with a second nucleic acid encoding a heterologous antigen.

Yet another embodiment provides a *Listeria* bacterium comprising one or more of the above polynucleotide. The polynucleotide can be genomic, it can be plasmid-based, or it can reside on both a plasmid and the listerial genome. Also provided is the above *Listeria* where the polynucleotide is not genomic, as well as the above *Listeria* where the polynucleotide is not plasmidic. The *Listeria* can be *Listeria monocytogenes, L. innocua,* or some other listerial species.

Methods for using ActA-N100 are also available. Provided is a method for stimulating immune response to an antigen from, or derived from, a tumor, cancer cell, or infectious agent, comprising administering to a mammal the above-disclosed *Listeria,* and wherein the heterologous antigen is shares at least one epitope with the antigen derived from the tumor, cancer cell, or infectious agent. What is also provided is the above method, where the stimulating is relative to antigen-specific immune response in absence of the administering the *Listeria* (specific to the antigen encoded by the second nucleic acid). Alternatively, the heterologous antigen can be identical to the antigen from, or derived from, the tumor, cancer cell, or infectious agent.

In some embodiments, the modified ActA consists of a fragment of ActA or other derivative of ActA in which the ActA signal sequence has been deleted. In some embodiments, the polynucleotides comprising nucleic acids encoding a fusion protein comprising such a modified ActA and the heterologous antigen further comprise a signal sequence that is not the ActA signal sequence. The ActA signal sequence is MGLNRFMRAMMVVFITANCITINPDIIFA (SEQ ID NO:125). In some embodiments, the modified ActA consists of amino acids 31-100 of ActA (i.e., ActA-N100 deleted of the signal sequence).

The present disclosure provides a polynucleotide comprising a first nucleic acid encoding a modified ActA, operatively linked and in frame with a second nucleic acid encoding a heterologous antigen. ActA contains a number of domains, each of which plays a part in binding to a component of the mammalian cytoskeleton, where the present disclosure contemplates removing one or more of these domains.

ActA contains a number of domains, including an N-terminal domain (amino acids 1-234), proline-rich domain (amino acids 235-393), and a C-terminal domain (amino acids 394-610). The first two domains have distinct effects on the cytoskeleton (Cicchetti, et al. (1999) J. Biol. Chem. 274:33616-33626). The proline-rich domain contains four proline-rich motifs. The proline-rich motifs are docking sites for the Ena/VASP family of proteins. Deletion of proline-rich domains of ActA strongly reduces actin filament assembly (Cicchetti, et al. (1999) J. Biol. Chem. 274:33616-33626). Machner, *et al.,* provides guidance for designing mutated proline-rich motifs that can no longer dock, where this guidance can be put to use for embodiments of the present disclosure (Machner, et al. (2001) J. Biol. Chem. 276:40096-40103). For example, the phenylalanine of the proline-rich motifs is critical. The present disclosure, in an alternate embodiment, provides a polynucleotide comprising a first nucleic acid encoding ActA, where the codons for the phenylalaline in each proline-rich motif is changed to an alanine codon, operably linked and in frame with a second nucleic acid encoding at least one heterologous antigen. In another aspect, the first nucleic acid encoding ActA comprises a proline to alanine mutation in only the first proline-rich motif, in only the second proline-rich motif, in only the third proline-rich motif, in only the fourth proline-rich motif, or any combination thereof. In another aspect, a nucleic acid encoding an altered ActA can encompass a mutation in a codon for one or more proline-rich motifs in combination with a mutation or deletion in, e.g., cofilin homology region and/or the core binding sequence for phospholipids interaction.

What is also embraced, is a mutation of proline to another amino acid, e.g., serine. The above guidance in designing mutations is not to be limited to changing the proline-rich motifs, but applies as well to the cofilin homology region, the core binding sequence for phospholipids interaction, and any other motifs or domains that contribute to interactions of ActA with the mammalian cytoskeleton.

ActA contains a domain that is a "core binding sequence for phospholipids interaction" at amino acids 185-201 of ActA, where the function in phospholipids binding was demonstrated by binding studies (Cicchetti, et al. (1999) J. Biol. Chem. 274:33616-33626). According to Cicchetti, *et al.,* supra, phospholipids binding regulates the activities of actin-binding proteins.

ActA contains a cofilin homology region KKRR (SEQ ID NO:23). Mutations of the KKRR (SEQ ID NO:23) region abolishes the ActA's ability to stimulate actin polymerization (see, e.g., Baoujemaa-Paterski, et al. (2001) Biochemistry 40:11390-11404; Skoble, et al. (2000) J. Cell. Biol. 150:527-537; Pistor, et al. (2000) J. Cell Sci. 113:3277-3287).

The following concerns expression, by *L. monocytogenes,* of truncated actA derivatives truncated down from amino acid 263 to amino acid 59. Unlike other truncated derivatives, actA N59 was not expressed whereas all of the longer ones were expressed (Skoble, J. (unpublished)). The next longest derivative tested was actA-N101. Fusion protein constructs expressed from actA promoter, consisting of a first fusion protein partner that is actA secretory sequence, and a second fusion protein partner, resulted in much less protein secretion than where the first fusion protein partner was actA-N100. Regarding deletion constructs, good expression was also found where the first fusion protein partner was soluble actA with amino acids 31-59 deleted. Moreover, good expression was found where the first fusion protein partner was soluble actA with amino acids 31-165 deleted (Skoble, J. (unpublished)).

The present disclosure, in certain embodiments, provides a polynucleotide comprising a first nucleic acid encoding a modified ActA, comprising at least one modification, wherein the at least one modification is an inactivating mutation in, deletion of, or termination of the ActA polypeptide sequence at or prior to, a domain required for ActA-mediated regulation of the host cell cytoskeleton, and a second nucleic acid encoding a heterologous antigen. The modified ActA can be one resulting in impaired motility and/or decreased plaque size, and includes a nucleic acid encoding one of the mutants 34, 39, 48, and 56 (Lauer, et al. (2001) Mol. Microbiol. 42:1163-1177). The present invention also contemplates a nucleic acid encoding one of the ActA mutants 49, 50, 51, 52, and 54. Also provides is a nucleic acid encoding one of the ActA mutants 40, 41, 42, 43, 44, 45, 45, and 47. Provided are mutants in the actin monomer binding region AB region, that is, mutants 41, 42, 43, and 44 (Lauer, et al. (2001) Mol. Microbiol. 42:1163-1177).

In another aspect, the modified ActA of the present invention can consist a deletion mutant, can comprise a deletion mutant, or can be derived from a deletion mutant ActA that is unable to polymerize actin in cells and/or unable to support plaque formation, or supported only sub-maximal plaque formation. These ActA deletion mutants include the nucleic acids encoding Δ31-165; Δ136-200; Δ60-165; Δ136-165; Δ146-150, Δ31-58; Δ60-101; and Δ202-263 and the like (Skoble, et al. (2000) J. Cell Biol. 150:527-537). Encompassed are nucleic acids encoding ActA deletion mutants that have narrower deletions and broader deletions. The following set of examples, which discloses deletions at the cofilin homology region, can optionally to each the ActA deletions set forth herein. The present invention provides nucleic acids encoding these deletions at the cofilin homology region: Δ146-150; Δ145-150; Δ144-150; Δ143-150; Δ142-150; Δ141-150; Δ140-150; Δ139-150; Δ138-150; Δ137-150; Δ136-150, and the like. Also encompassed are nucleic acids encoding ActA with the deletions: Δ146-150; Δ146-151; Δ146-152; Δ146-153; Δ146-154; Δ146-155; Δ146-156; Δ146-157; Δ146-158; Δ146-159; Δ146-160; and so on. Moreover, also embraced are nucleic acids encoding the deletion mutants: Δ146-150; Δ145-151; Δ144-152; Δ143-153; Δ142-154; Δ141-155; Δ140-156; Δ139-157; Δ138-158; Δ137-159; Δ136-160, and the like. Where there is a deletion at both the N-terminal end of the region in question, and at the C-terminal end, the sizes of these two deletions need not be equal to each other.

Deletion embodiments are also provided, including but not limited to the following. What is provided is a nucleic acid encoding full length actA, an actA missing the transmembrane anchor, or another variant of actA, where the actA is deleted in a segment comprising amino acids (or in the alternative, consisting of the amino acids):
31-59, 31-60, 31-61, 31-62, 31-63, 31-64, 31-65, 31-66, 31-67, 31-68, 31-69, 31-70, 31-71, 31-72, 31-73, 31-74, 31-75, 31-76, 31-77, 31-78, 31-79, 31-80, 31-81, 31-82, 31-83, 31-84, 31-85, 31-86, 31-87, 31-88, 31-89, 31-90, 31-91, 31-92, 31-93, 31-94, 31-95, 31-96, 31-97, 31-98, 31-99, 31-100, 31-101, 31-102, 31-103, 31-104, 31-105, 31-106, 31-107, 31-108, 31-109, 31-110, 31-111, 31-112, 31-113, 31-114, 31-115, 31-116, 31-117, 31-118, 31-119, 31-120, 31-121, 31-122, 31-123, 31-124, 31-125, 31-126, 31-127, 31-128, 31-129, 31-130, 31-131, 31-132, 31-133, 31-134, 31-135, 31-136, 31-137, 31-138, 31-139, 31-140, 31-141, 31-142, 31-143, 31-144, 31-145, 31-146, 31-147, 31-148, 31-149, 31-150, 31-151, 31-152, 31-153, 31-154, 31-155, 31-156, 31-157, 31-158, 31-159, 31-160, 31-161, 31-162, 31-163, 31-164, 31-165, and the like.

In yet another aspect, what is supplied is a polypeptide containing a first nucleic acid encoding an actA derivative, and a second nucleic acid encoding a heterologous nucleic acid, where the actA derivative is soluble actA comprising a deletion or conservative amino acid mutation, and where the deletion or conservative amino acid mutation comprises (or in another embodiment, where the deletion consists of) amino acid: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97,98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, and so on.

What is also provided, in other embodiments, is a polynucleotide comprising a first nucleic acid encoding an altered ActA, operably linked and in frame with a second nucleic acid, encoding a heterlogous antigen, where the first nucleic acid is derived from, for example, ΔActA3 (amino acids 129-153 deleted); ΔActA9 (amino acids 142-153 deleted); ΔActA6 (amino acids 68-153 deleted); ΔActA7 (amino acids 90-153 deleted); or ΔActA8 (amino acids 110-153 deleted), and so on (see, e.g., Pistor, et al. (2000) J. Cell Science 113:3277-3287).

A number of derivatives of ActA, encompassing the start methionine (N-terminus) and prematurely terminated, resulting in a novel C-terminus. Some of these derivatives are reported in Skoble, et al. (2000) J. Cell Biol. 150:527-537). Nucleic acids encoding these derivatives were introduced into *L. monocytogenes,* to test expression. The ActA derivative terminating at amino acid 59 (ActA-N59) was not expressed by *L. monocytogenes.* In contrast, ActA-N101, and longer derivatives of ActA, were expressed. Fusion proteins (expressed from the ActA promoter) consisting of only the ActA signal sequence and a fusion protein partner, showed much less secretion than fusion proteins consisting of ActA-N100 and a fusion protein partner.

The truncation, deletion, or inactivating mutation, can reduce or eliminate the function of one or more of ActA's four FP₄ domains ((E/D)FPPPX(D/E)) (SEQ ID NO:135). ActA's FP₄ domains mediate binding to the following proteins: mammalian enabled (Mena); Ena/VASP-like protein (Evl); and vasodilator-stimulated phosphoprotein (VASP) (Machner, et al. (2001) J. Biol. Chem. 276:40096-40103). Hence, the nucleic acid of the present invention encodes a truncated ActA, deleted or mutated in one or more of its FP₄ domains, thereby reducing or preventing biding to Mena, Evl, and/or VASP. Provided is a nucleic acid encoding a truncated, partially deleted or mutated ActA and a heterologous antigen, where the truncation, partial deletion, or mutation, occurs at amino acids 236-240; amino acids 270-274; amino acids 306-310; and/or amino acids 351-355 of ActA (numbering of Machner, et al. (2001) J. Biol. Chem. 276:40096-40103).

The present invention provides a polynucleotide comprising a first nucleic acid encoding an ActA variant, and a second nucleic acid encoding at least one heterologous antigen, where the ActA variant is ActA deleted in or mutated in one "long repeat," two long repeats, or all three long repeats of ActA. The long repeats of ActA are 24-amino acid sequences located in between the FP₄ domains (see, e.g., Smith, et al. (1996) J. Cell Biol. 135:647-660). The long repeats help transform actin polymerization to a force-generating mechanism.

As an alternate example, what is provided is a nucleic acid encoding the following ActA-based fusion protein partner, using consisting language: What is provided is a nucleic acid encoding a fusion protein partner consisting of amino acids 1-50 of human actA (for example, GenBank Acc. No. AY512476 or its equivalent, where numbering begins with the start amino acid), amino acids 1-60; 1-61; 1-62; 1-63; 1-64; 1-65; 1-66; 1-67; 1-68; 1-69; 1-70; 1-72; 1-73; 1-74; 1-75; 1-76; 1-77; 1-78; 1-79; 1-80; 1-81; 1-82; 1-83; 1-84; 1-85; 1-86; 1-87; 1-88; 1-89; 1-90; 1-91; 1-92; 1-93; 1-94; 1-95; 1-96; 1-97; 1-98; 1-99; 1-100; 1-101; 1-102; 1-103; 1-104; 1-105; 1-106; 1-107; 1-108; 1-109; 1-110; 1-111; 1-112; 1-113; 1-114; 1-115; 1-116; 1-117; 1-118; 1-119; 1-120; 1-121; 1-122; 1-123; 1-124; 1-125; 1-126; 1-127; 1-128; 1-129; 1-130; 1-131; 1-132; 1-133; 1-134; 1-135; 1-136; 1-137; 1-138; 1-139; 1-140; 1-141; 1-142; 1-143; 1-144; 1-145; 1-146; 1-147; 1-148; 1-149; 1-150; 1-151; 1-152; 1-153; 1-154; 1-155; 1-156; 1-157; 1-158; 1-159; 1-160, and so on.

As yet another alternate example, what is provided is a nucleic acid encoding the following ActA-based fusion protein partner, using comprising language: What is provided is a nucleic acid encoding a fusion protein partner comprising amino acids 1-50 of human actA (for example, GenBank Acc. No. AY512476 or its equivalent, where numbering begins with the start amino acid), amino acids 1-60; 1-61; 1-62; 1-63; 1-64; 1-65; 1-66; 1-67; 1-68; 1-69; 1-70; 1-72; 1-73; 1-74; 1-75; 1-76; 1-77; 1-78; 1-79; 1-80; 1-81; 1-82; 1-83; 1-84; 1-85; 1-86; 1-87; 1-88; 1-89; 1-90; 1-91; 1-92; 1-93; 1-94; 1-95; 1-96; 1-97; 1-98; 1-99; 1-100; 1-101; 1-102; 1-103; 1-104; 1-105; 1-106; 1-107; 1-108; 1-109; 1-110; 1-111; 1-112; 1-113; 1-114; 1-115; 1-116; 1-117; 1-118; 1-119; 1-120; 1-121; 1-122; 1-123; 1-124; 1-125; 1-126; 1-127; 1-128; 1-129; 1-130; 1-131; 1-132; 1-133; 1-134; 1-135; 1-136; 1-137; 1-138; 1-139; 1-140; 1-141; 1-142; 1-143; 1-144; 1-145; 1-146; 1-147; 1-148; 1-149; 1-150; 1-151; 1-152; 1-153; 1-154; 1-155; 1-156; 1-157; 1-158; 1-159; 1-160, and so on.

The contemplated nucleic acids encoding an actA-based fusion protein partner include nucleic acids encoding the actA-based fusion protein partner, where one or more nucleotides is altered to provide one or more conservative amino acid changes. What is contemplated is one conservative amino acid change, two, three, four, five, six, seven, eight, nine, ten, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or more, conservative amino acid changes. Moreover, what is contemplated is a nucleic acid encoding the actA-based fusion protein partner, comprising at least one mutation encoding at least one short deletion, or at least one short insertion, or any combination thereof

Regarding the identity of the nucleic acid encoding ActA, and derivatives thereof, the codon for the start methionine can be a valine start codon. In other words, *Listeria* uses a valine start codon to encode methionine.

The contemplated invention encompasses ActA, and ActA deleted in one or more cytoskeleton-binding domains, ActA-N100 fusion protein partners, from all listerial species, including *L. monocytogenes* and *L. ivanovii* (Gerstel, et al. (1996) Infection Immunity 64:1929-1936; GenBank Acc. No. X81135; GenBank Acc. No. AY510073).

In some embodiments, the modified ActA consists of a sequence having at least about 80% sequence identity, at least about 85% sequence identity, at least about 90% sequence identity, or at least about 95% sequence identity to a fragment of ActA comprising more than the first 59 amino acids of ActA and less than the first 380 amino acids of ActA. For instance, in some embodiments, the modified ActA consists of a sequence having at least about 80% sequence identity, at least about 85% sequence identity, at least about 90% sequence identity, or at least about 95% sequence identity to ActA-N100. In some embodiments, the nucleic acid molecule encoding the modified ActA hybridizes under stringent conditions to a nucleic acid molecule encoding the ActA-N100 sequence, or its complement.

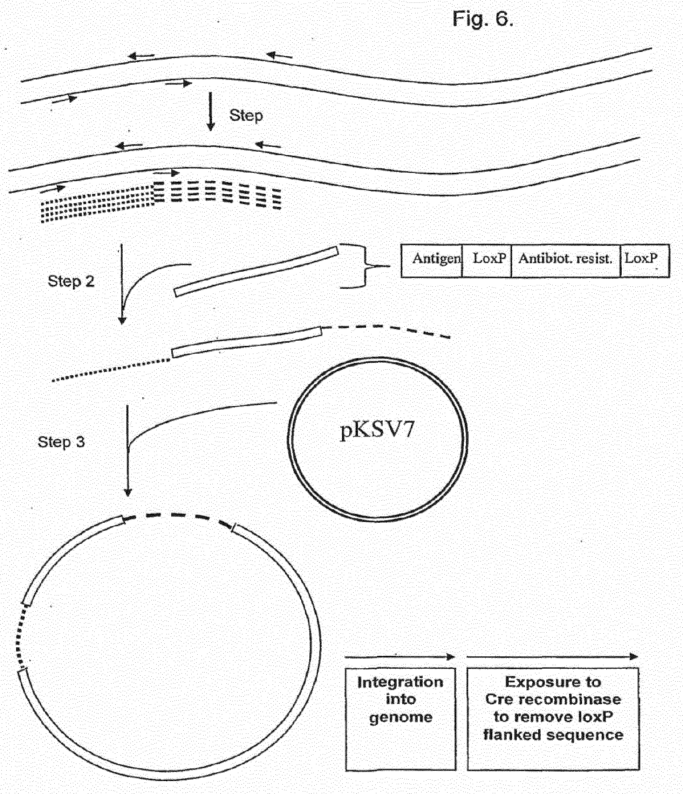

The present invention, in some embodiments, encompasses a polynucleotide comprising a first nucleic acid encoding actA-N100 operably linked and in frame with a second nucleic acid encoding a heterologous antigen, such as human mesothelin, or a derivative thereof. Human mesothelin was expressed from a number of constructs, where these constructs were created by site-directed integration or homologous integration into the *Listeria* genome. Some of these constructs are shown in Figure 6. Figure 6 discloses naturally occurring human mesothelin, which contains a signal sequence and a GPI-sequence. The signal sequence and GPI-sequence was deleted in the following examples, where the naturally occurring signal sequence was replaced with the *Bacillus anthracis* Protective Antigen secretory sequence (BaPA), with LLO-62, with LLO-60_{codon optimized} (LLO-60ₒₚₜ), or with ActA-N100 (Figure 6). The sequence of ActA-N100 includes the naturally occurring secretory sequence of ActA.

### ii. ABNORMAL CELL PHYSIOLOGY PRODUCED BY WILD TYPE ActA.

The modified ActA, of at least some embodiments of the invention, is changed to reduce or eliminate its interaction with the mammalian cytoskeleton. While the physiological function of ActA is to bind to the mammalian cytoskeleton and to allow actin-mediated movement of the *Listeria* bacterium through the cytoplasm, this binding is reduced or eliminated in the ActA component of the fusion protein.

Expression of soluble ActA in mammalian cytoplasm, by way of eukaryotic expression vectors, results in abnormalities of the cytoskeleton, e.g., "redistribution of F-actin," and sequestration of the recombinant ActA at the location of "membrane protrusions." In other words, the normal location of F-actin was changed, where its new location was in membrane protrusions. Moreover, "ActA stain co-distributed with that of F-actin in membrane protrusions." Other abnormalities in mammalian cells included "loss of stress fibres." It was observed that "the amino-terminal part of ActA is involved in the nucleation of actin filaments while the segment including the proline-rich repeat region promotes or conrols polymerization" (Friederich, et al. (1995) EMBO J. 14:2731-2744). Moreover, according to Olazabal and Machesky, overexpressing a protein demonstrated to be similar to ActA, the WASP protein, causes "defects in actin organization that lead to malfunctions of cells" (Olazabal and Machesky (2001) J. Cell Biol. 154:679-682). The title of a publication ("*Listeria* protein ActA mimics WASP family proteins") indicates this similarity (Boujemaa-Paterski, et al. (2001) Biochemistry 40:11390-11404).

Introducing certain domains of ActA into a mammalian cell disrupts the host cell cytoplasm. In detail, microinjecting ActA's repeat oligoproline sequence induces "loss of stress fibers," "dramatic retraction of peripheral membranes," and "accumulation of filamentous actin near the retracting peripheral membrane" (Southwick and Purich (1994) Proc. Natl. Acad. Sci. USA 91:5168-5172). ActA, a protein expressed by *Listeria,* sequesters or "highjacks" or utilizes various cytoskeleton related proteins, including the Arp2/3 complex and actin (Olazabal, et al. (2002) Curr. Biol. 12:1413-1418; Zalevsky, et al. (2001) J. Biol. Chem. 276:3468-3475; Brieher, et al. (2004) J. Cell Biol. 165:233-242).

The ActA-based fusion protein partner, of the present invention, has a reduced polypeptide length when compared to ActA lacking the transmembrane domain. The ActA-based fusion protein partner provides reduced disruption of actin-dependent activity such as immune presentation, host cell proliferation, cell polarity, cell migration, endocytosis, sealing of detached vesicles, movement of endocytotic vesicles, secretion, cell polarity, and response to wounds (wound healing) (see, e.g., Setterblad, et al. (2004) J. Immunol. 173:1876-1886; Tskvitaria-Fuller, et al. (2003) J. Immunol. 171:2287-2295). Without implying any limitation on the invention, reduced disruption in this context is relative to that found with full-length ActA, with ActA deleted only in the transmembrane domain, or with ActA truncated at the transmembrane domain. ActA lacking the membrane anchor sequence produces a "discernable redistribution of actin" in mammalian cells (see, e.g., Pistor, et al. (1994) EMBO J. 13:758-763).

Actin-dependent activities of the cell include immune cell functions, wound healing, capping, receptor internalization, phagocytosis, Fc-receptor clustering and Fc-receptor mediated phagocytosis, utilize actin (see, e.g., Kwiatkowska, et al (2002) J. Cell Biol. 116:537-550; Ma, et al. (2001) J. Immunol. 166:1507-1516; Fukatsu, et al. (2004) J. Biol. Chem. 279:48976-48982; Botelho, et al. (2002) J. Immunol. 169:4423-4429; Krishnan, et al. (2003) J. Immunol. 170:4189-4195; Gomez-Garcia and Komberg (2004) Proc. Natl. Acad. Sci. USA 101:15876-15880; Kusner, et al. (2002) J. Biol. Chem. 277:50683-50692; Roonov-Jessen and Peterson (1996) J. Cell Biol. 134:67-80; Choma, et al. (2004) J. Cell Science 117:3947-3959; Miki, et al. (2000) Am. J. Physiol. Lung Cell. Mol. Physiol. 278:L13-L18; Fujimoto, et al. (2000) Traffic 1:161-171; Zualmann, et al. (2000) J. Cell Biol. 150:F111-F116; Olazabal, et al. (2002) Curr. Biol. 12:1413-1418; Magdalena, et al. (2003) Molecular Biology of the Cell 14:670-684).

ActA is degraded (in the mammalian cytoplasm) by way of the "N-end rule pathway." (see, e.g., Moors, et al. (1999) Cellular Microbiol. 1:249-257; Varshavsky (1996) Proc. Natl. Acad. Sci. USA 93:12142-12149).

### B. Rare codons of ActA; immunogenicity of ActA.

The ActA coding region contains a number of codons that are non-optimal for *L. monocytogenes.* Of these, a number occur in the listerial genome at a frequency of 25% or less than that of the most commonly used codon. The following provides a codon analysis for *L. monocytogenes* 10403S ActA. In the codons encoding amino acids 101-400, rare codons for glutamate (GAG) occur 12 times; rare codons for lysine (AAG) occurs three times; rare codons for isoleucine (ATA) occurs three times; rare codons for arginine (CGC) occurs once; rare codons for glutamine (CAG) occurs once; and rare codons for leucine (CTG; CTC) occurs three times. The following commentary relates to non-optimal codons, not just to rare codons. Moreover, in the codons encoding amino acids 101-400 (300 codons), non-optimal codons (this is in addition to the rare codons) occur 152 times (out of 300 codons total).

ActA is a major target for immune response by humans exposed to *L. monocytogenes* (see, e.g., Grenningloh, et al. (1997) Infect. Immun. 65:3976-3980). In some embodiments, the present invention provides an ActA-based fusion protein partner, where the ActA-based fusion protein partner has reduced immunogenicity, e.g., contains fewer epitopes than full-length ActA or is modified to provide epitopes of reduced immunogenicity.

The reagents and methods of the present invention provide a nucleic acid encoding an ActA, a truncated ActA, and/or a mutated ActA (e.g., a point mutation or a deletion), having a reduced number of antigenic epitopes, or that lacks one or more regions of increased antigenicity. Regions of increased antigenicity, as determined by a Welling plot, include amino acids 85-90; 140-150; 160-190; 220-230; 250-260; 270-280; 305-315; 350-370; 435-445; 450-460; 490-520; 545-555; and 595-610, of GenBank Acc. No. X59723. ActA has been identified as an immunogenic protein (see, e.g., Grenningloh, et al. (1997) Infection Immunity 65:3976-3980; Darji, et al. (1998) J. Immunol. 161:2414-2420; Niebuhr, et al. (1993) Infect. Immun. 61:2793-2802; Lingnau, et al. (1995) Infect. Immun. 63:3896-3903). The immunogenic properties of ActA increase with expression of soluble forms of actin, e.g., actin lacking all or part of its C-terminal region (amino acids 394-610 using numbering of Mourrain, et al. (1997) Proc. Natl. Acad. Sci. USA 94:10034-10039) (see also, e.g., Darji, et al. (1998) J. Immunol. 161:2414-2420; Cicchetti, et al. (1999) J. Biol. Chem. 274:33616-33626). Hence, where a truncated, partially deleted, or mutated ActA of the present invention lacks (or functionally lacks) a domain used for membrane-binding, thereby resulting in increased immunogenicity, the present invention provides for further truncations or mutations in order to reduce immunogenicity of the truncated ActA.

### C. Assays to measure binding of ActA derivatives, to cytoskeletal proteins, and ActA-dependent movement of Listeria.

Assays for determining recruiting of actin, or other proteins, to ActA, or to variants of ActA, are available. Recruiting can reasonably be assessed by bacterial movement assays, that is, assays that measure actin-dependent rate of *Listeria* movement in eukaryotic cell extracts or inside a eukaryotic cell (see, e.g., Marchand, et al. (1995) J. Cell Biol. 130:331-343). Bacterial movement assays can distinguish between *Listeria* expressing wild type ActA, and *Listeria* expressing mutant versions of ActA, for example, mutant ActA that lacks FP₄ domains (Smith, et al. (1996) J. Cell Biol. 135:647-660).

Recruitment can also be assessed by measuring local actin concentration at the surface of ActA-coated beads or at the surface of ActA-expressing bacteria. Bead-based assays are described (see, e.g., Machner, et al. (2001) J. Biol. Chem. 276:40096-40103; Fradelizi, et al. (2001) Nature Cell Biol. 3:699-707; Theriot, et al. (1994) Cell 76:505-517; Smith, et al. (1995) Mol. Microbiol. 17:945-951; Cameron, et al. (1999) Proc. Natl. Acad. Sci. USA 96:4908-4913). Ultracentrifugation can assess the number of cytoskeletal proteins bound to ActA (see, e.g., Machner, *et al., supra*)*.*

Assays available to the skilled artisan include, e.g., the spontaneous actin polymerization assay; the elongation from the barbed end assay; and the elongation from the pointed end (see, e.g., Zalevsky, et al. (2001) J. Biol. Chem. 276:3468-3475). Methods are also available for assessing polarity of ActA-induced actin polymerization (see, e.g., Mogilner and Oster (2003) Biophys. J. 84:1591-1605; Noireauz, et al. (2000) Biophys. J. 78:1643-1654).

### (d). SecA2-secreted proteins for use as fusion protein partner.

The present disclosure provides a family of SecA2 listerial secretory proteins useful as fusion protein partners with a heterologous antigen. The secretory protein-derived fusion protein partner finds use in increasing expression, increasing stability, increasing secretion, enhancing immune presentation, stimulating immune response, improving survival to a tumor, improving survival to a cancer, increasing survival to an infectious agent, and the like.

The contemplated listerial secretory proteins include p60 autolysin; N-acetyl-muramidase (NamA); penicillin-binding protein 2B (PBP-2B) (GenBank Acc. No. NC_003210); pheromone transporter (OppA) (complement to nt 184,539-186,215 of GenBank Acc. No. AL591982); maltose/maltodextrin ABC transporter (complement to nt 104,857-105,708 of GenBank Acc. No. AL591982); antigenic lipoprotein (Csa) (nt 3646-4719 of GenBank Acc. No. AL591982); and conserved lipoprotein, e.g., of *L. monocytogenes* EGD (see, e.g., Lenz, et al. (2003) Proc. Natl. Acad. Sci. USA 100:12432-12437; Lenz and Portnoy (2002) Mol. Microbiol. 45:1043-1056).

p60 is encoded by an open reading frame of 1,452 bp, has an N-terminal signal sequence, an SH3 domain in the N-terminal region, a central region containig threonine-asparagine repeats, and a C-terminal region encompassing the autolysin catalytic site (see, e.g., Pilgrim, et al. (2003) Infect. Immun. 71:3473-3484). p60 is also known as invasion-associated protein (iap) (GenBank Acc. No. X52268; NC_003210).

The present disclosure provides a polynucleotide comprising a first nucleic acid encoding p60, or a p60 derivative, and a second nucleic acid encoding a heterologous antigen. The p60 or p60 derivatives encompass a full length p60 protein (e.g., from *L. monocytogenes, L. innocua*, *L. ivanovii*, *L. seeligeri*, *L. welshimeri*, *L. murrayi*, and/or *L. grayi*), truncated p60 proteins consisting essentially of the N-terminal 70 amino acids; a truncated p60 protein deleted in the region that catalyses hydrolysis; signal sequences from a p60 protein; or a p60 protein with its signal sequence replaced with a different signal sequence (e.g., the signal sequence of ActA, LLO, PFO, or BaPA), and a second nucleic acid encoding a heterologous antigen. The p60 signal sequence (27 amino acids) is: MNMKKATIAATAGIAVTAFAAPTIASA (SEQ ID NO:24) (Bubert, et al. (1992) J. Bacteriol. 174:8166-8171; Bubert, et al. (1992) Appl. Environ. Microbiol. 58:2625-2632; J. Bacteriol. 173:4668-4674). The N-acetyl-muramidase signal sequence (52 amino acids) is: MDRKFIKPGIILLIVAFLVVSINVGAETGGSRTAQVNLTTSQQAFIDEILPA (SEQ ID NO:25) (nt 2679599 to 2681125 of GenBank Acc. No. NC_003210; GenBank Acc. No. AY542872; nt 2765101 to 2766627 of GenBank Acc. No. NC_003212; Lenz, et al. (2003) Proc. Natl. Acad. Sci. USA 100:12432-12437).

The present disclosure provides a p60 variant, for example, where the codons for amino acids 69 (L) and 70 (Q) are changed to provide a unique *Pst I* restriction site, where the *Pst I* site finds use in insertion a nucleic acid encoding a heterologous antigen.

Contemplated is nucleic acid encoding a fusion protein comprising a SecA2-pathway secreted protein and a heterologous antigen. Also contemplated is a nucleic acid encoding a fusion protein comprising a derivative or truncated version of a SecA2-pathway secreted protein and a heterologous antigen. Moreover, what is contemplated is a *Listeria* bacterium comprising a nucleic acid encoding a fusion protein comprising a SecA2-pathway secreted protein and a heterologous antigen, or comprising a nucleic acid encoding a fusion protein comprising a derivative or truncated version of a SecA2-pathway secreted protein and a heterologous antigen.

### (e) Mesothelin.

Human mesothelin cDNA is 2138 bp, contains an open reading frame of 1884 bp, and encodes a 69 kD protein. The mesothelin precursor protein contains 628 amino acids, and a furin cleavage site (RPRFRR at amino acids 288-293). Cleavage of the 69 kd protein generates a 40 kD membrane-bound protein (termed "mesothelin") plus a 31 kD soluble protein called megakaryocyte-potentiating factor (MPF). Mesothelin has a lipophilic sequence at its C-terminus, which is removed and replaced by phosphatidyl inositol, which causes mesothelin to be membrane-bound. Mesothelin contains a glycosylphosphatidyl inositol anchor signal sequence near the C-terminus. Mesothelin's domains, expression of mesothelin by cancer and tumor cells, and antigenic properties of mesothelin, are described (see, e.g., Hassan, et al. (2004) Clin. Cancer Res. 10:3937-3942; Ryu, et al. (2002) Cancer Res. 62:819-826; Thomas, et al. (2003) J. Exp. Med. 200:297-306; Argani, et al. (2001) Clin. Cancer Res. 7:3862-3868; Chowdhury, et al. (1998) Proc. Natl. Acad. Sci. USA 95:669-674; Chang and Pastan (1996) Proc. Natl. Acad. Sci. USA 93:136-140; Muminova, et al. (2004) BMC Cancer 4:19; GenBank Acc. Nos. NM_005823 and NM_013404; U.S. Pat No. 5,723,318 issued to Yamaguchi, et al.)*.*

Human mesothelin, deleted in mesothelin's signal sequence, is shown below:

Human mesothelin, deleted in mesothelin's signal sequence and also deleted in mesothelin's GPI-anchor, is disclosed below:

### (f) Site of Integration

The present disclosure provides a polynucleotide comprising a first nucleic acid that mediates growth or spread in a wild type or parent *Listeria*, wherein the first nucleic acid is modified by integration of a second nucleic acid encoding at least one antigen. In one aspect, the integration results in attenuation of the *Listeria.* In another aspect, the integration does not result in attenuation of the *Listeria.* In yet another aspect, the parent *Listeria* is attenuated, and the integration results in further attenuation. Furthermore, as another non-limiting example, the parent *Listeria* is attenuated, where the integration does not result in further measurable attenuation.

Embodiments further comprising modification by integrating in the first nucleic acid, a third nucleic acid encoding at least one antigen, a fourth nucleic acid encoding at least one antigen, a fifth nucleic acid encoding at least one antigen, or the like, are also provided.

Without implying any limitation, the antigen can be a heterologous antigen (heterologous to the *Listeria*), a tumor antigen or an antigen derived from a tumor antigen, an infectious agent antigen or an antigen derived from an infectious agent antigen, and the like.

The first nucleic acid can be the actA gene or inlB gene. Integration can be at a promoter or regulatory region of actA or inlB, and/or in the open reading frame of actA or inlB, where the integration attenuates the *Listeria*, as determinable under appropriate conditions. Integration can be accompanied by deletion of a part or all of the promoter or regulatory region of actA or inlB, or with deletion of part or all of the open reading frame of actA or inlB, or with deletion of both the promoter or regulatory region plus part or all of the open reading frame of actA or inlB, where the integration attenuates the *Listeria,* as determinable under appropriate conditions.

For each of the above-disclosed embodiments, the present disclosure provides a *Listeria* bacterium containing the polynucleotide. The polynucleotide can be genomic.

In some embodiments, the first nucleic acid that is modified by integration of a second nucleic acid encoding at least one antigen mediates growth or spread in a wild type or parent *Listeria.* In some embodiments, the first nucleic acid that is modified mediates cell to cell spread. In some embodiments, the first nucleic acid is actA.

In some embodiments, the first nucleic acid that is modified by integration of a second nucleic acid encoding at least one antigen, comprises a gene identified as one of the following: hly gene (encodes listeriolysin O; LLO); internalin A; internalin B; actA; SvpA; p104 (a.k.a. LAP); lplA; phosphatidylinositol-specific phospholipase C (PI-PLC) (plcA gene); phosphatidylcholine-specific phospholipase C (PC-PLC) (plcB gene); zinc metalloprotease precursor (Mpl gene); p60 (protein 60; invasion associated protein (iap); sortase; listeriolysin positive regulatory protein (PrfA gene); PrfB gene; FbpA gene; Auto gene; Ami (amidase that mediates adhesion); dlt operon (dltA; dltB; dltC; dltD); any prfA boxe; or Htp (sugar-P transporter).

Moreover, what is embraced is a *Listeria* comprising the above polynucleotide. The polynucleotide can be genomic. In one aspect, the *Listeria* can be *Listeria monocytogenes.* Provided is each of the above-disclosed embodiments, wherein the integration results in attenuation of the *Listeria*, as determinable under appropriate conditions. Also provided is each of the above-disclosed embodiments, wherein the integration does not result in attenuation of the *Listeria*, as determinable under appropriate conditions. In yet another aspect, the parent *Listeria* is attenuated, and the integration results in further attenuation. Furthermore, as another example, the parent *Listeria* is attenuated, where the integration does not result in further measurable attenuation.

In another aspect, first nucleic acid can be genomic. In another aspect, the integration can be mediated by homologous recombination, where the integration does not result in any deletion of the first nucleic acid, where the integration results in deletion of all or part of the first nucleic acid, where the first nucleic acid contains a promoter or other regulatory region and where the second nucleic acid is operably linked and/or in frame with the promoter or other regulatory region, and where the first nucleic acid contains a promoter or other regulatory region and where the second nucleic acid is not at all operably linked and/or in frame with the promoter or other regulatory region.

The term "gene modified by integration" encompasses, but is not limited to, "a locus of integration that is the gene."

What is also embraced by the present disclosure is a polynucleotide comprising a first nucleic acid that mediates growth or spread in a wild type or parent *Listeria*, where the first nucleic acid comprises all or part of a pathogenicity island or virulence gene cluster, wherein the all or part of the pathogenicity island or virulence gene cluster is modified by integration of a second nucleic acid encoding at least one antigen, wherein the integration results in attenuation of the *Listeria*, as determinable under appropriate conditions. Pathogenicity islands and virulence gene clusters are disclosed (see, e.g., Chakraborty, et al. (2000) Int. J. Med. Microbiol. 290:167-174; Vazquez-Boland, et al. (2001) Clin. Microbiol. Revs. 14:584-640). The gene that mediates growth and spread is not limited to a gene that specifically mediates virulence, but encompasses growth-mediating genes such those that mediate energy production (e.g., glycolysis, Krebs cycle, cytochromes), anabolism and/or catabolism of amino acids, sugars, lipids, minerals, purines, and pyrimidines, and genes that mediate nutrient transport, transcription, translation, and/or replication, and the like.

In another aspect, what is provided is a polynucleotide comprising a first nucleic acid that mediates growth or spread in a wild type or parent *Listeria*, wherein the nucleic acid is modified by integration of a plurality of nucleic acids encoding an antigen or antigens.

The integration can be within the second nucleic acid without any corresponding deletion of the second nucleic acid. Alternatively, the integration can be within the second nucleic acid with a corresponding deletion of the second nucleic acid, or a portion thereof. Where the first nucleic acid in the wild type or parent *Listeria* comprises a promoter and/or other regulatory site, the integration can be in the promoter and/or regulatory site.

Where the first nucleic acid comprises a promoter and/or other regulatory site, the present disclosure provides an integrated second nucleic acid, where the second nucleic acid comprises a coding region that is operably linked and in-frame with the promoter and/or regulatory site. As an alternative, the present disclosure provides an integrated second nucleic acid, where the second nucleic acid comprises a coding region that is not operably linked and in-frame with the promoter and/or regulatory site. Provided is each of the above embodiments, where the integrated nucleic acid (second nucleic acid) comprises a promoter and/or regulatory site, where the promoter and/or regulatory site can take the place of, or alternatively can operate in addition to, a promoter and/or other regulatory site present in the first nucleic acid.

In one aspect, the first nucleic acid comprises (or in the alternative, consists of) a promoter or other regulatory element, and the second nucleic acid is operably linked with the promoter and/or other regulatory element. In another aspect, the second nucleic encoding an antigen further comprises a promoter and/or other regulatory element.

The first nucleic acid need not encode any polypeptide, as the first nucleic acid can be a regulatory region or box. The following concerns integration as mediated by, for example, homologous integration. This disclosure provides the above polynucleotide, wherein the second nucleic acid is integrated without deletion of any of the first nucleic acid.

In one embodiment, the first nucleic acid mediates growth but not spread. In another embodiment, the first nucleic acid mediates spread but not growth. In yet another embodiment, the first nucleic acid mediates both growth and spread. In one aspect, the integration reduces or eliminates the growth, reduces or eliminates the spread, or reduces or eliminates both growth and spread.

Moreover, in one embodiment the first nucleic acid has the property that its inactivation results in at least 10% reduction of growth, sometimes in at least 20% reduction of growth, typically in at least 30% reduction of growth, more typically in least 40% reduction of growth, most typically in at least 50% reduction in growth, often in at least 60% reduction in growth, more often in at least 70% reduction in growth, most often in at least 80% reduction in growth, conventionally at least 85% reduction in growth, more conventionally at least 90% reduction in growth, and most conventionally in at least 95% reduction in growth, and sometimes in at least 99% reduction in growth. In one aspect, the growth can be measured in a defined medium, in a broth medium, in agar, within a host cell, in the cytoplasm of a host cell, and the like.

Moreover, in one embodiment the first nucleic acid has the property that its inactivation results in at least 10% reduction of cell-to-cell spread, sometimes in at least 20% reduction of spread, typically in at least 30% reduction of spread, more typically in least 40% reduction of spread, most typically in at least 50% reduction in spread, often in at least 60% reduction in spread, more often in at least 70% reduction in spread, most often in at least 80% reduction in spread, conventionally at least 85% reduction in spread, more conventionally at least 90% reduction in spread, and most conventionally in at least 95% reduction in spread, and sometimes in at least 99% reduction in spread. In one aspect, the growth can be measured in a defined medium, in a broth medium, in agar, within a host cell, in the cytoplasm of a host cell, and the like.

Provided is a *Listeria* bacterium comprising each of the above-disclosed polynucleotides. In one aspect, the *Listeria* is *Listeria monocytogenes.* Without implying any limitation, the present disclosure contemplates each of the above polynucleotides that is genomic, plasmid based, or that is present in both genomic and plasmid based forms.

In each of the above-disclosed embodiments, integration can be mediated by site-specific integration. Site-specific integration involves a plasmidic attPP' site, which recognizes a genomic attBB' site. In certain embodiments, the attBB' site can be naturally present in a gene that mediates growth or spread. In other embodiments, the attBB' site can be integrated, e.g., by homologous integration, in the gene that mediates growth or spread, followed by site-specific integration of the above-disclosed second nucleic acid.

The present disclosure provides a *Listeria* containing a polynucleotide comprising a first nucleic acid that, in the wild type *Listeria* or parent *Listeria*, mediates growth or spread, or both growth and spread, wherein the nucleic acid is modified by integration of a second nucleic acid encoding an antigen. Yet one further example of each of the embodiments disclosed herein provides an integration that reduces or eliminates growth, reduces or eliminates spread, or reduces or eliminates both growth and spread.

What is also embraced is a polynucleotide comprising a first nucleic acid that mediates growth or spread of a wild type or parental *Listeria*, and where the first nucleic acid comprises a signal sequence or secretory sequence, wherein the first nucleic acid is modified by integration of a second nucleic acid encoding at least one antigen, and wherein the integration results an in attenuation of the *Listeria*, and where the integration operably links the signal or secretory sequence (encoded by the first nucleic acid) with an open reading frame encoding by the second nucleic acid. In one aspect, the above integration results in deletion of all of the polypeptide encoded by the first nucleic acid, except for the signal or secretory sequence encoded by the first nucleic acid (where the signal or secretory sequence remains intact).

Genomes comprising each of the polynucleotide embodiments described herein are further contemplated. Moreover, what is provided is a listerial genome comprising each of the above embodiments. Furthermore, this disclosure supplies a *Listeria* bacterium comprising each of the polynucleotide embodiments described herein.

In one embodiment, this disclosure provides *Listeria* (e.g., *Listeria monocytogenes*) in which the genome comprises a polynucleotide comprising a nucleic acid encoding a heterologous antigen. In some embodiments, the nucleic acid encoding the heterologous antigen has been integrated into the genome by site-specific recombination or homologous recombination. In some embodiments, the presence of the nucleic acid in the genome attenuates the *Listeria.* In some embodiments, the nucleic acid encoding the heterologous antigen has been integrated into the locus of a virulence gene. In some embodiments, the nucleic acid encoding the heterologous antigen has been integrated into the actA locus. In some embodiments, the nucleic acid encoding the heterologous antigen has been integrated into the inlB locus. In some embodiments, the genome of the *Listeria* comprises a first nucleic acid encoding a heterologous antigen that has been integrated into a first locus (e.g., the actA locus) and a second nucleic acid encoding a second heterologous antigen that has been integrated into a second locus (e.g., the inlB locus). The first and second heterologous antigens may be identical to each other or different. In some embodiments, the first and second heterologous antigens differ from each other, but are derived from the same tumor antigen or infectious agent antigen. In some embodiments, the first and second heterologous antigens are each a different fragment of an antigen derived from a cancer cell, tumor, or infectious agent. In some embodiments, the integrated nucleic acid encodes a fusion protein comprising a modified ActA and the heterologous antigen. In some embodiments, at least two, at least three, at least four, at least five, at least six, or at least seven nucleic acid sequences encoding heterologous antigens have been integrated into the Listerial genome.

In some embodiments, a polynucleotide (or nucleic acid) described herein has been integrated into a virulence gene in the genome of the *Listeria*, wherein the integration of the polynucleotide (a) disrupts expression of the virulence gene; and/or (b) disrupts a coding sequence of the virulence gene. In some embodiments, the *Listeria* is attenuated by the disruption of the expression of the virulence gene and/or the disruption of the coding sequence of the virulence gene attenuates the *Listeria.* In some embodiments, the virulence gene is necessary for mediating growth or spread. In other embodiments, the virulence gene is not necessary for mediating growth or spread. In some embodiments, the virulence gene is a prfA-dependent gene. In some embodiments, the virulence gene is not a prfA-dependent gene. In some embodiments, the virulence gene is *actA* or *inlB.* In some embodiments, the expression of the virulence gene in which the polynucleotide/nucleic acid is integrated is disrupted at least 10%, at least 25%, at least 50%, at least 75%, at least 90%, or about 100% (relative to the expression of the virulence gene in the absence of the integrated polynucleotide/nucleic acid, as determined by measuring expression levels. Disruption of the coding sequence of the virulence gene encompasses alterations of the coding sequence of any kind including frame-shift mutations, truncations, insertions, deletions, or replacements/substitutions. In some embodiments, all or part of the virulence gene is deleted during integration of the polynucleotide into the virulence gene. In other embodiments, none of the virulence gene is deleted during integration of the polynucleotide. In some embodiments, part or all of the coding sequence of the virulence gene is replaced by the integrated polynucleotide.

In some embodiments, multiple polynucleotides described herein have been integrated into the *Listeria* genome at one or more different sites. The multiple polynucleotides may be the same or different. In some embodiments, a first polynucleotide described herein has been integrated into the *actA* locus and/or a second polynucleotide described herein has been integrated into the *inlB* locus. In some embodiments, a first polynucleotide described herein has been integrated into the *actA* locus and a second polynucleotide described herein has been integrated into the *inlB* locus. The heterologous antigen encoded by the first polynucleotide may be the same or different as that encoded by the second polynucleotide. In some embodiments, the two heterologous antigens encoded by the integrated antigens differ, but are derived from the same antigen.

### IV. THERAPEUTIC COMPOSITIONS AND USES.

### (a). Therapeutic compositions.

The attenuated *Listeria,* vaccines, small molecules, biological reagents, and adjuvants that are provided herein can be administered to a host, either alone or in combination with a pharmaceutically acceptable excipient, in an amount sufficient to induce an appropriate immune response to an immune disorder, cancer, tumor, or infection. The immune response can comprise, without limitation, specific immune response, non-specific immune response, both specific and non-specific response, innate response, primary immune response, adaptive immunity, secondary immune response, memory immune response, immune cell activation, immune cell proliferation, immune cell differentiation, and cytokine expression.

"Pharmaceutically acceptable excipient" or "diagnostically acceptable excipient" includes but is not limited to, sterile distilled water, saline, phosphate buffered solutions, amino acid-based buffers, or bicarbonate buffered solutions. An excipient selected and the amount of excipient used will depend upon the mode of administration. Administration may be oral, intravenous, subcutaneous, dermal, intradermal, intramuscular, mucosal, parenteral, intraorgan, intralesional, intranasal, inhalation, intraocular, intramuscular, intravascular, intranodal, by scarification, rectal, intraperitoneal, or any one or combination of a variety of well-known routes of administration. The administration can comprise an injection, infusion, or a combination thereof. Administration of the *Listeria* of the present disclosure by a non-oral route can avoid tolerance (see, e.g., Lecuit, et al. (2001) Science 292:1722-1725; Kirk, et al. (2005) Transgenic Res. 14:449-462; Faria and Weiner (2005) Immunol. Rev. 206:232-259; Kraus, et al. (2005) J. Clin. Invest. 115:2234-2243; Mucida, et al. (2005) J. Clin. Invest. 115:1923-1933). Methods are available for administration of *Listeria*, e.g., intravenously, subcutaneously, intramuscularly, intraperitoneally, orally, mucosal, by way of the urinary tract, by way of a genital tract, by way of the gastrointestinal tract, or by inhalation (Dustoor, et al. (1977) Infection Immunity 15:916-924; Gregory and Wing (2002) J. Leukoc. Biol. 72:239-248; Hof, et al. (1997) Clin. Microbiol. Revs. 10:345-357; Schluter, et al. (1999) Immunobiol. 201:188-195; Hof (2004) Expert Opin. Pharmacother. 5:1727-1735; Heymer, et al. (1988) Infection 16(Suppl. 2):S106-S111; Yin, et al. (2003) Environ. Health Perspectives 111:524-530).

This disclosure provides immunogenic compositions comprising any of the *Listeria* described herein. This disclosure further provides pharmaceutical compositions comprising any of the *Listeria* described herein and a pharmaceutically acceptable excipient. In some embodiments, the immunogenic compositions or pharmaceutical compositions are vaccines. In some embodiments, the composition comprising the *Listeria* is a vaccine that further comprises an adjuvant.

The following applies, optionally, to each of the embodiments disclosed herein. Provided is an administered reagent that is pure or purified, for example where the administered reagent can be administered to a mammal in a pure or purified form, i.e., alone, as a pharmaceutically acceptable composition, or in an excipient. Moreover, the following also can apply, optionally, to each of the embodiments disclosed herein. Provided is an administered reagent that is pure or purified, where the administered reagent can be administered in a pure or purified form, i.e., alone, as a pharmaceutically acceptable composition, or in an excipient, and where the reagent is not generated after administration (not generated in the mammal). In one embodiment, what might optionally apply to each of the reagents disclosed herein, is a polypeptide reagent that is administered as a pure or purified polypeptide (e.g., alone, as a pharmaceutically acceptable composition, or in an excipient), where the administered polypeptide reagent is not administered in the form of a nucleic acid encoding that polypeptide, and as a consequence, there is no administered nucleic acid that can generate the polypeptide inside the mammal.

The *Listeria* of the present invention can be stored, e.g., frozen, lyophilized, as a suspension, as a cell paste, or complexed with a solid matrix or gel matrix.

An effective amount for a particular patient may vary depending on factors such as the condition being treated, the overall health of the patient, the route and dose of administration and the severity of side affects. An effective amount for a particular patient may vary depending on factors such as the condition being treated, the overall health of the patient, the route and dose of administration and the severity of side affects. Guidance for methods of treatment and diagnosis is available (see, e.g., Maynard, et al. (1996) A Handbook of SOPs for Good Clinical Practice, Interpharm Press, Boca Raton, FL; Dent (2001) Good Laboratory and Good Clinical Practice, Urch Publ., London, UK).

The *Listeria* of the present invention can be administered in a dose, or dosages, where each dose comprises at least 1000 *Listeria* cells/kg body weight; normally at least 10,000 cells; more normally at least 100,000 cells; most normally at least 1 million cells; often at least 10 million cells; more often at least 100 million cells; typically at least 1 billion cells; usually at least 10 billion cells; conventionally at least 100 billion cells; and sometimes at least 1 trillion *Listeria* cells/kg body weight. The present invention provides the above doses where the units of *Listeria* administration is colony forming units (CFU), the equivalent of CFU prior to psoralen-treatment, or where the units are number of *Listeria* cells.

The *Listeria* of the present invention can be administered in a dose, or dosages, where each dose comprises between 10⁷ and 10⁸ *Listeria* per 70 kg body weight (or per 1.7 square meters surface area; or per 1.5 kg liver weight); 2 x 10⁷ and 2 x 10⁸ *Listeria* per 70 kg body weight (or per 1.7 square meters surface area; or per 1.5 kg liver weight); 5 x 10⁷ and 5 x 10⁸ *Listeria* per 70 kg body weight (or per 1.7 square meters surface area; or per 1.5 kg liver weight); 10⁸ and 10⁹ *Listeria* per 70 kg body weight (or per 1.7 square meters surface area; or per 1.5 kg liver weight); between 2.0 x 10⁸ and 2.0 x 10⁹ *Listeria* per 70 kg (or per 1.7 square meters surface area, or per 1.5 kg liver weight); between 5.0 x 10⁸ to 5.0 x 10⁹ *Listeria* per 70 kg (or per 1.7 square meters surface area, or per 1.5 kg liver weight); between 10⁹ and 10¹⁰ *Listeria* per 70 kg (or per 1.7 square meters surface area, or per 1.5 kg liver weight); between 2 x 10⁹ and 2 x 10¹⁰ *Listeria* per 70 kg (or per 1.7 square meters surface area, or per 1.5 kg liver weight); between 5 x 10⁹ and 5 x 10¹⁰ *Listeria* per 70 kg (or per 1.7 square meters surface area, or per 1.5 kg liver weight); between 10¹¹ and 10¹² *Listeria* per 70 kg (or per 1.7 square meters surface area, or per 1.5 kg liver weight); between 2 x 10¹¹ and 2 x 10¹² *Listeria* per 70 kg (or per 1.7 square meters surface area, or per 1.5 kg liver weight); between 5 x 10¹¹ and 5 x 10¹² *Listeria* per 70 kg (or per 1.7 square meters surface area, or per 1.5 kg liver weight); between 10¹² and 10¹³ *Listeria* per 70 kg (or per 1.7 square meters surface area); between 2 x 10¹² and 2 x 10¹³ *Listeria* per 70 kg (or per 1.7 square meters surface area, or per 1.5 kg liver weight); between 5 x 10¹² and 5 x 10¹³ *Listeria* per 70 kg (or per 1.7 square meters surface area, or per 1.5 kg liver weight); between 10¹³ and 10¹⁴ *Listeria* per 70 kg (or per 1.7 square meters surface area, or per 1.5 kg liver weight); between 2 x 10¹³ and 2 x 10¹⁴ *Listeria* per 70 kg (or per 1.7 square meters surface area, or per 1.5 kg liver weight); 5 x 10¹³ and 5 x 10¹⁴ *Listeria* per 70 kg (or per 1.7 square meters surface area, or per 1.5 kg liver weight); between 10¹⁴ and 10¹⁵ *Listeria* per 70 kg (or per 1.7 square meters surface area, or per 1.5 kg liver weight); between 2 x 10¹⁴ and 2 x 10¹⁵ *Listeria* per 70 kg (or per 1.7 square meters surface area, or per 1.5 kg liver weight); and so on, wet weight.

The mouse liver, at the time of administering the *Listeria* of the present invention, weighs about 1.5 grams. Human liver weighs about 1.5 kilograms.

Also provided is one or more of the above doses, where the dose is administered by way of one injection every day, one injection every two days, one injection every three days, one injection every four days, one injection every five days, one injection every six days, or one injection every seven days, where the injection schedule is maintained for, e.g., one day only, two days, three days, four days, five days, six days, seven days, two weeks, three weeks, four weeks, five weeks, or longer. This disclosure also embraces combinations of the above doses and schedules, e.g., a relatively large initial dose of *Listeria*, followed by relatively small subsequent doses of *Listeria*, or a relatively small initial dose followed by a large dose.

A dosing schedule of, for example, once/week, twice/week, three times/week, four times/week, five times/week, six times/week, seven times/week, once every two weeks, once every three weeks, once every four weeks, once every five weeks, and the like, is available for this disclosure. The dosing schedules encompass dosing for a total period of time of, for example, one week, two weeks, three weeks, four weeks, five weeks, six weeks, two months, three months, four months, five months, six months, seven months, eight months, nine months, ten months, eleven months, and twelve months.

Provided are cycles of the above dosing schedules. The cycle can be repeated about, e.g., every seven days; every 14 days; every 21 days; every 28 days; every 35 days; 42 days; every 49 days; every 56 days; every 63 days; every 70 days; and the like. An interval of non-dosing can occur between a cycle, where the interval can be about, e.g., seven days; 14 days; 21 days; 28 days; 35 days; 42 days; 49 days; 56 days; 63 days; 70 days; and the like. In this context, the term "about" means plus or minus one day, plus or minus two days, plus or minus three days, plus or minus four days, plus or minus five days, plus or minus six days, or plus or minus seven days.

The present invention encompasses a method of administering *Listeria* that is oral. Also provided is a method of administering *Listeria* that is intravenous. Moreover, what is provided is a method of administering *Listeria* that is intramuscular. This disclosure supplies a *Listeria* bacterium, or culture or suspension of *Listeria* bacteria, prepared by growing in a medium that is meat based, or that contains polypeptides derived from a meat or animal product. Also supplied by the present invention is a *Listeria* bacterium, or culture or suspension of *Listeria* bacteria, prepared by growing in a medium that does not contain meat or animal products, prepared by growing on a medium that contains vegetable polypeptides, prepared by growing on a medium that is not based on yeast products, or prepared by growing on a medium that contains yeast polypeptides.

The present invention encompasses a method of administering *Listeria* that is not oral. Also provided is a method of administering *Listeria* that is not intravenous. Moreover, what is provided is a method of administering *Listeria* that is not intramuscular. This disclosure supplies a *Listeria* bacterium, or culture or suspension of *Listeria* bacteria, prepared by growing in a medium that is not meat based, or that does not contain polypeptides derived from a meat or animal product. Also supplied by the present invention is a *Listeria* bacterium, or culture or suspension of *Listeria* bacteria, prepared by growing in a medium based on vegetable products, that contains vegetable polypeptides, that is based on yeast products, or that contains yeast polypeptides.

Methods for co-administration with an additional therapeutic agent, e.g., a small molecule, antibiotic, innate immunity modulating agent, tolerance modulating agent, cytokine, chemotherapeutic agent, or radiation, are well known in the art (Hardman, et al. (eds.) (2001) Goodman and Gilman's The Pharmacological Basis of Therapeutics, 10th ed., McGraw-Hill, New York, NY; Poole and Peterson (eds.) (2001) Pharmacotherapeutics for Advanced Practice:A Practical Approach, Lippincott, Williams & Wilkins, Phila., PA; Chabner and Longo (eds.) (2001) Cancer Chemotherapy and Biotherapy, Lippincott, Williams & Wilkins, Phila., PA).

The present invention provides reagents for administering in conjunction with an attenuated *Listeria.* These reagents include biological reagents such as: (1) Cytokines, antibodies, dendritic cells, attenuated tumor cells cells; (2) Small molecule reagents such as 5-fluorouracil, methotrexate, paclitaxel, docetaxel, cis-platin, gemcitabine; (3) Reagents that modulate regulatory T cells, such as cyclophosphamide, anti-CTLA4 antibody, anti-CD25 antibody (see, e.g., Hawryfar, et al. (2005) J. Immunol. 174:344-3351); and (4) Vaccines (including polypeptide vaccines, nucleic acid vaccines, attenuated tumor cell vaccines, and dendritic cell vaccines). The reagents can be administered with the *Listeria* or independently (before or after) the *Listeria.* For example, the reagent can be administered immediately before (or after) the *Listeria*, on the same day as, one day before (or after), one week before (or after), one month before (or after), or two months before (or after) the *Listeria*, and the like.

Biological reagents or macromolecules of the present invention encompass an agonist or antagonist of a cytokine, a nucleic acid encoding an agonist or antagonist of a cytokine, a cell expressing a cytokine, or an agonistic or antagonistic antibody. Biological reagents include, without limitation, a TH-1 cytokine, a TH-2 cytokine, IL-2, IL-12, FLT3-ligand, GM-CSF, IFNgamma, a cytokine receptor, a soluble cytokine receptor, a chemokine, tumor necrosis factor (TNF), CD40 ligand, or a reagent that stimulates replacement of a proteasome subunit with an immunoproteasome subunit.

The present disclosure encompasses biological reagents, such cells engineered to express at least one of the following: GM-CSF, IL-2, IL-3, IL-4, IL-12, IL-18, tumor necrosis factor-alpha (TNF-alpha), or inducing protein-10. Other contemplated reagents include agonists of B7-1, B7-2, CD28, CD40 ligand, or OX40 ligand (OX40L), and novel forms engineered to be soluble or engineered to be membrane-bound (see, e.g., Karnbach, et al. (2001) J. Immunol. 167:2569-2576; Greenfield, et al. (1998) Crit. Rev. Immunol. 18:389-418; Parney and Chang (2003) J. Biomed. Sci. 10:37-43; Gri, et al. (2003) J. Immunol. 170:99-106; Chiodoni, et al. (1999) J. Exp. Med. 190:125-133; Enzler, et al. (2003) J. Exp. Med. 197:1213-1219; Soo Hoo, et al. (1999) J. Immunol 162:7343-7349; Mihalyo, et al. (2004) J. Immunol. 172:5338-5345; Chapoval, et al. (1998) J. Immunol. 161:6977-6984).

Without implying any limitation, the present disclosure provides the following biologicals. MCP-1, MIP1-alpha, TNF-alpha, and interleukin-2, for example, are effective in treating a variety of tumors (see, e.g., Nakamoto, et al. (2000) Anticancer Res. 20(6A):4087-4096; Kamada, et al. (2000) Cancer Res. 60:6416-6420; Li, et al. (2002) Cancer Res. 62:4023-4028; Yang, et al. (2002) Zhonghua Wai Ke Za Zhi 40:789-791; Hoving, et al. (2005) Cancer Res. 65:4300-4308; Tsuchiyama, et al. (2003) Cancer Gene Ther. 10:260-269; Sakai, et al. (2001) Cancer Gene Ther. 8:695-704).

The present disclosure provides reagents and methods encompassing an Flt3-ligand agonist, and an Flt3-ligand agonist in combination with *Listeria.* Flt3-ligand (Fms-like thyrosine kinase 3 ligand) is a cytokine that can generate an antitumor immune response (see, e.g., Dranoff (2002) Immunol. Revs. 188:147-154; Mach, et al. (2000) Cancer Res. 60:3239-3246; Furumoto, et al. (2004) J. Clin. Invest. 113:774-783; Freedman, et al. (2003) Clin. Cancer Res. 9:5228-5237; Mach, et al. (2000) Cancer Res. 60:3239-3246).

In another embodiment, the present disclosure contemplates administration of a dendritic cell (DC) that expresses at least one tumor antigen, or infectious disease antigen. Expression by the DC of an antigen can be mediated by way of, e.g., peptide loading, tumor cell extracts, fusion with tumor cells, transduction with mRNA, or transfected by a vector (see, e.g., Klein, et al. (2000) J. Exp. Med. 191:1699-1708; Conrad and Nestle (2003) Curr. Opin. Mol. Ther. 5:405-412; Gilboa and Vieweg (2004) Immunol. Rev. 199:251-263; Paczesny, et al. (2003) Semin. Cancer Biol. 13:439-447; Westermann, et al. (1998) Gene Ther. 5:264-271).

The methods and reagents of the present disclosure also encompass small molecule reagents, such as 5-fluorouracil, methotrexate, irinotecan, doxorubicin, prednisone, dolostatin-10 (D10), combretastatin A-4, mitomycin C (MMC), vincristine, colchicines, vinblastine, cyclophosphamide, fungal beta-glucans and derivatives therof, and the like (see, e.g., Hurwitz, et al. (2004) New Engl. J. Med. 350:2335-2342; Pelaez, et al. (2001) J. Immunol. 166:6608-6615; Havas, et al. (1990) J. Biol. Response Modifiers 9:194-204; Turk, et al. (2004) J. Exp. Med. 200:771-782; Ghiringhelli, et al. (2004) Eur. J. Immunol. 34:336-344; Andrade-Mena (1994) Int. J. Tissue React. 16:95-103; Chrischilles, et al. (2003) Cancer Control 10:396-403). Also encompassed are compositions that are not molecules, e.g., salts and ions.

Provided are analogues of cyclophosphamide (see, e.g., Jain, et al. (2004) J. Med. Chem. 47:3843-3852; Andersson, et al. (1994) Cancer Res. 54:5394-5400; Borch and Canute (1991) J. Med. Chem. 34:3044-3052; Ludeman, et al. (1979) J. Med. Chem. 22:151-158; Zon (1982) Prog. Med. Chem. 19:205-246).

Also embraced by this disclosure are small molecule reagents that stimulate innate immune response, e.g., CpG oligonucleotides, imiquimod, and alphaGalCer. CpG oligonucleotides mediate immune response via TLR9 (see, e.g., Chagnon, et al. (2005) Clin. Cancer Res. 11:1302-1311; Speiser, et al. (2005) J. Clin. Invest. Feb.3 (epub ahead of print); Mason, et al. (2005) Clin. Cancer Res. 11:361-369; Suzuki, et al. (2004) Cancer Res. 64:8754-8760; Taniguchi, et al. (2003) Annu. Rev. Immunol. 21:483-513; Takeda, et al. (2003) Annu. Rev. Immunol. 21:335-376; Metelitsa, et al. (2001) J. Immunol. 167:3114-3122).

Other useful small molecule reagents include those derived from bacterial peptidoglycan, such as certain NOD2 ligands (McCaffrey, et al. (2004) Proc. Natl. Acad. Sci. USA 101:11386-11391).

This disclosure includes reagents and methods for modulating activity of T regulatory cells (Tregs; suppressor T cells). Attenuation or inhibition of Treg cell activity can enhance the immune system's killing of tumor cells. A number of reagents have been identified that inhibit Treg cell activity. These reagents include, e.g., cyclophosphamide (a.k.a. Cytoxan®; CTX), anti-CD25 antitobody, modulators of GITR-L or GITR, a modulator of Forkhead-box transcription factor (Fox), a modulator of LAG-3, anti-IL-2R, and anti-CTLA4 (see, e.g., Pardoll (2003) Annu. Rev. Immunol. 21:807-839; Ercolini, et al. (2005) J. Exp. Med. 201:1591-1602; Haeryfar, et al. (2005) J. Immunol. 174:3344-3351; Mihalyo, et al. (2004) J. Immunol. 172:5338-5345; Stephens, et al. (2004) J. Immunol. 173:5008-5020; Schiavoni, et al. (2000) Blood 95:2024-2030; Calmels, et al. (2004) Cancer Gene Ther. Oct. 08 (epub ahead of print); Mincheff, et al. (2004) Cancer Gene Ther. Sept. 17 [epub ahead of print]; Muriglan, et al. (2004) J. Exp. Med. 200:149-157; Stephens, et al. (2004) J. Immunol. 173:5008-5020; Coffer and Burgering (2004) Nat. Rev. Immunol. 4:889-899; Kalinichenko, et al. (2004) Genes Dev. 18:830-850; Cobbold, et al. (2004) J. Immunol. 172:6003-6010; Huang, et al. (2004) Immunity 21:503-513). CTX shows a bimodal effect on the immune system, where low doses of CTX inhibit Tregs (see, e.g., Lutsiak, et al. (2005) Blood 105:2862-2868).

CTLA4-blocking agents, such as anti-CTLA4 blocking antibodies, can enhance immune response to cancers, tumors, pre-cancerous disorders, infections, and the like (see, e.g., Zubairi, et al. (2004) Eur. J. Immunol. 34:1433-1440; Espenschied, et al. (2003) J. Immunol. 170:3401-3407; Davila, et al. (2003) Cancer Res. 63:3281-3288; Hodi, et al. (2003) Proc. Natl. Acad. Sci. USA 100:4712-4717). Where the present disclosure uses anti-CTLA4 antibodies, and the like, this disclosure is not necessarily limited to use for inhibiting Tregs, and also does not necessarily always encompass inhibition of Tregs.

Lymphocyte activation gene-3 (LAG-3) blocking agents, such as anti-LAG-3 antibodies or soluble LAG-3 (e.g., LAG-3 Ig), can enhance immune response to cancers or infections. Anti-LAG-3 antibodies reduce the activity of Tregs (see, e.g., Huang, et al. (2004) Immunity 21:503-513; Triebel (2003) Trends Immunol. 24:619-622; Workman and Vignali (2003) Eur. J. Immunol. 33:970-979; Cappello, et al. (2003) Cancer Res. 63:2518-2525; Workman, et al. (2004) J. Immunol. 172:5450-5455; Macon-Lemaitre and Triebel (2005) Immunology 115:170-178).

Vaccines comprising a tumor antigen, a nucleic acid encoding a tumor antigen, a vector comprising a nucleic acid encoding a tumor antigen, a cell comprising a tumor antigen, a tumor cell, or an attenuated tumor cell, are encompassed by this disclosure. Provided are reagents derived from a nucleic acid encoding a tumor antigen, e.g., a codon optimized nucleic acid, or a nucleic acid encoding two or more different tumor antigens, or a nucleic acid expressing rearranged epitopes of a tumor antigen, e.g., where the natural order of epitopes is ABCD and the engineered order is ADBC, or a nucleic acid encoding a fusion protein comprising at least two different tumor antigens.

Where an administered antibody, binding compound derived from an antibody, cytokine, or other therapeutic agent produces toxicity, an appropriate dose can be one where the therapeutic effect outweighs the toxic effect. Generally, an optimal dosage of the present invention is one that maximizes therapeutic effect, while limiting any toxic effect to a level that does not threaten the life of the patient or reduce the efficacy of the therapeutic agent. Signs of toxic effect, or anti-therapeutic effect include, without limitation, e.g., anti-idiotypic response, immune response to a therapeutic antibody, allergic reaction, hematologic and platelet toxicity, elevations of aminotransferases, alkaline phosphatase, creatine kinase, neurotoxicity, nausea, and vomiting (see, e.g., Huang, et al. (1990) Clin. Chem. 36:431-434).

An effective amount of a therapeutic agent is one that will decrease or ameliorate the symptoms normally by at least 10%, more normally by at least 20%, most normally by at least 30%, typically by at least 40%, more typically by at least 50%, most typically by at least 60%, often by at least 70%, more often by at least 80%, and most often by at least 90%, conventionally by at least 95%, more conventionally by at least 99%, and most conventionally by at least 99.9%.

The reagents and methods of the present invention provide a vaccine comprising only one vaccination; or comprising a first vaccination; or comprising at least one booster vaccination; at least two booster vaccinations; or at least three booster vaccinations. Guidance in parameters for booster vaccinations is available (see, e.g., Marth (1997) Biologicals 25:199-203; Ramsay, et al. (1997) Immunol. Cell Biol. 75:382-388; Gherardi, et al. (2001) Histol. Histopathol. 16:655-667; Leroux-Roels, et al. (2001) ActA Clin. Belg. 56:209-219; Greiner, et al. (2002) Cancer Res. 62:6944-6951; Smith, et al. (2003) J. Med. Virol. 70:Suppl.1:S38-S41; Sepulveda-Amor, et al. (2002) Vaccine 20:2790-2795).

Provided is a first reagent that comprises a *Listeria* bacterium (or *Listeria* vaccine), and a second reagent that comprises, e.g., a cytokine, a small molecule such as cyclophosphamide or methotrexate, or a vaccine, such as an attenuated tumor cell or attenuated tumor cell expressing a cytokine. Provided are the following methods of administration of the first reagent and the second reagent.

The *Listeria* and the second reagent can be administered concomitantly, that is, where the administering for each of these reagents can occur at time intervals that partially or fully overlap each other. The *Listeria* and second reagent can be administered during time intervals that do not overlap each other. For example, the first reagent can be administered within the time frame of t = 0 to 1 hours, while the second reagent can be administered within the time frame of t = 1 to 2 hours. Also, the first reagent can be administered within the time frame of t = 0 to 1 hours, while the second reagent can be administered somewhere within the time frame of t = 2-3 hours, t = 3-4 hours, t = 4-5 hours, t = 5-6 hours, t = 6-7 hours, t = 7-8 hours, t = 8-9 hours, t = 9-10 hours, and the like. Moreover, the second reagent can be administered somewhere in the time frame of t = minus 2-3 hours, t = minus 3-4 hours, t = minus 4-5 hours, t = 5-6 minus hours, t = minus 6-7 hours, t = minus 7-8 hours, t = minus 8-9 hours, t = minus 9-10 hours, and the like.

To provide another example, the first reagent can be administered within the time frame of t = 0 to 1 days, while the second reagent can be administered within the time frame of t = 1 to 2 days. Also, the first reagent can be administered within the time frame of t = 0 to 1 days, while the second reagent can be administered somewhere within the time frame of t = 2-3 days, t = 3-4 days, t = 4-5 days, t = 5-6 days, t = 6-7 days, t = 7-8 days, t = 8-9 days, t = 9-1 0 days, and the like. Moreover, the second reagent can be administered somewhere in the time from of t = minus 2-3 days, t = minus 3-4 days, t = minus 4-5 days, t = minus 5-6 days, t = minus 6-7 days, t = minus 7-8 days, t = minus 8-9 days, t = minus 9-10 days, and the like.

In another aspect, administration of the *Listeria* can begin at t = 0 hours, where the administration results in a peak (or maximal plateau) in plasma concentration of the *Listeria,* and where administration of the second reagent is initiated at about the time that the concentration of plasma *Listeria* reaches said peak concentration, at about the time that the concentration of plasma *Listeria* is 95% said peak concentration, at about the time that the concentration of plasma *Listeria* is 90% said peak concentration, at about the time that the concentration of plasma *Listeria* is 85% said peak concentration, at about the time that the concentration of plasma *Listeria* is 80% said peak concentration, at about the time that the concentration of plasma *Listeria* is 75% said peak concentration, at about the time that the concentration of plasma *Listeria* is 70% said peak concentration, at about the time that the concentration of plasma *Listeria* is 65% said peak concentration, at about the time that the concentration of plasma *Listeria* is 60% said peak concentration, at about the time that the concentration of plasma *Listeria* is 55% said peak concentration, at about the time that the concentration of plasma *Listeria* is 50% said peak concentration, at about the time that the concentration of plasma *Listeria* is 45% said peak concentration, at about the time that the concentration of plasma *Listeria* is 40% said peak concentration, at about the time that the concentration of plasma *Listeria* is 35% said peak concentration, at about the time that the concentration of plasma *Listeria* is 30% said peak concentration, at about the time that the concentration of plasma *Listeria* is 25% said peak concentration, at about the time that the concentration of plasma *Listeria* is 20% said peak concentration, at about the time that the concentration of plasma *Listeria* is 15% said peak concentration, at about the time that the concentration of plasma *Listeria* is 1 0% said peak concentration, at about the time that the concentration of plasma *Listeria* is 5% said peak concentration, at about the time that the concentration of plasma *Listeria* is 2.0% said peak concentration, at about the time that the concentration of plasma *Listeria* is 0.5% said peak concentration, at about the time that the concentration of plasma *Listeria* is 0.2% said peak concentration, or at about the time that the concentration of plasma *Listeria* is 0.1%, or less than, said peak concentration.

In another aspect, administration of the second reagent can begin at t = 0 hours, where the administration results in a peak (or maximal plateau) in plasma concentration of the second reagent and where administration of the *Listeria* is initiated at about the time that the concentration of plasma level of the second reagent reaches said peak concentration, at about the time that the concentration of plasma second reagent is 95% said peak concentration, at about the time that the concentration of plasma second reagent is 90% said peak concentration, at about the time that the concentration of plasma second reagent is 85% said peak concentration, at about the time that the concentration of plasma second reagent is 80% said peak concentration, at about the time that the concentration of plasma second reagent is 75% said peak concentration, at about the time that the concentration of plasma second reagent is 70% said peak concentration, at about the time that the concentration of plasma second reagent is 65% said peak concentration, at about the time that the concentration of plasma second reagent is 60% said peak concentration, at about the time that the concentration of plasma second reagent is 55% said peak concentration, at about the time that the concentration of plasma second reagent is 50% said peak concentration, at about the time that the concentration of plasma second reagent is 45% said peak concentration, at about the time that the concentration of plasma second reagent is 40% said peak concentration, at about the time that the concentration of plasma second reagent is 35% said peak concentration, at about the time that the concentration of plasma second reagent is 30% said peak concentration, at about the time that the concentration of plasma second reagent is 25% said peak concentration, at about the time that the concentration of plasma second reagent is 20% said peak concentration, at about the time that the concentration of plasma second reagent is 15% said peak concentration, at about the time that the concentration of plasma second reagent is 10% said peak concentration, at about the time that the concentration of plasma second reagent is 5% said peak concentration, at about the time that the concentration of plasma reagent is 2.0% said peak concentration, at about the time that the concentration of plasma second reagent is 0.5% said peak concentration, at about the time that the concentration of plasma second reagent is 0.2% said peak concentration, or at about the time that the concentration of plasma second reagent is 0.1%, or less than, said peak concentration. As it is recognized that alteration of the *Listeria* or second reagent may occur *in vivo,* the above concentrations can be assessed after measurement of intact reagent, or after measurement of an identifiable degradation product of the intact reagent.

Formulations of therapeutic and diagnostic agents may be prepared for storage by mixing with physiologically acceptable carriers, excipients, or stabilizers in the form of, e.g., lyophilized powders, slurries, aqueous solutions or suspensions (see, e.g., Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

This disclosure also provides a kit comprising a *Listeria* cell, a listerial cell culture, or a lyophilized cell preparation, and a compartment. In addition, the present invention provides a kit comprising a *Listeria* cell, listerial cell culture, or a lyophilized cell preparation and a reagent. Also provided is a kit comprising a *Listeria* cell, a listerial cell culture, or a lyophilized cell preparation and instructions for use or disposal. Moreover, the present invention provides a kit comprising a *Listeria* cell, a listerial cell culture, or lyophilized cell preparation, and compartment and a reagent. Provided is a kit comprising *Listeria* bacteria, and instructions for using the *Listeria* bacteria with a small molecule anti-cancer agent, and/or small molecule immunomodulating agent (e.g., cyclophosphamide), and/or a small molecule anti-infection agent, and the like. Also provided is a kit comprising *Listeria* bacteria, and/or instructions for administering the *Listeria*, and/or instructions for monitoring immune response to the administered *Listeria*, and/or instructions for monitoring immune response to a heterologous antigen encoded by the administered *Listeria.*

### (b). Uses.

This disclosure provides, in certain embodiments, a modified *Listeria* bacterium, e.g., *L. monocytogenes,* engineered to express at least one heterologous antigen. This disclosure is useful for enhancing immune response, stimulating immune response, enhancing immune presentation, increasing stability of an expressed mRNA or polypeptide, increasing proteolytic processing of an expressed polypeptide, increasing immune response to a mutated self antigen, increasing survival to a cancer or infection, and/or for treating a cancer or infection. This disclosure is also useful for enhanced expression of a heterologous antigen, e.g., for industry, agriculture, or medicine.

For methods to stimulate, enhance, or increase immune response to a cancer, tumor, or infectious agent; and for methods to stimulate, enhance, or increase survival to a cancer, tumor, or infectious agent; an increase can occur with administration of a *Listeria* containing a nucleic acid encoding a heterologous antigen. For purposes of providing an experimental control, the increase can be relative to response with administration of a *Listeria* not containing a nucleic acid encoding that particular heterologous antigen. As another alternative, the increase can be relative to response with administering a *Listeria* not containing any nucleic acid that encodes a heterologous antigen, e.g., a parental or wild type *Listeria.* As still another alternative, the increase can be relative to response without administering any *Listeria.*

For methods to stimulate, enhance, or increase immune response to a cancer, tumor, or infectious agent; and for methods to stimulate, enhance, or increase survival to a cancer, tumor, or infectious agent; an increase can occur with administration of a *Listeria* (containing or not containing a nucleic acid encoding a heterologous antigen) with an immune modulator, such as an agonist antibody, a cytokine, or an antibody that specifically binds to an antigen of the cancer, tumor, or infectious agent. For purposes of providing an experimental control, the increase can be relative to response with administration of a *Listeria* but without administering the immune modulator. As an alternative, the increase can be relative to any response with administering the immune modulator, but without administering any *Listeria.* As still another alternative, the increase can be relative to response without administering any *Listeria* and without administering the immune modulator.

In some embodiments, this disclosure provides methods of stimulating an immune response to an antigen in a mammal, comprising administering an effective amount of a *Listeria* bacterium described herein, or an effective amount of a composition comprising the *Listeria*, to the mammal. In some embodiments, this disclosure provides methods of stimulating an immune response to an antigen from, or derived from, a cancer or infectious agent, comprising administering an effective amount of a *Listeria* bacterium described herein, or an effective amount of a composition comprising the *Listeria*, to the mammal. In some embodiments, the heterologous antigen expressed by the Listeria shares at least one epitope with, or is immunologically cross-reactive with, the antigen from, or derived from, the cancer or infectious agent. In some embodiments, the immune response is a CD8+ T-cell immune response. In some embodiments, the immune response is a CD4+ T-cell immune response.

This disclosure provides a *Listeria* bacterium, or a *Listeria* strain, that is killed but metabolically active (KBMA) (see, e.g., Brockstedt, et al (2005) Nat. Med. 11:853-860). A KBMA *Listeria* bacterium is metabolically active, but cannot form a colony, e.g., on agar. An inactivating mutation in at least one DNA repair gene, e.g., ΔuvrAB, enables killing of *Listeria* using concentrations of a nucleic acid cross-linking agent (e.g., psoralen) at low concentrations, where these concentrations are sufficient to prevent colony formation but not sufficient to substantially impair metabolism, or to detectably impair metabolism. The result of limited treatment with psoralen/UVA light, and/or of treatment with a nucleic acid cross-linking agent that is highly specific for making interstrand genomic cross links, is that the bacterial cells are killed but remain metabolically active.

In some embodiments, the present invention results in the reduction of the number of abnormally proliferating cells, reduction in the number of cancer cells, reduction in the number of tumor cells, reduction in the tumor volume, reduction of the number of infectious organisms or pathogens per unit of biological fluid or tissue (e.g., serum), reduction in viral titer (e.g., serum), where it is normally reduced by at least 5%, more normally reduced by at least 10%, most normally reduced by at least 15%, typically reduced by at least 20%, more typically reduced by at least 25%, most typically reduced by at least 30%, usually reduced by at least 40%, more usually reduced by at least 50%, most usually reduced by at least 60%, conventionally reduced by at least 70%, more conventionally reduced by at least 80%, most conventionally reduced by at least 90%, and still most conventionally reduced by at least 99%. The unit of reduction can be, without limitation, number of tumor cells/mammalian subject; number of tumor cells/liver; number of tumor cells/spleen; mass of tumor cells/mammalian subject; mass of tumor cells/liver; mass of tumor cells/spleen; number of viral particles or viruses or titer per gram of liver; number of viral particles or viruses or titer per cell; number of viral particles or viruses or titer per ml of blood; and the like.

The growth medium used to prepare a *Listeria* can be characterized by chemical analysis, high pressure liquid chromatography (HPLC), mass spectroscopy, gas chromatography, spectroscopic methods, and the like. The growth medium can also be characterized by way of antibodies specific for components of that medium, where the component occurs as a contaminant with the *Listeria*, e.g., a contaminant in the listerial powder, frozen preparation, or cell paste. Antibodies, specific for peptide or protein antigens, or glycolipid, glycopeptide, or lipopeptide antigens, can be used in ELISA assays formulated for detecting animal-origin contaminants. Antibodies for use in detecting antigens, or antigenic fragments, of animal origin are available (see, e.g., Fukuta, et al. (1977) Jpn. Heart J. 18:696-704; DeVay and Adler (1976) Ann. Rev. Microbiol. 30:147-168; Cunningham, et al. (1984) Infection Immunity 46:34-41; Kawakita, et al. (1979) Jpn. Cir. J. 43:452-457; Hanly, et al. (1994) Lupus 3:193-199; Huppi, et al. (1987) Neurochem. Res. 12:659-665; Quackenbush, et al. (1985) Biochem. J. 225:291-299). This disclosure supplies kits and diagnostic methods that facilitate testing the *Listeria's* influence on the immune system. Testing can involve comparing one strain of *Listeria* with another strain of *Listeria*, or a parent *Listeria* strain with a mutated *Listeria* strain. Methods of testing comprise, e.g., phagocytosis, spreading, antigen presentation, T cell stimulation, cytokine response, host toxicity, LD₅₀, and efficacy in ameliorating a pathological condition.

The present disclosure provides methods to increase survival of a subject, host, patient, test subject, experimental subject, veterinary subject, and the like, to a cancer, a tumor, a precancerous disorder, an immune disorder, and/or an infectious agent. The infectious agent can be a virus, bacterium, or parasite, or any combination thereof. The method comprises administering an attenuated *Listeria*, for example, as a suspension, bolus, gel, matrix, injection, or infusion, and the like. The administered *Listeria* increases survival, as compared to an appropriate control (e.g., nothing administered or an administered placebo, and the like) by usually at least one day; more usually at least four days; most usually at least eight days, normally at least 12 days; more normally at least 16 days; most normally at least 20 days, often at least 24 days; more often at least 28 days; most often at least 32 days, conventionally at least 40 days, more conventionally at least 48 days; most conventionally at least 56 days; typically by at least 64 days; more typically by at least 72 days; most typically at least 80 days; generally at least six months; more generally at least eight months; most generally at least ten months; commonly at least 12 months; more commonly at least 16 months; and most commonly at least 20 months, or more.

In some embodiments, the subject/host/patient to which the *Listeria* is administered is a mammal. In some embodiments, the mammal is a primate. In some embodiments, the primate is a human.

Each of the above disclosed methods contemplates administering a composition comprising a *Listeria* and an excipient, a *Listeria* and a carrier, a *Listeria* and buffer, a *Listeria* and a reagent, a *Listeria* and a pharmaceutically acceptable carrier, a *Listeria* and an agriculturally acceptable carrier, a *Listeria* and a veterinarily acceptable carrier, a *Listeria* and a stabilizer, a *Listeria* and a preservative, and the like.

The present invention provides reagents and methods for treating conditions that are both cancerous (neoplasms, malignancies, cancers, tumors, and/or precancerous disorders, dysplasias, and the like) and infectious (infections). Provided are reagents and methods for treating disorders that are both cancerous (neoplasms, malignancies, cancers, tumors, and/or precancerous disorders, dysplasias, and the like) and infectious. With infection with certain viruses, such as papillomavirus and polyoma virus, the result can be a cancerous condition, and here the condition is both cancerous and infectious. A condition that is both cancerous and infectious can be detected, as a non-limiting example, where a viral infection results in a cancerous cell, and where the cancerous cell expresses a viral-encoded antigen. As another non-limiting example, a condition that is both cancerous and infectious is one where immune response against a tumor cell involves specific recognition against a viral-encoded antigen (See, e.g., Montesano, et al. (1990) Cell 62:435-445; Ichaso and Dilworth (2001) Oncogene 20:7908-7916; Wilson, et al. (1999) J. Immunol. 162:3933-3941; Daemen, et al. (2004) Antivir. Ther. 9:733-742; Boudewijn, et al. (2004) J. Natl. Cancer Inst. 96:998-1006; Liu, et al. (2004) Proc. Natl. Acad. Sci. USA 101:14567-14571).

In some embodiments, the *Listeria* described herein that express a heterologous antigen (or compositions comprising the *Listeria*) are used to induce immune responses against cells in a subject that express the antigen. In some embodiments, the *Listeria,* vaccines, and other compositions described herein are used to treat cancerous disorders, precancerous disorders, tumors, infections, and/or angiogenesis of tumors and cancers. In some embodiments, the *Listeria*, vaccines, and other compositions described herein are used to treat cancer in a subject.

The following embodiments relate to the individual embodiments disclosed herein.

The present invention, in certain embodiments, comprises a method of stimulating the immune system against an infectious disorder, where the infectious disorder is a *Listeria* infection. Also comprised, is a method of stimulating the immune system against an infectious disorder, where the infectious disorder is not a *Listeria* infection, that is, excludes *Listeria* infections.

Each of the embodiments encompasses, as an alternate or additional reagent, a *Listeria* that is not attenuated. Also, each of the embodiments encompasses, as an alternate or additional reagent, a *Listeria* that is attenuated. Each of the embodiments encompasses, as an alternate or additional method, using *a Listeria* that is not attenuated. Also, each of the embodiments encompasses, as an alternate or additional method, using a *Listeria* that is attenuated.

Each of the embodiments disclosed herein encompasses methods and reagents using a *Listeria* that comprises a nucleic acid encoding at least one tumor antigen, a *Listeria* that comprises a nucleic acid encoding at least one cancer antigen, a *Listeria* that comprises a nucleic acid encoding at least one heterologous antigen, as well as a *Listeria* that expresses at least one tumor antigen, cancer antigen, and/or heterologous antigen.

Each of the embodiments disclosed herein encompasses methods and reagents using a *Listeria* that does not comprise a nucleic acid encoding a tumor antigen, a *Listeria* that does not comprise a nucleic acid encoding a cancer antigen, a *Listeria* that does not comprise a nucleic acid encoding a heterologous antigen, as well as a *Listeria* that does not express a tumor antigen, cancer antigen, and/or a heterologous antigen.

Each of the embodiments disclosed herein encompasses methods and reagents using a *Listeria* that comprises a nucleic acid encoding an antigen from a non-listerial infectious organism. Each of the above-disclosed embodiments encompasses methods and reagents using a *Listeria* that comprises a nucleic acid encoding at least one antigen from a virus, parasite, bacterium, tumor, self-antigen derived from a tumor, or non-self antigen derived from a tumor.

Each of the embodiments disclosed herein encompasses methods and reagents using a *Listeria* that does not comprise a nucleic acid encoding an antigen from a non-listerial infectious organism. Each of the above-disclosed embodiments encompasses methods and reagents using a *Listeria* that does not comprise a nucleic acid encoding at least one antigen from a virus, parasite, bacterium, tumor, self-antigen derived from a tumor, or non-self antigen derived from a tumor.

Each of the embodiments disclosed herein also encompasses a *Listeria* that is not prepared by growing on a medium based on animal protein, but is prepared by growing on a different type of medium. Each of the above-disclosed embodiments also encompasses a *Listeria* that is not prepared by growing on a medium containing peptides derived from animal protein, but is prepared by growing on a different type of medium. Moreover, each of the above-disclosed embodiments encompasses administration of a *Listeria* by a route that is not oral or that is not enteral. Additionally, each of the above-disclosed embodiments includes administration of a *Listeria* by a route that does not require movement from the gut lumen to the lymphatics or bloodstream.

Each of the embodiments disclosed herein further comprises a method wherein the *Listeria* are not injected directly into the tumor or are not directly injected into a site that is affected by the cancer, precancerous disorder, tumor, or infection.

Additionally, each of the embodiments disclosed herein encompasses administering the *Listeria* by direct injection into a tumor, by direct injection into a cancerous lesion, and/or by direct injection into a lesion of infection. Also, this disclosure includes each of the above embodiments, where administration is not by direct injection into a tumor, not by direct injection into a cancerous lesion, and/or not by direct injection into a lesion of infection.

Provided is a vaccine where the heterologous antigen, as in any of the embodiments disclosed herein, is a tumor antigen or is derived from a tumor antigen. Also provided is a vaccine where the heterologous antigen, as in any of the embodiments disclosed herein, is a cancer antigen, or is derived from a cancer antigen. Moreover, what is provided is a vaccine where the heterologous antigen, as in any of the embodiments disclosed herein, is an antigen of an infectious organism, or is derived from an antigen of an infectious organism, e.g., a virus, bacterium, or multi-cellular organism.

A further embodiment provides a nucleic acid where the heterologous antigen, as in any of the embodiments disclosed herein, is a tumor antigen or derived from a tumor antigen. Also provided is a nucleic acid where the heterologous antigen, as in any of the embodiments disclosed herein, is a cancer antigen, or is derived from a cancer antigen. Moreover, what is provided is a nucleic acid, where the heterologous antigen, as in any of the embodiments disclosed herein, is an antigen of an infectious organism, or is derived from an antigen of an infectious organism, e.g., a virus, bacterium, or multi-cellular organism.

In another embodiment, what is provided is a *Listeria* where the heterologous antigen, as in any of the embodiments disclosed herein, is a tumor antigen or derived from a tumor antigen. Also provided is a *Listeria* where the heterologous antigen, as in any of the examples disclosed herein, is a cancer antigen, or is derived from a cancer antigen. Moreover, what is provided is a *Listeria*, where the heterologous antigen, as in any of the embodiments disclosed herein, is an antigen from an infectious organism or derived from an antigen of an infectious organism, e.g., a virus, bacterium, parasite, or multi-cellular organism.

Each of the above-disclosed embodiments also encompasses an attenuated *Listeria* that is not prepared by growing on a medium based on animal or meat protein, but is prepared by growing on a different type of medium. Provided is an attenuated *Listeria* not prepared by growing on a medium based on meat or animal protein, but is prepared by growing on a medium based on yeast and/or vegetable derived protein.

Unless specified otherwise, each of the embodiments disclosed herein encompasses a bacterium that does not contain a nucleic acid encoding a heterologous antigen. Also, unless specified otherwise, each of the embodiments disclosed herein encompasses a bacterium that does not contain a nucleic acid encoding a heterologous regulatory sequences. Optionally, every one of the embodiments disclosed herein encompasses a bacterium that contains a nucleic acid encoding a heterologous antigen and/or encoding a heterologous regulatory sequence.

The following concerns bacterial embodiments, e.g., of *Listeria*, *Bacillus anthracis*, or another bacterium, that encode secreted antigens, non-secreted antigens, secreted antigens that are releasable from the bacterium by a mechanism other than secretion, and non-secreted antigens that are releasable by a mechanism other than secretion. What is embraced is a bacterium containing a polynucleotide comprising a nucleic acid, where the nucleic acid encodes a polypeptide that contains a secretory sequence and is secreted under appropriate conditions; where the nucleic acid encodes a polypeptide that does not contain a secretory sequence; where the nucleic acid does contain a secretory sequence and where the polypeptide is releasable by some other mechanism such as enzymatic damage or perforation to the cell membrane or cell wall; and where the nucleic acid encodes a polypeptide that does not contain any secretory sequence but where the polypeptide is releasable by some other mechanism, such as enzymatic damage or perforation to the cell membrane and/or cell wall.

Without implying any limitation, as to narrowness or breadth, of the present invention, this disclosure can be modified by the skilled artisan to comprise any one of the following embodiments, or to consist of any one of the following embodiments (Table 10).

| Table 10. Spread of the bacterium of the present invention, i.e., transmission of a bacterium from a first host cell to a second host cell. | | |
|---|---|---|
| Without implying any limitation to the bacterium of the present invention, e.g., with regard to its ability to spread from cell to cell, the spread of the bacterium of the present invention can encompass one or more of the following. Without implying any lack of limitation to the bacterium of the present invention, e.g., with regard to its ability to spread from cell to cell, the spread of the bacterium of the present invention can encompass one or more of the following. | | |
| The spread of the bacterium of the present invention can be | at most 1%; at most 5%; at most 10%; at most 20%; at most 30%; at most 40%; at most 50%; at most 60%; at most 70%; at most 80%; at most 90%; at most 95%; at most 100%; at most 200%; at most 300%; at most 400%; at most 500%, | as compared to the spread of a suitable control or parent bacterium. |
| The spread of the bacterium of the present invention can be | at least 1%; at least 5%; at least 10%; at least 20%; at least 30%; at least 40%; at least 50%; at least 60%; at least 70%; at least 80%; at least 90%; at least 95%; at least 100%; at least 200%; at least 300%; at least 400%; at least 500%, | as compared to the spread of a suitable control or parent bacterium. |
| The spread of the bacterium of the present invention can be | 0 to 1%; 1% to 5%; 5% to 10%; 10% to 20%; 20% to 30%; 30% to 40%; 40% to 50%; 50% to 60%; 60% to 70%; 70% to 80%; 80% to 90%; 90% to 95%; 90% to 100%; 100% to 200%; 200% to 300%; 300% to 400%; 400% to 500%, or greater than 500%, | as compared to the spread of a suitable control or parent bacterium. |

| Table 10 (continued). Growth of the *Listeria* strain of the present invention. Without implying any limitation to the present invention, e.g., as to narrowness or to breadth, the present invention can encompass any one, or any of combination, of the following embodiments. Without implying any lack of limitation to the present invention, the present invention can encompass any one, or any combination, of the following embodiments. | | |
|---|---|---|
| Growth of the *Listeria* strain of the present invention is at least | 0.1%; 0.5%; 1.0%; 5%; 10%; 15%; 20%; 25%; 30%; 35%; 40%; 45%; 50%; 55%; 60%; 65%; 70%; 75%; 80%; 85%; 90%; 95%; 99%; 99.5%; 99.5%, 2-fold; 5-fold; 10-fold; or greater than 10-fold, | as compared with the parent *Listeria* or with a suitable control *Listeria.* |
| Growth of the *Listeria* strain of the present invention is at most | not detectable, 0.1%; 0.5%; 1.0%; 5%; 10%; 15%; 20%; 25%; 30%; 35%; 40%; 45%; 50%; 55%; 60%; 65%; 70%; 75%; 80%; 85%; 90%; 95%; 99%; 99.5%; 99.5%, 2-fold; 5-fold; 10-fold; or greater than 10-fold, | as compared with the parent *Listeria* or with a suitable control *Listeria.* |
| Growth of the *Listeria* strain of the present invention is less than | 0.1%; 0.5%; 1.0%; 5%; 1 0%; 15%; 20%; 25%; 30%; 35%; 40%; 45%; 50%; 55%; 60%; 65%; 70%; 75%; 80%; 85%; 90%; 95%; 99%; 99.5%; or 99.5%; 2-fold; 5-fold; 10-fold; or greater than 10-fold, | as compared with the parent *Listeria* or with a suitable control *Listeria.* |
| Growth of the *Listeria* strain of the present invention is more than | 0.1%; 0.5%; 1.0%; 5%; 10%; 15%; 20%; 25%; 30%; 35%; 40%; 45%; 50%; 55%; 60%; 65%; 70%; 75%; 80%; 85%; 90%; 95%; 99%; 99.5%; 99.5%, 2-fold; 5-fold; 10-fold; or greater than 10-fold, | as compared with the parent *Listeria* or with a suitable control *Listeria.* |
| Growth of the *Listeria* of the present invention is between | 0-0.1%; 0.1-0.5%; 0.5-1.0%; 1.0-5%; 5-10%; 10-15%, 15-20%; 20-25%; 25-30%; 30-35%; 35-40%; 40-45%; 45-50%; 50-55%; 55-60%; 60-65%; 65-70%; 70-75%; 75-80%; 80-85%; 85-90%; 90-95%; 95-99%; 99-99.5%; 99.5-99.5%, 99.5%-greater, 100% to 2-fold; 2-fold to 10-fold; 10-fold to 10-fold, | as compared with the parent *Listeria* or with a suitable control *Listeria.* |

| Table 10 (continued). Growth of the *Listeria* strain of the present invention. | | |
|---|---|---|
| Extracellular growth of the *Listeria* strain of the present invention is at least | 0.1%; 0.5%; 1.0%; 5%; 10%; 15%; 20%; 25%; 30%; 35%; 40%; 45%; 50%; 55%; 60%; 65%; 70%; 75%; 80%; 85%; 90%; 95%; 99%; 99.5%; or greater than 99.5%, 100%, 2-fold greater; 5-fold greater; or 10-fold greater, | as compared with intracellular growth of the same *Listeria* strain. |
| Extracellular growth of the *Listeria* strain of the present invention is | 0-0.1%; 0.1-0.5%; 0.5-1.0%; 1.0-5%; 5-10%; 10-15%; 15-20%; 20-25%; 25-30%; 30-35%; 35-40%; 40-45%; 45-50%; 50-55%; 55-60%; 60-65%; 65-70%; 70-75%; 75-80%; 80-85%; 85-90%; 90-95%; 95-99%; 99-99.5%; 99.5-100%, 100-200%; 200-500%; 500-1000%; or greater than 1000%, | as compared with intracellular growth of the same *Listeria* strain. |
| Growth related genes. A growth related gene of the present invention can include, but is not necessarily limited in narrowness or in breadth, by the following. | | |
| A growth related gene embraces one that stimulates the rate of intracellular growth by | the same amunt, by at least 10% greater; by at least 20% greater; by at least 30% greater; by at least 40% greater; by at least 50% greater; by at least 60% greater; by at least 70% greater; by at least 80% greater; by at least 90% greater; by at least 100% (2-fold) greater; by at least 3-fold greater; by at least 4-fold greater; by at least 10-fold greater; by at least 20-fold greater; by at least 40-fold greater, | than the rate the gene stimulates extracellular growth. |
| Growth of a *Listeria* strain of the present invention can be compared with a parent, or suitable control, *Listeria* strain, where only intracellular growth is compared. Growth of a *Listeria* strain of the present invention can be compared with a parent, or suitable control, *Listeria* strain, where only extracellular growth is compared. Growth of a *Listeria* strain of the present invention can be compared with a parent, or suitable control, *Listeria* strain, where intracellular growth of the present invention strain is compared with extracellular growth of a parent or suitable control strain. | | |
| Growth of a *Listeria* strain of the present invention can be compared with a parent, or suitable control, *Listeria* strain, where extracellular growth of the present invention strain is compared with intracellular growth of a parent or suitable control strain. | | |

| Table 10 (continued). Metabolically active bacteria. Without implying any limitation to the present invention, e.g., as to narrowness or to breadth, the present invention can encompass any one, or any of combination, of the following embodiments. Without implying any lack of limitation to the present invention, e.g., as to narrowness or to breadth, the present invention can encompass any one, or any of combination, of the following embodiments. | | |
|---|---|---|
| A metabolically active but colony formation impaired (and/or cell division or replication impaired) *Listeria* bacterium of the present invention encompasses a *Listeria* bacterium where the rate of colony formation, cell division, and/or replication is under 40% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%, | that of the control or parent *Listeria* bacterium. |
| A metabolically active but colony formation impaired (and/or cell division or replication impaired) *Listeria* bacterium of the present invention encompasses a *Listeria* bacterium where the rate of colony formation, cell division, and/or replication is under 30% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; or 40% to 30, | that of the control or parent *Listeria* bacterium. |
| A metabolically active but colony formation impaired (and/or cell division or replication impaired) *Listeria* bacterium of the present invention encompasses a *Listeria* bacterium where the rate of colony formation, cell division, and/or replication is under 20% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; or 30 to 20%, | that of the control or parent *Listeria* bacterium. |
| A metabolically active but colony formation impaired (and/or cell division or replication impaired) *Listeria* bacterium of the present invention encompasses a *Listeria* bacterium where the rate of colony formation, cell division, and/or replication is under 10% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; or 20 to 10%, | that of the control or parent *Listeria* bacterium. |
| A metabolically active but colony formation impaired (and/or cell division or replication impaired) *Listeria* bacterium of the present invention encompasses a *Listeria* bacterium where the rate of colony formation, cell division, and/or replication is under 5% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20 to 10%; or 10 to 5%, | that of the control or parent *Listeria* bacterium. |

| Table 10 (continued). Metabolically active bacteria. | | |
|---|---|---|
| A metabolically active but colony formation impaired (and/or cell division or replication impaired) *Listeria* bacterium of the present invention encompasses a *Listeria* bacterium where the rate of colony formation, cell division, and/or replication is under 1% that of a parent or control *Listeria* bacterium, | and where metabolism is greater than 10-fold; 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20-10%; 10-5%; or 5% to 1%, | that of the control or parent *Listeria* bacterium. |
| A "killed but metabolically active" (KMBA) bacterium, is a *Listeria* bacterium that is unable to form colonies and where metabolism is, e.g., 10-fold to 5-fold (an indicator of metabolism occurring at a level higher than normally found); 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20 to 10%; or 10 to 5%, that of a control or parent *Listeria* bacterium. In another aspect, a KBMA bacterium is a *Listeria* bacterium where the rate of colony formation is under 1% that of a control or parent *Listeria* bacterium, and where metabolism is, e.g., 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20 to 10%; or 10 to 5%, that of the control or parent *Listeria* bacterium. In yet another aspect, a KBMA bacterium is a *Listeria* bacterium where the rate of colony formation is under 2% that of a control or parent *Listeria* bacterium, and where metabolism is, e.g., 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20 to 10%; or 10 to 5%, that of the control or parent *Listeria* bacterium. In another embodiment, a KBMA bacterium is a *Listeria* bacterium where the rate of colony formation is under 5% that of a control or parent *Listeria* bacterium, and where metabolism is, e.g., 10-fold to 5-fold; 5-fold to 4-fold; 4-fold to 2-fold; 2-fold to 100%; essentially 100%; 100% to 95%; 95% to 90%; 90% to 80%; 80% to 70%; 70% to 60%; 60% to 50%; 50% to 40%; 40% to 30; 30 to 20%; 20 to 10%; or 10 to 5%, that of the control or parent *Listeria* bacterium. | | |

The rate of metabolism can be measured by various indicia, e.g., translation, respiration, secretion, transport, fermentation, glycolysis, amino acid metabolism, or the Krebs cycle. Various indicia of metabolism for *L. monocytogenes* are disclosed (see, e.g., Karlin, et al. (2004) Proc. Natl. Acad. Sci. USA 101:6182-6187; Gilbreth, et al. (2004) Curr. Microbiol. 49:95-98). Often, metabolism is assessed with intact bacteria by way of radioactive, heavy isotope, or fluorescent tagged metabolites. The skilled artisan can choose a suitable gene for measuring translation, or a suitable enzyme for measuring glycolysis, amino acid metabolism, or the Krebs cycle. A heat-killed bacterium generally is essentially or totally metabolically inactive. Residual apparent metabolic activity of an essentially or totally metabolically inactive bacterium can be due, e.g., to oxidation of lipids, oxidation of sulfhydryls, reactions catalyzed by heavy metals, or to enzymes that are stable to heat-treatment.

### (c) Methods for assessing immune response; methods of diagnosis.

Reagents and methods useful for determining, assessing, monitoring, and/or diagnosing immune response are available. The present disclosure, in some situations, provides the following methods for diagnosing a mammalian subject administered with the compositions of the present invention. In other aspects, what is provided are the following methods for assessing immune response to one or more of the administered compositions of the present invention. These methods, which can be applied, e.g., *in vivo, in vitro, ex vivo, in utero*; to living or deceased mammals; to cells; to recombinant, chimeric, or hybrid cells; to biological fluids, to isolated nucleic acids, and the like, include:
i. Methods for measuring cellular parameters. What can be measured includes effector T cells; central memory T cells (T_{CM}); effector memory T cells (T_{EM}), and constituents thereof. What can be measured are biological functions of these cells including cytotoxic function, expression of markers, affinity for antigen, number of cells in a biological compartment such as serum, preferred location in the body such as in lymph node or spleen, and rate of response when exposed or re-exposed to antigen.
ii. Methods for measuring antibodies. What can be measured is affinity maturation of antibodies (see, e.g., McHeyzer-Williams and McHeyzer-Williams (2005) Ann. Rev. Immunol. 23:487-513), antibody titer or isotype, including IgG (IgG₁; IgG₂; IgG₃; IgG₄); IgA (IgA₁; IgA₂); IgM; IgD; IgE; isotype switching of antibodies, for example, decreases in IgM and increases in IgG (see, e.g., Hasbold, et al. (2004) Nature Immunol. 5:55-63; Ryffel, et al. (1997) J. Immunol. 158:2126-2133; Lund, et al. (2002) J. Immunol. 169:5236-5243; Palladino, et al. (1995) J. Virol. 69:2075-2081; Karrer, et al. (2000) J. Immunol. 164:768-778); isotype switching that is a function of Th1-type or Th2-type response (Delate, et al. (2005) J. Immunol. 175:6723-6732; McKenzie, et al. (1999) J. Exp. Med. 189:1565-1572; Fayette, et al. (1997) J. Exp. Med. 185:1909-1918).
iii. Parameters of B cells. What can be measured includes naive B cells (high in membrane IgD and low in CD27), memory B cells (low in IgD and high in CD27), and constituents of these cells (see, e.g., Fecteau and Neron (2003) J. Immunol. 171:4621-4629). What can be measured is formation of memory B cells within germinal centers (see, e.g., Ohkubo, et al. (2005) J. Immunol. 174:7703-7710). What can be measured includes terminally differentiated B cells, for example, cell's ability to respond to CXCL12 (see, e.g., Roy, et al. (2002) J. Immunol. 169:1676-1682). What can be measured includes commitment antibody-secreting cells (ASCs) (see, e.g., Hasbold, et al. (2004) Nature Immunol. 5:55-63).
iv. Parameters of T cells. What can be measured is affinity of a peptide for T cell receptor, affinity maturation of T cell receptor (see, e.g., Rees, et al. (1999) Proc. Natl. Acad. Sci. USA 96:9781-9786; McKinney, et al. (2004) J. Immunol. 173:1941-1950). What can be measured is affinity of a cytotoxic T cell for a target cell (see, e.g., Montoya and Del Val (1999) J. Immunol. 163:1914-1922). What can be measured includes markers, for example, effector memory T cells (T_{EM}) can be identified as CD62L^{LOW} and CCR7^{LOW}, where these cells show immediate effector function with antigen re-encounter. Central memory T cells (T_{CM}) can be identified by relatively high expression of CD62L and CCR7, where the cells show a relatively slow activation kinetics. Other available markers include, e.g., CCL4, CCL5, XCL1, granulysin, granzyme A, granzyme B, and so on (see, e.g., Chtanova, et al. (2005) J. Immunol. 175:7837-7847; Kondrack, et al. (2003) J. Exp. Med. 198:1797-1806; Huster, et al. (2004) Proc. Natl. Acad. Sci. USA 101:5610-5615; Ahmadzadeh, et al. (2001) J. Immunol. 166:926-935; Goldrath, et al. (2004) Proc. Natl. Acad. Sci. USA 101:16885-16890; Wherry, et al. (2003) Nature Immunol. 4:225-234; Sallusto, et al. (2004) Ann. Rev. Immunol. 22:745-763). Different types of immune cells, as well as different stages of maturation of a particular cell, or different stages of activation of a cell, can be distinguished by titrating with a reagent specific to any given marker (see, e.g., Ahmadzah, et al. (2001) J. Immunol. 166:926-935).
v. Parameters of antigen presenting cells (APCs), including dendritic cells (DCs). What can be measured is mmoles of peptide presented (or bound) per mmole MHC Class I. Moreover, what can be measured is mmoles peptide presented or bound per mmol of MHC Class II. Also, what can be measured is the amino acid sequence of the bound peptides (see, e.g., Velazquez, et al. (2001) J. Immunol. 166:5488-5494). In addition, what can be measured is relative ability of the APC to present epitopes derived from peptides versus epitopes derived from proteins, as well as ability to present epitopes acquired from low levels of peptides versus high levels of peptides and, in other aspects, the identity of the APC suitable for presentation (see, e.g., Constant, et al. (1995) J. Immunol. 154:4915-4923).

A number of specific examples of generally applicable methods for assessing expression, secretion, presentation, immunogenicity, and/or therapeutic efficacy of candidate expression constructs and candidate *Listeria*-based vaccines are exemplified in the Examples below. See, e.g., Example IX, below. Assays for assessing immunogenicity of a particular candidate *Listeria*-based vaccine are further provided, e.g., in U.S. Patent Publication Nos. 2005/0249748, 2005/0281783, and 2004/0228877.

Guidance is available for the skilled artisan in designing diagnostic appropriate controls (see, e.g., Wilson (1991) An Introduction to Scientific Research, Dover Publications, Mineola, NY).

The broad scope of this invention is best understood with reference to the following examples, which are not intended to limit the invention to any specific embodiments.

### EXAMPLES

### I. General methods.

Standard methods of biochemistry and molecular biology are described (see, e.g., Maniatis, et al. (1982) Molecular Cloning, A Laboratory Manual, Cold Spring Harbor Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA; Innis, et al. (eds.) (1990) PCR Protocols:A Guide to Methods and Applications, Academic Press, N.Y. Standard methods are also found in Ausbel, et al. (2001) Curr. Protocols in Mol. Biol., Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4)). Methods for producing fusion proteins are described (see, e.g., Invitrogen (2005) Catalogue, Carlsbad, CA; Amersham Pharmacia Biotech. (2005) Catalogue, Piscataway, NJ; Liu, et al. (2001) Curr. Protein Pept. Sci. 2:107-121; Graddis, et al. (2002) Curr. Pharm. Biotechnol. 3:285-297).

Splice overlap extension PCR, and other methods, for creating mutations, restriction sites, loxP sites, and the like, are described (see, e.g., Horton, et al. (1990) Biotechniques 8:528-535; Horton, et al. (1989) Gene 77:61-68; Horton (1995) Mol Biotechnol. 3:93-99; Cutrone and Langer (2001) J. Biol. Chem. 276:17140-17148; Cox, et al. (2002) Nucleic Acids Res. 30:e108; Warrens, et al. (1997) Gene 186:29-35; Guo and Bi (2002) Methods Mol. Biol. 192:111-119; Johnson (2000) J. Microbiol. Methods 41:201-209; Lantz, et al. (2000) Biotechnol. Annu. Rev. 5:87-130; Gustin and Burk (2000) Methods Mol. Biol. 130:85-90; QuikChange® Mutagenesis Kit, Stratagene, La Jolla, CA). Engineering codon preferences of signal peptides, secretory proteins, and heterologous antigens, to fit the optimal codons of a host are described (Sharp, et al. (1987) Nucl. Acids Res. 15:1281-1295; Uchijima, et al. (1998) J. Immunol. 161:5594-5599). Engineering codon preferences of signal peptides, secretory proteins, and heterologous antigens, to fit the optimal codons of a host are described (Sharp, et al. (1987) Nucl. Acids Res. 15:1281-1295; Uchijima, et al. (1998) J. Immunol. 161:5594-5599). Polynucleotides and nucleic acids are available, e.g., from Blue Heron Biotechnology, Bothell, WA).

Methods for effecting homologous recombination in, e.g., bacteria, phages, and plasmids, are available (see, e.g., Kuzminov (1999) Microb. Mol. Biol. Rev. 63:751-813; Camerini-Otero and Hsieh (1995) Annu. Rev. Genet. 29:509-552; Amundsen and Smith (2003) Cell 112:741-744; Cox (2001) Annu. Rev. Genet. 35:53-82; Quiberoni, et al. (2001) Res. Microbiol. 152:131-139; Fernandez, et al. (2000) Res. Microbiol. 151:481-486; Wedland (2003) Curr. Genet. 44:115-123; Muttucumaru and Parish (2004) Curr. Issues Mol. Biol. 6:145-157; Bhattacharyya, et al. (2004) Infect. Genet. Evol. 4:91-98).

A number of transducing listeriophages, as well as techniques for infecting *L. monocytogenes* with listeriophages are available. These listeriophages include, e.g., P35, U153, and derivatives thereof (see, e.g., Lauer, et al. (2002) J. Bact. 184:4177-4186; Hodgson (2000) Mol. Microbiol. 35:312-323; Mee-Marquet, et al. (1997) Appl. Environ. Microbiol. 63:3374-3377; Zink and Loessner (1992) Appl. Environ. Microbiol. 58:296-302; Loessner, et al. (1994) Intervirol. 37:31-35; Loessner, et al. (1994) J. Gen. Virol. 75:701-710; Loessner, et al. (2000) Mol. Microbiol. 35:324-340).

Methods for using electroporation and *E.coli-*mediated conjugation for introducing nucleic acids into *Listeria* are described. Plasmids suitable for introducing a nucleic acid into a bacterium include, e.g., pPL1 (GenBank assession no:AJ417488), pPL2 (Acc. No. AJ417449), pLUCH80, pLUCH88, and derivatives thereof (see, e.g., Lauer, et al. (2002) J. Bact. 184:4177-4186; Wilson, et al. (2001) Infect. Immunity 69:5016-5024; Chesneau, et al. (1999) FEMS Microbiol. Lett. 177:93-100; Park and Stewart (1990) Gene 94:129-132; Luchansky, et al. (1988) Mol. Microbiol. 2:537-646; He and Luchansky (1997) Appl. Environ. Microbiol. 63:3480-3487).

Methods for protein purification such as immunoprecipitation, column chromatography, electrophoresis, isoelectric focusing, centrifugation, and crystallization, are described (Coligan, et al. (2000) Current Protocols in Protein Science, Vol. 1, John Wiley and Sons, Inc., New York). Chemical analysis, chemical modification, post-translational modification, and glycosylation of proteins is described. See, e.g., Coligan, et al. (2000) Current Protocols in Protein Science, Vol. 2, John Wiley and Sons, Inc., New York; Walker (ed.) (2002) Protein Protocols Handbook, Humana Press, Towota, NJ; Lundblad (1995) Techniques in Protein Modification, CRC Press, Boca Raton, FL. Techniques for characterizing binding interactions are described (Coligan, et al. (2001) Current Protocols in Immunology, Vol. 4, John Wiley and Sons, Inc., New York; Parker, et al. (2000) J. Biomol. Screen. 5: 77-88; Karlsson, et al. (1991) J. Immunol. Methods 145:229-240; Neri, et al. (1997) Nat. Biotechnol. 15:1271-1275; Jonsson, et al. (1991) Biotechniques 11:620-627; Friguet, et al. (1985) J. Immunol. Methods 77: 305-319; Hubble (1997) Immunol. Today 18:305-306; Shen, et al. (2001) J. Biol. Chem. 276:47311-47319).

Software packages for determining, e.g., antigenic fragments, leader sequences, protein folding, functional domains, glycosylation sites, and sequence alignments, are available (see, e.g., Vector NTI® Suite (Informax, Inc, Bethesda, MD); GCG Wisconsin Package (Accelrys, Inc., San Diego, CA); DeCypher® (TimeLogic Corp., Crystal Bay, Nevada); Menne, et al. (2000) Bioinformatics 16: 741-742; Menne, et al. (2000) Bioinformatics Applications Note 16:741-742; Wren, et al. (2002) Comput. Methods Programs Biomed. 68:177-181; von Heijne (1983) Eur. J. Biochem. 133:17-21; von Heijne (1986) Nucleic Acids Res. 14:4683-4690). Methods for determining coding sequences (CDS) are available (Furono, et al. (2003) Genome Res. 13:1478-1487).

Computer algorithms (e.g., BIMAS; SYFPEITHI) for identifying peptides that bind to MHC Class I and/or MHC Class II are available (Thomas, et al. (2004) J. Exp. Med. 200:297-306). These algorithms can provide nucleic acids of the present invention that encode proteins comprising the identified peptides.

Sequences of listerial proteins and nucleic acids can be found on the world wide web at: (1) ncbi.nlm.nih.gov; (2) genolist.Pasteur.fr (with clicking on "listilist"); and (3) tigr.org (with clicking on "databases," then on "comprehensive microbial resource").

Methods are available for assessing internalization of a *Listeria* by an APC, and for assessing presentation of listerial-encoded antigens by the APC. Methods are also available for presentation of these antigens to T cell, and for assessing antigen-dependent priming of the T cell. A suitable APC is murine DC 2.4 cell line, while suitable T cell is the B3Z T cell hybridoma (see, e.g., U.S. Provisional Pat. Appl. Ser. No. 60/490,089 filed July 24, 2003; Shen, et al. (1997) J. Immunol. 158:2723-2730; Kawamura, et al. (2002 J. Immunol. 168:5709-5715; Geginat, et al. (2001) J. Immunol. 166:1877-1884; Skoberne, et al. (2001) J. Immunol. 167:2209-2218; Wang, et al. (1998) J. Immunol. 160:1091-1097; Bullock, et al. (2000) J. Immunol. 164:2354-2361; Lippolis, et al. (2002) J. Immunol. 169:5089-5097). Methods for preparing dendritic cells (DCs), *ex vivo* modification of the DCs, and administration of the modified DCs, e.g., for the treatment of a cancer, pathogen, or infective agent, are available (see, e.g., Ribas, et al. (2004) J. Immunother. 27:354-367; Gilboa and Vieweg (2004) Immunol. Rev. 199:251-263; Dees, et al. (2004) Cancer Immunol. Immunother. 53:777-785; Eriksson, et al. (2004) Eur. J. Immunol. 34:1272-1281; Goldszmid, et al. (2003) J. Immunol. 171:5940-5947; Coughlin and Vonderheide (2003) Cancer Biol. Ther. 2:466-470; Colino and Snapper (2003) Microbes Infect. 5:311-319).

Assays for *Listeria* plaque size, LD₅₀, and motility are described. Plaque diameter is a function of a bacterium's ability to grow, to move from from cell to cell, and to escape from a secondary vesicle formed in an adjacent cell (see, e.g., Lauer, et al. (2001) Mol. Microbiol. 42:1163-1177; Theriot, et al. (1994) Cell 76:505-517; Theriot, et al. (1998) Meth. Enzymol. 298:114-122; Portnoy, et al. (1988) J. Exp. Med. 167:1459-1471).

Elispot assays and intracellular cytokine staining (ICS) for characterizing immune cells are available (see, e.g., Lalvani, et al. (1997) J. Exp. Med. 186:859-865; Waldrop, et al. (1997) J. Clin. Invest. 99:1739-1750; Hudgens, et al. (2004) J. Immunol. Methods 288:19-34; Goulder, et al. (2001) J. Virol. 75:1339-1347; Goulder, et al. (2000) J. Exp. Med. 192:1819-1831; Anthony and Lehman (2003) Methods 29:260-269; Badovinac and Harty (2000) J. Immunol. Methods 238:107-117). The "tetramer staining" method is also available (see, e.g., Serbina and Pamer (2003) Curr. Opin. Immunol. 15:436-442; Skinner and Haase (2002) J. Immunol. Methods 268:29-34; Pittet, et al. (2001) Int. Immunopharmacol. 1:1235-1237).

Methods are available for determining if an antigen or epitope is presented via direct presentation or by cross-presentation. These methods include use of TAP-deficient mice with administration of cells (from another source) that contain an antigen of interest. Another method involves preparing a mouse genetically deficient in an MHC Class I or Class II molecule that is required for presenting a specific epitope, e.g., MHC Class I H-2^{b}, and administering H-2^{b}-expressing antigen presenting cells (APCs) (from another source) that contain the antigen of interest (or that were pulsed with an epitope of interest) (see, e.g., van Mierlo, et al. (2004) J. Immunol. 173:6753-6759; Pozzi, et al. (2005) J. Immunol. 175:2071-2081).

Methods for determining binding affinities, binding specificities, and affinity maturation are available. The present disclosure provides methods for stimulating and/or diagnosing affinity maturation, as it applies to, e.g., maturation of antibodies and/or of T cells (see, e.g., Chen, et al. (2004) J. Immunol. 173:5021-5027; Rees, et al. (1999) Proc. Natl. Acad. Sci. USA 96:9781-9786; Busch and Pamer (1999) J. Exp. Med. 189:701-709; Ploss, et al. (2005) J. Immunol. 175:5998-6005; Brams, et al. (1998) J. Immunol. 160:2051-2058; Choi, et al. (2003) J. Immunol. 171:5116-5123).

Methods for using animals in the study of cancer, metastasis, and angiogenesis, and for using animal tumor data for extrapolating human treatments are available (see, e.g., Hirst and Balmain (2004) Eur J Cancer 40:1974-1980; Griswold, et al. (1991) Cancer Metastasis Rev. 10:255-261; Hoffman (1999) Invest. New Drugs 17:343-359; Boone, et al. (1990) Cancer Res. 50:2-9; Moulder, et al. (1988) Int. J. Radiat. Oncol. Biol. Phys. 14:913-927; Tuveson and Jacks (2002) Curr. Opin. Genet. Dev. 12:105-110; Jackson-Grusby (2002) Oncogene 21:5504-5514; Teicher, B.A. (2001) Tumor Models in Cancer Research, Humana Press, Totowa, NJ; Hasan, et al. (2004) Angiogenesis 7:1-16; Radovanovic, et al. (2004) Cancer Treat. Res. 117:97-114; Khanna and Hunter (2004) Carcinogenesis Sept.9 [epub ahead of print]; Crnic and Christofori (2004) Int. J. Dev. Biol. 48:573-581).

Colorectal cancer hepatic metastases can be generated using primary hepatic injection, portal vein injection, or whole spleen injection of tumor cells (see, e.g., Suh, et al. (1999) J. Surgical Oncology 72:218-224; Dent and Finley-Jones (1985) Br. J. Cancer 51:533-541; Young, et al. (1986) J. Natl. Cancer Inst. 76:745-750; Watson, et al. (1991) J. Leukoc. Biol. 49:126-138).

### EXAMPLE II. Vectors for use in mediating site-specific recombination and homologous recombination.

The *Listeria monocytogenes* strains used in the present work are described (see, Brockstedt, et al. (2004) Proc. Natl. Acad. Sci. USA 101:13832-13837). *L. monocytogenes* ΔActAΔinlB (also known as DP-L4029inlB) was deposited with the American Type Culture Collection (ATCC), 10801 University Blvd., Manassas, Virginia 20110-2209, United States of America, on October 3, 2003, under the provisions of the Budapest Treaty on the International Recognition of the Deposit of Microorganisms for the Purposes of Patent Procedure, and designated with accession number PTA-5562. *L. monocytogenes* ΔActAΔuvrAB (also known as DP-L4029uvrAB) was deposited with the American Type Culture Collection (ATCC), 10801 University Blvd., Manassas, Virginia 20110-2209, United States of America, on October 3, 2003, under the provisions of the Budapest Treaty on the International Recognition of the Deposit of Microorganisms for the Purposes of Patent Procedure, and designated with accession number PTA-5563. Yeast medium without glucose contained 25 grams/L yeast extract (Bacto®yeast extract) (BD Biosciences, Sparks, MD); 9 grams/L potassium phosphate monobasic, pH 7.2.

Homologous recombination can be mediated by pKSV7 (SEQ ID NO:3) (see also, Smith and Youngman (1992) Biochimie 74:705-711; Camilli, et al. (1993) Mol. Microbiol. 8:143-157; Camilli (1992) Genetic analysis of Listeria monocytogenes Determinants of Pathogenesis, Univ. of Pennsylvania, Doctoral thesis).

Site-specific integration can be mediated by pPL1, pPL2, pINT, or variants thereof (see, e.g., Lauer, et al. (2002) J. Bacteriol. 184:4177-4186; Int. Appl. No. PCT/US03/13492 (Int. Publ. No. WO 03/092600) of Portnoy, Calendar, and Lauer).

The pINT plasmid has loxP sites that allow the specific removal of most of the plasmid from the listerial chromosome, leaving behind the attP and MCS (multiple cloning site), and the contents of the multi-cloning site (MCS) (e.g., an antigen cassette). pINT can work differently from pPL2 as follows. Up to a 100 microliters aliquot of a 10:1 dilution of a pPL2 conjugation can be plated on double selection plates. Plating up to a 100 microliters aliquot of a 10:1 dilution of a pPL2 conjugation generally results in 50-100 colonies. Plating more than 100 microliters of a 10:1 dilution of pPL2 conjugation gives little or no colonies due to a background growth from the *E. coli* donor. pINT, on the other hand, can be plated without diluting and even concentrating the conjugation mix because erythromycin (Erm) is more selective than chloramphenicol against *E. coli.* The use of pINT broadens the dynamic range for successful integration by approximately 2 logs.

### pINT vector.

### EXAMPLE III. ActA-based fusion protein partners, including ActA derivatives that are truncated or deleted in one or more motifs.

The present invention, in some embodiments, provides reagents and methods comprising a first nucleic acid encoding an ActA-based fusion protein partner operably linked to and in frame with a second nucleic acid encoding at least one heterologous antigen. Provided is a nucleic acid that can hybridize under stringent conditions to any of the disclosed nucleic acids.

What is encompassed is a first nucleic acid and second nucleic acid that are operably linked with each other, and in frame with each other. In this context, "operably linked with each other" means that any construct comprising the first and second nucleic acids encode a fusion protein. In another embodiment, the second nucleic acid can be embedded in the first nucleic acid.

The ActA-based fusion protein partner can comprise one or more of the following. "Consisting" embodiments are also available, and here the ActA-based fusion protein partner can consist of one or more of the following embodiments:
(1) ActA-N100 (amino acids 1-100 of GenBank Acc. No. X59723, or of a similar or homologous ActA sequence).
(2) Full length ActA, where a nucleic acid encoding at least one heterologous antigen is connected to (and in frame with) the C-termmus of full length ActA, residing at an internal position of ActA, or both connected to the C-terminus of the full length ActA and also residing at an internal position of ActA.
(3) A truncated ActA that normally supports less than 90% the activity of nucleating the Arp2/3 complex, as compared with the activity of full length ActA; conventionally supports less than 80% the nucleating activity of full length ActA; characteristically supports less than 70% the nucleating activity of full length ActA; typically supports less than 60% the nuceating activity of full length ActA; more typically supports less than 50% the nucleating activity of full length ActA; most typically supports less than 40% the nucleating activity of full length ActA; often supports less than 30% the nucleating activity of full length ActA; more often supports less than 20% the nucleating activity of full length ActA; most often supports less than 10% the nucleating activity of full length ActA; usually supports less than 5% the nucleating activity of full length ActA; more usually supports less than 2% the nucleating activity of full length ActA; and most usually is undetectable in any ability to nucleate the Arp2/3 complex.
   The reduced, or eliminated, nucleation activity of progressively truncated ActA was demonstrated by Skoble (Skoble, et al. (2000) J. Cell Biol. 150:527-537). It was demonstrated that ActA truncated at amino acid-101, and ActA truncated at amino acid-135, have little or no nucleating activity, while ActA trunated at amino acids 165, 201, and 263, are as potent as full length ActA in nucleating the Arp2/3 complex.
(4) A truncated ActA, wherein the ActA is truncated at about amino acid-40; truncated at about amino acid-45; truncated at about amino acid-50; truncated at about amino acid-55; truncated at about amino acid-60; truncated at about amino acid-65; truncated at about amino acid-70; truncated at about amino acid-75; truncated at about amino acid-80; truncated at about amino acid-85; truncated at about amino acid-90; truncated at about amino acid-95; truncated at about amino acid-100; truncated at about amino acid-105; truncated at about amino acid-110; truncated at about amino acid-115; truncated at about amino acid-120; truncated at about amino acid-125; truncated at about amino acid-130; truncated at about amino acid-135; truncated at about amino acid-140; truncated at about amino acid-145; truncated at about amino acid-150; truncated at about amino acid-150; truncated at about amino acid-155; and truncated at about amino acid-160. The term "about" in this context means plus or minus one amino acid, plus or minus two amino acids, plus or minus three amino acids, plus or minus four amino acids, or plus or minus five amino acids.
(5) ActA secretory sequence (amino acids 1-29 of GenBank Acc. No. X59723, or of a similar or homologous ActA sequence).
(6) Does not comprise an ActA secretory sequence (amino acids 1-29 of GenBank Acc. No. X59723, or of a similar or homologous ActA sequence).
(7) ActA secretory sequence and the mature N-terminal domain (amino acids 1-263 of GenBank Acc. No. X59723, or of a similar or homologous ActA sequence).
(5) Mature N-terminal domain without the secretory sequence (amino acids 30-263 of GenBank Acc. No. X59723, or of a similar or homologous ActA sequence).
(9) ActA sequence with reduced ability to directly stimulate actin polymerization. The reduced ability can be, e.g., normally at most 90% maximal, more normally at most 80% maximal, most normally at most 70% maximal, usually at most 60% maximal, more usually at most 50% maximal, most usually at most 40% maximal, often at most 30% maximal, more often at most 20% maximal, most often at most 10% maximal, and typically at most 5% maximal.
(10) ActA sequence with a reduced ability to bind to a member of the Ena/VASP family of proteins (mammalian Enabled (Mena); Ena/VASP-like protein (Ev1); vasodilator-stimulated phosphoprotein (VASP) (see, e.g., Machner, et al. (2001) J. Biol. Chem. 276:40096-40103). The reduced ability can be, e.g., normally at most 90% maximal, more normally at most 80% maximal, most normally at most 70% maximal, usually at most 60% maximal, more usually at most 50% maximal, most usually at most 40% maximal, often at most 30% maximal, more often at most 20% maximal, most often at most 10% maximal, and typically at most 5% maximal.
(11) ActA that is truncated at the point of, deleted in, or mutated in amino acids 93-98 of GenBank Acc. No. X59723, or of a similar or homologous ActA sequence (LKEKAE (SEQ ID NO:124)) (homologous to actin binding domain of caldesmon (see, e.g., Pistor, et al. (2000) J. Cell Science 113:3277-3287; Lasa, et al. (1997) EMBO J. 16:1531-1540).
(12) ActA that is truncated at the point of, deleted in, or mutated in, amino acids 126-155 (PAIQ, etc.) of GenBank Acc. No. X59723, or of a similar or homologous ActA sequence, that are critical for ActA dimer formation (see, e.g., Mourrain, et al. (1997) Proc. Natl. Acad. Sci. USA 94:10034-10039).
(13) ActA that is truncated at the point of, deleted in, or mutated in, amino acids 121-170 of GenBank Acc. No. X59723, or of a similar or homologous ActA sequence (minimal ARP2/3 activating domain) (see, e.g., Zalevsky, et al. (2001) J. Biol. Chem. 276:3468-3475).
(14) ActA that is truncated at the point of, deleted in, or mutated in, amino acids 146-150 KKRRK (SEQ ID NO:30)) of GenBank Acc. No. X59723, or of a similar or homologous ActA sequence (a region essential for recruiting Arp2/3 complex) (Lasa, et al. (1997) EMBO J. 16:1531-1540; Pistor, et al. (2000) J. Cell Science 113:3277-3287).
(15) ActA that is truncated at the point of, deleted in, or mutated in, amino acids 41-46 DEWEEE (SEQ ID NO:31) of GenBank Acc. No. X59723, or of a similar or homologous ActA sequence (a region involved in Arp2/3 complex binding) (see, e.g., Boujemaa-Paterski, et al. (2001) Biochemisty 40: 11390-11404).
(16) ActA that is truncated at the point of, deleted in, or mutated in, amino acids 481-492 (DRLADLRDRGTG (SEQ ID NO:32)), which is a vinculin homology region. Vinculin mediates cell-to-cell spread of *S. flexneri* (see, e.g., Kocks, et al. (1992) Cell 68:521-531).
(17) ActA that is truncated at the point of, deleted in, or mutated in, the cofilin homology domain (IKKKRRKAIASSD (SEQ ID NO:33)) (amino acids 145-156 of GenBank Acc. No. X59723, or of a similar or homologous ActA sequence) (see, e.g., Skoble, et al. (2000) J. Cell Biol. 150:527-537).
(18) ActA that is truncated at the point of, deleted in, or mutated in, amino acids 50-125 of GenBank Acc. No. X59723, or of a similar or homologous ActA sequence (continuity of filament elongation region) (see, e.g., Lasa, et al. (1997) EMBO J. 16:1531-1540).
(16) ActA that is truncated at the point of, deleted in, or mutated in, the first FP₄ motif (amino acids 265-269, or 264-269, and the like), second FP₄ motif (amino acids 300-304, or 299-304, and the like), third FP₄ motif (amino acids 335-339, or 334-339, and the like), fourth FP₄ motif (amino acids 380-384, or 379-384, and the like), all four FP₄ motifs, or any combination of the above, where the amino acids refer to GenBank Acc. No. X59723, or a similar or homologous ActA sequence (see, e.g., Machner, et al. (2001) J. Biol. Chem. 276:40096-40103). The FP₄ motifs enhance actin polymerization and bacterial motility by recruiting focal contact proteins (e.g., VASP and Mena) and profilin, which promote elongation of filaments nucleated by interactions between motifs at the N-terminal region of ActA and Arp2/3 complex (see, e.g., Welch, et al. (1998) Science 281:105-108; Skoble, et al. (2000) J. Cell Biol. 150:527-537); Pistor, et al. (2000) J. Cell Science 113:3277-3287).
(17) ActA that is truncated at the point of, deleted in, or mutated in, amino acids 136-165 of GenBank Acc. No. X59723, or of a similar or homologous ActA sequence (cofilin homology region, a region that stimulates Arp2/3 complex) (see, e.g., Lauer, et al. (2001) Mol. Microbiol. 42:1163-1177).
(18) ActA that is truncated at the point of, deleted in, or mutated in, the "acidic stretch," that is, amino acids 31-58 (TDSED (SEQ ID NO:34), etc.) of GenBank Acc. No. X59723, or of a similar or homologous ActA sequence. The acidic stretch contributes to actin polymerization, movement of *Listeria* in the host cell cytoplasm, cell to cell spreading, and to plaque size (see, e.g., Skoble, et al. (2000) J. Cell Biol. 150:527-537; Lauer, et al. (2001) Mol. Microbiol. 42:1163-1177).
(19) ActA that is truncated at the point of, deleted in, or mutated in, amino acids 60-101 (AB region, an actin binding domain) of GenBank Acc. No. X59723, or of a similar or homologous ActA sequence (see, e.g., Lauer, et al. (2001) Mol. Microbiol. 42:1163-1177).
(20) ActA that is truncated at the point of, deleted in, or containing the mutation of mutant 34 (no movement; no plaque) amino acids 117-121 (KKRRK (SEQ ID NO:30)) of GenBank Acc. No. X59723, or of a similar or homologous ActA sequence (Lauer, et al. (2001) Mol. Microbiol. 42:1163-1177.
(21) ActA that is truncated at the point of, deleted in, or containing the mutation of mutant 34 (no movement; no plaque) amino acids 244-249 (DKSAGLID (SEQ ID NO:123)) of GenBank Acc. No. X59723, or of a similar or homologous ActA sequence. The mutation can be, e.g., replacement of the D, K, and D by alanines (Lauer, et al. (2001) Mol. Microbiol. 42:1163-1177).
(22) ActA that is truncated at the point of, deleted in, or containing the mutation of mutants 39, 47-52, 54 and/or 48 (reduced movement) (Lauer, et al. (2001) Mol. Microbiol. 42:1163-1177).
(23) ActA that is truncated at the point of, deleted in, or mutated in, amino acids 264-390 (central repeat region) of GenBank Acc. No. X59723, or of a similar or homologous ActA sequence (see, e.g., Lauer, et al. (2001) Mol. Microbiol. 42:1163-1177; Skoble, et al. (2000) J. Cell Biol. 150:527-537; Skoble, et al. (2001) J. Cell Biol. 155:89-100).

The present disclosure provides an ActA-based fusion protein partner that can comprise any one, or any combination of, the above-disclosed embodiments. "Consisting" embodiments are also available, and here the ActA-based fusion protein partner can consist of one or more of the above-disclosed embodiments.

When provided with the present disclosure, the skilled artisan can envision and prepare embodiments containing conservative modifications, or modifications where one or more amino acids is deleted, or where one or more amino acids is replaced with alanine, and the like.

In the present context, "fusion protein partner" encompasses, but is not limited to, a nucleic acid encoding a polypeptide, or the polypeptide itself, that occurs as a fusion protein with a heterologous antigen, where the fusion protein partner enhances, e.g., transcription, translation, stability, processing by an antigen presenting cell (APC), presentation by an APC, immune presentation, cytotoxic T cell response, CD8⁺ T cell response, CD4⁺ T cell response, reduction in tumor size, number, or metastasis, increase in survival to a tumor or infective agent, and the like.

The present disclosure provides nucleic acids and polypeptides of ActA-N100, and fusion proteins thereof, including fusion proteins that comprise at least one antigen. Without implying any limitation on the invention, the at least one antigen can comprise mesothelin, H-ras, a mesothelin derivative, a H-ras derivative, or any combination thereof. The nucleic acid encoding at least one antigen can be operably linked to, and in frame with, the N-terminus of an ActA-based fusion protein partner. Alternatively, the nucleic acid encoding the at least one antigen can be operably linked to, and in frame with, the C-terminus of the ActA fusion protein partner. Or the nucleic acid encoding the at least one antigen can be operably linked with, and reside within a nucleic acid encoding an ActA-based fusion protein partner.

### EXAMPLE IV. Building blocks used for assembling nucleic acids encoding ActA fusion proteins.

The following discloses nucleic acids and polypeptides used for making constructs that contain ActA-NlOO as a fusion protein partner. Sequences codon optimized for expression in *L. monocytogenes,* and non-codon optimized sequences, are identified.

| | |
|---|---|
| Nucleic acid encoding ActA-N100 native sequence (not codon optimized), including Shine-Dalgarno sequence. (SEQ ID NO:122) | |
| ActA promoter *L. monocytogenes* 10403S. (SEQ ID NO:35) | |
| Native actA promoter together with the 5' UTR (including the Shine Delgarno sequence), and GUG translation site. (SEQ ID NO: 157) | |
| | |
| Underlined and lowercase is a KpnI site added for cloning. | |
| | |
| PrfA box is in italics and bold. | |
| +1 for transcription is shown as "T" | |
| | |
| Shine-Dalgarno sequence near end is underlined. | |
| | |
| Lowercase "gtg" at end is start site for ActA in *Listeria.* | |
| ActA-N100 native sequence (not codon optimized), including Shine-Dalgarno sequence, with human mesothelin (codon optimized) with SS deleted and GPI deleted. The *BamHI* (GGATCC) and *SacI* (GAGCTC) sites are shown in **BOLD**. (SEQ ID NO:36) | |
| Nucleic acid encoding full-length ActA *L. monocytogenes* 10403S. | |
| (SEQ ID NO:37) | |
| ActA polypeptide from *L. monocytogenes* 10403S. (SEQ ID NO:38) (Predicted amino acid sequence if not at N-terminus when expressed in *Listeria.*) | |
| ActA polypeptide from *L. monocytogenes* 10403S. (SEQ ID NO:152) | |
| (Actual amino acid sequence when expressed in *Listeria.* Although the coding sequence contains a valine codon at the N-terminus, the *Listeria* actually biosynthesizes a polypeptide starting with methionine, not valine.) | |
| Nucleic acid encoding ActA-N100 fragment used in our constructs, including promoter and restriction enzyme sites (*KpnI* site and *BamHI* site underlined, promoter sequence lowercase, N100 ORF sequence in UPPERCASE). (SEQ ID NO:39) | |
| Amino acid sequence of ActA-N100. (SEQ ID NO:40) (Predicted amino acid sequence if not at N-terminus when expressed in *Listeria*.) | |
| Amino acid sequence of ActA-N100. (SEQ ID NO: 153) Actual amino acid sequence when expressed in *Listeria.* The nucleic acid encoding ActA-N100 contains a valine codon at the N-terminus, but the *Listeria* actually biosynthesizes a polypeptide starting with methionine, not valine.) | |
| Amino acid sequence of fusion protein of ActA-N100 with human mesothelin (ss deleted; GPI deleted). The *BamHI* site adds two amino acids (GS). (SEQ ID NO:41) | |
| Amino acid sequence of fusion protein of ActA-N100 with human mesothelin (ss deleted; GPI deleted). (SEQ ID NO: 154) (Actual amino acid sequence when expressed in *Listeria.* The nucleic acid encoding ActA-N100, or a fusion protein thereof, contains a valine codon at the N-terminus, but the *Listeria* actually biosynthesizes a polypeptide starting with methionine, not valine.) The *BamHI* site adds two amino acids | |
| Nucleic acid sequence of 12ras. (SEQ ID NO:42) | |
| Amino acid sequence of 12ras. (SEQ ID NO:43) | AMTEYKLVVVGADGVGKSALTIQLIQ |
| Nucleic acid of fusion protein of ActA-N100 with codon optimized human mesothelin (deleted SS; deleted GPI) and 12ras. 12ras is fused to the 3'-end of mesothelin (deleted in SS; deleted in GPI). The mesothelin-ras fusion construct is codon optimized and cloned (as a *BamHI-SacI* fragment) downstream of the ActA-N100-fusion protein partner. The **BOLD** nucleotides indicate restriction sites. *BamHI* is GGATCC. *SacI* is GAGCTG. (SEQ ID NO:44) | |
| | |
| Fusion protein of ActA-N100 with human mesothelin (deleted SS; deleted GPI) and 12ras. The *BamHI* site adds two amino acids (GS). (SEQ ID NO:45) | |
| Fusion protein of ActA-N 100 with human mesothelin (deleted SS; deleted GPI) and 12ras. (SEQ ID NO: 155) (Actual amino acid sequence when expressed in *Listeria.* The nucleic acid encoding ActA-N100, or a fusion protein thereof, contains a valine codon at the N-terminus, but the *Listeria* actually biosynthesizes a polypeptide starting with methionine, not valine.) The *BamHI* site adds two amino acids (GS). | |
| **ActA promoter and ActA-N100: N100 coding sequence is native. Tumor antigens are inserted at the BamHI site (GGATCC). (SEQ ID NO:46)** | |
| Amino acid sequence of ActAN100: the BamHI site adds two amino acids (GS). (SEQ ID NO:47) (Predicted amino acid sequence if not at N-terminus when expressed in *Listeria*.) | |
| Amino acid sequence of ActAN100: the BamHI site adds two amino acids (GS). (SEQ ID NO:156) (Actual amino acid sequence when expressed in *Listeria,* since the *Listeria* actually biosynthesizes a polypeptide starting with methionine, not valine.) | |
| Nucleic acid sequence of fusion protein of ActA-N100 with human mesothelin (codon optimized, deleted SS). The *BamHI* site adds two amino acids (GS). (SEQ ID NO: 158) | |
| | |
| Amino acid sequence of fusion protein of ActA-N100 with human mesothelin (codon optimized, deleted SS). The *BamHI* site adds two amino acids (GS). (SEQ ID NO: 159) (Actual amino acid sequence when expressed in *Listeria,* since the *Listeria* actually biosynthesizes a polypeptide starting with methionine, not valine.) | |

### EXAMPLE V. Building blocks used for assembling listeriolysin (LLO; hly gene) fusion proteins.

| | |
|---|---|
| Nucleic acid of LLO open reading frame (ORF) from wild type *Listeria* 10403S. (SEQ ID NO:48) | |
| Codon optimized LLO (GGATCC is a BamHI site added at the 3' end for in-frame fusions). (SEQ ID NO:49) | |
| | |
| One mutant variation on codon optimized LLO (as a translational fusion - GGATCC is a BamHI site added at the 3' end for in-frame fusions; mutant variation is in CAPS, changes TGGTGG to TTTTTT amino acid changes WW to FF). (SEQ ID NO:50) | |
| | |
| Nucleic acid of LLO59 (not codon optimized). (SEQ ID NO:51) | |
| Nucleic acid of LLO59, codon optimized for expression in *Listeria.* (SEQ ID NO:52) | |
| Amino acids of LLO59. (SEQ ID NO:53) | |
| Nucleic acid of LLO59, codon optimized for expression in *Listeria*, with codon optimized human mesothelin (deleted SS; deleted GPI), cloned in frame with LLO as a BamHI/SacI fragment. The BamHI (GGATCC) and SacI (GAGCTC) sites are indicated in **BOLD**. | |
| This construct can be called: LLOopt59-hMesothelin(d eleted SS; deleted gpi) fusion. (SEQ ID NO:54) | |
| Amino acids of fusion protein of LLO59, codon optimized, with codon optimized human mesothelin (deleted SS; deleted GPI). (SEQ ID NO:55) | |
| | |
| Nucleic acid of fusion protein of LLO59 (not codon optimized) with human mesothelin (codon optimized) with deleted SS and deleted GPI, as BamHI-SacI fragment. The *BamHI* site (GGATCC) and the *SacI* site (GAGCTC) are shown in **BOLD.** | |
| This sequence can be called: LLOnat59 hMesothelin (deleted SS; deleted gpi) fusion. "nat" means natural, not codon optimized. Regarding the amino acid sequences, the amino acid encoded by this sequence is the same as that encoded by the corresponding sequence where mesothelin is codon optimized. (SEQ ID NO:56) | |
| *hly* promoter. (SEQ ID NO:57) | |
| Nucleic acid for codon-optimized BaPA signal peptide. (SEQ ID NO:58) | |
| Amino acids of BaPA signal peptide. (SEQ ID NO:59) | MKKRKVLIPLMALSTILVSSTGNLEVIQAEVGS |
| The *hly* promoter and BaPA signal peptide are fused seamlessly together. The *hly* promoter and BaPA signal peptide are fused seamlessly together (no restriction sites) and the promoter-signal peptide assembly is inserted into plasmids as a *KpnI* (GGTACC)-*BamHI* (GGATCC) fragment. The tumor antigen is inserted at the *BamHI* site. (SEQ ID NO:60) | |

### EXAMPLE VI. Building blocks used for assembling p60 fusion proteins and fusion proteins other polypeptides that mediate SecA2-dependent secretion.

The present disclosure provides a polynucleotide comprising a first nucleic acid encoding a protein secreted by a SecA2-dependent pathway and a second nucleic acid encoding a heterologous antigen. Autolysins, such as p60 and NamA (N-acetyl-muramidase), are proteins secreted from *Listeria* by the SecA2-dependent pathway (Lenz, et al. (2003) Proc. Natl. Acad. Sci. USA 100:12432-12437). In one embodiment, the fusion protein partner (e.g., p60 or NamA) retains its enzymatic or structural activity. In another embodiment, the fusion protein partner lacks its enzymatic or structural activity. Yet another embodiment places or insertes a nucleic acid encoding a heterologous protein between the signal sequence (SS) and nucleic acids encoding the cell wall binding domains (LysSM) and catalytic domains Lyz-2 (NamA) and p60-dom (p60).

The following discloses, as a non-limiting example, nucleic acids encoding fusion proteins comprising p60 and human mesothelin (hMeso). Mesothelin was inserted into *Listeria*'s p60 protein as follows. A nucleic acid encoding mesothelin was inserted into a nucleic acid encoding p60, so that when expressed, mesothelin would be inserted into p60 at amino acid 70. A polynucleotide encoding the resulting fusion protein was prepared for use in expression by a *Listeria* bacterium.

In another embodiment, protein chimera contained optimal codons for expression in *Listeria* in the p60 amino acids 1-70 as well as in the entire mesothelin coding sequence. In yet another embodiment, the p60-human mesothelin protein chimera was functionally linked to the *L. monocytogenes* hly promoter, into the pPL2 vector, which was used subsequently to generate recombinant *L. monocytogenes* strains expressing and secreting human mesothelin.

The sequence of the first 70 amino acids of p60 from *L. monocytogenes,* strain 10403 S is disclosed.
M N M K K A T I A A T A G I A V T A F A A P T I A S A S T V V V E A G D T L W G I A Q S K G T T V D A I K K A N N L T T D K I V P G Q K L Q (SEQ ID NO:61)

The synthesized DNA sequence corresponding to the hly promoter-70 N-terminal p60 amino acids is shown below. The codons encoding p60 amino acid residues 69 (L) and 70 (Q), were modified to contain a unique *Pst I* enzyme recognition sequence, to facilitate functional insertion of a heterologous sequence (e.g., a nucleic acid encoding mesothelin). Moreover, the 5' end of the synthesized sub-fragment contains a unique *KpnI* enzyme recognition sequence.

At this point in the commentary on vector synthesis, the nucleic acid sequence corresponds to the following:

### hly promoter-p60 (70 N-terminal amino acids of p60).

The unique PstI site (CTGCAG) is visible at the 3'-end.

The 447 bp *KpnI* and *PstI* digested sub-fragment is ligated into the corresponding *KpnI* and *PstI* sites of the pPL2 vector, treated by digestion with *KpnI* and *PstI* enzymes and digestion with calf intestinal alkaline phosphatase (CIAP). This plasmid is known as pPL2-hlyP-Np60 CodOp. Subsequently, the remainder of the native p60 gene was cloned into the pPL2-hlyP-Np60 CodOp plasmid, between the unique *Pst I* and *BamHI* sites. The remainder of the p60 gene was cloned by PCR, using a proof-reading containing thermostable polymerase, and the following primer pair:
Forward primer:
   5'-CGCCTGCAGGTAAATAATGAGGTTGCTG (SEQ ID NO:63)
Reverse primer:
   5'-CGCGGATCCTTAATTATACGCGACCGAAG (SEQ ID NO:64)

The 1241 bp amplicon is digested with *PstI* and *BamHI,* and the purified 1235 bp is ligated into the pPL2-hlyP-Np60 CodOp plasmid, digested with *PstI* and *BamHI,* and treated with CIAP. The resulting plasmid contains the full p60 gene with optimal codons corresponding to amino acids 1-77, and native codons corresponding to amino acids 78-478. The full p60 gene is linked functional to the *L. monocytogenes hly* promoter.

At this point in the commentary on vector synthesis, the nucleic acid sequence corresponds to the following:

### hly promoter-p60-[70 N-terminal amino acids 1-77 of p60 (codon optimized)]-[PstI]-[C-terminal amino acids 78-478 of p60 (non-codon optimized)].

At this point, the construct has not yet received a nucleic acid encoding a heterologous antigen. In commentary to follow, the unique *PstI site* will receive a nucleic acid encoding a heterologous antigen (mesothelin). This plasmid, which contains full length p60, but with the N-terminal region codon optimized, and the C-terminal region non-codon optimized, is known as: pPL2-hlyP-Np60 CodOp (1-77). The sequence of the *KpnI-BamHI* sub-fragment that contains the hlyP linked functionally to the p60 encoding sequence is shown below (SEQ ID NO:65). The expected sequence of the pPL2-hlyP-Np60 CodOp(1-77) plasmid was confirmed by sequencing.

The next step in the construction is the functional insertion of a heterologous protein encoding sequence at the unique *PstI* site of plasmid as pPL2-hlyP-Np60 CodOp(1-77).

A nucleic acid encoding human mesothelin that was codon-optimized for optimal expression in *L. monocytogenes* was inserted into the unique *PstI* site of plasmid as pPL2-hlyP-Np60 CodOp (1-77). Specifically, full-length mesothelin, or mesothelin that was deleted of the signal peptide and GPI linker domains (mesothelin ΔSP/ΔGPI) was cloned from a plasmid that contains the full-length human mesothelin, containing optimal codons for expression in *L. monocytogenes,* using a thermostable polymerase with proof-reading activity, and the primer pair shown below. The present invention provides for other nucleic acids encoding antigens other than mesothelin or in addition to mesothelin, for use in the present protocol. Moreover, the present invention provides for codon-optimization of nucleic acids encoding an antigen, codon-optimization of nucleic acids encoding a fusion protein partner, and/or codon-optimization of nucleic acids encoding a fusion protein partner.

The skilled artisan will understand that expressions that recite "an antigen was inserted into a polypeptide," or expressions to that effect, can encompass "a first nucleic acid encoding an antigen was inserted into a second nucleic acid encoding a polypeptide," and the like.

PCR Primers used to amplify full length human mesothelin:
Forward Primer (huMeso 3F):
   5'-AAACTGCAGGCATTGCCAACTGCACGTCC (SEQ ID NO:66)
Reverse Primer (hMeso 1935R):
   5'-AAACTGCAGAGCTAATGTACTGGCTAATAATAATGCTAAC (SEQ ID NO:67)
PCR primers used to amplify human mesothelin (ΔSSΔGPI anchor).
Forward Primer (huMeso 133F):
   5'- CGCCTGCAGCGTACATTAGCAGGTGAAACAGG (SEQ ID NO:68)
Reverse Primer (huMeso 1770R):
   5'-CGCCTGCAGGCCTTGTAAACCTAAACCTAATGTATC (SEQ ID NO:69)

In viewing the following embodiments of mesothelin, the skilled artisan will recognize that the disclosed nucleic acids and polypeptides of mesothelin can be inserted or used in into a variety of polypeptide constructs including fusion proteins, nucleic acids encoding fusion proteins and the like, multicistronic constructs, plasmids, vectors, fusion proteins, bacterial vaccines, and the like.

| | |
|---|---|
| Nucleic acid of the signal peptide of human mesothelin. (SEQ ID NO:70) | |
| Nucleic acid of the GPI anchor of human mesothelin. (SEQ ID NO:71) | |
| Human mesothelin nucleic acid cassette, codon optimized for expression in *Listeria,* with 5'-*BamHI* (GGATCC) and 3'-*SacI* (GAGCTC) cloning sites. As this is full length mesothelin, it contains the C-terminal gpi anchor domain. (SEQ ID NO:72) | |
| Amino acids of full length human mesothelin. (SEQ ID NO:73) (If sequence is fused at the N-terminus to a heterologous sequence, the initial methionine is optionally deleted (by deletion of the corresponding codon).) | |

| | |
|---|---|
| | |
| Human mesothelin nucleic acid (codon ' optimized), deleted SS, deleted GPI anchor. This is a cassette encoding human mesothelin, where the cassette contains the restriction sites 5'*-BamHI* and 3*'*-*SacI.* (SEQ ID NO:74) | |
| Human mesothelin amino acid, deleted SS, deleted GPI anchor. (SEQ ID NO:75) | |

| | |
|---|---|
| | |

The PCR amplicons of 1932 bps (full-length mesothelin) and 1637 bps (mesothelin ΔSP/ΔGPI) were purified, digested with *PstI,* purified, and ligated into the unique *PstI* site of plasmid pPL2-htyP-Np60 CodOp(1-77), treated by digestion with *PstI,* and digestion with CIAP. The consistent amino terminus to carboxy terminus orientation of the p60 and Mesothelin domains was confirmed by restriction endonuclease mapping. These plasmids are known as pPL2-hlyP-Np60 CodOp(1-77)-mesothelin and pPL2-blyP-Np60 CodOp(1-77)-mesothelin ΔSP/ΔGPI, and were introduced into selected *L. monocytogenes* strains.

The sequence of the *KpnI-BamHI* sub-fragment of plasmid pPL2-hlyP-Np60 CodOp(1-77)-mesothelin containing the *hly* promoter linked functionally to the p60-human mesothelin protein chimera encoding gene has the sequence shown below.

The sequence of the *KpnI-BamHI* sub-fragment of plasmid pPL2-hlyP-Np60 CodOp(1-77)-mesothelin ΔSS/ΔGPI containing the *hly* promoter linked functionally to the p60-human mesothelin ΔSS/ΔGPI protein chimera encoding gene has the sequence shown below.

### EXAMPLE VII. ActA-N 100-based fusion proteins; LLO-based fusion proteins (synthesis; vaccination; immunogenicity).

Table 11 discloses some of the bacterial strains that were prepared. The bacteria were used for vaccination into tumor-bearing mice. Where indicated, vaccination resulted in anti-tumor immune responses, reduction in tumor number and size, and increased survival.

| Table 11. Recombinant *L. monocytogenes* bacteria of the present invention. "Delta" means deleted. The E30R mutation and the E30M mutation, where indicated, occur in the Bacillus Protective Antigen (BaPA) secretory sequence. The S28D mutation and S28R mutation, where indicated, occur in p60. | | | | | |
|---|---|---|---|---|---|
| Strain (trivial name) | Construct | Genetic background | Locus of integration | Promoter | Secretory sequence (SS) |
| -- | Full length (FL) hMesothelin | ΔActA ΔinlB | tRNA Arg | Hly | BaPA |
| hMeso1 | hMeso [deltaSS deltaGPI] | ΔActA ΔinlB | tRNA Arg | Hly | BaPA |

| Strain (trivial name) | Construct | Genetic background | Locus of integration | Promoter | Secretory sequence (SS) |
|---|---|---|---|---|---|
| hMeso2 | HMeso[deltaSS deltaGPI] | ΔActA ΔinlB prfA* | tRNA Arg | Hly | BaPA |
| hMeso3 | hMeso [deltaSS deltaGPI] | ΔActA ΔinlB | ActA | ActA | BaPA |
| hMeso4 | HMeso [deltaSS deltaGPI] | ΔActA ΔinlB | inlB | Hly | BaPA |
| hMeso5 | p60-hMeso [deltaSS deltaGPI] | ΔActA ΔinlB | tRNA Arg | Hly | p60 |
| hMeso6 | ActA-N 100 hMeso [deltaSS deltaGPI] | ΔActA ΔinlB | ActA | act | ActA |
| hMeso8 | hMeso [deltaSS deltaGPI]-rasG12D | ΔActA ΔinlB | tRNA Arg | hly | BaPA |
| hMeso 10 | ActA-N100 hMeso [deltaSS deltaGPI]-rasG 12D | ΔActA ΔinlB | ActA | ActA | ActA |
| hMeso 11 | HMeso [deltaSS deltaGPI]-rasG12D | ΔActA ΔinlB | inlB | Hly | BaPA |
| hMeso12 | hMeso [deltaSS deltaGPI]-rasG12D | ΔActA ΔinlB | tRNA Arg | Hly | BaPA (E30R) |
| hMeso 13 | hMeso [deltaSS deltaGPI]-rasG12D | ΔActA ΔinlB | tRNA Arg | hly | BaPA (E30M) |
| hMeso 14 | LLO62-hMeso [deltaSS deltaGPI]-rasG12D | ΔActA ΔinlB | tRNAArg | hly | LLO(62) |
| hMeso 15 | LLOopt62 hMeso [deltaSS deltaGPI]-rasG 12D | ΔActA ΔinlB | tRNA Arg | Hly | LLO(opt62) |
| hMeso18 | A30R ActA-N100-hMeso [deltaSS deltaGPI]-12ras (the ras has a G12D mutation) | ΔActA ΔinlB | ActA | ActA | ActA (A30R) |
| hMeso 19 | S28D p60hMeso [deltaSS deltaGPI] | ΔActA ΔinlB | tRNA Arg | hly | p60 |
| hMeso20 | S28R deltap60hMeso [deltaSS deltaGPI] | ΔActA ΔinlB | tRNA Arg | hly | p60 |
| hMeso22 | LLO441-hMeso [deltaSS deltaGPI]-rasG12D | ΔActA ΔinlB | tRNA Arg | hly | LLO |
| hMeso26 | ActA-N 100 hMeso [deltaSS deltaGPI] | ΔActA ΔinlB | inlB | ActA | ActA |
| hMeso31 | ActA-N100 (A30R in ActA-N100)-hMeso [deltaSS deltaGPI] diploid | ΔActA ΔinlB | ActA and inlB | ActA and ActA | ActA and ActA |
| hMeso32 | ActA-N100-hMeso [deltaSS deltaGPI] diploid | ΔActA ΔinlB | inlB and TRNA^{Arg} | ActA and ActA | ActA and ActA |
| hMeso33 | ActA-N100 deltaSS (containing GPI) | ΔActA ΔinlB | tRNA^{Arg} integrated with pINT | ActA | ActA |
| hMeso37 | ActA-N100 [deltaSS] (containing GPI) | ΔActA ΔinlB | tRNA^{Arg} integrated with pINT | ActA | ActA |
| hMeso37 differs from hMeso33 in that hMeso37 was treated with a plasmid encoding Cre recombinase to effect removal of loxP-flanked DNA. Cre recombinase was provided via the plasmid pCON2. pCON2 is temperature sensitive. Shifting temperature results in removal of loxP-flanked DNA and results in loss of pCON2 from the cell. pCON is described (see, e.g., Behari, et al. (1998) J. Bacteriol. 180:6316-6324; Milenbachs, et al. (2004) Microbiology 150:321-333). | | | | | |
| hMeso38 | ActA-N100-hmeso [deltaSS] (not deleted in GPI). (hmeso33allele) | ΔActA ΔinlB | inlB | ActA | ActA |
| hMeso40 (see Table 12) | hMeso26 with this additional integration: pINT-ActA-N100-db12ras3 | ΔActA ΔinlB | inlB and tRNA^{Arg} | ActA and ActA | ActA and ActA |
| hMeso41 (see Table 12) | hmeso26 with this additional integration: pINT-ActA-N100-db1-12ra s4 | ΔActA ΔinlB | inlB and tRNA^{Arg} | ActA and ActA | ActA and ActA |
| hMeso42 (see Table 12) | hMeso26 with this additional integration: pINT-ActA-N100-db1-12ra s5 | ΔActA ΔinlB | inlB and tRNA^{Arg} | ActA and ActA | ActA and ActA |
| hMeso43 (see Table 12) | hMeso26 with this additional integration: pINT-ActA-N100-db1-12ras6 | ΔActA ΔinlB | inlB and tRNA^{Arg} | ActA and ActA | ActA and ActA |
| Where a polynucleotide is integrated at the ActA locus, the ActA gene is deleted during homologous recombination, unless otherwise specified. Where a polynucleotide is integrated at the ActA locus, and where the construct comprises a fusion protein that includes ActA-N100, and where the secretory sequence is listed as the ActA secretory sequence, the ActA secretory sequence comes from the ActA-N100 fusion protein partner (not from the genomic ActA gene, for the reason that the genomic ActA gene was deleted during homologous recombination), as in hMeso6, hMeso10, and hMeso1 8. | | | | | |

**Table 12. Sequences in expression cassettes of hMeso40, hMeso41, hMeso42, and hMeso43. "ActA-N100" indicates that the ActA-N100 sequence immediately precedes the indicated amino acids that follow.**

| | |
|---|---|
| Db112ras3 sequence of hMeso40 | |
| Db112ras4 sequence of hMeso41 | |
| Db112ras5 sequence of hMeso42 | |
| Db1 12ras6 sequence of hMeso43 | |

| Identification of details within above sequences | |
|---|---|
| rasG12D (a.k.a. 12rasD) | MTEYKLVVVGA**D**GVGKSALTIQLIQ (SEQ ID NO:81) |
| rasG12V (a.k.a. 12rasV) | MTEYKLVVVGA**V**GDGKSALTIQLIQ (SEQ ID NO:82) |
| Meso secretory sequence (MesoSS) | ALPTARPLLGSCGTPALGSLLFLLFSLGWVQPSRTLAGETGQEAA (SEQ ID NO:83) |
| MesoA2 epitope occurring within MesoSS | TPALG**SLLFLLFSL**GWVQP (SEQ ID NO:84) |
| Spacer | EEDEEE (SEQ ID NO:85) |

The following *Listeria* ΔActA ΔinlB constructs, suitable as control constructs, were found not to detectably express: (1) hMeso deltaSS deltaGPI ras (the ras had a G12D mutation). This construct had an ActA promoter, BaPA signal sequence, with an ActA locus of integration; (2) A30R ActA-N100 hMesoΔSSΔGPI ras (the ras had a G12D mutation). This construct had an ActA promoter, BaPA signal sequence, with an ActA locus of integration; and (3) A30L ActA-N100 hMeso ΔSSΔGPI ras (the ras had a G12D mutation). This particular construct had an ActA promoter, ActA signal sequence, with an ActA locus of integration.

Promoters of the present invention can include one or more of the following operably linked with a nucleic acid encoding an antigen: hly, ActA, p60, pHyper, and so on. pHyper is disclosed (see, e.g., U.S. Pat. Appl. 2005/0147621 of Higgins, et al.). The present invention provides signal sequences, such as one or more of the signal sequence of LLO, ActA, BaPA, BsPhoD, p60, and so on. Fusion protein partners of the present disclosure can include one or more of LLO₁₋₆₂, LLO₁₋₄₄₁, ActA-N100, p60, PFO₁₋₃₉₀, BaPA₁₋₈₂, and the like.

Constructs containing an ActA-based fusion protein partner or an LLO-based fusion protein partner were synthesized as follows. When synthesis was complete, the construct was integrated into the genome of *L. monocytogenes.* While integration was mediated by vectors such as pKSV7, pPL2, and pINT, the present disclosure is not limited to any particular integration vector or mechanism of integration. Various polynucleotides were assembled in a modular fashion, that is, by ligating prefabricated nucleic acids together on the pKSV7 scaffold. These prefabricated nucleic acids were as follows:

The ActA promoter/ActA-N100/human Mesothelin ΔSSΔGPI construct was assembled using the following components: A first polynucleotide consisting of a first nucleic acid encoding native listerial ActA promoter sequence (including the Shine Dalgarno site) connected directly to a second nucleic acid encoding ActA-N100 (the first polynucleotide had a 5'-*HindIII* site and a 3'-*BamHI* site.) A second polynucleotide consisted of human Mesothelin ΔSSΔGPI (5'-*BamHI* site and 3'-SacI site). The pKSV7 received an insert consisting of the first polynucleotide connected directly to the second polynucleotide. In a variation of this construct, the second polynucleotide consisted of a first nucleic acid encoding human Mesothelin ΔssΔGPI connected directly to a second nucleic acid encoding 12ras (5'-*BamHI* site and 3'-*SacI* site). Human mesothelin is intended as a non-limiting example.

The hly promoter/LLO62/human Mesothelin ΔSSΔGPI/12ras construct was assembled using the following components. LLO62 means a nucleic acid encoding amino acids 1-62 of listeriolysin (LLO). A first polynucleotide was prepared that consisted of a first nucleic acid encoding native listerial hly promoter sequence (including the Shine Dalgarno site) connected directly to a second nucleic acid encoding LLO62 (the first polynucleotide had a 5'-*KpnI* site and a 3'-*BamHI* site). A second polynucleotide was prepared that consisted of a first nucleic acid encoding human Mesothelin ΔSSΔGPI connected directly to a second nucleic acid encoding 12ras (the second polynucleotide had a 5'-*BamHI* site and a 3'-*SacI* site.) The pKSV7 received an insert consisting of the first polynucleotide connected directly to the second polynucleotide. A variation of this construct used LLO60 (codon optimized) in place of LLO62.

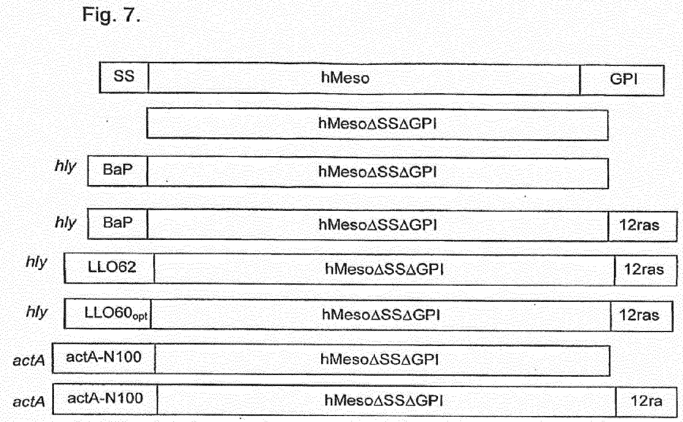

Figure 7 discloses a number of embodiments of the present invention, including LLO-based fusion proteins and actA-N100-based fusion proteins.

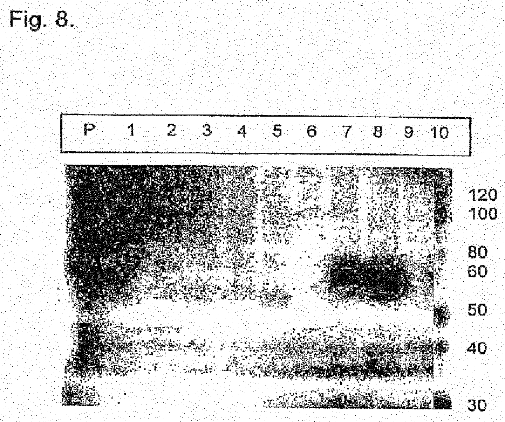

Figure 8 discloses expression of various constructs from cell cultures of engineered *Listeria.* In this context, expression means protein biosynthesis and secretion into the medium, where the indicated construct had been integrated into the listerial genome. Expression was conducted in a medium containing yeast extract without glucose at a bacterial density corresponding to OD₆₀₀ = 0.8. The term pPL2 indicates that the construct was inserted by way of site-specific recombination using the vector pPL2, pKSV7 means that the construct was inserted by homologous recombination using the vector pKSV7 (see Table 13).

The antibody for detecting mesothelin expression was a rabbit polyclonal antibody, produced by immunizing rabbit with three peptides from human mesothelin, where the antibody was purified by a single peptide that is completely conserved between mouse and human mesothelin (SEADVRALGGLAC (SEQ ID NO:86)).

**Table 13. Legend for Figure 8.**

| | Construct | Promoters | Secretory sequences (SS) of construct: | Integration mediated by: |
|---|---|---|---|---|
| Lane P. | Parent *L. monocytogenes* ΔActAΔinlB. | N.A. | N.A. | N.A. |
| Lane 1. | pPL2 LLO BaPA ΔSS hMeso ΔSSΔGPI-12-ras. | hly | LLO | pPL2 |
| Lane 2. | pPL2 LLO BaPA E30R hMeso ΔSSΔGPI-12-ras. | hly | LLO and BaPA | pPL2 |
| Lane 3. | pPL2 LLO BaPA E30M hMeso ΔSSΔGPI-12-ras. | hly | LLO and BaPA | pPL2 |
| Lane 4. | pPL2 LLOₙₐₜᵤᵣₐₗ hmeso ΔSSΔGPI-12-ras. | hly | LLO | pPL2 |
| Lane 5. | pPL2 LLOₒₚₜ hmeso ΔSSΔGPI-12-ras. | hly | LLO | pPL2 |
| Lane 6. | pKSV7 ActA::ActA-N100 mMeso ΔSSΔGPI. | ActA | ActA | pKSV7 |
| Lane 7. | pKSV7 ActA:: ActA-N 100 hMeso ΔSSΔGPI-12-ras. | ActA | ActA | pKSV7 |
| Lane 8. | pKSV7 ActA:: ActA N100 hMeso ΔSSΔGPI. | ActA | ActA | pKSV7 |
| Lane 9. | pKSV7 inlB::BaPA hMeso ΔSSΔGPI-12-ras. | inlB | BaPA | pKSV7 |
| Lane 10. | Molecular weight markers. | N.A. | N.A. | N.A. |
| The double colon of "ActA::ActA-N100" means that the locus of insertion was the ActA gene. LLO means listeriolysin. The hly gene encodes listeriolysin. | | | | |

The results from the gel (Figure 8) show proteins in the supernatant (secreted proteins).

Lane P, a control experiment using the parental *Listeria,* does not show any obvious stained band.

Lanes 1-4 show little or no bands.

Lane 5 shows some secretion of LLOₒₚₜ hmeso ΔSSΔGPI-12-ras, where integration was by pPL2-mediated integration in the listerial tRNA^{Arg} gene.

Lane 6, which represents an attempt to secrete mouse mesothelin, does not show any obvious stained band.

Lane 7 shows marked secretion of the ActA-N100 hMeso ΔSSΔGPI-12-ras, where integration was mediated by pKSV7 at the ActA site of the listerial genome.

Lane 8 shows even greater secretion, where the construct was ActA N100 hMeso ΔSSΔGPI, and where integration was mediated by pKSV7 at the ActA site of the listerial genome (Figure 7).

Lane 9 shows little or no band.

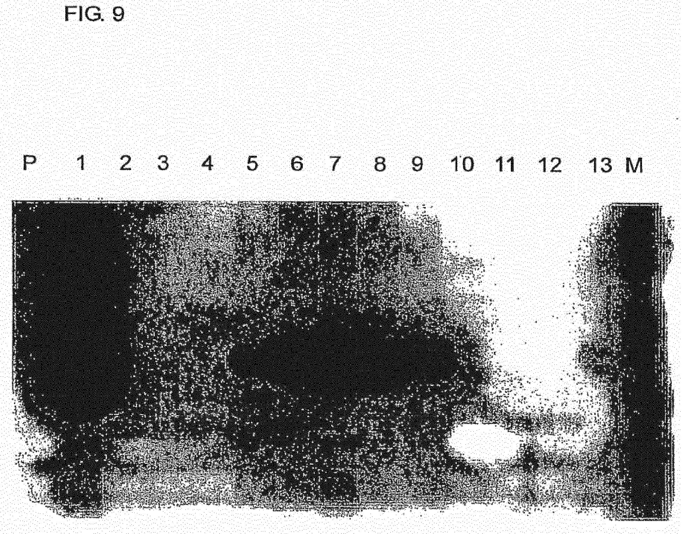

Figure 9 demonstrates protein secretion from *L. monocytogenes* ΔActAΔinlB, where the *Listeria* expressed various fusion proteins comprising human mesothelinΔSSΔGPI. All mesothelin constructs were expressed from *L. monocytogenes* by nucleic acids codon optimized for *L*. *monocytogenes.* Various constructs were prepared for the secretion study (see Table 14). In these experiments also, the antibody for detecting mesothelin expression was a rabbit polyclonal antibody, produced by immunizing rabbit with three peptides from human mesothelin, where the antibody was purified by a single peptide that is completely conserved between mouse and human mesothelin (SEADVRALGGLAC (SEQ ID NO:86)).

**Table 14. Legend for Figure 9. Western blot analysis for secretion of human mesothelin (hMeso).**

| Lane | Construct | Promoters | Secretory sequences (SS) of construct: | Integration mediated by: |
|---|---|---|---|---|
| P. | Parent *L. monocytogenes* ΔActAΔinlB (no mesothelin). | N.A. | N.A. | N.A. |
| 1. | *L. monocytogenes* ΔActAΔinlB LLO441ₒₚₜ human mesothelinΔSSΔGPI-12-ras | ActA | LLO | pPL2. |
| 2. | *L. monocytogenes* ΔActAΔinlB ActA::BaPA ActA-N100(A30R)-human mesothelinΔSSΔGPI (clone 2.25). | ActA | BaPA | pKSV7 at ActA locus. |
| 3. | *L. monocytogenes* ΔActAΔinlB ActA::BaPA ActA-N100 (A30R)-human mesothelinΔSSΔGPI (clone 2.69). | ActA | BaPA | pKSV7 at ActA locus. |
| 4. | *L. monocytogenes* ΔActAΔinlB ActA::BaPA ActA-N100 (A30R)-human mesothelinΔSSΔGPI-12-ras (clone 1.1) | ActA | BaPA. | pKSV7 at ActA locus. |
| 5. | *L. monocytogenes* ΔActAΔinlB ActA::ActA-N 100 (A30R)-human mesothelinΔSSΔGPI (clone 1.46). A30R indicates mutation in the ActA upon which ActA-N100 is based. | ActA | ActA | pKSV7 at ActA locus. |
| 6. | *L. monocytogenes* ΔActAΔinlB ActA::ActA-N100 (A30R)-human mesothelinΔSSΔGPI (clone 2.14). A30R indicates mutation in the ActA upon which ActA-N100 is based. | ActA | ActA | pKSV7 at ActA locus. |
| 7. | *L. monocytogenes* ΔActAΔinlB inlB::ActAN100-human mesothelinΔSSΔGPI (clone BH77). ActA-N100 is based on wild type ActA. | inlB | ActA | pKSV7 at inlB locus. |
| 8. | *L. monocytogenes* ΔActAΔinlB inlB::ActAN100-human mesothelinΔSSΔGPI (clone BH78). ActA-N100 is based on wild type ActA. | inlB | ActA | pKSV7 at inlB locus. |
| 9. | *L. monocytogenes* ΔActAΔinlB inlB::ActA-N100(A30R)-human mesothelinΔSSΔGPI (clone BH85). A30R indicates mutation the ActA upon which ActA-N100 is based. | inlB | ActA | pKSV7 at inlB locus. |
| 10. | *L. monocytogenes* ΔActAΔinlB inlB::ActA-N100(A30R)-human mesothelinΔSSΔGPI (clone BH85). A30R indicates mutation the ActA upon which ActA-N100 is based. | inlB | ActA | pKSV7 at ActA locus. |
| 11. | *L. monocytogenes* ΔActAΔinlB ActA-N100 Ndegcon-human mesothelin (clone A11-2). | ActA | ActA. | pKSV7 at ActA locus. |
| 12. | *L. monocytogenes* ΔActAΔinlB ActA-N 100 Ndegcon-human mesothelin (clone A 11-2). | ActA | ActA. | pKSV7 at ActA locus. |
| 13. | *L. monocytogenes* ΔActAΔinlB ActA-N100 Ndegcon human mesothelinΔSSΔGPI-12-ras (clone 1-3). | ActA | ActA. | pKSV7 at ActA locus. |
| 14. | Molecular weight markers. | N.A. | N.A. | N.A. |
| N.A. means not applicable. The double colon found in "inlB::ActAN100" indicates the locus of the construct, i.e., at the inlB gene. "Ndegcon" refers to constructs that include consensus sequences modeled after the sequences set forth by Suzuki and Varshavsky (1999) EMBO J. 18:6017-6026. | | | | |

The construct used for Lane 1 used LLO441 as the source of secretory sequence, where the nucleic acid for LLO441 had been codon optimized for expression in *L. monocytogenes,* and where the heterologous antigen was human mesothelinΔSSΔGPI (Lane 1). This construct produced the highest level of secretion in this particular experiment (Lane 1). The high molecular weight material shown in the western blot represents LLO₄₄₁ fused to mesothelin, where the lower molecular weight material likely represents degradation products.

The constructs used for Lanes 2 and 4 were based on ActA-N100, but with the ActA's signal sequence deleted and replaced with the the signal sequence of BaPA. Expression from these constructs was relatively low (Lanes 2 and 4) (Figure 9).

All of the remaining constructs contained full-length ActA-N100 as the source of secretory sequence, but where ActA-N100 had an A30R mutation (Lane 5); where ActA-N100 had no mutation (Lane 7); where ActA-N100 had an A30R mutation (Lane 9); and where ActA-N100 had four mutations (designated "Ndegcon") (Lane 11). The four mutations in ActA-N100 designated by "Ndegcon" were Arg-29, Lys-32, Lys-37, and Lys-44. The Ndegcon was situated (or inserted) in between ActA-N100 and the mesothelin. Secreted protein was collected by precipitation with trichloroacetic acid from mid-exponential cultures grown in yeast extract without glucose.

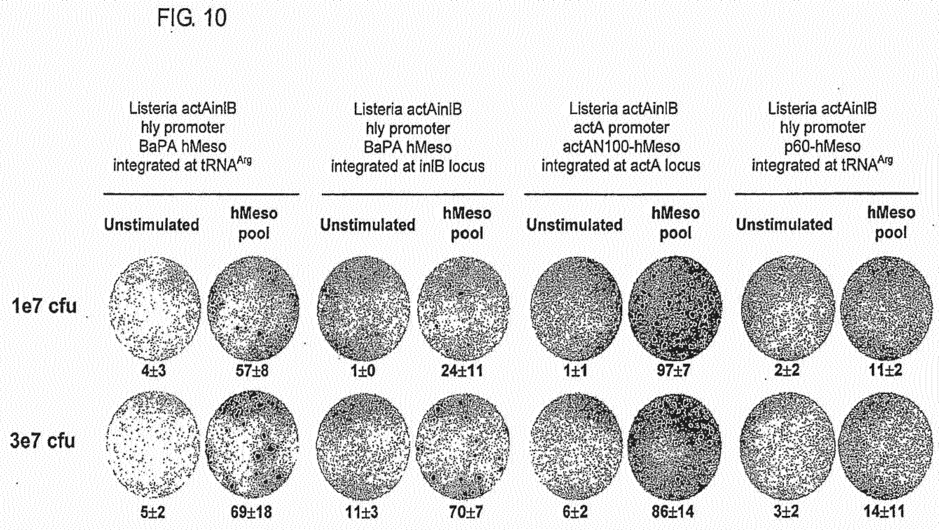

Figure 10 shows immune stimulation, as determined after a single vaccination with the indicated *L. monocytogenes* ΔActAΔinlB construct, where spleens were harvested seven days after vaccination and used as the source of splenocytes. Mesothelin-specific immune responses were found after vaccination with each of the four constructs: (1) hly promoter was operably linked with BaPA signal sequence and hMeso (integrated at tRNA^{Arg} locus); (2) hly prmoter was operably linked with BaPA signal sequence and hMeso (integrated at inlB locus); (3) ActA promoter was operably linked with ActA-N100 and hMeso (integrated at ActA); and (4) hly promoter was operably linked with p60 and hMeso (integrated at tRNA^{Arg} locus).

The results indicate a role of the ActA promoter in stimulating immune response; a role of the ActA-N100 fusion partner in enhancing immune response; as well as a role of integration at ActA locus in increasing immune response; and demonstrate enhanced ability to stimulate immune response where the ActA promoter is operably linked with ActA-N100 fusion protein partner and integration is at ActA locus (Figure 10).

Further details of the above study are described as follows. Mice were injected with *Listeria,* followed by a period of time (7 days) to allow the *Listeria* to be taken up and processed by antigen presenting cells (APCs)- After uptake of the *Listeria,* the APC presented *Listeria*-encoded antigens to T cells, resulting in the activation and clonal expansion of the T cells. Spleens were removed, and the splenocytes (including T cells and APCs) were isolated. To the isolated splenocytes was added either buffer or a pool of human mesothelin peptides (0.002 mg/ml final concentration of pool). After adding the peptides, the dendritic cells (DCs) in the splenocyte preparation were allowed to present peptide to any activated T cells. Successful presentation resulted in the T cell's secretion of interferon-gamma, as reflected by signals in spot forming assays (spot forming cells; SFC) (Figure 10).

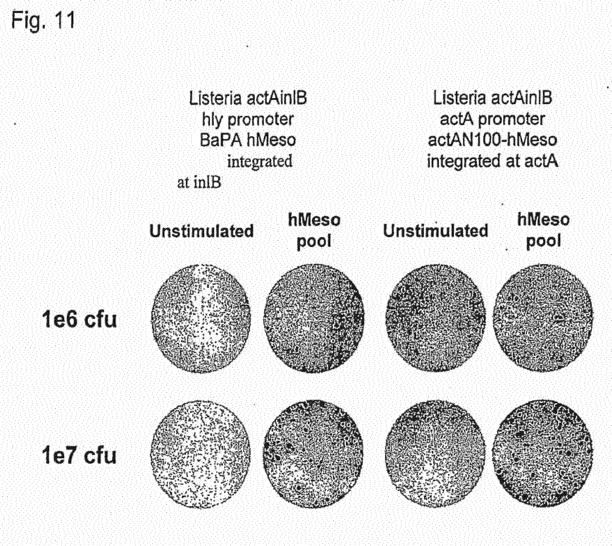

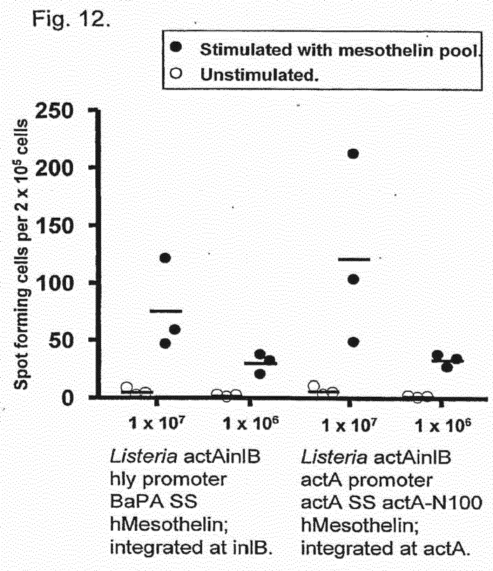

The mesothelin peptide pool (also known as 15 x 11 pool) consisted of 153 different peptides, all of them 15 mers, spanning the entire sequence of human mesothelin, where succeeding peptides overlapped by eleven amino acids. The results demonstrated that interferon-gamma (IFGgamma) expression was greater where the peptide pool had been added to the splenocytes, than where no peptide pool was used. Figures 10-12 compare immune response where mice were vaccinated with 1 x 10⁷ CFU or 3 x 10⁷ CFU (Figure 10); 1 x 10⁶ CFU or 1 x 10⁷ CFU (Figure 11); or 1 x 10⁶ CFU or 1 x 10⁷ CFU of *L. monocytogenes* (Figure 12). In most cases disclosed here, immune response was greater where mice were injected with greater numbers of bacteria.

Figures 11 and 12 disclose similar studies using spot forming cell assays.

The raw data (photographs of spot forming cell assays) from Figure 11 are graphed in Figure 12. Figure 12 discloses the number of cells that produce an IFNgamma signal (spot forming cell; SPC) per number of splenocytes. The data disclose comparable mesothelin-specific immune responses, where the construct was with hly promoter operably linked with BaPA signal sequence and hMeso (inlB locus), or where the construct was with ActA promoter operably linked with ActA signal sequence and ActA-N100 and hMeso (ActA locus).

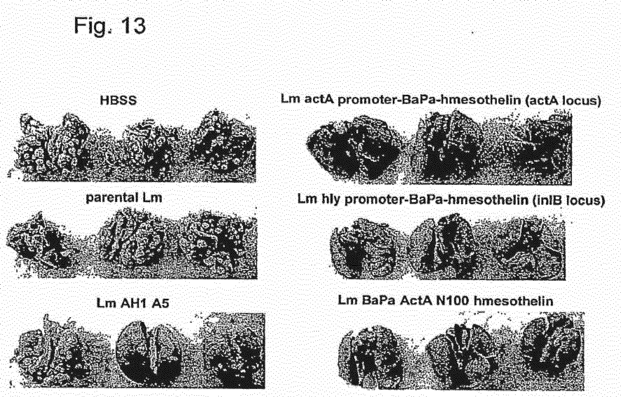

Figures 13-14 disclose tumor metastasis data. The study measured metastasis of CT-26 human mesothelin expressing cells to the lungs. At t = 0 days, CD-26 tumor cells were injected i.v. (2e5 cells). At t = 3 days, mice were administered the indicated *Listeria* vaccine. At t = 18 days, lungs were harvested. "2e5 cells" means 2 x 10⁵ cells.

Tumor cell-inoculated mice were treated as follows: (1) Salt water only (HBSS); (2) *L. monocytogenes* ΔActAΔinlB encoding no heterologous antigen (negative control); (3) *L. monocytogenes* ΔActAΔinlB encoding the AH1-A5 peptide derived from the gp70 tumor antigen (an antigen different from mesothelin -- positive control); and (4)-(7) *Listeria* ΔActAΔinlB encoding various mesothelin constructs. The AH1-A5 peptide is derived from the gp70 tumor antigen. AH1-A5 is used as a positive control in the present experiments (see, e.g., Brockstedt, et al. (2004) Proc. Natl. Acad. Sci. USA 101:13832-13837; Slansky, et al. (2000) Immunity 13:529-538).

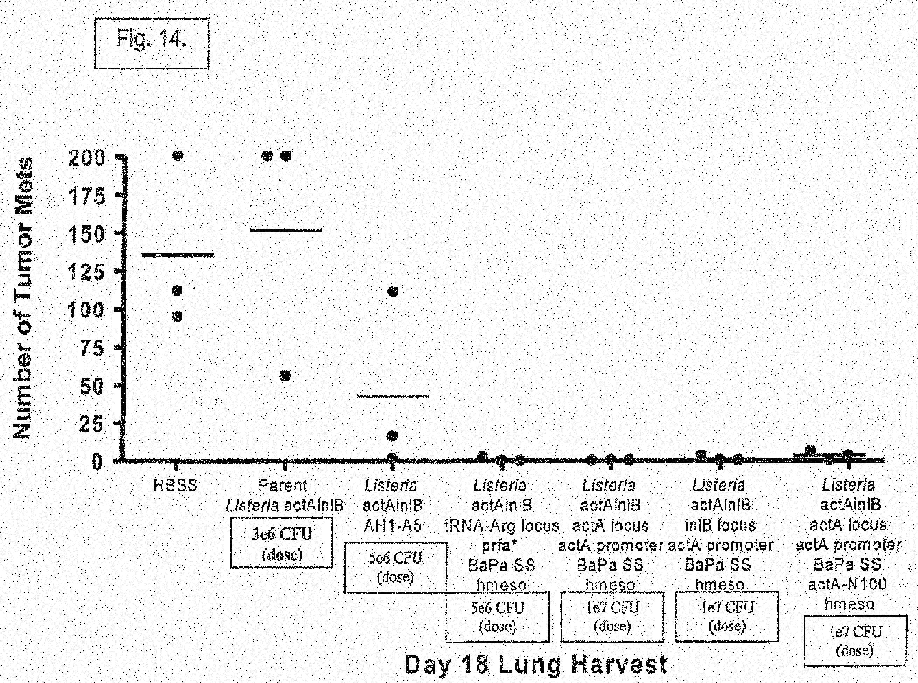

Figure 14 reveals equivalent effects of the four mesothelin-expressing *Listeria* constructs in eliminating tumor metastasis.

**Table 15. Groups of mice challenged with CT26 tumor cells and treated with Listeria vaccines.**

| Group | *Listeria* vaccine | Site of integraton. |
|---|---|---|
| 1 | Hanks Buffered Salt Solution only (HBSS) (no *Listeria*) (negative control). | no *Listeria* |
| 2 | *L. monocytogenes* ΔActAΔinlB (parental strain) (negative control). | none |
| 3 | *L. monocytogenes* ΔActAΔinlB-OVA-AH1-A5. The AH1-A5 epitope was inserted in-frame within OVA by using a unique AvaII site (expressed from hly promoter as part of pPL2 vector) (positive control). | tRNA^{Arg} locus |
| 4 | *L. monocytogenes* ΔActAΔinlB prfA* (E77K)-BaPa signal sequence-human Mesothelin ΔSSΔGPI (see, e.g., Mueller and Freitag (2005) Infect. Immun. 73:1917-1926). | ActA locus |
| 5 | *L. monocytogenes* ΔActAΔinlB-BaPa signal sequence-human mesothelin ΔSSΔGPI (expressed from ActA promoter). | ActA locus |
| 6 | *L. monocytogenes* ΔActAΔinlB-BaPa signal sequence-human mesothelin ΔSSΔGPI (expressed from hly promoter). | inlB locus |
| 7 | *L. monocytogenes* ΔActAΔinlB-ActA signal sequence-ActA-N100-human mesothelin ΔSSΔGPI (expressed from ActA promoter). | ActA locus |

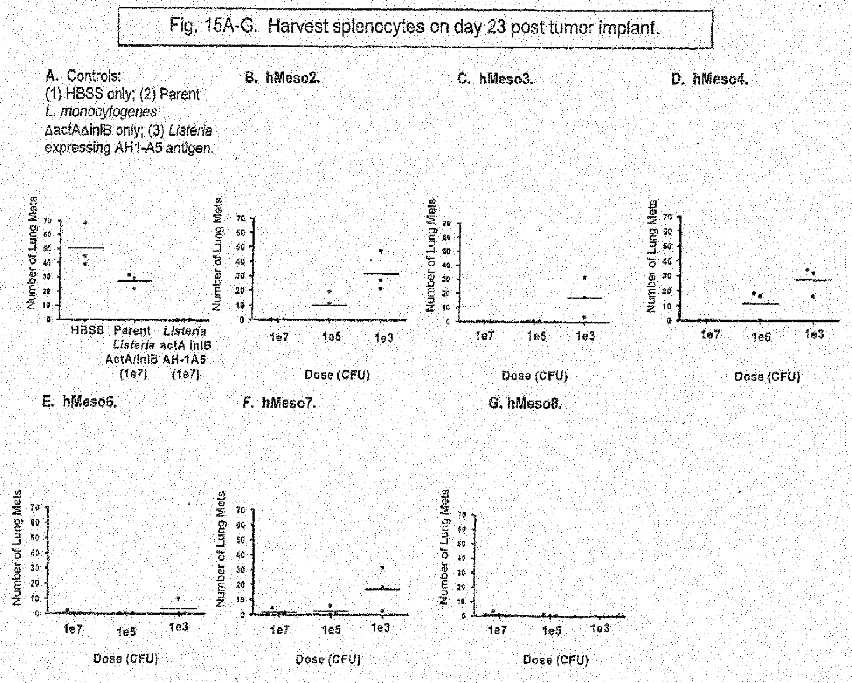

Figure 15 demonstrates that various mesothelin-expressing *Listeria* are effective in reducing lung tumors, where three different doses of each mesothelin-expressing *Listeria* were tested. hMeso6 is more effective than, for example, hMeso2 or hMeso4, in stopping lung metastasis (Figure 15).

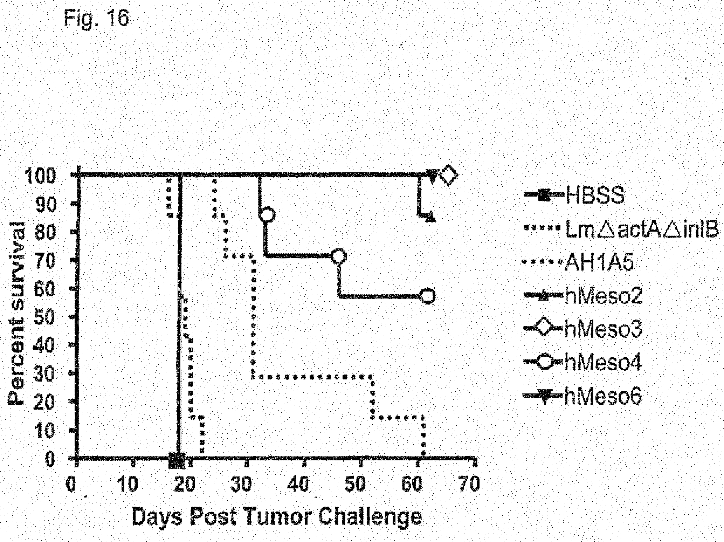

Figure 16 discloses survival to tumors with various listerial vaccines. With negative control treatments (HBSS; parental *Listeria*), none of the mice survived beyond 22 days. The positive control *Listeria* expressed an antigen derived from gp70. The antigen (AH1-A5) was derived from the immunodominant antigen from CT26 cells (Slansky, et al. (2000) Immunity 13:529-538). Mice treated with the positive control vaccine survived up to or beyond 60 days (Figure 16).

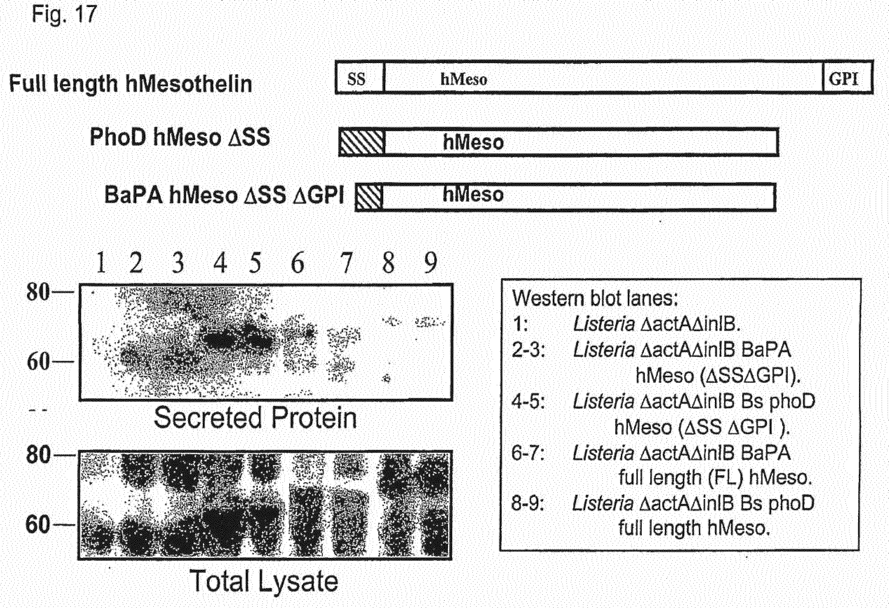

Figure 17 discloses gels, with western blot analysis, for detecting secreted mesothelin (top blot) and total expressed mesothelin (lower blot). *L. monocytogenes* ΔActAΔinlB engineered to contain a polynucleotide encoding the indicated secretory sequences and antigens were cultured, and the total or secreted mesothelin was measured. The secretory sequences were BaPA or Bs phoD, as indicated. The antigens were full length (FL) human mesothelin or human mesothelin deleted in its secretory sequence and GPI anchor (hMesoΔSSΔGPI), as indicated. The results indicate that total expression was somewhat greater with Bs phoD (lanes 4-5; lower gel) than with BaPA (lanes 2-3; lower gel). The results also demonstrate that, at least with the Bs phoD containing constructs, secretion was greater with hMeso (ΔSSΔGPI) (lanes 4-5; top gel) than with full length hMeso (lanes 8-9; top gel).

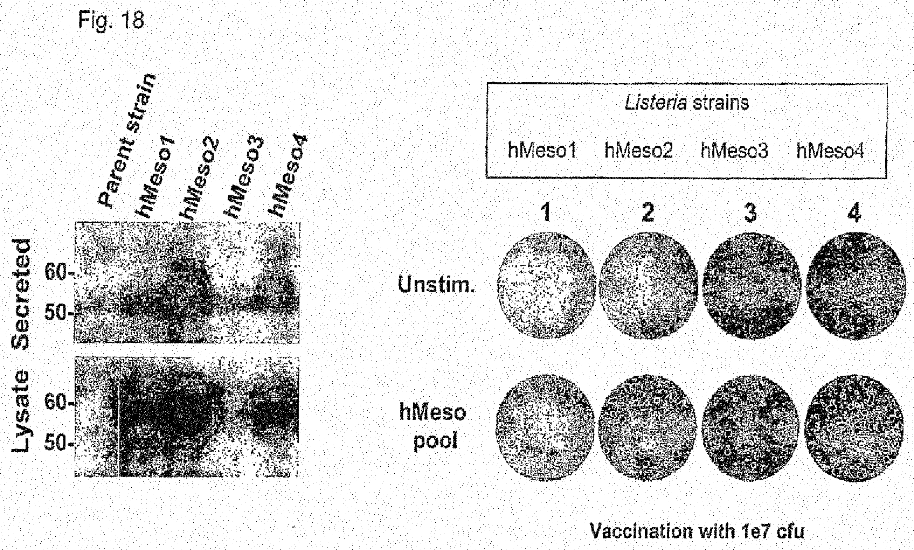

Figure 18 compares the mesothelin-specific immune response to vaccination with hMeso1, hMeso2, hMeso3, and hMeso4. Side-by-side comparison of hMeso1 and hMeso2 reveals that a *Listeria* construct comprising a nucleic acid encoding for constitutively active PrfA (prfA*) increases immune response, as compared to a *Listeria* construct not comprising that nucleic acid. Side-by-side comparisons of hMeso1 and hMeso4 reveals that increased immune response is found with genomic integration at the inlB locus (hMeso4), as compared to immune response where genomic integration is at the tRNA^{Arg} locus (hMeso1). Comparison of immune response to hMeso3 and hMeso4 suggests that immune response can be enhanced by using hly promoter, as compared to immune response with ActA promoter. Elispot analysis was used to assess immune response. Splenocytes (plus or minus stmulation of splenocytes with a pool of mesothelin peptides) for elispot assays, where the elispot assays measured IFNgamma expression.

The gels of Figure 18 disclose western blots sensitive to total expression of mesothelin or to secretion of mesothelin. hMeso2 produced the highest levels of secretion, indicating the usefulness of the following combination for increasing secretion: (1) prfA* nucleic acid; (2) Integration at tRNAArg locus; (3) The hly promoter; and (4) BaPa secretory sequence. Again, the usefulness of the prfA* nucleic acid is demonstrated.

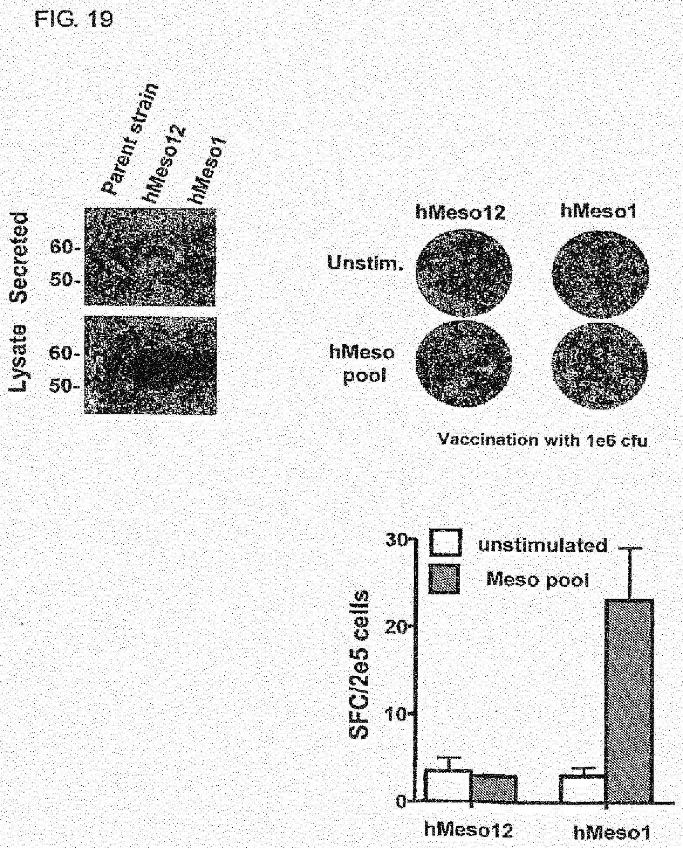

Figure 19 compares immune response to hMeso12 and hMeso1. Mesothelin-specific immune response is depicted by the raw data (elispot assays) and by histograms showing the number of spot forming splenocytes per 2 x 10⁵ spenocytes. The results indicate that the ras sequence present in the fusion protein of hMeso ΔSSΔGPI (hMeso12) results in lower immune response (elispot assays) and lower expression (western blots), as compared to results where the fusion protein did not comprise ras (hMeso1) (Figure 19).

Mice were vaccinated with the two strains (hMeso12 or hMeso1), and splenocytes were removed and used for elispot assays, where assay mixtures were pulsed with the standard hMeso pool of peptides. As disclosed above, hMeso1 (the BaPA secretory sequence is wild type) stimulated a greater mesothelin-specific immune response than hMeso12 (the BaPA secretory sequence is E30R).

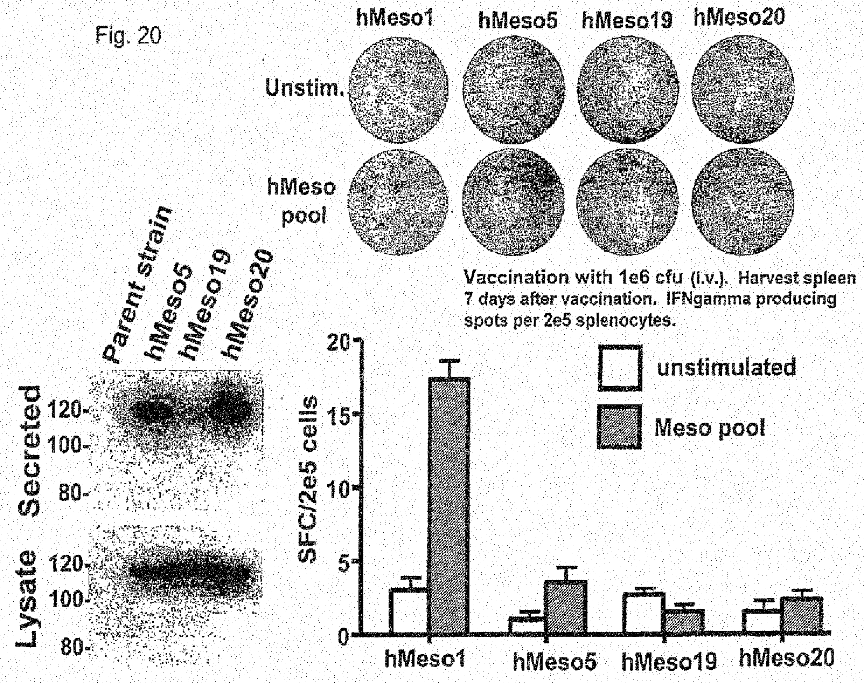

Figure 20 compares immune response to hMeso1, hMeso5, hMeso19, and hMeso20. The results demonstrate that the greatest mesothelin-specific immune response was to hMeso1, where there was also some detectable mesothelin-specific response to hMeso5. The results demonstrate that BaPA secretory sequence results in greater immune response, as compared to p60 secretory sequence, or to derivatives of p60 secretory sequence. The gel demonstrates that the p60 secretory sequence supports secretion of mesothelin. See lanes labeled hMeso5 or hMeso20 (Figure 20).

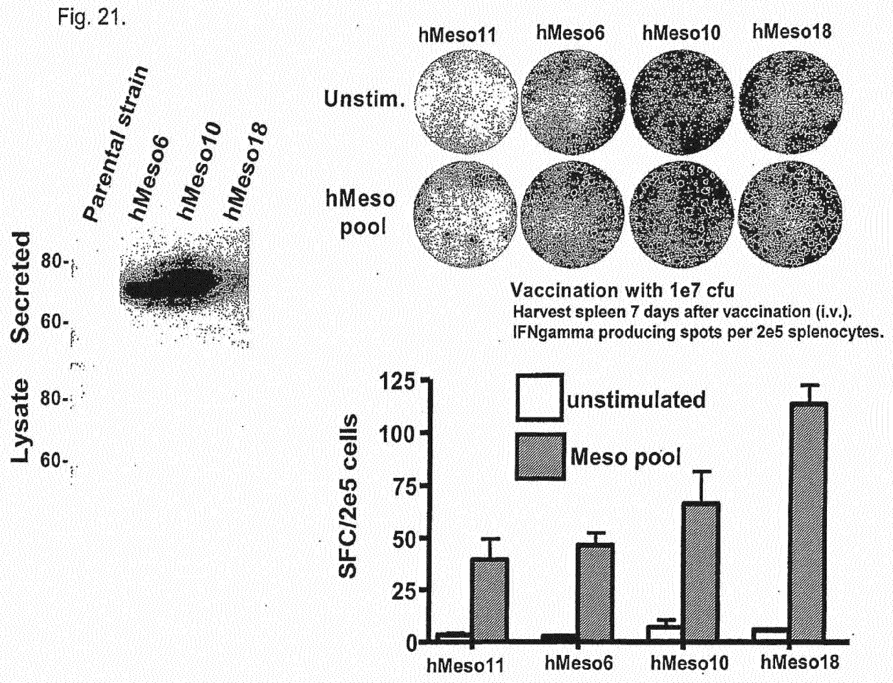

Figure 21 compares immune responses to hMeso11, hMeso6, hMeso10, and hMeso18. Mesothelin-specific immune responses occurred with each of these vaccines, where the highest responses were provoked by hMeso10 and hMeso18. In comparing hMeso6 and hMeso10, it can be seen that the ras (hMesolO) can enhance mesothelin-specific immune response. Here, both *Listeria* strains ActA secretory sequence was used, ActA promoter was used, and ActA locus of integration was used. The high degree of immune response to hMeso18 can be due to the use of the ActA (A30R) secretory sequence. The present invention provides a *Listeria* containing a polynucleotide comprising a first nucleic acid encoding ActA (A30R), operably linked with and in frame with a second nucleic acid encoding a heterologous antigen, e.g., an antigen derived from a tumor, such as mesothelin antigen, or an antigen derived from an infectious agent. The gel reveals that the hMeso18 *Listeria* strain secreted relatively low amounts of mesothelin, as compared with secretion by hMeso10 and hMeso6 (Figure 21).

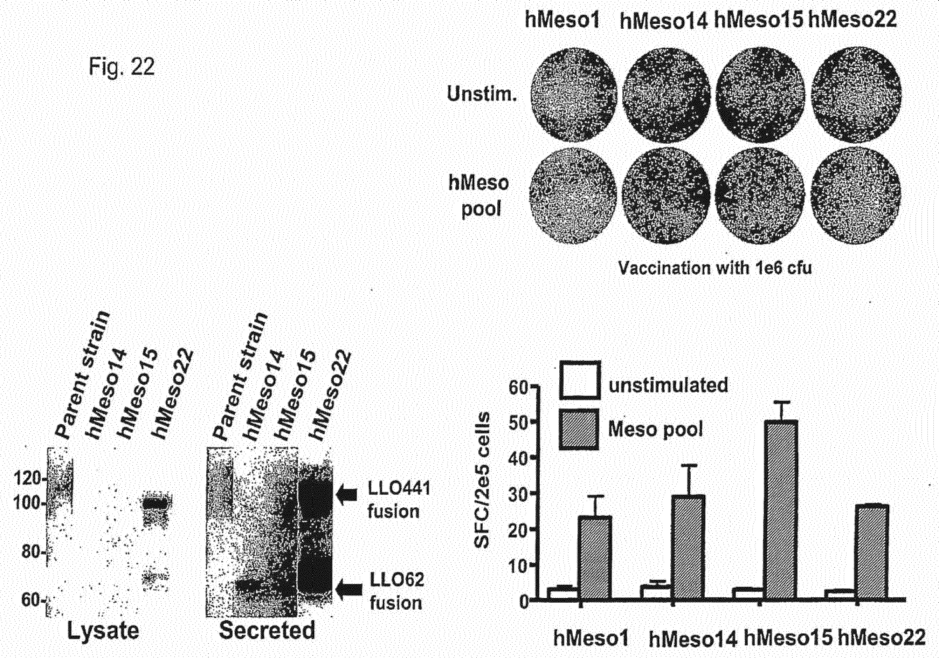

Figure 22 compares immune responses to hMeso1, hMeso14, hMeso 15, and hMeso22. Mesothelin-specific immune responses to hMeso1, hMeso14, and hMeso22 were comparable, while that to hMeso15 was greater. The secretory sequences (SS) of each these four vaccine strains are different. The secretory sequences (SS) of hMeso1 is BaPA; hMeso14 (LLO62); hMeso15 (LLO opt62); hMeso22 (LLO441).

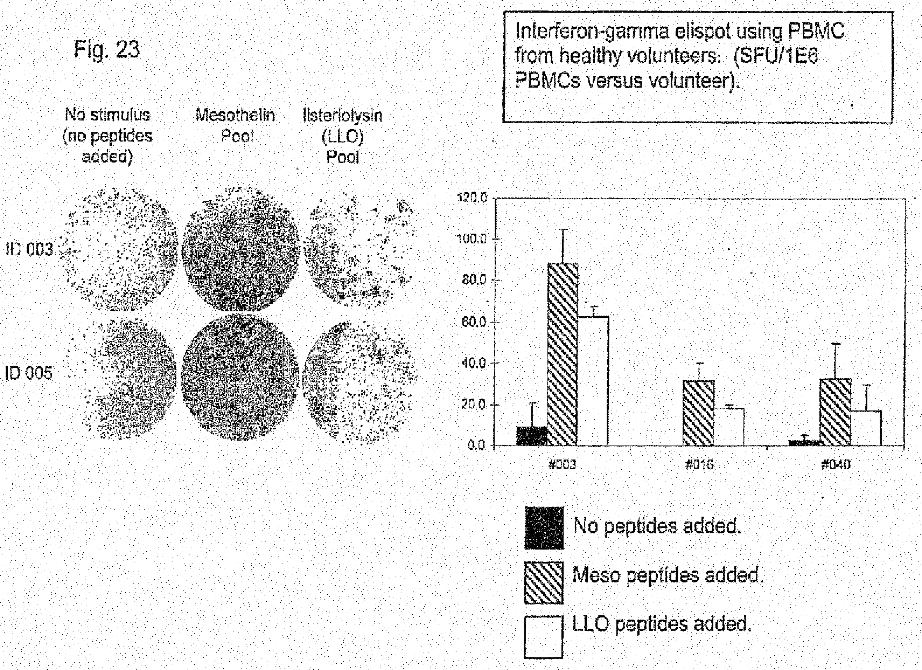

Figure 23 reveals immune response in healthy human volunteers, to listeriolysin (LLO) and to mesothelin. Immune response to epitopes of LLO and to mesothelin was found in all three subjects tested.

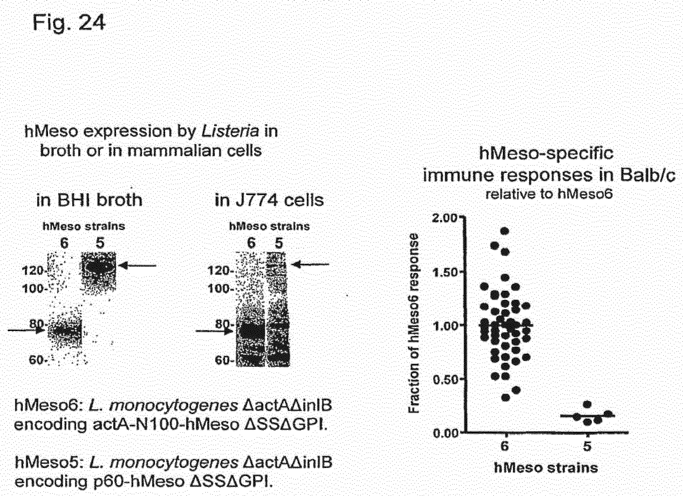

Figure 24 illustrates expression of human mesothelin by hMeso6 or hMeso5, in BHI broth and in J774 macrophages, where expression was assessed by gel separation and detection by the western blot method. The results demonstrate relatively low expression by hMeso6 in broth (and high expression by hMeso5 in broth), and relatively high expression by hMeso6 inside mammalian cells (and low expression by hMeso5 inside mammalian cells). The graph demonstrates relatively high immune response (meso-specific response; elispot assays) after vaccination with hMeso6, and low immune response after vaccination with hMeso5 (Figure 24).

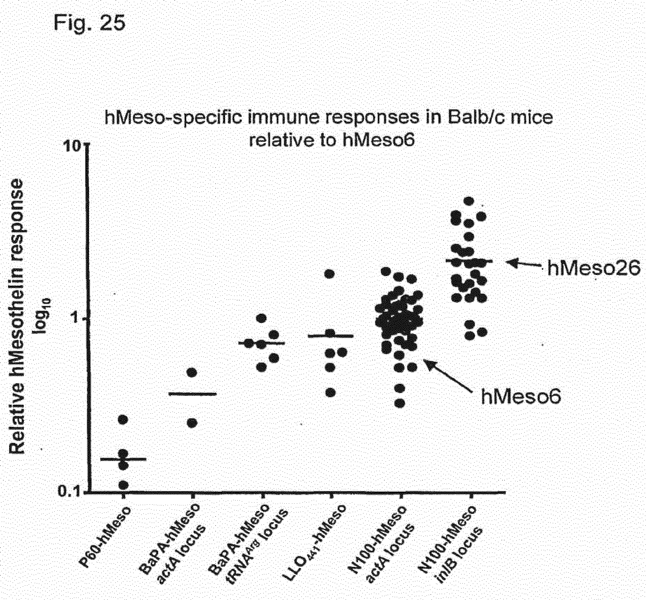

Figure 25 discloses mesothelin-specific immune response, where mice had been vaccinated with *Listeria* containing a polynucleotide comprising a first nucleic acid encoding p60, BaPA, LLO441, ActA-N100, as indicated, and a second nucleic acid encoding hMeso. Integration was at the tRNA^{Arg} locus, ActA locus, or inlB locus, of the listerial genome, as indicated.

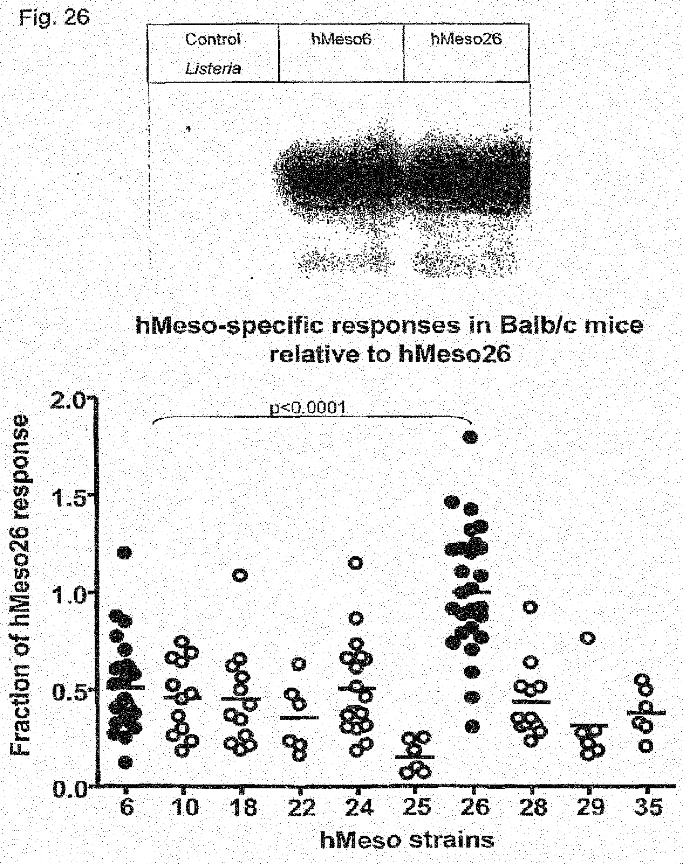

Figure 26 illustrates *in vivo* expression of mesothelin from J774 macrophages, as detected by western blotting using an anti-mesothelin antibody. Similar *in vivo* expression occurred when the J744 macrophages were infected with hMeso6 or with hMeso26.

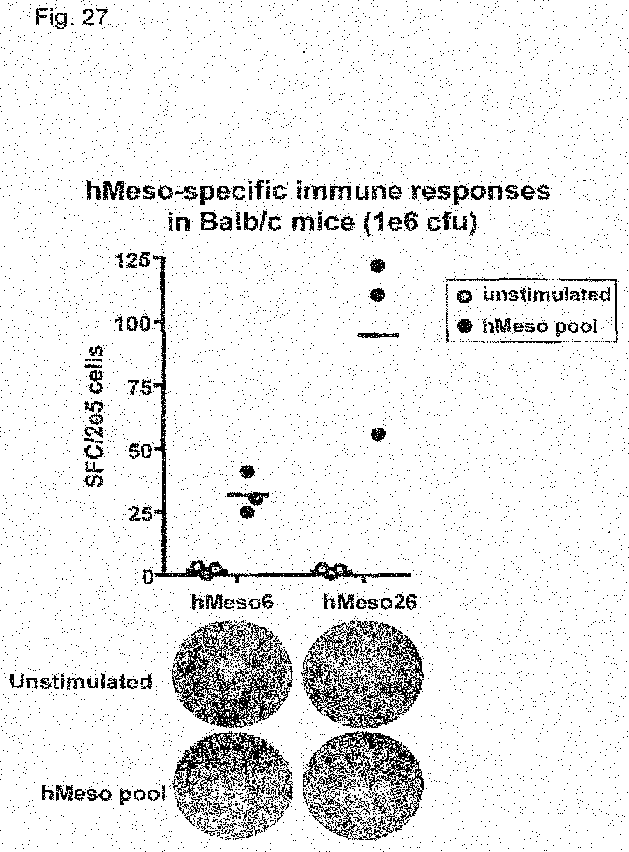

Figure 26, as well as Figure 27, shows show that mesothelin-specific mounted after vaccination with various engineered *Listeria* were greater with the hMeso26 strain than with the other tested strains.

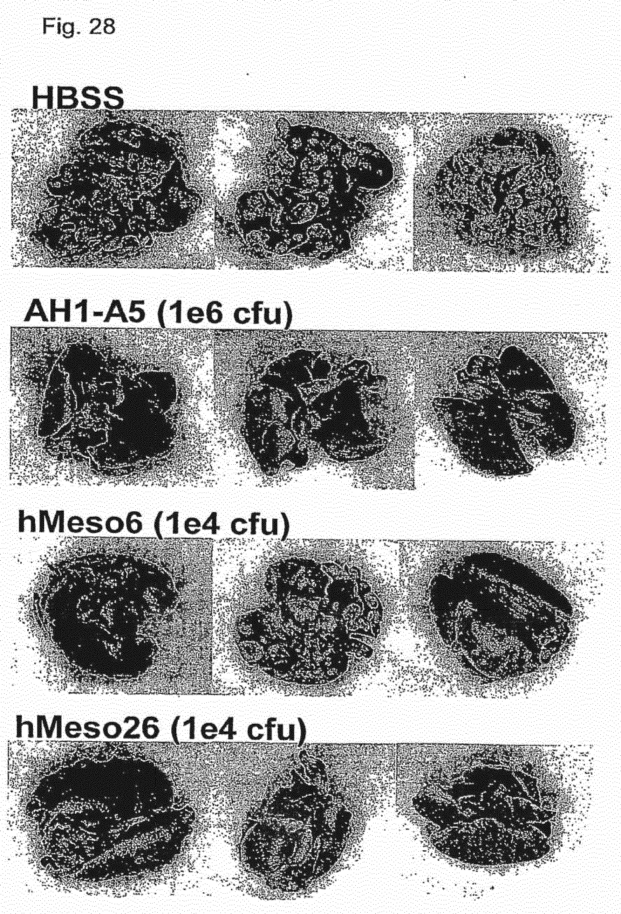

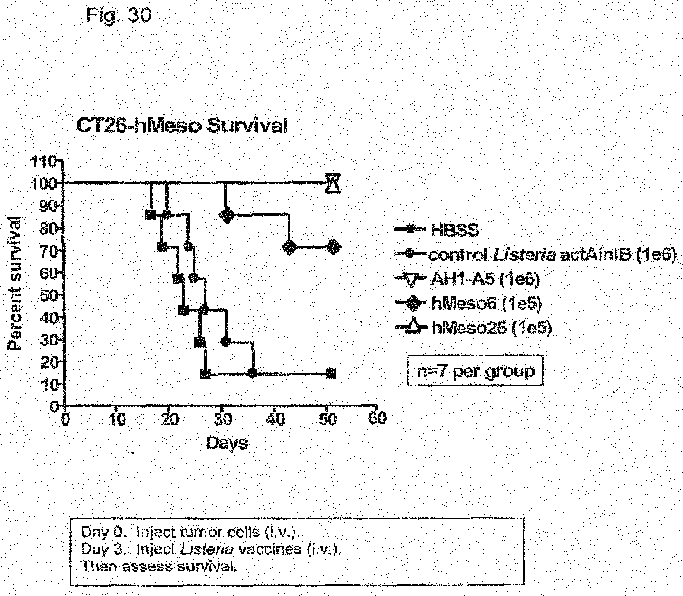

Figures 28 (photographs of lungs), 29 (histograms of lung data), and 30 (mouse survival) reveal the successful treatment of lung tumors by administering hMeso6 and hMeso26. Mice were treated with a negative control (HBSS); positive control (*Listeria* expressing AH1-A5); or the indicated numbers of hMeso6 or hMeso26. The tumors were induced by an injection with CT26 cells. The results demonstrate that both hMeso6 and hMeso26 were effective in reducing tumor metastasis, where hMeso26 was more effective than hMeso6 (Figure 30).

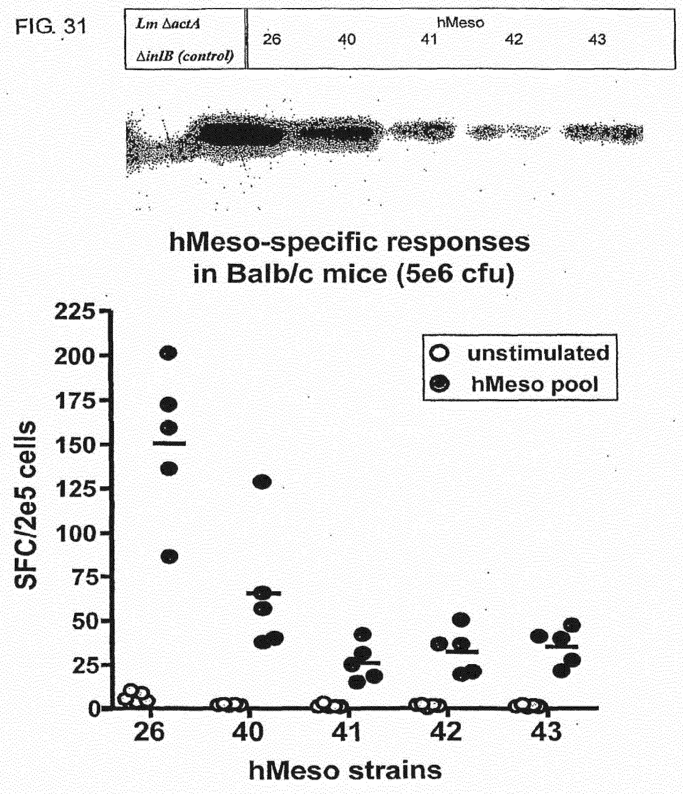

Figure 31 compares expression, and immune responses to vaccination, with various *Listeria* strains engineered to contain integrated expression cassettes at different points in the listerial genome. The control bacterium (*L. monocytogenes* ΔActAΔinlB) contained no expression cassette, while hMeso26 contained only one integrated expression cassette. The strains hMeso40, hMeso41, hMeso42, and hMeso43, each contained two different expression cassettes (integrated at two different points in the genome), where expression from these *Listeria* strains and immune response to these *Listeria* strains are shown (Figure 31).

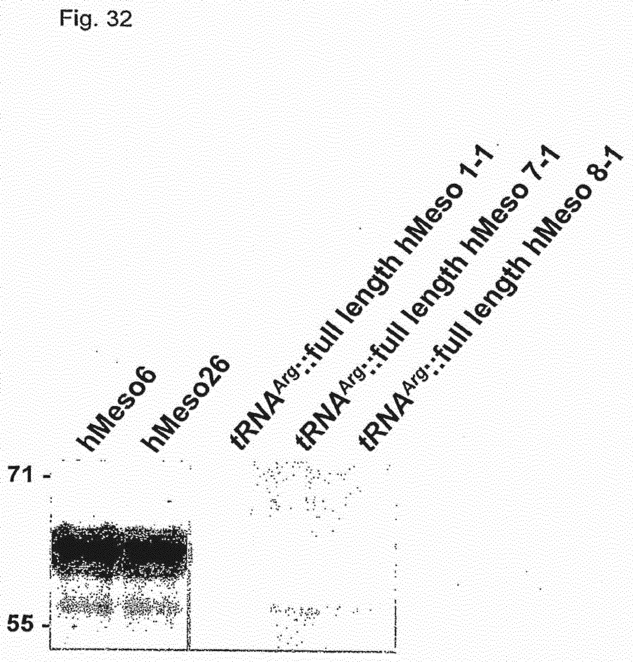

Figure 32 shows *in vivo* expression of mesothelin, that is, *in vivo* within J744 macrophages, after infection with (1) hMeso6; (2) hMeso26; or (3) *L. monocytogenes* ΔActAΔinlB (three identical constructs) bearing an expression cassette encoding full length human mesothelin, and integrated at tRNA^{Arg} locus. The three identical constructs, or siblings, are labeled 1-1, 7-1, and 8-1.

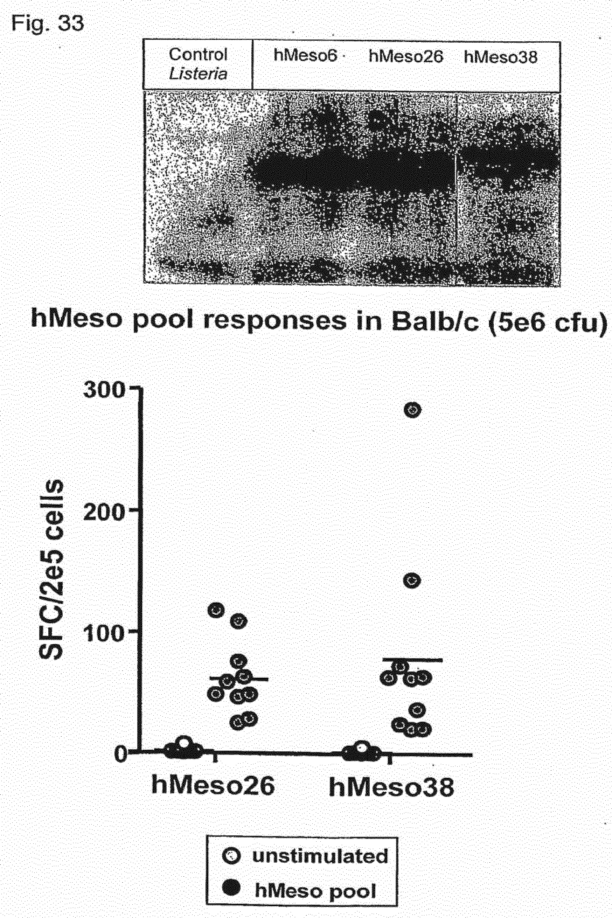

Figure 33 discloses *in vivo* expression of mesothelin by hMeso6, hMeso26, and hMeso38 within J774 murine macrophages (gels with western blots). The control bacterium was *L. monocytogenes* ΔActAΔinlB. Also shown are mesothelin-specific immune responses (elispot assays). The results demonstrate comparable expression of mesothelin where hMeso6, hMeso26, and hMeso38 are located in macrophages, and comparable immune response to hMeso26 and hMeso38.

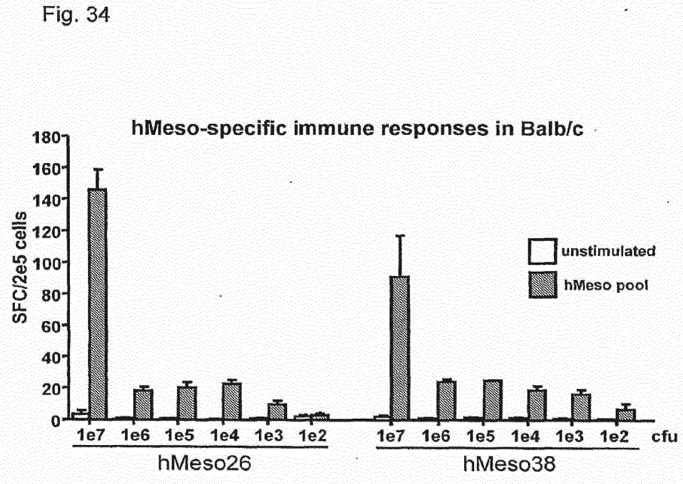

Figure 34 discloses mesothelin-specific immune response generated seven days after a single injection of hMeso26 or hMeso38, at the indicated doeses. The dose response curves reveal a marked increase in going from one million bacteria to ten million bacteria. The dose response curves found with the two strains are similar to each other (Figure 34). The present invention provides hMeso26; hMeso38; a vaccine comprising hMeso26 and/or hMeso38; a method of administering hMeso26 and/or hMeso38 to a mammalian subject; a method of stimulating mesothelin-specific immune response against a cancer or tumor comprising administering hMeso26 and/or hMeso38; a method of increasing survival to a cancer or tumor comprising administering hMeso26 and/or hMeso28, and so on (Figure 34).

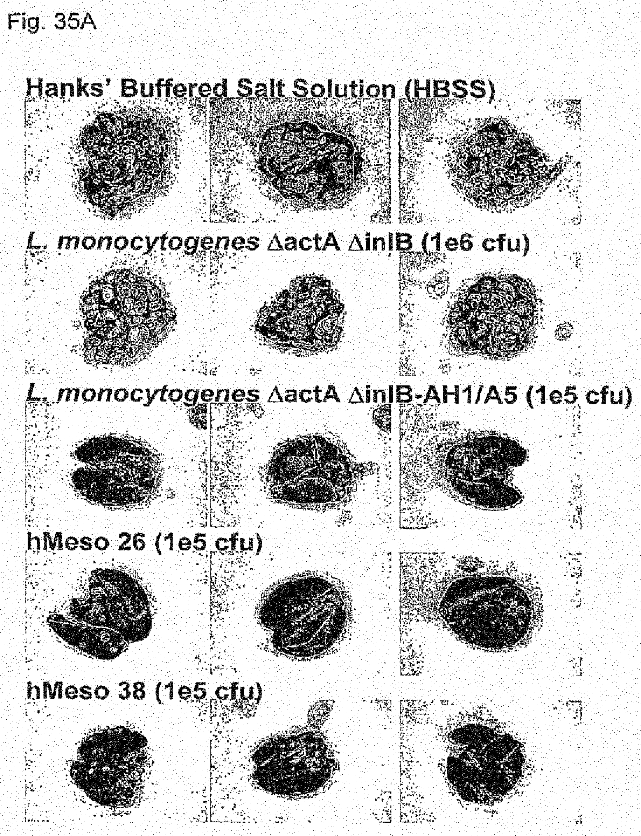

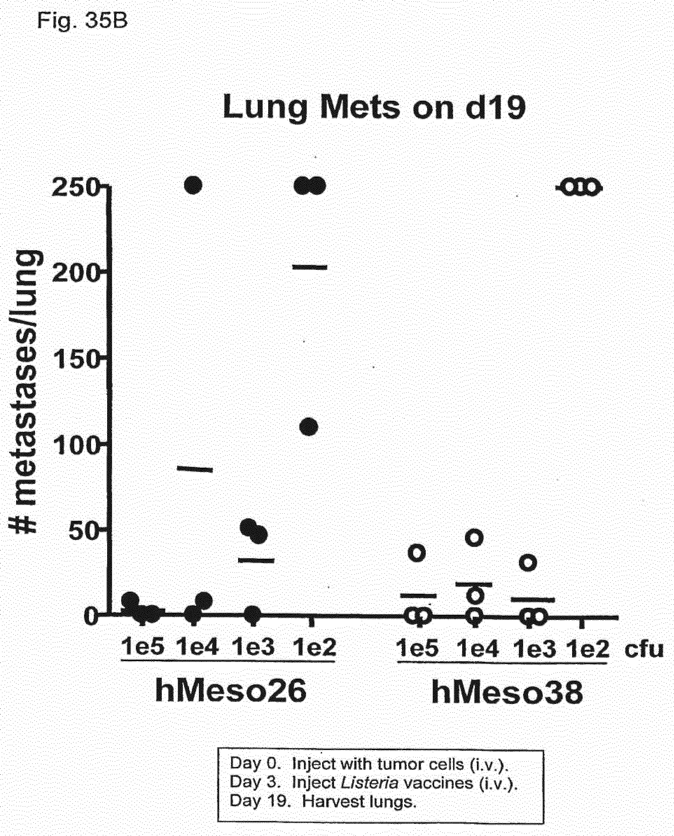

Figures 35A and 35B continue the narrative on hMeso26 and hMeso38, and shows photographs of fixed lungs. Tumor cells were injected at t = 0 days. *Listeria* vaccines were injected (i.v.) at T = 3 days. Lungs were harvested at t = 19 days, where the histograph quantitates the metastasis results represented by the lung photographs (Figure 35A,B). With titration of mice with the indicated numbers of bacteria, the results show similar responses for both listerial strains, hMeso26 and hMeso38.

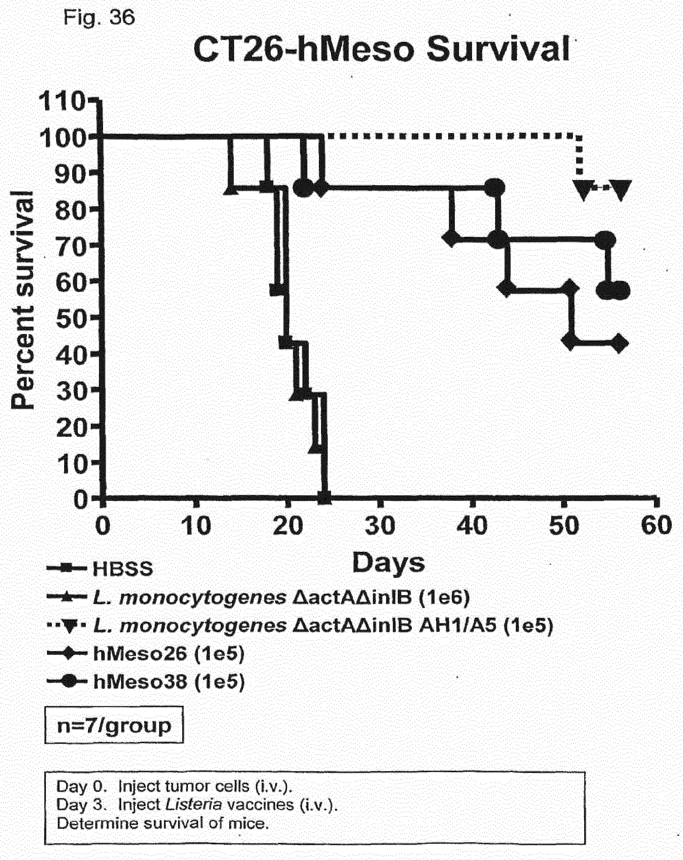

Figure 36 also continues the narrative of *Listeria* strains hMeso26 and hMeso38. The results demonstrate that both strains result in similar increases in survival to innoculated CT26 tumor cells.

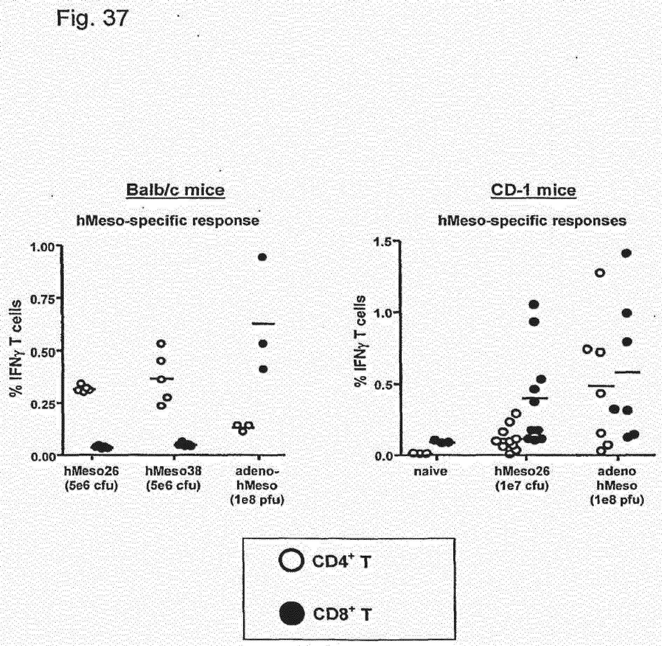

Figure 37 dissects mesothelin-specific immune response to *Listeria* strains hMeso26 and hMeso38 into CD4⁺ T cell response and CD8⁺ T cell response. Immune response was monitored by intracellular staining assays (ICS). Both strains of *Listeria* were tested with Balb/c mice, while only the hMeso26 *Listeria* strain was tested with CD-1 mice. The results demonstrate that the proportion of immune response that is CD4⁺ T cell response, or CD8⁺ T cell response, can differ in different strains of mice.

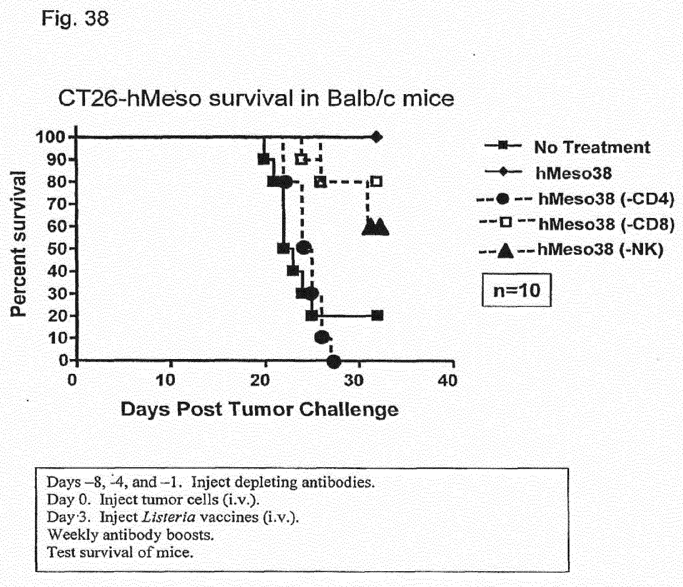

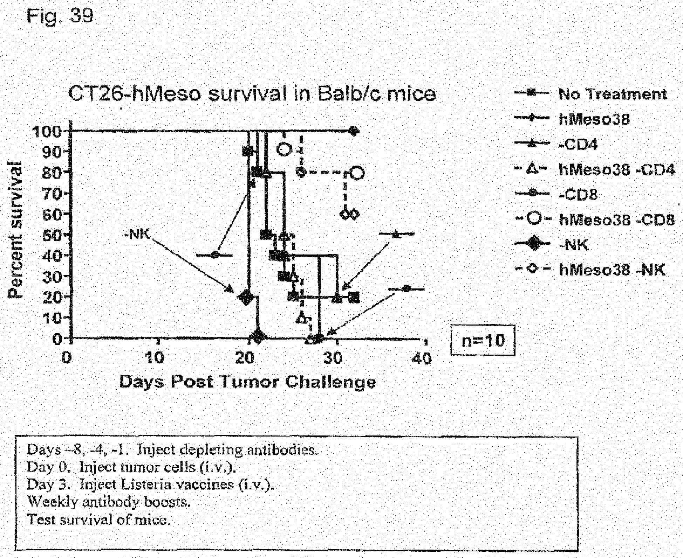

Figure 38 demonstrates that hMeso38 increases survival to tumors, and dissects the contribution to survival by cells that are CD4+, CD8+, and NK cells. Mice were treated with antibodies that depleted one of CD4+ cells, CD8+ cells, or NK cells. Treating with the anti-CDS antibodies resulted in only slight impairment of hMeso38-mediated increased survival. Treating with anti-NK cell antibodies resulted in moderate impairments of hMeso38-mediated increased survival. Treating with anti-CD4 antibodies resulted in a large impairment in hMeso38-medicated increased survival (Figure 38). Antibody-mediated depletion of the mouse's cells were effected by administering antibodies on t = minus 8 days, minus 4 days, and on minus 1 days. At t = 0 days, mice were injected (i.v.) with tumor cells. At t = 3 days, mice were injected with *Listeria* vaccine (i.v.). Weekly antibody boosts were given to provoke depletion of the mouse's cells. Figure 39 shows a similar experiment, but where only antibody was administered, where only hMeso38 was administered, or where both hMeso38 and the indicated antibody were administered.

The above-disclosed data are not intended to limit the present invention to embodiments comprising *L. monocytogenes* ΔActAΔinlB containing a nucleic acid encoding human mesothelin. The present disclosure provides other attenuated listerial vaccine platforms, e.g., KBMA *L. monocytogenes, L. monocytogenes* ΔinlB; *L. monocytogenes* ΔActA; *L. monocytogenes* Δhly; KBMA *L. monocytogenes* ΔinlB; KBMA *L. monocytogenes* ΔActA; KBMA *L. monocytogenes* ΔActAΔinlB; KBMA *L. monocytogenes* Δhly. Moreover, what is also provided are constructs encoding antigens other than, or in addition to, human mesothelin.

### EXAMPLE VIII. Nucleic acids encoding phage integrases, phage attachment sites (attPP'), and bacterial attachment sites (attBB').

Site-specific integration of a first nucleic acid into a polynucleotide can be mediated by a phage integrase, an attPP' site residing in the first nucleic acid, and a corresponding or compatible attBB' site residing in the polynucleotide. The present disclosure provides a number of nucleic acids, encoding phage integrases, attPP' sites, and attBB' sites, useful for mdiating integration of a first nucleic acid into a polynucleotide, where the polynucleotide can be a plasmid or bacterial genome, to provide some non-limiting examples.

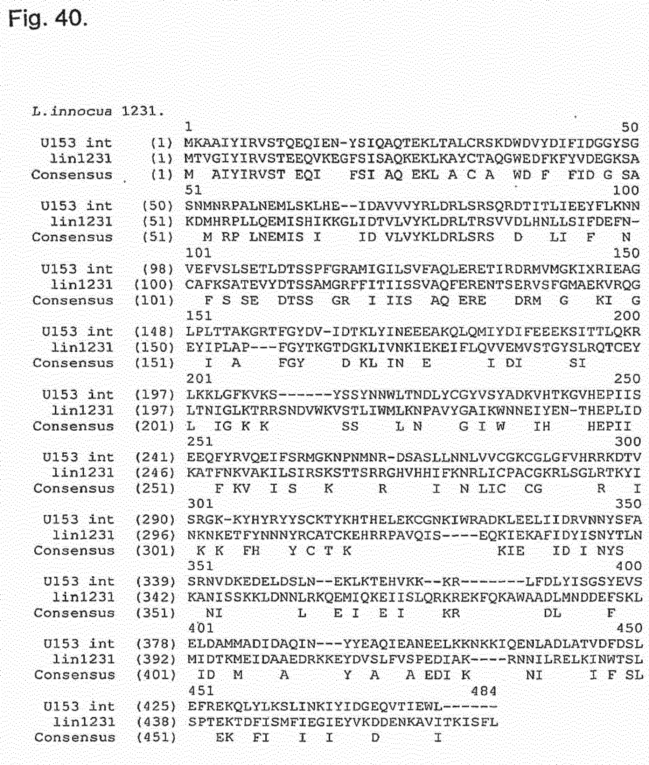

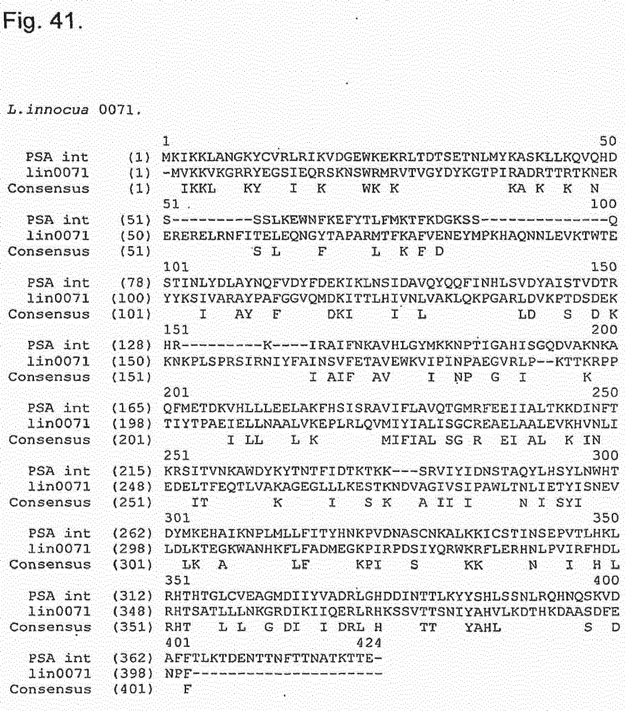

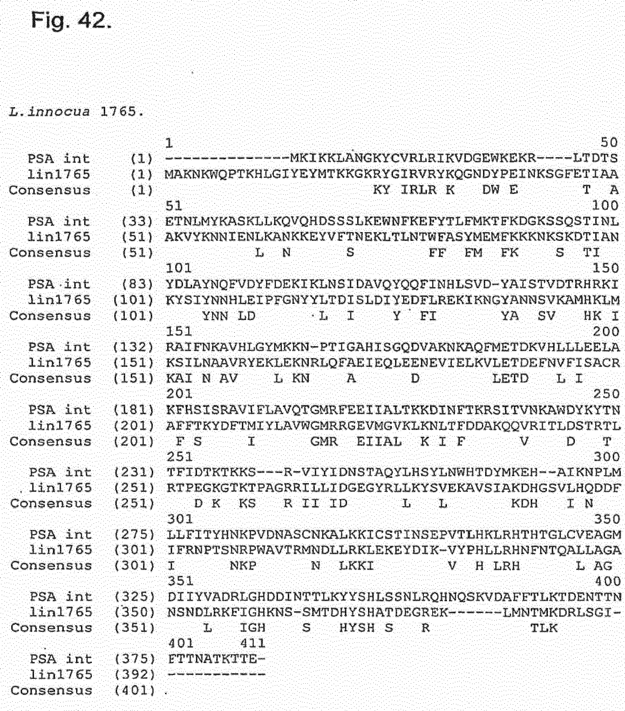

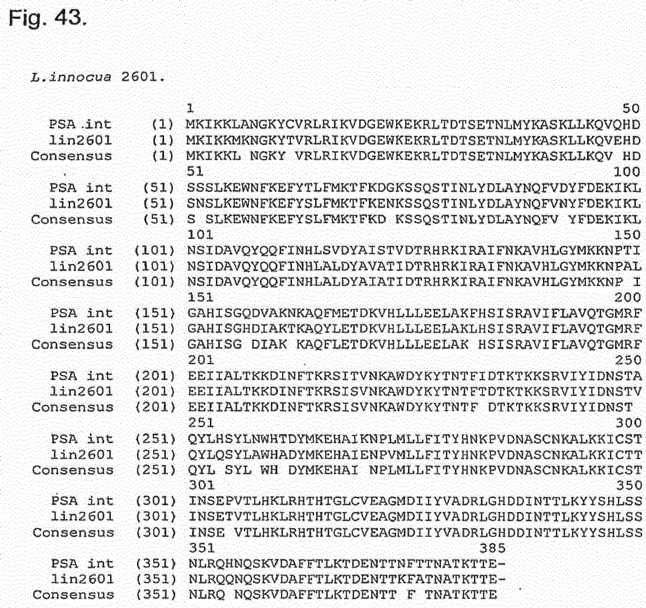

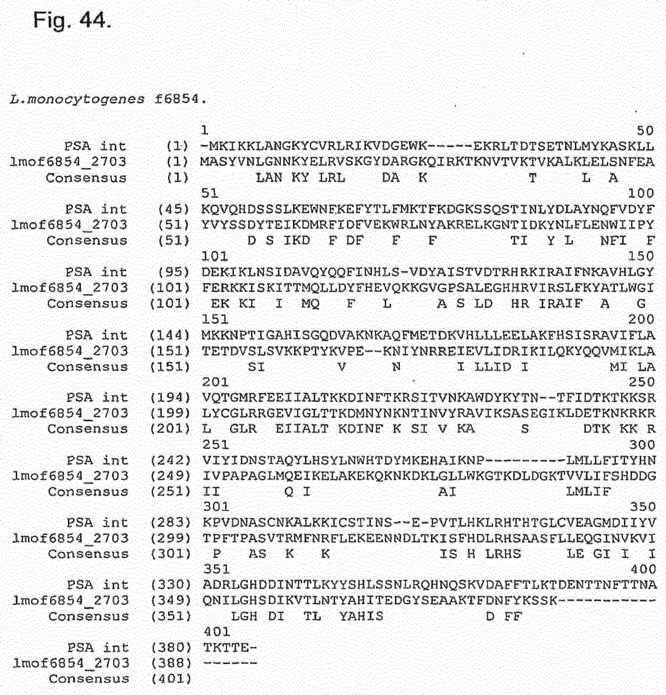

Figure 40, Figure 41, Figure 42, Figure 43, and Figure 44, disclose the amino acid sequences of some of the phage integrases of the present disclosure. What is encompassed is polynucleotides encoding these phage integrases, nucleic acids that hybridize under stringent conditions to these polynucleotides where the nucleic acids encode functional phage integrases. Also encompassed are other polynucleotides that are bracketed by a pair of PCR primers, where the pair of PCR primers corresponds exactly to two positions of a polynucleotide encoding a phage integrase of the present disclosure.

Provided are nucleic acids encoding the following phage integrases, the phage integrase polypeptides, nucleic acids encoding relevant phage attachment sites (attPP') and nucleic acids encoding corresponding bacterial attachment sites (attBB'). The present disclosure encompasses the following integrases: (1) *L. innocua* 0071 integrase; (2) *L. innocua* 1231 integrase; (3) *L. innocua* 1765 integrase; (4) *L. innocua* 2610 integrase; and *(5) L. monocytogenes* f6854_2703 integrase.

Identification of a nucleic acids encoding integrases, attPP' sites, and attBB' sites, was according to the following multi-step procedure. Candidate nucleic acid sequences were initially acquired, and homologies can be identified, using, e.g., the protein or nucleotide BLAST feature on the world wide web at ncbi.nlm.nih.gov, and using the completed microbial genomes feature on the world wide web at tigr.org.

Step 1. Novel phage integrase sequences were identified as follows. Nucleic acids of a known phage integrase were used to search for a similar sequence in a listerial genome, where the listerial genome harbors a prophage. The known phage integrases sequences used at this step of the search were those encoding PSA integrase and U153 integrase.

Step 2. Once a nucleic acid encoding a new phage integrase is identified, review the DNA 3-prime to the nucleic acid encoding the integrase for the appearance of an attachment site. The attachment site typically takes the form of a hybrid of the phage attachment site and the bacterial attachment site (attPB'). The attachment site takes the form of this hybrid because the phage has integrated itself into the listerial genome.

Step 3. Regions of the listerial genome containing a putative attPB' site were compared with the corresponding region of another listerial strain or listerial species, where this other listerial strain or species is not expected to contain an integrated phage. The crossover point (crossover point in between phage sequence and bacterial sequence in attPB') takes the form of a discontinuity. The crossover point can occur in an open reading frame or in an intergenic region.

Step 4. The sequence of nucleotides residing immediately downstream from (immediately 3-prime end of) the integrase gene, and upstream to the crossover point, is identified as phage-derived sequence, and constitutes "a first half" of the phage attachment site.

Step 5. The "second half" of the phage attachment site can be identified by reviewing the nucleic acid sequences residing upstream to (5-prime to) the integrase gene, comparing with the corresponding regions of a listerial strain or species expected not to contain any integrated phage (no integrated phage in the genomic region of interest), and identifying a region of discontinuity. The combination of the first half of the phage attachment site and the second half of the phage attachment site is attPP'.

Step 6. Phage attachment sites and bacterial attachment sites typically contain a region of identity, for example, of between three to 10, 20, 30, or more nucleotides. A region of identity can help in finding the general location of the phage attachment site and bacterial attachment site.

Step 7. Where the listerial species of interest is a species other than *L. monocytogenes,* e.g., *L. innocua,* the identified bacterial attachment site in the *L. innocua* genome can be used as a computer-probe to search the *L. monocytogenes* genome for homologous sequences. The result of this search of the *L. monocytogenes* genome where the result of the probe will be the bacterial attachment site (attBB').

Step 8. Where the region of identity is relatively long, e.g., 40-50 nucleotides, this region of identity can constitute the entire phage attachment site (attPP') and entire bacterial attachment site (attBB').

Most site-specific integrases are of the tyrosine recombinase family or serine recombinase family. About 100 phage-encoded integrase genes have been identified. These genes, encoded by the phage genome, can be found in the phage genome and/or also with a bacterial genome after integration of the phage into the bacterial genome.

The serine recombinases have a catalytic domain at the N-terminus, which includes a number of invariant residues, including Arg-8, Ser-10, and Arg-68. The N-terminal catalytic domain is followed by a region of about 220 amino acids, which contains at least ten conserved residues (including three cysteines). This region is followed by about 125 amino acids on non-conserved residues, by a 30-amino acid region rich in Leu, Ile, Val, and/or Met, and finally a C-terminal tail of 4-200 amino acids in length (see, e.g., Smith and Thorpe (2002) Mol. Microbiol. 44:299-307; Nunes-Darby, et al. (1998) Nucleic Acids Res. 26:391-406; Esposito and Scocca (1997) Nucleic Acids Res. 25:3605-3614).

Phage integrases of the tyrosine recombinase family can be identified by a conserved R-H-R-Y motif. The R-H-R-Y motif is a hallmark for the integrase family of recombinases. The histidine (H) can be substituted by arginine, lysine, asparagine, or tyrosine. In phage lambda integrase, for example, the amino acids of the R-H-R-Y motif occur at amino acids R212, H308, R311, and Y342 (see, e.g., GenBank Acc. No. P03700) (Nunes-Duby, *et al.,* supra). Phage integrases are further identified by Box I (see,e.g., A202-G225 of phage lambda integrase), Box II (see, e.g., T306-D344 of phage lambda integrase), and by certain motifs occurring before or between Box I and Box II. Box II can include the consensus sequence LLGH, where the glycine (G) can be replaced by A, S, or T (SEQ ID NO: 137) (Nunes-Duby, *et al.,* supra). In addition to the Box I motif and Box II motif, three "patches" of conserved sequences occur in prokaryotic integrases, such as phage integrases. Patch I is upstream of Box I, and has the consensus sequence LT-EEV--LL (SEQ ID NO:88). In phage lambda integrase, Patch I has the sequence LTADEYLKIY (SEQ ID NO:87) (amino acids 180-189 of GenBank Acc. No. P03700). Patch II is lysine (K235 of phage lambda integrase) flanked on both sides by serine, threonine, glycine, or methionine. In phage lambda integrase, Patch II occurs as SKT, while in Cre recombinase Patch II occurs as TKT, and in XerD recombinase it occurs as GKG. Patch III, which occurs between Boxes I and II, is [D,E]-[F,Y,W,V,L,I,A]₃₋₆[S,T] (SEQ ID NO:89, 138, 139, 140). In phage lambda integrase, Patch III occurs at amino acids 269-274 (Nunes-Duby, *et al.,* supra). In using a candidate phage integrase sequence as a query sequence, for comparison with established phage integrase sequences, it might be useful to introduce a gap or extension to bring Box I and Box II into alignment.

The conserved R-H-R-Y motif (Table 16) resides in the phage integrases of the present invention. The positions were determined by manual inspection. Esposito and Scocca provide additional conserved sequences within Box I (a.k.a. Box A) and Box II (a.k.a. Box B) (Esposito and Scocca (1997) Nucleic Acids Res. 25:3605-3614). Esposito and Scocca disclose that that Arginine (in Box I (Box A) of the R-H-R-Y motif) resides in the following context: TGL**R**XTEL (SEQ ID NO:91), and that the histidine and the second arginine (in Box II (Box B) of the R-H-R-Y motif) reside in the following context: **H**XL**R**HAXATXLXXXG (SEQ ID NO:90). The histdine (H) and second arginine (R) of the R-H-R-Y motif is bolded and underlined. Sequences corresponding to these two contexts can readily be found, by manual inspection, in Boxes I and II of *L. innocua* 0071. Esposito and Scocca place the Tyrosine (Y) of the R-H-R-Y motif in a motif identified as Box C, where the Box C of Esposito and Scooca is: VXXXLGHXXXXXTXX**Y**XH (SEQ ID NO:92). The Y of the of R-H-R-Y motif is bolded and underlined. Inspection of the *L. innocua* 0071 integrase sequence demonstrates that the Box C consensus sequence resides in *L. innocua* 0071 integrase of the present invention.

Inspection reveals that Esposito and Scocca's Box B and Box C exists in *L. innocua* 1765 integrase of the present invention. Furthermore, inspection demonstrates that Esposito and Scocca's Box A resides in *L. innocua* 2601 integrase of the present invention. In addition, inspection of the *L. monocytogenes* f6854_2703 integrase sequence shows the occurrence of Box A, B, and C. Taken together, the consensus sequences of Nunes-Duby, *et al., supra,* and of Esposito and Scocca, *supra,* confirm the identified sequences as phage integrases. Inspection of PSA phage integrase sequence reveals motifs similar to Esposito and Scocca's Boxes A, B, and C.

*L. innocua* 1231 integrase of the present can be identified as a serine recombinase. Yang and Steitz disclosed a number of invariant motifs, and conservatively substituted motifs, of the serine recombinase family (Yang and Steitz (1995) Cell 82:193-207). The YxRVSTxxQ (SEQ ID NO:93) motif of Yang and Steitz occurs in *L. innocua* 1231 integrase. Also, the VLVxxLDRLxR (SEQ ID NO:141) motif of Yang and Steitz can be found in *L. innocua* 1231 integrase. Furthermore, Yang and Steitz's VAQAERxxxxERxxxG (SEQ ID NO:94) motif is found in *L. innocua* 1231 integrase of the present disclosure.

**Table 16. Conserved R-H-R-Y motifs in phage integrases.**

| | Arginine (R) | Histidine (H) | Arginine (R) | Tyrosine (Y) |
|---|---|---|---|---|
| *L. innocua* 0071 integrase. | 382 | 595 | 598 | 631 |
| *L. innocua* 1765 integrase. | 241 | 334 | 337 | 369 |
| *L. innocua* 2601 integrase (90.9% identical to PSA integrase). | 199 | 309 | 312 | 344 |
| *L. monocytogenes* f6854_2703 integrase. | 204 | 328 | 331 | 364 |
| Lambda phage. GenBank Acc. No. P03700. | 212 | 308 | 311 | 342 |
| PSA phage. GenBank Acc. No. CAC85582. | 199 | 309 | 312 | 344 |

| | |
|---|---|
| *L. innocua* 0071 integrase. Coding sequence plus Shine Dalgarno and terminator. See, e.g., GenBank Acc. No. AL596163.1 (Segment 1/12). (SEQ ID NO:95) | |
| Coding sequence only of integrase, | |
| | |
| *L. innocua* 0071. (SEQ ID NO:96) | |
| *L. innocua* 0071 integrase amino acid sequence. (SEQ ID NO:97) | |
| | |
| *L. innocua* 0071. | |
| | |
| Bacterial attachment site (between *L. mono-cytogenes* f2365_0095 *& L. mono-cytogenes* f2365_0096, in the tRNA-lys gene (attachment site underlined). (SEQ ID NO:98) | |
| *L. innocua* 0071 phage attachment site. (Common sequence between phage and chromosome (attP and attB)). (SEQ ID NO:99) | ACTCTTAATCAGCGGGTCGGGGGTTCGAAACCCTCACAACCCATA |
| *L. innocua* 1231 integrase nucleic acid sequence. | |
| | |
| *L. innocua* Clip11262 complete genome GenBank Acc. No. AL596168.1 (segment 6/12 nucleotides 29,995 to 28,563). (SEQ ID NO:100) | |
| *L. innocua* 1231 integrase amino acid sequence. (SEQ ID NO:101) | |
| *L. innocua* 1231 phage attachment site attPP'. This site resides in *L. mono-cytogenes* strain 4bF2365 (complement to 2495122 to 2495193), and is essentially the same as a sequence found in *L. mono-cytogenes* strain EGD (nt 145171 to 145423 of GenBank Acc. No. AL59I983.1 segment 11/12). (SEQ ID NO:1 02) | |
| *L. innocua* 1231 attachment site attBB' within *L. mono-cytogenes* 1263: (SEQ ID NO:103) | |
| *L. innocua* 1765 integrase. See also *L. innocua* Clip11262 complete genome, segment 7/12 (nucleotide 210,321 to 211,089). (SEQ ID NO:104) | |
| *L. innocua* 1765 integrase amino acid sequence. (SEQ ID NO:105) | |
| *L. innocua* 1765 Phage attachment site. (SEQ ID NO:106) | |
| *L. innocua* 1765. bacterial attachment site. This sequence resides in *L. monocytogenes* EGDe (complete genome) GenBank Acc. No. AL591824 at nt 1,705,630 to nt 1,706 ,203. Similar sequences occur in *L. mono-cytogenes* strain 4bF2365 (nt 216008 to 216262 of section 6) and in *L. innocua* Clip 11262 nt 77369 to 77270. (SEQ ID NO:107) | |
| *L. innocua* 2610. Integrase gene from *L. innocua.* The present invention also provides the nucleic acid and polypeptide of *L. innocua* Clip11262 complete genome segment 11/12 GenBank Acc. No. AL596173.1 (nucleotides 14,676 to 15,804). (SEQ ID NO:108) | |
| *L. innocua 2610* integrase, amino acid sequence (90.9% identical to PSA integrase). (SEQ ID NO:109) | |
| | |
| *L. innocua* 2610. This sequence is an attBB' site from *L. innocua.* Attachment site (tRNA-ArgS gene plus surrounding sequences, integrates *Listeria innocua* strain). Core attachment site in **bold (atgccctcggaggga).** (SEQ ID NO:110) | |
| Core attachment site (in **bold**). (SEQ ID NO:111) | **atgccctcggaggga** |
| This sequence is an attBB' site from *L. monocytogenes* f2365. Attachment site of non-integrated strain (*L. mono-cytogenes* F2365; attachment site in tRNA-Arg5 gene underlined. Core att site is in **BOLD (atgccctcggaggga).** (SEQ ID NO:112) | |
| *L. monocytogenes* f6854_2703 integrase - 2680803: 2681963 (Most of this sequence is available at tigr.org). (SEQ ID NO: 113) | |
| *L. monocytogenes* f6854_2703 integrase 2,680,803: 2,681,963. (SEQ ID NO: 114) | |
| *L. monocytogenes* f6854_2703. Phage attachment site. (SEQ ID NO:115) | |
| Phage attachment site (attPP'). Phi6854.3 attachment site is within the tRNA-Thr-4 gene Phage attachment site highlighted in **bold** and underlined, and is annotated as a phage attachment site in the F2365 genome (Nelson, et al. (2004) Nucleic Acids Res. 332:2386-2395). (SEQ ID NO:116) | |
| Phage (attPP') Phi6854.3 attachment site (same as above) is within the tRNA-Thr-4 gene, where the tRNA-Thr-4 gene is shown outlined in a box. (SEQ ID NO:117) | |
| Bacterial (attBB') Phi6854.3 attachment site is within the tRNA-Thr-4 gene Phage attachment site highlighted in bold and underlined, and is annotated as a phage attachment site in the F2365 genome (Nelson, *et al., supra*). tRNA-Thr-4 gene is 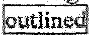 . (SEQ ID NO:118) | |
| Nucleic acid sequences can be found on the world wide web at tigr.org and, clicking: | |
| (1) Comprehensive microbial resources; | |
| (2) Searches; | |
| (3) CMR BLAST; and | |
| (4) inputting a listerial integrase sequence as a query sequence. | |
| If an accession number is known, a sequence can be found on the world wide web at tigr.org, by clicking: | |
| (1) Comprehensive microbial resource; | |
| (2) Genomes; | |
| (3) *Listeria monocytogenes* 1/2a F6854; | |
| (4) Searches; | |
| (5) Locus; | |
| (6) typing "LMOf6854_2703" in the box; and | |
| (7) clicking at TIGR sequences on the sidebar. | |

A phage attachment site (attPP') or bacterial attachment site (attBB') of the present invention can be implanted into a polynucleotide by way of site-specific recombination, homologous recombination, by use of restriction sites, by methods of synthetic organic chemistry, or by other methods. In particular, where homologous recombination is used, an attBB' site can be implanted into a virulence gene, where integration results in a simple insertion or, alternatively, in insertion with deletion of a corresponding region of the virulence gene.

Thus, the present disclosure provides methods for implanting a phage attachment site (attPP') into a plasmid. Provided are methods for implanting a bacterial attachment site (attBB') into a plasmid, as well as downstream methods where the plasmid can later be used to transfer the attBB' into a bacterial genome. In one aspect, the plasmid contains a first nucleic acid encoding an attPP' site and a second nucleic acid encoding a heterologous antigen. In this case, this disclosure contemplates methods for incorporating the second nucleic acid into an attBB' site residing in a target polynucleotide, where the target polynucleotide can be a bacterial genome.

The target polynucleotide of site-specific recombination, homologous recombination, or engineering by using restriction sites, is not to be limited to virulence genes, but also encompasses without limitation any polynucleotide, plasmid, episome, extrachromosomal element, bacterial genome, listerial genome, genome of *Bacillus anthracis,* or genome of *Francisella tularensis.*

The present disclosure encompasses a nucleic acid encoding a phage integrase, an attPP' site, or an attBB' site, where the nucleic acid can hybridize under stringent condition to one of the nucleic acids claimed as part of the present disclosure, that is, to one of the nucleic acids encoding a phage integrase, attPP' site, or attBB site, and where the hybridizing polynucleotide can encode a functional phage integrase, attPP' site, or attBB' site.

Also encompassed is a nucleic acid derived from a polymerase chain reaction (PCR), where the pair of PCR primers matches exactly and brackets a functional region of one of the nucleic acids of the present disclosure, disclosed herein, encoding a phage integrase, attPP' site, or attBB site. The PCR reaction can be carried out in silico. The present disclosure encompasses a nucleic acid derived from the PCR reaction, where the nucleic acid encodes a functional phage integrase, attPP' site, or attBB' site. The PCR primers can be designed to bracket the entire nucleic acid encoding the phage integrase, attPP' site, or attBB' site, disclosed herein, or they can be designed to bracket a shorter, functionally active, part of the nucleic acid.

### EXAMPLE IX. Reagents and methods used for Examples X and XI

The heterologous antigen in recombinant *L. monocytogenes* (Lm) strains can be alternatively constructed to contain the nucleotide sequence encoding the eight amino SIINFEKL (SEQ ID NO:142) peptide (also known as SL8 and ovalbumin₂₅₇₋₂₆₄), positioned in-frame at the carboxyl terminus of an ActAN100-PSCA fusion protein. Compositions such as the SL8 epitope serve as a surrogate to demonstrate the ability of recombinant *L. monocytogenes* vaccine strains to be taken up by antigen presenting cells and subsequently program presentation of antigens via the MHC class I pathway, using an *in vitro* antigen presentation assay.

This presentation assay can be performed using the cloned C57BL/6-derived dendritic cell line DC2.4 together with the B3Z T cell hybridoma cell line. The DC2.4 line was generated from bone marrow-derived DC that were subsequently transformed with the oncogenes *myc* and *raf.* DC2.4 cells have been shown previously to present exogenously added antigen by both MHC class I and class II pathways. Presentation of peptide by DC2.4 cells on class I molecules following phagocytosis of recombinant *L. monocytogenes* was measured after incubation with B3Z cells. B3Z is a lacZ-inducible CD8⁺ T-cell hybridoma specific for the SL8 epitope presented on the murine K^{b} class I molecule. Class I-restricted presentation of SL8 to B3Z cells results in the induction of beta-galactosidase synthesis by B3Z. The amount of beta-galactosidase produced can be measured by the hydrolysis of the chromogenic substrate CPRG and is an indication of the amount of SL8 / K^{b} complexes presented on the surface of antigen presenting cells. Thus, the *in vitro* antigen presentation assay is a direct measure of the ability of recombinant *L. monocytogenes* strains utilizing any predetermined ActAN100-heterologous antigen fusion protein to express and secrete the antigen fusion protein within infected host cells, leading to appropriate processing and presentation on MHC molecules and stimulation of antigen-specific T lymphocytes (Dubensky, et al., Pat. Appl. Publ. No. US 2004/0228877).

Alternatively, the ability of recombinant *L. monocytogenes* strains utilizing any selected ActAN100-heterologous antigen composition to express and secrete the antigen fusion protein within infected host cells can be measured by Western Blot analysis of recombinant *L. monocytogenes*-infected host cell lysates, using an antibody that is specific for the ActAN100 N-terminal region fusion partner. The anti-ActA antibody used in this Example was a rabbit polyclonal antibody raised against a synthetic peptide (ATDSEDSSLNTDEWEEEK (SEQ ID NO:143)) corresponding to the mature N-terminal 18 amino acids of ActA (Mourrain, et al. (1997) Proc. Natl. Acad. Sci. USA 94:10034-10039).

The following lysis buffer was used to extract *L. monocytogenes*-encoded proteins that had been expressed and secreted within a mammalian host cell, e.g., into the cytosol of the mammalian host cell. Lysis buffer was prepared in a total volume of 50 ml, and contained 2.5 ml 1 M Tris HCl, pH 6.8; 10 ml 10% sodium dodecylsulfate; 5 ml glycerol; 0.1 g bromophenol blue; and 5 ml 1 M dithiothreitol. Extraction was with heating at 100° for 5 min.

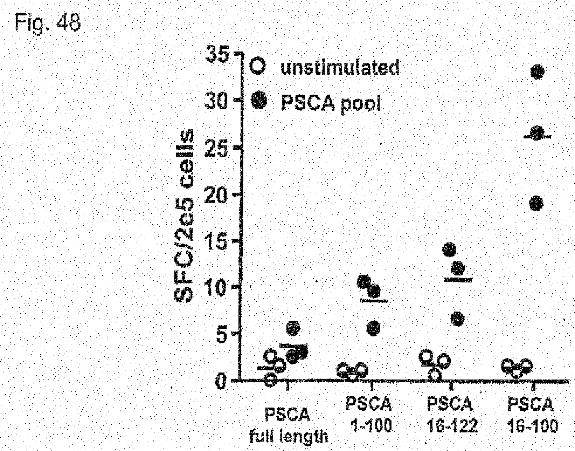

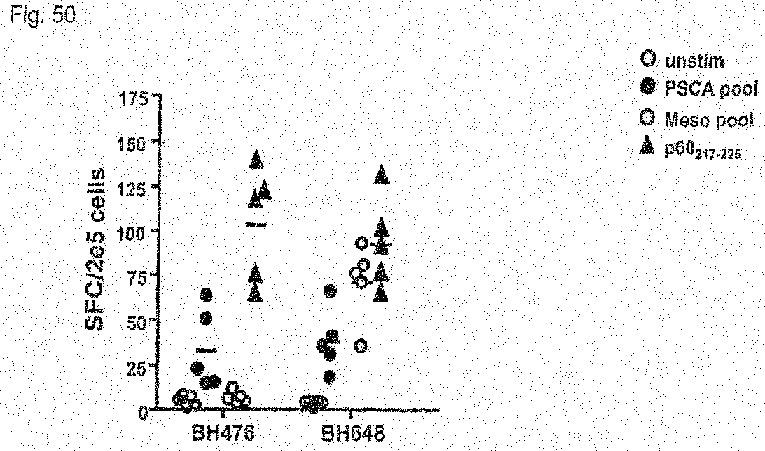

Immune response in mice vaccinated with recombinant *L. monocytogenes* strains was assessed as follows. Elispot assays were used to assess immune responses that had been generated in mice inoculated with *L. monocytogenes* vaccine constructs. In short, Balb/c mice were inoculated with the indicated *L. monocytogenes* strain (5 x 10⁶ cfu). After one week, spleens were harvested, and the isolated splenocytes (2 x 10⁵ splenocytes) were incubated overnight with or without the indicated peptides. The overnight incubations were conducted in wells coated with immobilized anti-interferon-γ antibody. The immobilized antibody served to capture any secreted IFN-γ, thus permitting subsequent measurement of secreted IFN-γ, and assessment of the immune response to the vaccine. To view the overall picture, immune responses generated in the mouse were visualized in the *in vitro* splenocyte IFN-γ expression assays. In Figure 48, the peptide pool spanned the amino acid sequence of human PSCA. In Figure 50, the added peptides were either the PSCA pool, the mesothelin pool, or a single peptide corresponding to an epitope of the listerial protein, p60. The p60 peptide was p60₂₁₇₋₂₂₅ (KYGVSVQDI(SEQ ID NO:144)).

In Elispot assays, the PSCA peptide pool was a pool of 28 peptides, where the 28 peptides spanned the length of human PSCA. Each peptide was a 15-mer, where adjacent peptides overlapped each other by 11 amino acids. The concentration of each peptide was 1 microgram/ml.

The Mesothelin peptide pool was a pool of 153 different peptides spanning the entire sequence of human Mesothelin, where succeeding peptides overlapped by 11 amino acids. The concentration of each peptide was 1 microgram/ml. Each peptide was a 15-mer.

### EXAMPLE X. Construction and Immunogenicity of recombinant L. monocytogenes strains encoding Prostate Stem Cell Antigen (PSCA)

The construction and immunogenicity of recombinant *L. monocytogenes* containing an expression cassette encoding an ActAN100-Prostate Stem Cell Antigen (PSCA) based fusion protein is yet a further non-limiting example of the utility of the compositions and methods set forth in this invention. The Examples disclose expression and secretion of heterologous antigens from recombinant *Listeria* within infected mammalian host cells, leading to a cellular immune response specific for the antigen.

Prostate stem cell antigen (PSCA) is a cell surface antigen associated with human cancer. A number of PSCA sequences and PSCA-derived sequences are shown below. What is disclosed is the amino acid sequence of human PSCA; the nucleic acid sequence of codon optimized PSCA (codon optimized for expression in *Listeria monocytogenes*), where the PSCA was deleted in its signal sequence and the GPI-anchor sequence; the amino acid sequence encoded by this codon optimized nucleic acid sequence; and the amino acid sequence of an ActAN100-human PSCA fusion protein, modified to contain a standard immunogenic peptide (SIINFEKL (SEQ ID NO:142)). GPI-anchor sequence refers to a sequence of amino acids occurring at the C-terminus of PSCA for post-translational modification by glycosylphosphatidylinositol (GPI).

Human PSCA amino acid sequence (SS & GPI in *italics*):

Codon-optimized PSCA, BamHI-SpeI delta SS, delta GPI (amino acids 18-101):

Translation of codon-optimized PSCA BamHI-SpeI delta SS, delta GPI (GS and TS are BamHI and SpeI restriction sites, respectively):

ActAN100-hPSCA 18-101-SIINFEKL (predicted sequence based on nucleic acid sequence, the first amino acid is Valine). (PSCA sequence is underlined):

ActAN100-hPSCA 18-101-SHNFEKL, (the first amino acid is methionine) (Actual sequence expressed in *Listeria monocytogenes* when first amino acid is the amino-terminal amino acid (PSCA sequence is underlined):

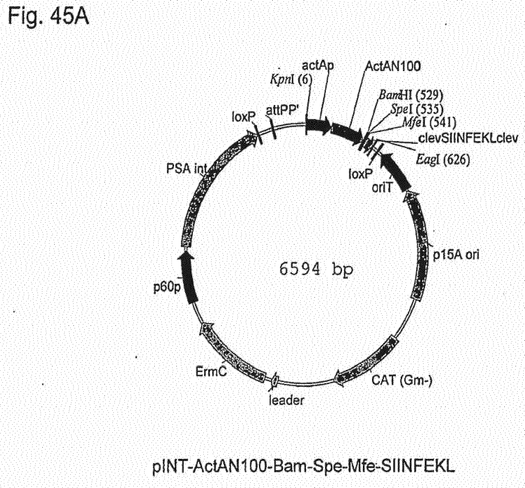

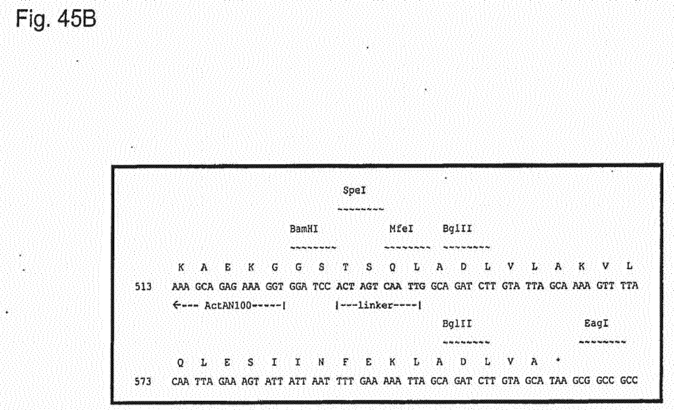

Figure 45 discloses the schematic configuration of the vector, pINT-ActAN100-BamHI-SpeI-MfeI-SIINFEKL plasmid DNA. This vector contains an attPP' site, which mediates site-specific integration into a nucleic acid that contains a corresponding attBB' site. The vector also contains an actA promoter, actA-N100, a cloning site containing a number of restriction sites, and a SIINFEKL (SEQ ID NO: 142) sequence. The vector also encodes antibiotic resistance genes (ErmC and CAT), which are flanked by loxP sites, where the loxP sites facilitate Cre-recombinase catalyzed elimination of the antibiotic resistance genes. Elimination of these genes is useful, for example, after the vector has been integrated into a bacterial genome, and after isolation of the bacterium with the successful integration.

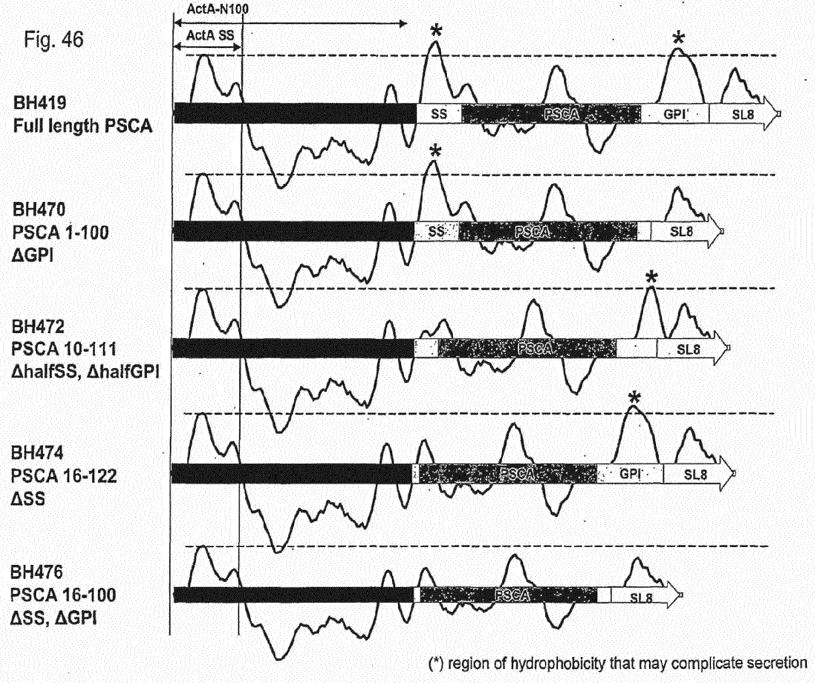

Figure 46 discloses the schematic configuration of PSCA molecular constructs with a Kyte-Doolittle overlay. The diagrams include the following domains, namely, actA signal sequence (actA SS); actA-N100; PSCA signal sequence; PSCA; the PSCA GPI anchor region, and SIINFEKL ((SEQ ID NO:142) abbreviated as "SL8"). As indicated in the Figure, "delta half SS, delta half GPI" refers to the PSCA SS with half of this sequence deleted, and the PSCA GPI anchor with half of this sequence deleted. Amino acids 1-9 of the signal sequence were deleted, while amino acids 112-123 of the GPI anchor had been deleted.

The following concerns the terminology in Figure 46. The diagram separately identifies ActA SS and ActA-N100. ActA-N100 includes, within it, an ActA signal sequence. However, the amino acid sequence of ActA signal sequence was not duplicated in the constructs, and the separate identification of ActA SS and ActA-N100 is only for convenience. In this diagram, the notation PSCA means full length PSCA, but without the PSCA SS and without the PSCA GPI anchor. Thus, where the diagram shows that a construct contains both "SS" and "PSCA," there exists only one PSCA signal sequence in the construct.

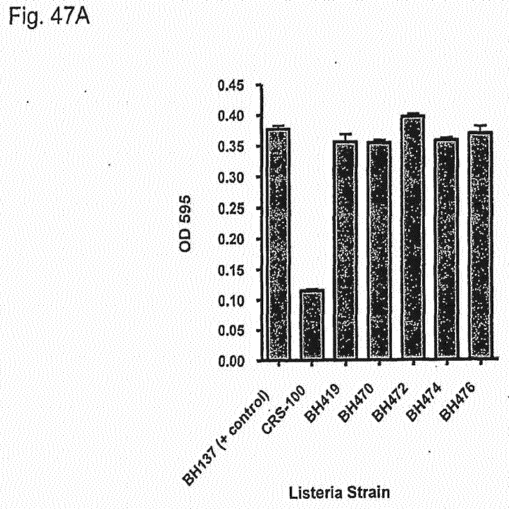

Figure 47A discloses response of a reporter T cell line (B3Z cells) to peptides presented by dendritic cells. The bar graph on the left demonstrates that heterologous ActA-N100-PSCA fusion proteins encoded by recombinant *L. monocytogenes,* were expressed and secreted within infected host dendritic cells, and subsequently processed and presented on MHC molecules by the dendritic cells. Various recombinant *L. monocytogenes* were used to infect DC2.4 cells, as indicated in the Figure. With infection, the recombinant *L. monocytogenes* expressed and secreted the polypeptides within the DC2.4 cells. The DC2.4 cells presented peptides, where detection of presented peptides was by way of a reporter T cell hybridoma line (B3Z T cell hybridoma). CRS-100 is a *L. monocytogenes* strain with deletions in both the actA and inlB genes (Lm ΔactAΔinlB). The strain CRS-100 is not engineered to express any heterologous antigen, though it can be modified to contain further mutations or to express heterologous antigens.

**Table 17. Listeria monocytogenes strains expressing ActA-N100-PSCA fusion proteins**

| **Strain** | **Construct** | **Background** |
|---|---|---|
| BH137 | pPL2_ActAN100-AH1A5-OVA | CRS-100 |
| CRS-100 | | |
| BH419 | p221_ActAN100_PSCA-fl_SL8 | CRS-100 |
| BH470 | p221_ActAN100_PSCA-1-100_SL8 | CRS-100 |
| BH472 | p221_ActAN100_PSCA-10-110_SL8 | CRS-100 |
| BH474 | p221_ActAN100_PSCA-16-122_SL8 | CRS-100 |
| BH476 | p221_ActAN100_PSCA-16-100_SL8 | CRS-100 |

Figure 47A discloses results from the B3Z T cell assays. Various *L. monocytogenes* strains (2 x 10⁷ cfu) were incubated for one hour with 100,000 DC2.4 cells at 37°, to allow infection of the DC2.4 cells. After the one-hour incubation, 100,000 B3Z cells were added, together with gentamicin at a final concentration of 0.1 mg/ml. The final volume was 0.2 ml/well. The mixture was incubated overnight, followed by collection of all cells by centrifugation, lysis of cells, and assay of the beta-galactosidase expressed by the reporter B3Z T cell line. The results were a function of a number of steps, for example, bacterial expression and secretion, and the DC2.4 cell's presentation of the SL8 peptide by way of MHC Class I. Similar results were found with all of the indicated recombinant *L. monocytogenes* that encoded the SL8 epitope.

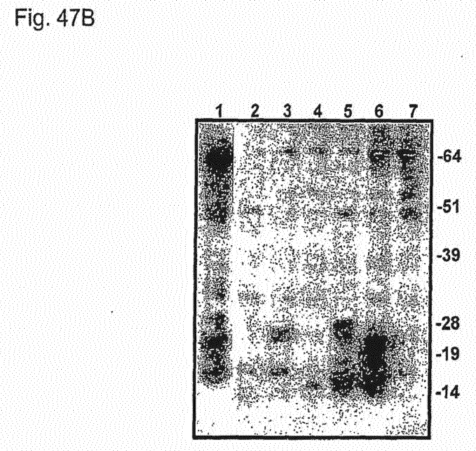

The Western blot (Figure 47B) demonstrates that recombinant *L. monocytogenes* infected J774 macrophage cells express and secrete the encoded heterologous ActA-N100-PSCA fusion proteins. The Western blot discloses results from recombinant *L. monocytogenes* infections of J774 cells. J774 macrophages were infected with recombinant *L. monocytogenes,* where the figure identifies each of the recombinant *L. monocytogenes* strains. J774 cells (1 x 10⁶ cells) were infected with *L. monocytogenes* (50 x 10⁶ cfu), at a multiplicity of infection of 50. The mixture was incubated for 30 mint, washed, then supplemented with gentamicin and incubated for 5-7 hours at 37°. After this incubation, the J774 cells were lysed, using lysis buffer, in order to release the expressed and secreted ActA-N100-PSCA fusion protein, while leaving the bacteria intact.

The Western blot demonstrates that the delta SS, delta GPI construct was expressed and secreted most efficiently (lane 6). The SS and GPI anchor domains contain regions of hydrophobicity that may complicate secretion. The invention provides antigen constructs deleted in SS and GPI domains.

**Table 18. Legend for Figure 47B. Western blot analysis for expression and secretion of ActA-N100-PSCA fusion proteins**

| **lane** | **strain** | **antigen** |
|---|---|---|
| 1 | hMeso26 | ActAN100-hMesoΔSS, ΔGPI |
| 2 | BH419 | ActAN100-PSCAfl_SL8 |
| 3 | BH470 | ActAN100-PSCA 1-100_SL8 |
| 4 | BH472 | ActAN100-PSCA 10-110_SL8 |
| 5 | BH474 | ActAN100-PSCA 16-122_SL8 |
| 6 | BH476 | ActAN100-PSCA 16-100_SL8 |
| 7 | CRS-100 | - |

The expected molecular weight of the polypeptide expressed from *L. monocytogenes* strain BH419 (actA-N100-full length PSCA-SL8) is 24 kDa. The expected molecular weight for the polypeptide expressed from *L. monocytogenes* BH476 (actA-N100-PSCA 16-100-SL8) is 20.3 kDa.

*L. monocytogenes* strain BH476 produced the greatest levels of expression and secretion as demonstrated by the gel, where the gel revealed dense staining of polypeptides in a molecular weight range in the vicinity of the 19 kDa molecular weight marker.

Figure 48 discloses vaccine-induced PSCA-specific T cell immune responses in mice vaccinated with various recombinant *L. monocytogenes* strains expressing ActA-N100-PSCA fusion proteins.

Balb/c mice were inoculated with the indicated recombinant *L. monocytogenes* strain. After one week, spleens were harvested and the isolated splenocytes were incubated overnight with or without the indicated peptides, in Elispot assays. Incubations were in the presence or absence of the PSCA peptide pool. The results demonstrate that recombinant *L. monocytogenes* encoding full length PSCA induced the lowest immune response, while Lm-PSGA-ΔSS-ΔGPI (Lm-PSCA-16-100) induced the highest immune response (Figure 48). These results demonstrate that the extent of expression and secretion of the ActA-N100-PSCA fusion protein by recombinant *L. monocytogenes* within infected host cells is directly correlated with its immunogenicity in vaccinated animals. This correlation can be observed by comparing the Western blots with the Elispot results.

### EXAMPLE XI. Construction and immunogenicity of recombinant bivalent L. monocytogenes strains encoding ActA-N100-Prostate Stem Cell Antigen (PSCA) and ActA-N 1 00-Mesothelin.

It is well known in the art that selected tumors express several antigens that are related to the tumor, and that inducing cellular immune responses to more than one of these tumor associated antigens can enhance the potency of a selected cancer vaccine. This disclosure provides compositions and methods for recombinant *L. monocytogenes* vaccine strains that express and secrete more than one heterologous antigen, where vaccination leads to the induction of cellular immunity in vaccinated animals to each of the expressed heterologous antigens. Example XI discloses, as a non-limiting example, the utility of compositions and methods of recombinant bivalent *L. monocytogenes* strains encoding two distinct ActAN100-heterologous antigen fusion proteins.

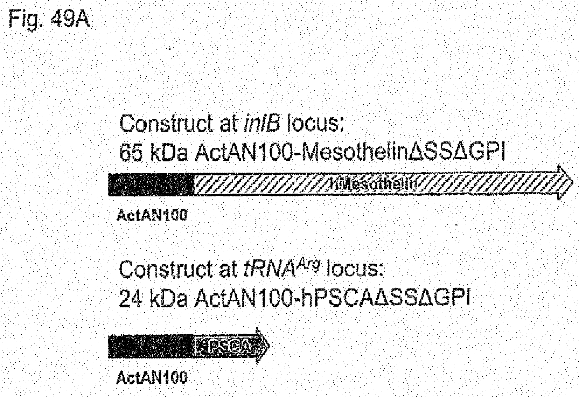

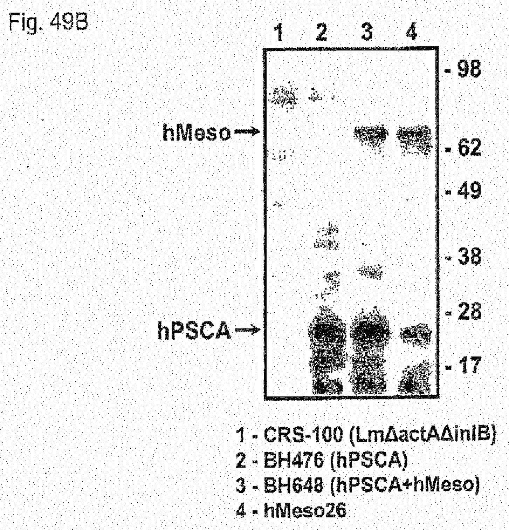

Figures 49A and 49B disclose a recombinant bivalent *L. monocytogenes* vaccine strain expressing two different human cancer antigens, Mesothelin and PSCA, each antigen taking the form of an ActA-N 100 fusion protein. Utility was demonstrated by the induced T cell immunity, where T cell immunity was induced against each expressed and secreted antigen in animals vaccinated with the bivalent vaccine.

The schematic shown in Figure 49A shows a first fusion protein of actA-N100 and Mesothelin ΔSSΔGPI, and a second fusion protein of actA-N100 and PSCAΔSSΔGPI. The diagram identifies the *L. monocytogenes* internalin B gene (*inlB* gene) as the site of integration of a first nucleic acid encoding the first fusion protein, and the *L. monocytogenes* tRNA^{Arg} locus as the site of integration of a second nucleic acid encoding the second fusion protein.

The Western blot (Figure 49B) demonstrates expression and secretion of both fusion proteins, i.e., the actA-N100-mesothelin ΔSSΔGPI and actA-N100-PSCA ΔSSΔGPI, by recombinant bivalent *L. monocytogenes* vaccine strains in infected J774 macrophage cells. The recombinant bivalent *L. monocytogenes* vaccine strain (Lm strain BH648) expressed and secreted both the PSCA and Mesothelin ActA-N100 fusion proteins in the infected J774 macrophages, as demonstrated by the Western blot (lane 3). Expression of ActAN100-PSCA was similar to that found with the recombinant monovalent *L. monocytogenes* strain encoding only actA-N100-PSCA ΔSSΔGPI (lane 2), while expression of actAN100-Mesothelin was similar to that found with the recombinant monovalent *L. monocytogenes* strain encoding only actA-N100-mesothelin ΔSSΔGPI (lane 4).

Figure 50 discloses immunization of mice with recombinant monovalent *L. monocytogenes* encoding only ActAN100-PSCA (BH476) or with recombinant bivalent *L. monocytogenes* encoding both PSCA and Mesothelin ActA-N100 fusion proteins (BH648). The PSCA and Mesothelin sequences in these constructs are delta SS and delta GPI.

Balb/c mice were immunized with the indicated recombinant *L. monocytogenes* (5 x 10⁶ cfu) vaccine strain. After one week, spleens were harvested, and the isolated splenocytes were incubated overnight with or without the indicated peptides, in Elispot assays.

Elispot assays disclose the results of immunizing with one of two different *L. monocytogenes* vaccine strains. Balb/c mice were immunized with recombinant monovalent *L. monocytogenes* strain BH476, which encoded only one human cancer antigen (PSCA), or with recombinant bivalent *L. monocytogenes* strain BH648, which encoded two human cancer antigens (PSCA and Mesothelin). The results demonstrate that the recombinant monovalent *L. monocytogenes* strain stimulated an immune response against PSCA, while the recombinant bivalent *L. monocytogenes* strain stimulated immune response against both PSCA and Mesothelin. The results demonstrate that the magnitude of PSCA-specific cellular immunity elicited in mice immunized with either recombinant monovalent or bivalent *L. monocytogenes* strains expressing ActAN100-PSCA fusion protein were the same, thus illustrating the utility of recombinant *L. monocytogenes* vaccines strains containing two or more antigen expression cassettes encoding ActA-N100-heterologous antigen fusion proteins, as applied to any desired selected heterologous antigen.

## Claims

1. A method of integrating a plasmid at a genomic attBB' site in a *Listeria* genome, wherein said attBB' site has been integrated into the genome into a gene that mediates growth or spread, wherein said plasmid comprises a phage attachment attPP' site and comprises:
a first nucleic acid encoding a phage integrase;
a second nucleic acid encoding said phage attPP' attachment site;
a third nucleic acid encoding a heterologous antigen;
fourth and fifth nucleic acids encoding first and second lox sites, or first and second Frt sites, and;
a sixth nucleic acid encoding a selection marker, wherein the first lox or Frt sites and second lox or Frt sites are operably linked with the sixth nucleic acid, and wherein the operably linked lox or Frt sites are useful for mediating Cre or FLP recombinase, respectively, catalyzed excision of the sixth nucleic acid;
said method comprising transfecting the *Listeria* with the plasmid and allowing integrase-catalysed integration of the heterologous antigen into the *Listeria* genome under conditions suitable for integration.

2. The method of claim 1, wherein the selection marker is an antibiotic resistance gene.

3. The method of claim 1, wherein the heterologous antigen is, or is derived from, a cancer cell, tumor, or infectious agent.

4. The method of claim 1, wherein the heterologous antigen is integrated into the *actA* locus or into the *inIB* locus.

5. The method of any one of claims 1-4, further comprising excising the nucleic acid encoding the selection marker from the bacterial genome, comprising contacting the genome with Cre recombinase or FLP recombinase, and allowing the recombinase to catalyze excision of said nucleic acid, under conditions allowing or facilitating excision,
wherein when the nucleic acid encoding the selection marker is flanked by lox sites the recombinase is Cre recombinase; and
wherein when the nucleic acid encoding the selection marker is flanked by Frt sites the recombinase is FLP recombinase.

6. The method of claim 5, wherein the recombinase is transiently expressed in the bacterium.

## Patentansprüche

1. Verfahren zum Integrieren eines Plasmids an einer genomischen attBB'-Stelle in ein Listerien-Genom, wobei die attBB'-Stelle in das Genom in einem Gen, das Wachstum oder Verbreitung vermittelt, integriert wurde, wobei das Plasmid eine Phagenbindungs-attPP'-Stelle umfasst und Folgendes umfasst:
eine erste Nucleinsäure, die für eine Phagenintegrase kodiert;
eine zweite Nucleinsäure, die für die Phagen-attPP'-Bindungsstelle kodiert;
eine dritte Nucleinsäure, die für ein heterologes Antigen kodiert;
vierte und fünfte Nucleinsäuren, die für erste und zweite Iox-Stellen oder erste und zweite Frt-Stellen kodieren, und;
eine sechste Nucleinsäure, die für einen Selektionsmarker kodiert, wobei die ersten lox- oder Frt-Stellen und die zweiten lox- oder Frt-Stellen an die sechste Nucleinsäure operabel gebunden sind und wobei die operabel gebundenen lox- oder Frt-Stellen für das Vermitteln von Cre- bzw. FLP-Rekombinase-katalysierter Exzision der sechsten Nucleinsäure zweckdienlich sind;
wobei das Verfahren das Transfizieren der Listerien mit dem Plasmid und das Ermöglichen von Integrase-katalysierter Integration des heterologen Antigens in das Listerien-Genom unter Bedingungen, die für Integration geeignet sind, umfasst.

2. Verfahren nach Anspruch 1, wobei der Selektionsmarker ein Antibiotikaresistenzgen ist.

3. Verfahren nach Anspruch 1, wobei das heterologe Antigen eine Krebszelle, ein Tumor oder Infektionserreger ist oder davon abgeleitet ist.

4. Verfahren nach Anspruch 1, wobei das heterologe Antigen in den actA-Locus oder in den inIB-Locus integriert wird.

5. Verfahren nach einem der Ansprüche 1 bis 4, das weiters das Exzidieren der für den Selektionsmarker kodierenden Nucleinsäure aus dem Bakteriengenom, umfassend das Kontaktieren des Genoms mit Cre-Rekombinase oder FLP-Rekombinase, und das Ermöglichen, dass die Rekombinase Exzision der Nucleinsäure katalysiert, unter Bedingungen, die Exzision ermöglichen oder erleichtern, umfasst,
wobei, wenn die für den Selektionsmarker kodierende Nucleinsäure durch lox-Stellen flankiert ist, die Rekombinase Cre-Rekombinase ist; und
wobei, wenn die für den Selektionsmarker kodierende Nucleinsäure durch Frt-Stellen flankiert ist, die Rekombinase FLP-Rekombinase ist.

6. Verfahren nach Anspruch 5, wobei die Rekombinase in dem Bakterium vorübergehend exprimiert wird.

## Revendications

1. Procédé d'intégration d'un plasmide au niveau d'un site attBB' génomique dans un génome de *Listeria,* dans lequel ledit site attBB' a été intégré dans le génome dans un gène qui médie la croissance ou la propagation, où ledit plasmide comprend un site attPP' d'attachement de phage et comprend :
un premier acide nucléique codant pour une intégrase de phage ;
un deuxième acide nucléique codant pour ledit site d'attachement attPP' de phage ;
un troisième acide nucléique codant pour un antigène hétérologue ;
un quatrième et un cinquième acide nucléique codant pour un premier et un deuxième site lox, ou un premier et un deuxième site Frt, et ;
un sixième acide nucléique codant pour un marqueur de sélection, où le premier site lox ou Frt et le deuxième site lox ou Frt sont en liaison fonctionnelle avec le sixième acide nucléique, et où les sites lox ou Frt en liaison fonctionnelle sont utiles pour médier une excision du sixième acide nucléique catalysée par une Cre ou FLP recombinase, respectivement ;
ledit procédé comprenant la transfection de la *Listeria* avec le plasmide et le fait de permettre une intégration, catalysée par une intégrase, de l'antigène hétérologue dans le génome de *Listeria* dans des conditions appropriées pour une intégration.

2. Procédé selon la revendication 1, dans lequel le marqueur de sélection est un gène de résistance aux antibiotiques.

3. Procédé selon la revendication 1, dans lequel l'antigène hétérologue est, ou est dérivé de, une cellule cancéreuse, une tumeur, ou un agent infectieux.

4. Procédé selon la revendication 1, dans lequel l'antigène hétérologue est intégré dans le locus actA ou dans le locus *inlB.*

5. Procédé selon l'une quelconque des revendications 1 à 4, comprenant en outre l'excision de l'acide nucléique codant pour le marqueur de sélection à partir du génome bactérien, comprenant la mise en contact du génome avec une Cre recombinase ou une FLP recombinase, et le fait de permettre à la recombinase de catalyser l'excision dudit acide nucléique, dans des conditions permettant ou facilitant une excision,
dans lequel lorsque l'acide nucléique codant pour le marqueur de sélection est flanqué par des sites lox, la recombinase est une Cre recombinase ; et
dans lequel lorsque l'acide nucléique codant pour le marqueur de sélection est flanqué par des sites Frt, la recombinase est une FLP recombinase.

6. Procédé selon la revendication 5, dans lequel la recombinase est exprimée de manière transitoire dans la bactérie.
